# EUROPEAN PATENT SPECIFICATION

(11) **EP 1 014 966 B1**
(45) Date of publication and mention of the grant of the patent: **02.08.2006**
(21) Application number: 97926871.1
(22) Date of filing: 29.05.1997
(51) Int. Cl.: A61K 31/24, A61K 31/165, A61K 31/04, C07F 9/02, C07C 229/00, C07C 233/00, C07C 235/00, C07C 237/00, C07C 239/00, C07C 255/00, C07C 229/14

(54) **PHARMACEUTICAL FOR TREATMENT OF NEUROLOGICAL AND NEUROPSYCHIATRIC DISORDERS**
PHARMAZEUTISCHE ZUSAMMENSETZUNG ZUR BEHANDLUNG VON NEUROLOGISCHEN UND NEUROPSYCHIATRISCHEN ERKRANKUNGEN
PRODUIT PHARMACEUTIQUE POUR LE TRAITEMENT DE TROUBLES NEUROLOGIQUES ET NEUROPSYCHIATRIQUES

(30) Priority: 31.05.1996 US 656063; 31.05.1996 US 655912; 27.02.1997 US 808754; 27.02.1997 US 808755
(43) Date of publication of application: 05.07.2000
(73) Proprietor: Allelix Neuroscience Inc., Salt Lake City, UT 84018 (US)
(72) Inventor: OGNYANOV, Vassil, Iliya, Franklin Park, NJ 08823 (US); BORDEN, Laurence, Hackensack, NJ 07601 (US); BELL, Stanley, Charles, Narberth, PA 19072 (US); ZHANG, Jing, Parsippany, NJ 08816 (US)
(74) Representative: Warcoin, Jacques
(86) International application number: PCT/US1997/009450
(87) International publication number: WO 1997/045115

(56) References cited:
- EP-A- 0 528 172
- EP-A- 0 645 378
- EP-A- 0 672 677
- BE-A- 885 303
- DE-A- 3 010 599
- JP-A- 2 129 158
- JOURNAL OF MEDICINAL CHEMISTRY, October 1993, Vol. 36, No. 20, CARCELLER et al., "Synthesis and Structure-Activity Relationships of 1-Acyl-4-[(2-Methyl-3-Pyridyl)Cyanomethyl]P iperazines as PAF Antagonists", pages 2984-2997, XP002946792
- ARCHIV DER PHARMAZIE, April 1983, Vol. 316, No. 4, LEHMANN J., "Synthese Dihydroxylierter Diphenylalkylamine Uber Azalactone", pages 339-346, XP002947833
- J. AM. CHEM. SOC., 07 April 1982, Vol. 104, No. 7, LEWIS et al., "Photochemical Addition of Tertiary Amines to Stilbene. Free-Radical and Electron-Transfer Mechanisms for Amine Oxidation", pages 1924-1929, XP002946793
- JOURNAL OF PHARMACEUTICAL SCIENCES, September 1981, Vol. 70, No. 9, OMAR et al., "Thiourea and Thiosemicarbazide Derivatives Structurally Related to Hexestrol: Synthesis and Anticancer and Other Pharmaceutical Properties", pages 1075-1079, XP002946794
- CHEMICAL AND PHARMACEUTICAL BULLETIN, September 1980, Vol. 28, No. 9, INAYAMA et al., "A Rapid and Simple Screening Method for Methamphetamine in Urine by Radioimmunoassay Using a I-Labeled Methamphetamine Derivative", pages 2779-2782, XP002946795
- TETRAHEDRON, 1979, Vol. 35, No. 11, VAULTIER et al., "Protonation en Milieu Anhydre D'Aziridines, Ylures D'Azomethine Potentiels. Nature Des Entites Formees Et Etude Des Divers Equilibres", pages 1357-1364, XP002946796

### SEQUENCE LISTING

<110> Allelix Neuroscience, Inc.
<120> PHARMACEUTICAL TREATMENT OF NEUROLOGICAL AND NEUROPSYCHIATRIC DISORDERS
<130> 317743-103B/EPO
<140> 97926871.1
   <141>
<160> 4
<170> FastSEQ for Windows Version 4.0
<210> 1
   <211> 28
   <212> DNA
   <213> homo sapiens
<400> 1
   gggggaagct tatggattgc agtgctcc
   28
<210> 2
   <211> 29
   <212> DNA
   <213> homo sapiens
<400> 2
   gggggggtac ccaacaccac tgtgctctg
   29
<210> 3
   <211> 20
   <212> DNA
   <213> homo sapiens
<400> 3
   ccacattgta gtagatgccg
   20
<210> 4
   <211> 24
   <212> DNA
   <213> homo sapiens
<400> 4
   gcaaactggc cgaaggagag ctcc
   24

## Description

The present invention relates to a class of substituted amines, pharmaceutical compositions and methods of treating neurological and neuropsychiatric disorders.

Synaptic transmission is a complex form of intercellular communication that involves a considerable array of specialized structures in both the pre- and post-synaptic neuron. High-affinity neurotransmitter transporters are one such component, located on the pre-synaptic terminal and surrounding glial cells (Kanner and Schuldiner, CRC Critical Reviews in Biochemistry, 22, 1032 (1987)). Transporters sequester neurotransmitter from the synapse, thereby regulating the concentration of neurotransmitter in the synapse, as well as its duration therein, which together influence the magnitude of synaptic transmission. Further, by preventing the spread of transmitter to neighboring synapses, transporters maintain the fidelity of synaptic transmission. Last, by sequestering released transmitter into the presynaptic terminal, transporters allow for transmitter reutilization.

Neurotransmitter transport is dependent on extracellular sodium and the voltage difference across the membrane; under conditions of intense neuronal firing, as, for example, during a seizure, transporters can function in reverse, releasing neurotransmitter in a calcium-independent non-exocytotic manner (Attwell et al., Neuron, 11, 401-407 (1993)). Pharmacologic modulation of neurotransmitter transporters thus provides a means for modifying synaptic activity, which provides useful therapy for the treatment of neurological and psychiatric disturbances.

The amino acid glycine is a major neurotransmitter in the mammalian central nervous system, functioning at both inhibitory and excitatory synapses. By nervous system, both the central and peripheral portions of the nervous system are intended. These distinct functions of glycine are mediated by two different types of receptor, each of which is associated with a different class of glycine transporter. The inhibitory actions of glycine are mediated by glycine receptors that are sensitive to the convulsant alkaloid strychnine, and are thus referred to as "strychnine-sensitive." Such receptors contain an intrinsic chloride channel that is opened upon binding of glycine to the receptor; by increasing chloride conductance, the threshold for firing of an action potential is increased. Strychnine-sensitive glycine receptors are found predominantly in the spinal cord and brainstem, and pharmacological agents that enhance the activation of such receptors will thus increase inhibitory neurotransmission in these regions.

Glycine functions in excitatory transmission by modulating the actions of glutamate, the major excitatory neurotransmitter in the central nervous system. See Johnson and Ascher, Nature. 325. 529-531 (1987); Fletcher et al., Glycine Transmission. (Otterson and Storm-Mathisen, eds., 1990), pp. 193-219. Specifically, glycine is an obligatory co-agonist at the class of glutamate receptor termed N-metghyl-D-aspartate (NMDA) receptor. Activation of NMDA receptors increases sodium and calcium conductance, which depolarizes the neuron, thereby increasing the likelihood that it will fire an action potential. NMDA receptors are widely distributed throughout the brain, with a particularly high density in the cerebral cortex and hippocampal formation.

Molecular cloning has revealed the existence in mammalian brains of two classes of glycine transporters, termed GlyT-1 and GlyT-2. GIyT-1 is found predominantly in the forebrain, and its distribution corresponds to that of glutamatergic pathways and NMDA receptors (Smith, et al., Neuron, 8, 927-935 (1992)). Molecular cloning has further revealed the existence of three variants of GIyT-1, termed GlyT-1a, GlyT-1b and GIyT-1 (Kim, et al., Molecular Pharmacology, 45, 608-617 (1994)), each of which displays a unique distribution in the brain and peripheral tissues. These variants arise by differential splicing and exon usage, and differ in their N-terminal regions. GlyT-2, in contrast, is found predominantly in the brain stem and spinal cord, and its distribution corresponds closely to that of strychnine-sensitive glycine receptors (Liu et al., J. Biological Chemistry, 268, 22802-22808 (1993); Jursky and Nelson, J. Neurochemistry, 64, 1026-1033 (1995)). These data are consistent with the view that, by regulating the synaptic levels of glycine, GIyT-1 and GlyT-2 selectively influence the activity of NMDA receptors and strychnine-sensitive glycine receptors, respectively.

Compounds that inhibit or activate glycine transporters would thus be expected to alter receptor function, and provide therapeutic benefits in a variety of disease states. For example, inhibition of GlyT-2 can be used to diminish the activity of neurons having strychnine-sensitive glycine receptors via increasing synaptic levels of glycine, thus diminishing the transmission of pain-related (i.e., nociceptive) information in the spinal cord, which has been shown to be mediated by these receptors. Yaksh, Pain, 37, 111-123 (1989). Additionally, enhancing inhibitory glycinergic transmission through strychnine-sensitive glycine receptors in the spinal cord can be used to decrease muscle hyperactivity, which is useful in treating diseases or conditions associated with increased muscle contraction, such as spasticity, myoclonus, and epilepsy (Truong et al., Movement Disorders 3, 77-87 (1988); Becker, FASEB J., 4, 2767-2774 (1990)). Spasticity that can be treated via modulation of glycine receptors is associated with epilepsy, stroke, head trauma, multiple sclerosis, spinal cord injury, dystonia, and other conditions of illness and injury of the nervous system.

NMDA receptors are critically involved in memory and learning (Rison and Stanton, Neurosci. Biobehav. Rev., 19, 533-552 (1995); Danysz et al., Behavioral Pharmacol., 6, 455-474 (1995)): and furthermore, decreased function of NMDA-mediated neurotransmission appears to underlie, or contribute to, the symptoms of schizophrenia (Olney and Farber. Archives General Psychiatry, 52, 998-1007 (1996). Thus, agents that inhibit GlyT-1 and thereby increase glycine activation of NMDA receptors can be used as novel antipsychotics and anti-dementia agents. and to treat other diseases in which cognitive processes are impaired, such as attention deficit disorders and organic brain syndromes. Conversely, over-activation of NMDA receptors has been implicated in a number of disease states, in particular the neuronal death associated with stroke and possibly neurodegenerative diseases, such as Alzheimer's disease, multi-infarct dementia, AIDS dementia. Huntington's disease, Parkinson's disease, amyotrophic lateral sclerosis or other conditions in which neuronal cell death occurs, such as stroke or head trauma. Coyle & Puttfarcken, Science, 262, 689-695 (1993); Lipton and Rosenberg, New Engl. J. of Medicine, 330, 613-622 (1993); Choi, Neuron, 1, 623-634 (1988). Thus, pharmacological agents that increase the activity of GlyT-1 will result in decreased glycine-activation of NMDA receptors, which activity can be used to treat these and related disease states. Similarly, drugs that directly block the glycine site on the NMDA receptors can be used to treat these and related disease states.

### SUMMARY OF THE INVENTION

By the present invention, a class of compounds has been identified that inhibit glycine transport via the GtyT-1 or GlyT -2 transporters, or are precursors, such as pro-drugs, to compounds that inhibit such transport. or are synthetic intermediates for preparing compounds that inhibit such transport. Thus, the invention provides a class of compounds formula: 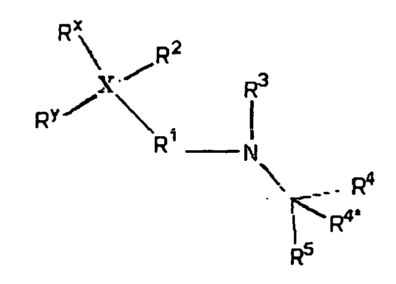 or a pharmaceutically acceptable salt thereof,
wherein:
**(1)** X is nitrogen or carbon, and R² is not present when X is nitrogen;
**(2)** R² (a) is hydrogen, (C1-C6) alkyl, (C1-C6) alkoxy, cyano, (C2-C7) alkanoyl, aminocarbonyl, (C1-C6) alkylaminocarbonyl or dialkylaminocarbonyl wherein each alkyl is independently C 1 to C6, (b) comprises (where R¹ is not aminoethylene, -O-R⁸ or -S-R^{8*}) hydroxy, fluoro, chloro, bromo or (C2-C7) alkanoyloxy, (c) forms a double bond with an adjacent carbon or nitrogen from one of either R¹, R^{xb} or R^{yb}, or (d) is R^{2a} linked by R^{2b} to X, or (e) ethylene forming a third bridging structure as set forth in (2ⁱⁱⁱ)(b)(i);
**(2**^{**i**}**)** R^{x} is R^{xa} linked by R^{xb} to X;
**(2**^{**ii**}**)** RY is R^{ya} linked by R^{yb} to X;
**(2**^{**iii**}**)** R^{xa}, R^{ya} and R^{2a}, are independently Ar which is aryl or heteroaryl, adamantyl or a 5 to 7-membered non-aromatic ring having from 0 to 2 heteroatoms selected from the group consisting of oxygen, sulfur and nitrogen, wherein at least one of R^{xa}, R^{ya} and R^{2a} is phenyl, and wherein:
   (a) aryl is phenyl or naphthyl,
   (b) heteroaryl comprises a five-membered ring, a six-membered ring, a six-membered ring fused to a five-membered ring, a five-membered ring fused to a six-membered ring, or a six-membered ring fused to a six-membered ring, wherein the heteroaryl is aromatic and contains heteroatoms selected from the group consisting of oxygen, sulfur and nitrogen, with the remaining ring atoms being carbon,
   (c) each of R^{xa}, R^{ya} and R^{2a} can be substituted or independently substituted with one of R^{q}, R^{r}O- or R^{s}S-, wherein each of R^{q}, R^{r} and R^{S} are independently Ar, adamantyl or a 5 to 7-membered non-aromatic ring as these ring structures are defined for R^{xa}, and
   (d) R^{xa}, R^{ya}, R^{2a}, R^{q}, R^{r} and R^{s} can be additionally substituted with one or more substituents selected from the group consisting of fluoro, chloro, bromo, nitro, hydroxy, cyano, trifluoromethyl, amidosulfonyl which can have up to two independent (C1-C6) N-alkyl substitutions, adamantyl, (C1-C12) alkyl, (C2-C12) alkenyl, amino, (C1-C6) alkylamino, dialkylamino wherein each alkyl is independently C1 to C6, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be substituted for hydrogen with up to two independent (C1-C6) alkyl, (C1-C6) alkylsulfonyl, amidino that can be independently substituted with up to three (C1-C6) alkyl group, or methylenedioxy or ethylenedioxy with the two oxygens bonded to adjacent positions on the aryl or heteroaryl ring structure, which methylenedioxy or ethylenedioxy can be substituted with up to two independent (C1-C6) alkyl, wherein: (i.) the substitutions of R^{xa}, R^{ya} and R^{2a} can be combined to form a second bridge between two of R^{xa}, R^{ya} and R^{2a} comprising (1) (C1-C2) alkyl or (C2) alkenyl, which can be independently substituted with one or more (C1-C6) alkyl or by R², wherein R² is ethylene to form a third bridging structure, (2) sulfur, (3) oxygen, (4) amino, which can be substituted for hydrogen with one (C1-C6) alkyl, (5) carbonyl, (6) -CH₂C(=O)-, which can be substituted for hydrogen with up to two independent (C1-C6) alkyl, (7) -C(=O)-O-, (8) - CH₂-O-, which can be substituted for hydrogen with up to two independent (C1-C6) alkyl, (9) -C(=O) N(R²⁴) , wherein R²⁴ is hydrogen or (C1-C6) alkyl, (10) -CH₂-NH-, which can be substituted for hydrogen with up to three (C1-C6) alkyl, or (11) -CH=N-, which can be substituted for hydrogen with (C1-C6) alkyl, or wherein two of R^{xa}, R^{ya} and R^{2a} can be directly linked by a single bond;
**(2**^{**iv**}**)** R^{xb} and R^{2b} are independently a single bond or (C1-C2) alkylene;
**(2**^{**v**}**)** R^{yb} is a single bond, oxo, (C1-C2) alkylene, ethenylene or -CH= (where the double bond is with X), thio, methyleneoxy or methylenethio, or either -N(R⁶) or -CH₂=N(R^{6*})·, wherein R⁶ and R^{6*} are hydrogen or (C1-C6) alkyl, wherein when X is nitrogen X is not bonded to another heteroatom;
**(3)** R¹ comprises: a straight-chained (C2-C3) aliphatic group; where X is carbon, =N-O-(ethylene), wherein the unmatched double bond is linked to X, in which X is carbon and Ryb does not include a heteroatom attached to X, -O-R⁸ or -S-R^{8*} wherein R⁸ or R^{8*}is a ethylene or ethenylene and O or S is bonded to X, in which X is carbon and Ryb does not include a heteroatom attached to X; aminoethylene where the amino is bonded to X:
   wherein R¹ can be substituted with up to one hydroxy, up to one (C1-C6) alkoxy or up to one (C2-C7) alkanoyloxy, with up to two independent (C1-C6) alkyl, with up to one oxo, up to one (C1-C6) alkylidene, with the proviso that the hydroxy, alkoxy, alkanoyloxy or oxo substituents are not bonded to a carbon that is bonded to a nitrogen or oxygen;
   wherein the alkyl or alkylidene substituents of R¹ can be linked to form a 3 to 7-membered non-aromatic ring; and
   wherein if X is nitrogen, X is linked to R¹ by a single bond and the terminal carbon of R¹ that links R¹ to N is saturated;
**(4)** R³ is hydrogen, (C1-C6) alkyl, or phenyl or phenylalkyl wherein the alkyl is C1 to C6 and either such phenyl can be substituted with the same substituents defined above for the aryl or heteroaryl of R^{xa} ; (5) and R4 and R⁴* are hydrogen;
**(6)** R⁵ is (CO)NR¹³R¹⁴, (CO)OR¹⁵, (CO)SR¹⁶, (SO₂)NR¹⁷R¹⁸, (pp)(OR¹⁹)(OR²⁰), (CR²²)(OR²³)(OR²⁴), CN or tetrazol-5-yl, wherein (a) R¹³, R¹⁴, R¹⁵, R¹⁶, R¹⁷, R¹⁸ R¹⁹ and R²⁰ are independently hydrogen, (C1-C8) alkyl which can include a (C3-C8) cycloalkyl, wherein the carbon linked to the oxygen of R¹⁵ or the sulfur of R¹⁶ has no more than secondary branching and , (C2-C6) hydroxyalkyl, aminoalkyl where the alkyl is C2 to C6 and the amino can be substituted with up to two independent (C1-C6) alkyls, Ar-alkyl wherein the alkyl is C1-C6 or Ar, (b) R²² is hydrogen or OR²⁵ and (c) R²³, R²⁴ and R²⁵ are (C1-C6) alkyl, phenyl, benzyl, acetyl or, where R²² is hydrogen, the alkyls of R²³ and R²⁴ can be combined to include 1,3-dioxolane or 1,3-dioxane:
   wherein the Ar groups of R¹³, R¹⁴, R¹⁵, R¹⁶, R¹⁷, R¹⁸, R¹⁹, R²⁰, R²², R²³ or R²⁴ can be substituted with substituents selected from the group consisting of fluoro, chloro, bromo, nitro, cyano, hydroxy, trifluoromethyl, amidosulfonyl which can have up to two independent (C1-C6) N-alkyl substitutions, (C1-C6) alkyl, (C2-C6) alkenyl, (C1-C6) alkylamine, dialkylamine wherein each alkyl is independently C1 to C6, amino, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be N-substituted with up to two independent (C1-C6) alkyl, (C1-C6) alkylsulfonyl, amidino that can substituted with up to three (C1-C6) alkyl, or methylenedioxy or ethylenedioxy with the two oxygens bonded to adjacent positions on the aryl or heteroaryl ring structure, which methylenedioxy or ethylenedioxy can be substituted with up to two independent (C1-C6) alkyl;
   wherein R¹³ and R¹⁴ together with the nitrogen can form a 5 to 7-membered ring that can contain one additional heteroatom selected from oxygen and sulfur;
   and wherein the following provisos apply:
   if R¹⁵ is hydrogen and R¹ is propylene, then at least one of the following applies (1) both R^{x} and R^{y} are not *p*-fluorophenyl, (2) one of R^{x} and R^{y} includes a heteroaryl, (3) RY is Ar-(C1-C2)alkyl, Ar-oxy, Ar-methoxy, Ar-thio, Ar-methylthio, Ar-N(R⁶)- or Ar-CH₂-N(R^{6*})-, (4) R² is R^{2a}R^{2b}-, (5) R² is not hydrogen, or (6) R³ is not hydrogen;
   if R¹⁵ is hydrogen and R¹ is ethylene or X-R¹ is prop-1-enylene, then at least one of the following applies (1) an aryl of at least one of R^{x} and RY is substituted with a radical different from hydrogen, (2) one of R^{x} and RY comprises a heteroaryl, (3) RY is Ar-(C1-C2)alkyl, Ar-oxy, Ar-methoxy, Ar-thio, Ar-methylthio, Ar-N(R⁶)- or Ar-CH₂₋N(R^{6*})-, (4) R² is R^{2a} R^{2b}-, (5) R^{2*} is not hydrogen, or (6) R³ is not hydrogen;
   if R⁵ is C(O)NR¹³R¹⁴, wherein R¹³ and R¹⁴ are hydrogen, (C1-C8)alkyl, phenyl or substituted phenyl, then at least one of the following applies (1) an aryl of at least one of R^{x} and R^{y} is substituted with a radical different from hydrogen, (2) one of R^{x} and RY comprises a heteroaryl, (3) RY is Ar-(C1-C2)alkyl, Ar-oxy, Ar-methoxy, Ar-thio, Ar-methylthio, Ar-N(R⁶)- or Ar-CH₂-N(R^{6*})-, (4) R² is R^{2a} R^{2b}-, (5) R^{2*} is not hydrogen, (6) R³ is not hydrogen, or (7) R¹ is not ethylene;
   wherein if R² is phenyl or *p*-methylphenyl, then at least one of the following applies (1) the aryls of R^{x} and RY are not substituted with *p*-methylphenyl or *p-*methoxyphenyl, (2) an aryl of at least one of R^{x} and RY is substituted with a radical different from hydrogen, (3) one of R^{x} and RY comprises a heteroaryl, (4) RY is Ar-(C1-C2)alkyl, Ar-oxy, Ar-methoxy, Ar-thio, Ar-methylthio, Ar-N(R⁶)- or Ar-CH₂-N(R^{6*})-, or (5) R¹ is not aminoethylene, OR⁸ or SR^{8*};
   wherein if R^{2a} is *p*-methoxyphenyl, then at least one of the following applies (1) an Ar of at least one of R^{x} and R^{y} is substituted with a radical different from hydrogen, (2) RY is Ar-(C1-C2)alkyl, Ar-oxy, Ar-methoxy, Ar-thio, Ar-methylthio, Ar-N(R6)- or ArCH₂-N(R^{6*})-, or (3) R¹ is not OR⁸ or SR^{8*}.

In one preferred embodiment, the invention relates to a compound of the following formula: 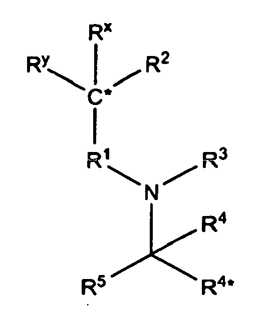 or a pharmaceutically acceptable salt thereof,
wherein:
(1) C* is a substituted carbon;
(2) R² (a) is hydrogen, (C1-C6) alkyl, (C1-C6) alkoxy, cyano, (C2-C7) alkanoyl, aminocarbonyl, (C1-C6) alkylaminocarbonyl, or dialkylaminocarbonyl wherein each alkyl is independently C1 to C6, (b) comprises (where R¹ is not aminoethylene, -O-R⁸ or -S-R⁸*) hydroxy, fluoro, chloro, bromo or (C2-C7) alkanoyloxy, (c) forms a double bond with an adjacent carbon or nitrogen from one of either R¹, R^{xb}, R^{yb}, (d) is R^{2a} linked by R^{2b} to C*, or (e) ethylene forming a third bridging structure as set forth in (2ⁱⁱⁱ)(b)(i);
(2ⁱ) R^{x} is R^{xa} linked by R^{xb} to C*;
(2ⁱⁱ) R^{y} is R^{ya} linked by R^{yb} to C*;
(2ⁱⁱⁱ) R^{xa}, R^{ya} and R^{2a}, are independently Ar, which is phenyl or naphthyl, or a 5 to 7-membered non-aromatic ring having 0 heteroatoms wherein:
   (a) each of R^{xa} and R^{ya} can be independently substituted with one of R^{q}, R^{r}O- or R^{s}S-, wherein each of R^{q}, R^{r} and R^{s} are independently Ar or adamantly, and
   (b) R^{xa}, R^{ya}, R^{2a}, R^{q}, R^{r} and R^{s} can be substituted or additionally substituted with one or more substituents selected from the group consisting of fluoro, chloro; bromo, nitro, hydroxy, cyano, trifluoromethyl, amidosulfonyl which can have up to two independent (C1-C6) N-alkyl substitutions, (C1-C12) alkyl, (C2-C12) alkenyl, amino, (C1-C6) alkylamino, dialkylamino wherein each alkyl of dialkylamino is independently -C1 to C6, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be substituted for hydrogen with up to two independent (C1-C6) alkyl, (C1-C6) alkylsulfonyl, or amidino that can independently substituted with up to three (C1-C6) alkyl, wherein:
      (i.) the substitutions of R^{xa} and R^{ya} can be combined to form a second bridge between R^{xa} and R^{ya} comprising (1) methylene or ethylene, which methylene or ethylene can be substituted by an R² when R² is ethylene to form the third bridging structure, or (2) -CH=CH- or wherein R^{xa} and R^{ya} can be directly linked by a single bond;
(2^{iv}) R^{xb} and R^{2b} are independently a single bond or (C1-C2) alkylene;
(2^{v}) R^{yb} is a single bond, oxa, (C1-C2) alkylene, ethenylene or -CH= (where the double bond is with C*), thia, methyleneoxy or methylenethio, or either -N(R⁶) or -CH₂-N(R^{6*})-, wherein R⁶ and R^{6*} are hydrogen or (C1-C6) alkyl;
(3) R¹ comprises: a straight-chained (C2-C3) aliphatic group; =N-O-(ethylene), wherein the unmatched double bond is linked to C*; -O-R⁸ or -S-R^{8*} wherein R⁸ or R^{8*} is a ethylene or ethenylene and O or S is bonded to C*; aminoethylene where the amino is bonded to C*:
   wherein R¹ can be substituted with up to one hydroxy, up to one (C1-C6) alkoxy or up to one (C2-C7) alkanoyloxy, with up to two independent (C1-C6) alkyl, with up to one oxo, up to one (C1-C6) alkylidene, with the proviso that the hydroxyl, alkoxy, alkanoyloxy or oxo substituents are not bonded to a carbon that is bonded to a nitrogen or oxygen; and
   wherein the alkyl or alyklidene substituents of R¹ can be linked to form a 3 to 7-membered non aromatic ring;
(4) R³ (a) is hydrogen, (C1-C6) alkyl, or phenyl or phenylalkyl wherein the alkyl is C1 to C6 and the phenyl or phenyl of phenylalkyl can be substituted with the same substituents defined above for the phenyl of R^{xa}, (b) is -R¹²C(R^{xx})(R^{yy})(R¹¹) , wherein R¹² is bonded to N, R^{xx} is independently the same as R^{x}, R^{yy} is independently the same as R^{y}, R¹¹ is independently the same as R² and R¹² is independently the same as R¹;
(5) R⁴ and R^{4*} are independently hydrogen or (C1-C6) alkyl, or one of R⁴ and R^{4*} can be (C1-C6) hydroxyalkyl; and
(6) R⁵ is (CO)NR¹³R¹⁴, (CO)OR¹⁵, (CO)SR¹⁶, (SO₂)NR¹⁷R¹⁸, (PO)(OR¹⁹)(OR²⁰), (CR²²)(OR²³)(OR²⁴), CN or tetrazol-5-yl, wherein (a) R¹³, R¹⁴, R¹⁵, R¹⁶, R¹⁷, R¹⁸ R¹⁹ and R²⁰ are independently hydrogen, (C1-C8) alkyl which can include a (C3-C8) cycloalkyl, wherein the carbon linked to the oxygen of R¹⁵ or the sulfur of R¹⁶ has no more than secondary branching and, (C2-C6) hydroxyalkyl, aminoalkyl where the alkyl is C2 to C6 and the amino can be substituted with up to two independent (C1-C6) alkyls, Ar-alkyl wherein the alkyl is C1-C6 or Ar, and (b) R²² is hydrogen or OR²⁵ and R²³, R²⁴ and R²⁵ are (C1-C6) independently alkyl, phenyl, benzyl or acetyl or, the alkyls of R²³ and R²⁴ can be combined to include 1,3-dioxolane or 1,3-dioxane:
wherein the phenyl or naphthyl groups of R¹³, R¹⁴, R¹⁵, R¹⁶, R¹⁷, R¹⁸, R¹⁹, R²⁰, R²², R²³ or R²⁴ can be substituted with substituents selected from the group consisting of fluoro, chloro, bromo, nitro, cyano, hydroxy, trifluoromethyl, amidosulfonyl which can have up to two independent (C1-C6) N-alkyl substitutions, (C1-C6) alkyl, (C2-C6) alkenyl, (C1-C6) alkylamine, dialkylamine wherein each alkyl is independently C1 to C6, amino, (C1-C6) alkoxy, (C2-C7) alkanoyl, (C2-C7) alkanoyloxy, trifluoromethoxy; hydroxycarbonyl, (C2-C7) alkyloxycarbonyl, aminocarbonyl that can be N-substituted with up to two independent (C1-C6) alkyl, (C1-C6) alkylsulfonyl, or amidino that can substituted with up to three (C1-C6) alkyl;
wherein R¹³ and R¹⁴ together with the attached nitrogen can form a 5 to 7-membered ring; and wherein further the following provisos apply:
if R¹⁵ is hydrogen and R' is propylene, then at least one of the following applies (1) both R^{xa} and R^{ya} are not *p*-fluorophenyl, (2) R^{y} is Ar-alkyl, Ar-oxy, Ar-methoxy, Ar-thio, Ar-methylthio, Ar-N(R⁶)- or Ar-CH₂-N(R^{6*})-, (3) R² is R^{2a} R^{2b}-, (4) R² is not hydrogen, or (5) R³ is not hydrogen;
if R¹⁵ is hydrogen and R¹ is ethylene or C*R¹ is prop-1-enylene, then at least one of the following applies (1) an aryl of at least one of R^{xa} and R^{ya} is substituted with a radical different from hydrogen, (2) R^{y} is Ar-alkyl, Ar-oxy, Ar-methoxy, Ar-thio, Ar-methylthio, Ar-N(R⁶)- or Ar-CH₂-N(R^{6*})-, (3) R² is R^{2a} R^{2b}-, (4) R² is not hydrogen, or (5) R³ is not hydrogen;
if R⁵ is C(O)NR¹³R¹⁴, wherein R¹³ and R¹⁴ are hydrogen, (C1-C8)alkyl, phenyl or substituted phenyl, then at least one of the following applies (1) an aryl of at least one of R^{x} and R^{y} is substituted with a radical different from hydrogen, fluoro, chloro, or bromo (2) R^{y} is Ar-alkyl, Ar-oxy, Ar-methoxy, Ar-thio, Ar-methylthio, Ar-N(R⁶)- or Ar-CH₂-N(R⁶*)-, (3) R² is R^{xa} R^{2a} R^{2b}-, (4) R²is not hydrogen, (5) R³ is not hydrogen, or (6) R¹ is not ethylene;
if R^{2a} is phenyl orp-methylphenyl, then at least one of the following applies (1) the aryls of R^{x} and R^{y} are not substituted with *p*-methylphenyl or *p*-methoxyphenyl, (2) an aryl of at least one of R^{x} and R^{y} is substituted with a radical different from hydrogen, (3) R^{y} is Ar-alkyl, Ar-oxy, Ar-methoxy, Ar-thio, Ar-methylthio, Ar-N(R⁶)- or Ar-CH₂-N(R^{6*})-, or (5) R¹ is not aminoethylene, OR⁸ or SR^{8*};
if R^{2a} is *p*-methoxyphenyl, then at least one of the following applies (1) an Ar of at least one of R^{x} and R^{y} is substituted with a radical different from hydrogen, (2) R^{y} is Ar-(C1-C2)alkyl, Ar-oxy, Ar-methoxy, Ar-thio, Ar methylthio, Ar-N(R⁶)-, or Ar-CH2N(R^{6*})-, or (3) R¹ is not -OR⁸ or -SR^{8*}.

In another preferred embodiment, (A) at least one of R^{xa}, R^{ya} and R^{2a} is substituted with fluoro, chloro, bromo, hydroxy, trifluoromethyl, trifluoromethoxy, nitro, cyano, (C3-C8) alkyl, R^{q}, R^{r}O-, R^{s}S-, (B) R³ is hydrogen, (C1-C6) alkyl, or phenyl or phenylalkyl wherein the alkyl is C1 to C6 and either such phenyl can be substituted with the same substituents defined above for the aryl or heteroaryl of R^{xa} or (C) the ring structures of R^{xa}, R^{ya} and R^{2a}, including substituents thereto, otherwise include at least two aromatic ring structures that together include from 15 to 20 ring atoms.

In another preferred embodiment, at least one of R^{xa}, R^{ya} and R^{2a} is substituted with fluoro, trifluoromethyl, trifluoromethoxy, nitro, cyano, or (C3-C8) alkyl.

In another preferred embodiment, at least one of R^{xa}, R^{ya} and R^{2a} is substituted with R^{q}, R^{r}O-, or R^{s}S-.

In another preferred embodiment, R^{yb} is oxy, methyleneoxy, thio or methylenethio.

In another preferred embodiment, R^{yb} is oxy or thio.

In another preferred embodiment, R⁵ is (CO)NR¹³R¹⁴, (CO)OR¹⁵ or (CO)SR¹⁶.

In another preferred embodiment, R¹⁵ is (C2-C6) alkyl, (C2-C4) hydroxyalkyl, phenyl, phenylalkyl wherein the alkyl is C1-C3, or aminoalkyl where the alkyl is C2-C6 and the amino can be substituted with up to two independent (C1-C3) alkyls, wherein the phenyl or the phenyl of phenylalkyl can be substituted.

R¹⁵ is preferably hydrogen.

In another preferred embodiment, R⁴ is hydrogen, methyl or hydroxymethyl and R⁴* is hydrogen.

In another preferred embodiment, at least one of R^{xa}, R^{ya} and R^{2a} is a heteroaryl comprising diazolyl, triazolyl, tetrazolyl, thiazolyl, isothiazolyl, oxazolyl, isoxazolyl, thiolyl, diazinyl, triazinyl, benzoazolyl, benzodiazolyl, benzothiazolyl; benzoxazolyl, benzoxolyl, benzothiolyl, quinolyl, isoquinolyl, benzodiazinyl, benzotriazinyl, pyridyl, thienyl, foranyl, pyrrolyl, indolyl, isoindoyl or pyrimidyl.

Preferably, R¹ is -O-R⁸ or -S-R^{8*}.

In another preferred embodiment, said second bridge between two of R^{xa}, R^{ya} and R^{2a} is L, and satisfies the following formula: 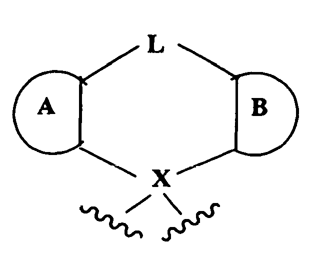 , wherein A and B are aryl or heteroaryl groups of R^{xa} and R^{ya}, respectively.

Preferably, R^{xa}-R^{xb}-, R^{ya}-R^{yb}- and X form: 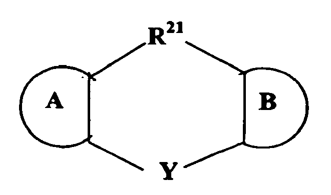 wherein Y is a carbon bonded to R¹ by a single or double bond or a nitrogen that is bonded to R' and wherein R²¹ either (i.) completes a single bond linking two aryl or heteroaryl rings of R^{x} and R^{y}, (ii.) is (C1-C2) alkylene or alkenylene, (iii.) is sulfur or (iv.) is oxygen, and wherein R^{x} and R^{y} can be substituted as set forth above.

In another preferred embodiment, R²¹ is CH₂CH₂ or CH=CH.

In another preferred embodiment, wherein the alkylenedioxy substitution of R^{xa}, R^{ya} or R^{2a} is as follows: 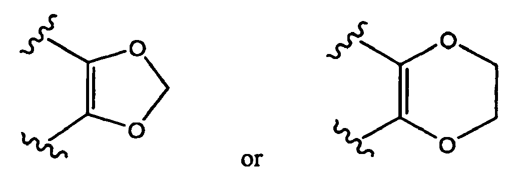 wherein the alkylenedioxy can be substituted with up to two independent (C1-C3) alkyl.

In another preferred embodiment, R^{xa} and R^{ya} together can be substituted with up to six substituents, R^{2a}, R^{q}, R^{r} and R^{s} can each be substituted with up to 3 substituents, and wherein the presence of each of R^{q}, R^{r} or R^{s} is considered a substitution to the respective ring structure of R^{xa}, R^{ya} and R^{2a}.

Preferably, a phenyl of R³ is substituted with up to three substituents.

In another preferred embodiment, the aryl, heteroaryl, aryl of arylalkyl or the heteroaryl of heteroarylalkyl of R¹³, R¹⁴, R¹⁵, R¹⁶ R¹⁷, R¹⁸ R¹⁹ or R²⁰ is substituted with up to three substituents.

Preferably, the compound as defined above is an optically pure enantiomer.

In another preferred embodiment,
(1) R² is hydrogen,
(2) R^{xa} and R^{ya} are both phenyl and at least one of R^{xa} and R^{ya} is substituted with one of phenyl, phenoxy, or phenylthio,
(3) R^{xb} is a single bond and R^{yb} is a single bond or oxa, and
(4) R⁵ is (CO)NR¹³R¹⁴ or (CO)OR¹⁵, wherein R¹³, R¹⁴, and R¹⁵ are independently hydrogen; (C1-C8) alkyl which can include a (C3-C8) cycloalkyl, wherein the carbon linked to the oxygen of OR¹⁵ has no more than secondary branching; (C2-C6) hydroxyalkyl or amirioalkyl where the alkyl is C2 to C6 and the amino can be substituted with up to two independent (C1-C6) alkyls or phenylalkyls, wherein the alkyl is C1-C6 and the phenyl can be substituted with substituents selected from the group consisting of fluoro, chloro, bromo, nitro, cyano, hydroxy, trifluoromethyl, amidosulfonyl which can have up to two independent (C1-C6) N-alkyl substitutions, (C1-C6) alkyl, (C2-C6) alkenyl, (C1-C6) alkylamine, dialkylamine
wherein each alkyl is independently C1 to C6, amino, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be N-substituted with up to two independent (C1-C6) alkyl, (C1-C6) alkylsulfonyl, amidino that can substituted with up to three (C1-C6) alkyl.

The invention alos relates to a pharmaceutical composition comprising the compound as depicted above and a pharmaceutically acceptable excipient.

In the pharmaceutical composition, the compound of claim 1 or 2 is present in an effective amount for:
(1) treating or preventing schizophrenia,
(2) enhancing treating or preventing dementia,
(3) treating or preventing epilepsy,
(4) treating or preventing spasticity,
(5) treating or preventing muscle spasm,
(6) treating or preventing pain,
(7) preventing neural cell death after stroke,
(8) preventing neural cell death in an animal suffering from a neurodegenerative disease,
(9) treating or preventing mood disorders,
(10) enhancing memory or learning, or
(11) treating or preventing learning disorders.

The invention also contemplates the use of a compound of formula: 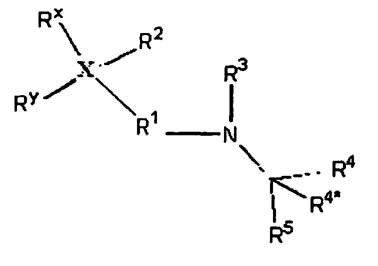 or a pharmaceutically acceptable salt thereof,
wherein:
(1) X is nitrogen or carbon, and R² is not present when X is nitrogen;
(2) R² (a) is hydrogen, (C1-C6) alkyl, (C1-C6) alkoxy, cyano, (C2-C7) alkanoyl, aminocarbonyl, (C1-C6) alkylaminocarbonyl or dialkylaminocarbonyl wherein each alkyl is independently C1 to C6, (b) comprises (where R¹ is not aminoethylene, -O-R⁸ or -S-R^{8*}) hydroxy, fluoro, chloro, bromo or (C2-C7) alkanoyloxy, (c) forms a double bond with an adjacent carbon or nitrogen from one of either R¹, R^{xb} or R^{yb}, or (d) is R^{2a} linked by R^{2b}to X, or (e) ethylene forming a third bridging structure as set forth in (2ⁱⁱⁱ)(b)(i);
(2ⁱ) R^{x} is R^{xa} linked by R^{xb} to X;
(2") R^{y} is R^{ya} linked by R^{yb} to X;
(2ⁱⁱⁱ) R^{xa}, R^{ya} and R^{2a}, are independently Ar which is aryl or heteroaryl, adamantyl or a 5 to 7-membered non-aromatic ring having from 0 to 2 heteroatoms selected from the group consisting of oxygen, sulfur and nitrogen, wherein at least one of R^{xa}, R^{ya} and R^{2a} is phenyl, and wherein:
   (a) aryl is phenyl or naphthyl,
   (b) heteroaryl comprises a five-membered ring, a six-membered ring, a six-membered ring fused to a five-membered ring, a five-membered ring fused to a six-membered ring, or a six-membered ring fused to a six-membered ring, wherein the heteroaryl is aromatic and contains heteroatoms selected from the group consisting of oxygen, sulfur and nitrogen, with the remaining ring atoms being carbon,
   (c) each of R^{xa}, R^{ya} and R^{2a} can be substituted or independently substituted with one of R^{q}, R^{r}O- or R^{s}S-, wherein each of R^{q}, R^{r} anf R^{s} are independently Ar, adamantly or a 5 to 7-membered non-aromatic ring as these ring structures are defined for R^{xa}, and
   (d) R^{xa}, R^{ya}, R^{2a}, R^{q}, R^{r} and R^{s} can be additionally substituted with one or more substituents selected from the group consisting of fluoro, chloro, bromo, nitro, hydroxy, cyano, trifluoromethyl, amidosulfonyl which can have up to two independent (C1-C6) N-alkyl substitutions, adamantyl, (C1-C12) alkyl, (C2-C12) alkenyl, amino, (C1-C6) alkylamino, dialkylamino wherein each alkyl is independently C1 to C6, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be substituted for hydrogen with up to two independent (C1-C6) alkyl, (C1-C6) alkylsulfonyl, amidino that can be independently substituted with up to three (C1-C6) alkyl group, or methylenedioxy or ethylenedioxy with the two oxygens bonded to adjacent positions on the aryl or heteroaryl ring structure, which methylenedioxy or ethylenedioxy can be substituted with up to two independent (C1-C6) alkyl, wherein:
      (i.) the substitutions of R^{xa}, R^{ya} and R^{2a} can be combined to form a second bridge between two of R^{xa}, R^{ya} and R^{2a} comprising (1) (C1-C2) alkyl or (C2) alkenyl, which can be independently substituted with one or more (C1-C6) alkyl or by R², wherein R² is ethylene to form a third bridging structure, (2) sulfur, (3) oxygen, (4) amino, which can be substituted for hydrogen with one (C1-C6) alkyl, (5) carbonyl, (6) -CH₂C(=O)-, which can be substituted for hydrogen with up to two independent (C1-C6) alkyl, (7) -C(=O)-O-, (8) -CH₂-O-, which can be substituted for hydrogen with up to two independent (C1-C6) alkyl, (9) -C(=O) N(R²⁴), wherein R²⁴ is hydrogen or (C1-C6) alkyl, (10) -CH₂-NH-, which can be substituted for hydrogen with up to three (C1-C6) alkyl, or (11) -CH=N-, which can be substituted for hydrogen with (C1-C6) alkyl, or wherein two of R^{xa}, R^{ya} and R^{2a} can be directly linked by a single bond;
(2^{iv}) R^{xb} and R^{2b} are independently a single bond or (C1-C2) alkylene;
(2^{v}) R^{yb} is a single bond, oxo, (C1-C2) alkylene, ethylene or -CH= (where the double bond is with X), thio, methyleneoxy or methylenethio, or either -N(R⁶) or -CH₂-N(R⁶*)-, wherein R⁶ and R⁶* are hydrogen or (C1-C6) alkyl, wherein when X is nitrogen X is not bonded to another heteroatom;
(3) R¹ comprises: a straight-chained (C2-C3) aliphatic group; where X is carbon, =NO-(ethylene), wherein the unmatched double bond is linked to X; (where X is carbon and R^{yb} does not include a heteroatom attached to X), -O-R⁸ or -S-R⁸* wherein R⁸ or R⁸* is a ethylene or ethenylene and O or S is bonded to X; (where X is carbon and R^{yb} does not include a heteroatom attached to X), aminoethylene where the amino is bonded to X:
   wherein R¹ can be substituted with up to one hydroxy, up to one (C1-C6) alkoxy or up to one (C2-C7) alkanoyloxy, with up to two independent (C1-C6) alkyl, with up to one oxo, up to one (C1-C6) alkylidene, with the proviso that the hydroxy, alkoxy, alkanoyloxy or oxo substituents are not bonded to a carbon that is bonded to a nitrogen or oxygen;
   wherein the alkyl or alkylidene substituents of R¹ can be linked to form a 3 to 7-membered non-aromatic ring; and
   wherein if X is nitrogen, X is linked to R¹ by a single bond and the terminal carbon of R¹ that links R¹ to N is saturated;
(4) R³ is hydrogen, (C1-C6) alkyl, or phenyl or phenylalkyl wherein the alkyl is C1 to C6 and either such phenyl can be substituted with the same substituents defined above for the aryl or heteroaryl of R^{xa},
(5) R⁴ and R⁴* are independently hydrogen;
(6) R⁵ is (CO)NR¹³R¹⁴, (CO)OR¹⁵, (CO)SR¹⁶, (SO₂)NR¹⁷R¹⁸, (PO)(OR¹⁹)(OR²⁰), (CR²²)(OR²³)(OR²⁴), CN or tetrazol-5-yl, wherein (a) R¹³, R¹⁴, R¹⁵, R¹⁶, R¹⁷, R¹⁸ R¹⁹and R²⁰ are independently hydrogen, (C1-C8) alkyl which can include a (C3-C8) cycloalkyl, wherein the carbon linked to the oxygen of R¹⁵ or the sulfur of R¹⁶ has no more than secondary branching and, (C2-C6) hydroxyalkyl, aminoalkyl where the alkyl is C2 to C6 and the amino can be substituted with up to two independent (C1-C6) alkyls, Ar-alkyl wherein the alkyl is C1-C6 or Ar, (b) R²² is hydrogen or OR²⁵ and (c) R²³, R²⁴ and R²⁵ are (C1-C6) alkyl, phenyl, benzyl, acetyl or, where R²² is hydrogen, the alkyls of R²³ and R²⁴ can be combined to include 1,3-dioxolane or 1,3-dioxane:
wherein the Ar groups of R¹³, R¹⁴, R¹⁵, R¹⁶, R¹⁷, R¹⁸, R¹⁹, R²⁰, R²², R²³ or R²⁴ can be substituted with substituents selected from the group consisting of fluoro, chloro, bromo, nitro, cyano, hydroxy, trifluoromethyl, amidosulfonyl which can have up to two independent (C1-C6) N-alkyl substitutions, (C1-C6) alkyl, (C2-C6) alkenyl, (C1-C6) alkylamine, dialkylamine wherein each alkyl is independently C1 to C6, amino, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be N-substituted with up to two independent (C1-C6) alkyl, (C1-C6) alkylsulfonyl, amidino that can substituted with up to three (C1-C6) alkyl, or methylenedioxy or ethylenedioxy with the two oxygens bonded to adjacent positions on the aryl or heteroaryl ring structure, which methylenedioxy or ethylenedioxy can be substituted with up to two independent (C1-C6) alkyl;
wherein R¹³ and R¹⁴ together with the nitrogen can form a 5 to 7-membered ring that can contain one additional heteroatom selected from oxygen and sulfur;
for the manufacture of a medicament for treating or preventing schizophrenia, (2) for treating or preventing dementia, (3) for treating or preventing epilepsy, (4) for treating or preventing spasticity, (5) for treating or preventing muscle spasm, (6) for treating or preventing pain, (7) for preventing neural cell death after stroke, (8) for preventing neural cell death in an animal suffering from a neurodegenerative disease, (9) for treating or preventing mood disorders, (10) for enhancing memory or learning, or (11) for treating or preventing learning disorders.

Preferably, (A) at least one of R^{xa}. R^{ya} and R^{2a} is substituted with fluoro. chloro. bromo, hydroxy, trifluoromethyl, trifluoromethoxy, nitro, cyano, (C3-C8) alkyl. R^{q}. R^{r}O-. R^{s}S-. (B) R³ is hydrogen, (C1-C6) alkyl, or phenyl or phenylalkyl wherein the alkyl is C I to C6 and either such phenyl can be substituted with the same substituents defined for the aryl or heteroaryl of R^{xa} or (C) the ring stuctures of R^{xa}, R^{ya} and R^{2a}, including substituents thereto. otherwise include at least two aromatic ring structures that together include from 15 to 20 ring atoms. Examples of preferred structures under clause (C) include A45, A53, A56, A57, A60-5, A73-74. A78-81, A86-89, A93-96, A99, A100, A102, A106-106, A108-109, A116, A122-123 and A176. Preferably, at least one of R^{xa}, R^{ya} and R^{2a} is substituted with fluoro, trifluoromethyl, trifluoromethoxy, nitro, cyano, or (C3-C8) alkyl. Preferably, R^{xa}, R^{ya} and R^{2a} is substituted with R^{q}, R^{r}O-, or R^{s}S-. Preferably, an aryl or heteroaryl of at least one of R^{xa}, R^{ya} and R^{2a} is phenyl. Preferably, R^{yb} is oxa, methyleneoxy, thia, methylenethia. Preferably, R^{yb} is oxa or thia. Preferably, R⁵ is (CO)N R¹³R¹⁴, (CO)OR¹⁵ or (CO)SR¹⁶.

In one embodiment, R¹⁵ is (C2-C6) alkyl, (C2-C4) hydroxyalkyl, phenyl, phenylalkyl wherein the alkyl is C1-C3. or aminoalky where the alkyl is C2-C6 and the amino can be substituted with up to two independent (C1-C3) alkyls, wherein the phenyl or the phenyl of phenylalkyl can be substituted as recited above. Preferably, n is zero. Preferably, R¹⁵ is hydrogen. Preferably, R⁴ is hydrogen, methyl or hydroxymethyl and R^{4*} is hydrogen. Preferably, at least one of R^{xa}, R^{ya} and R^{2a} is a heteroaryl comprising diazolyl, triazolyl, tetrazolyl, thiazolyl, isothiazolyl, oxazolyl, isoxazolyl, thiolyl, diazinyl, triazinyl, benzoazolyl, benzodiazolyl, benzothiazolyl, benzoxazolyl, benzoxolyl, benzothiolyl, quinolyl, isoquinolyl, benzodiazinyl, benzotriazinyl, pyridyl, thienyl, furanyl, pyrrolyl, indolyl, isoindoyl or pyrimidyl. Preferably, R¹ is -0-R⁸ or -S-R^{8*}. Preferably, the second bridge between two of R^{xa}, R^{ya} and R^{2a} (of Section (2ⁱⁱⁱ)(d)(i.)) is L. and satisfies the following formula: 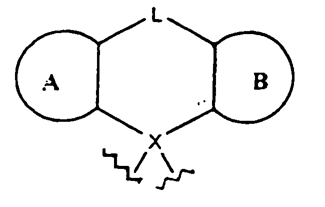 , wherein A and B are aryl or heteroaryl groups of R^{xa} and R^{ya}, respectively. Preferably, R^{xa}-R^{xb}-, R^{ya}-R^{yb}- and X form: 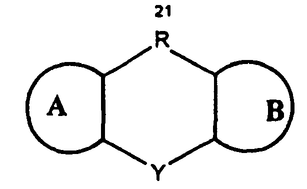 wherein Y is a carbon bonded to R¹ by a single or double bond or a nitrogen that is bonded to R¹ and wherein R²¹ either (i.) completes a single bond linking two aryl or heteroaryl rings of R^{x} and RY, (ii.) is (C1-C2) alkylene or alkenylene, (iii.) is sulfur or (iv.) is oxygen, and wherein R^{x} and R^{y} can be substituted as set forth above. Preferably, R²¹ is CH₂CH₂ or CH=CH. Preferably, the alkylenedioxy substitution of R^{xa}, R^{ya}, R^{2a}, R^{q}, R^{r} or R^{s} is as follows: 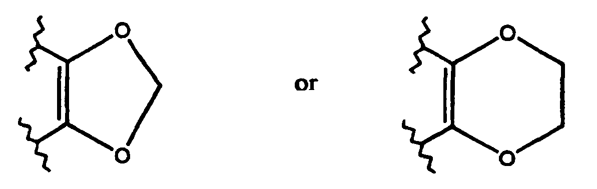 wherein the alkylenedioxy can be substituted with up to two independent (C1-C3) alkyl.

In one preferred embodiment, R^{xa} and R^{ya} together can be substituted with up to six substituents, R^{2a}, R^{q}, R^{r} and R^{s} can each be substituted with up to 3 substituents, and wherein the presence of each of R^{q}, R^{r} or R^{s} is considered a substitution to the respective ring structure of R^{xa}, R^{ya} and R^{2a}. Preferably, a phenyl of R³ is substituted with up to three substituents. Preferably, the compound is an optically pure enantiomer (i.e., at least about 80% ee, preferably at least about 90% ee, more preferably at least about 95% ee). Preferably, the compound is part of a pharmaceutical composition comprising a pharmaceutically acceptable excipient. Preferably, the compound of the composition is present in an effective amount for:
(1) treating or preventing schizophrenia,
(2) enhancing treating or preventing dementia,
(3) treating or preventing epilepsy,
(4) treating or preventing spasticity,
(5) treating or preventing muscle spasm,
(6) treating or preventing pain,
(7) preventing neural cell death after stroke,
(8) preventing neural cell death in an animal suffering from a neurodegenerative disease
(9) treating or preventing mood disorders such as depression,
(10) enhancing memory or learning, or
(11) treating or preventing learning disorders.

In another embodiment, the invention provides a method (1) of treating or preventing schizophrenia comprising administering a schizophrenia treating or preventing effective amount of a compound, (2) of treating or preventing dementia comprising administering a dementia treating or preventing effective amount of a compound, (3) of treating or preventing epilepsy comprising administering an epilepsy treating or preventing effective amount of a compound, (4) of treating or preventing spasticity comprising administering a spasticity treating or preventing effective amount of a compound, (5) of treating or preventing muscle spasm comprising administering a muscle spasm treating or preventing effective amount of a compound, (6) of treating or preventing pain comprising administering a pain treating or preventing effective amount of a compound, (7) of preventing neural cell death after stroke comprising administering a neural cell death preventing effective amount of a compound, (8) of preventing neural cell death in an animal suffering from a neurodegenerative disease, (9) treating or preventing mood disorders such as depression, (10) enhancing memory or learning, or (11) treating or preventing learning disorders, comprising administering an amount effective for said treating, preventing or enhancing of a compound of formula XI or a pharmaceutically acceptable salt thereof, wherein the substituents are as defined above, except that R²⁵ differs from R¹ in that it can be a straight-chained C4 aliphatic group. Preferably, the spasticity treated or prevented is associated with epilepsy, stroke, head trauma, multiple sclerosis, spinal cord injury or dystonia. Preferably, the neurodegenerative disease treated or prevented is Alzheimer's disease, multi-infarct dementia, AIDS dementia, Parkinson's disease, Huntington's disease, amyotrophic lateral sclerosis or stroke or head trauma (such as can result in neuronal cell death).

In another embodiment, the invention provides a method of synthesizing a compound of the invention comprising:
A) reacting a compound of one of the following formulas 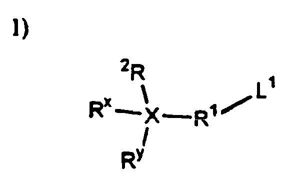 , wherein L¹ is a nucleophilic substitution leaving group, with a compound of the formula 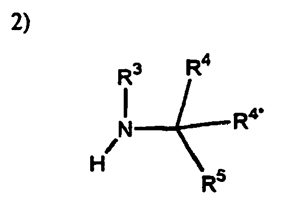
B) reacting a compound of the formula 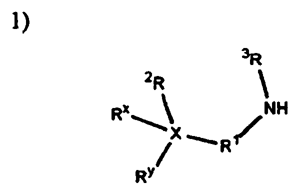 , with a compound of the formula 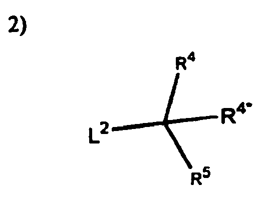 , wherein L² is a nucleophilic substitution leaving group.

In another embodiment, the invention provides a method of synthesizing a compound of the invention comprising:
A) reductively alkylating a compound of the formula 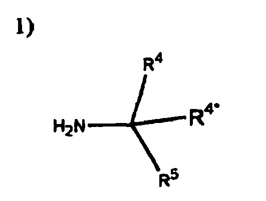 with a compound of the formula 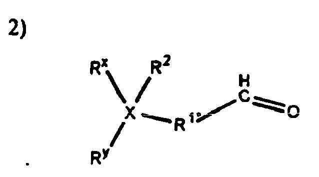 , where R^{1*} differs from R¹ in that it lacks the carbon that is part of the illustrated aldehyde carbonyl,
OR B) reductively alkylating a compound of the formula 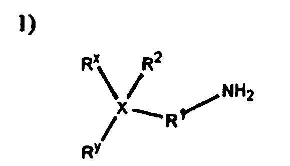 with a compound of the formula

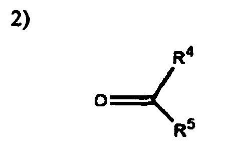

In another embodiment, the invention provides a method of synthesizing a compound of the invention comprising reductively alkylating R^{d}NH₂ with a compound of the formula 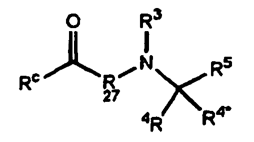 wherein R^{d} and R^{c} are independently the same as defined for R^{x}, and wherein R²⁷ has the same definition as R¹ except that it does not include a nitrogen, oxygen or sulfur and does not include any double bonds conjugated with the above-illustrated carbonyl.

In another embodiment, the invention provides a method of synthesizing a compound of the invention comprising reacting R^{f}OH or R^{f*}SH with a compound of the formula 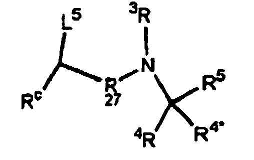 to form an ether or a thioether, respectively, wherein R^{f} and R^{f*} are independently the same as defined for R^{x}, wherein R²⁷ has the same definition as R¹ except that it does not include a nitrogen, oxygen or sulfur and does not include any double bonds at the atom bonded to the above-illustrated L⁵-substituted carbon and wherein L⁵ is a nucleophilic substitution leaving group.

The method of claim 28, further comprising synthesizing the compound of formula 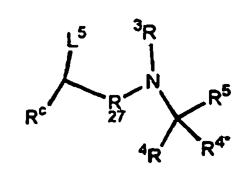 by replacing the hydroxyl of formula 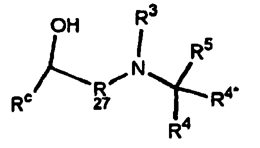 with another nucleophilic substitution leaving group. Preferably, the method comprises reacting a compound of formula 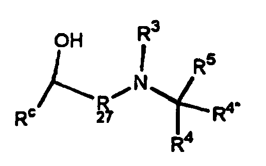 with an azodicarboxylate in the presence of a phosphine compound.

In another embodiment, the invention provides a method of synthesizing a compound of the invention comprising reacting R^{e}M with a compound of the formula 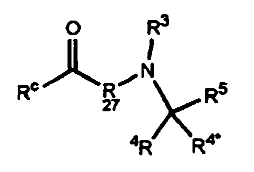 to form a compound of the formula 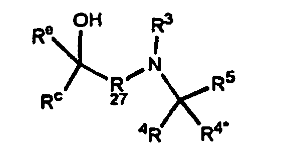 wherein R^{e} is independently the same as defined for R^{x}, wherein M is a metal-containing substituent such that R^{e}M is a organometallic reagent.

In another embodiment, the invention provides a method of synthesizing a compound of the invention comprising dehydrating a compound of the formula 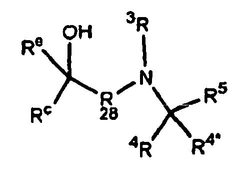 to form a compound of the formula 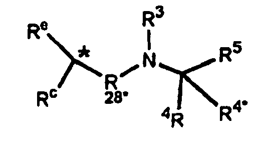 wherein C^{*} (the tertiary carbon marked with an adjacent "*") has a double bond with an adjacent carbon, R^{28*} and R²⁸ have the same definition as R¹ except that R^{28*} and R²⁸ do not include a heteroatom.

In another embodiment, the invention provides a method of synthesizing a compound of the invention comprising reducing a compound of the formula 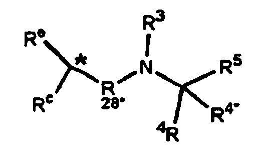 wherein C^{*} has a double bond with an adjacent carbon and R^{c} is independently the same as defined for R^{x}, to form a compound of the formula

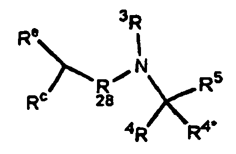

In another embodiment, the invention provides a method of synthesizing a compound that can be used to synthesize the compound of the invention, the method comprising synthesizing the compound of formula: 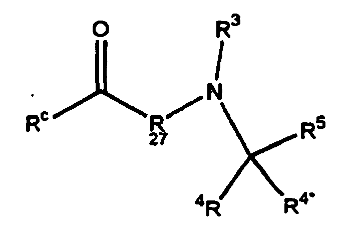 with a compound of formula 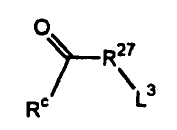 with a compound of formula 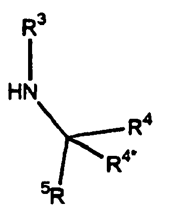 , wherein L³ is a nucleophilic substitution leaving group.

In another embodiment, the invention provides a method of synthesizing of a compound of the invention, the method comprising reacting a compound of formula 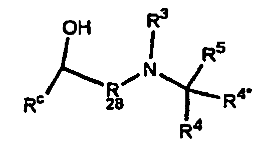 with Ar-Q wherein Ar is aryl which is substituted with an electron-withdrawing group or heteroaryl which is substituted with an electron-withdrawing group, and wherein Q is halide (preferably fluoro or chloro), to form

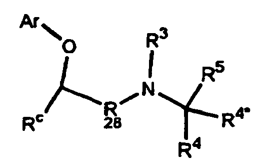

In another embodiment, the invention provides a method of synthesizing a compound that can be used to synthesize the compound of the invention, the method comprising synthesizing a compound of formula X: 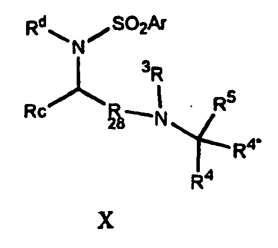 by reacting a compound of formula: 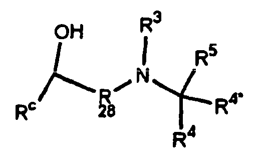 with R^{d}NHSO₂Ar. The method can further comprise converting the compound of formula X to:

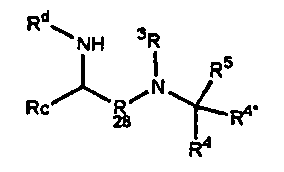

In another embodiment, the invention provides a method of synthesizing a compound that can be used to synthesize the compound of the invention, the method comprising reacting a compound of formula 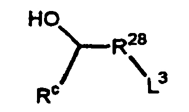 with a compound of formula 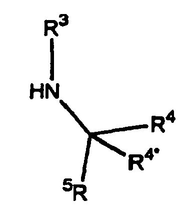 to form a compound of formula

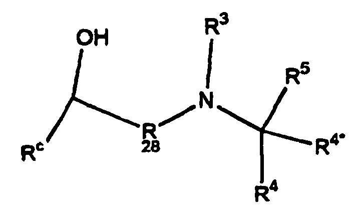

In another embodiment, the invention provides a method of synthesizing a compound that can be used to synthesize the compound of the invention, the method comprising synthesizing the compound of formula: 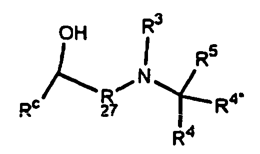 said synthesis comprising reducing the ketone of a compound of formula

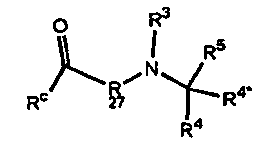

### BRIEF DESCRIPTION OF THE DRAWING

**Figure 1** depicts several reactions that can be employed in the synthesis of the compounds of the invention.
**Figure 2** depicts representative syntheses utilized in making compounds of the invention.
**Figure 3** shows additional representative syntheses utilized in making compounds of the invention.
**Figure 4** shows additional representative syntheses utilized in making compounds of the invention.

### DEFINITIONS

The following terms shall have the meaning set forth below:

### ◆ excipient

Excipients are pharmaceutically acceptable organic or inorganic carrier substances suitable for parenteral, enteral (e.g., oral or inhalation) or topical application that do not deleteriously react with the active compositions. Suitable pharmaceutically acceptable carriers include but are not limited to water, salt solutions, alcohols, gum arabic, benzyl alcohols, gelatine, carbohydrates such as lactose, amylose or starch, magnesium stearate, talc, silicic acid, hydroxymethylcellulose, polyvinylpyrrolidone, and the like.

### ◆ effective amount

The meaning of "effective amount" will be recognized by clinicians but includes amount effective to (1) reduce, ameliorate or eliminate one or more symptoms of the disease sought to be treated, (2) induce a pharmacological change relevant to treating the disease sought to be treated, or (3) prevent or lessen the frequency of occurrence of a disease.

### ◆ neuronal cell death prevention

Neuronal cell death is "prevented" if there is a reduction in the amount of cell death that would have been expected to have occurred but for the administration of a compound of the invention.

### ◆ oxo substitution

References to oxo as a "substituent" refer to "=O" substitutions.

### DETAILED DESCRIPTION

The compounds of the invention are generally prepared according to one of the following synthetic schemes, although alternative schemes will be recognized by those of ordinary skill. 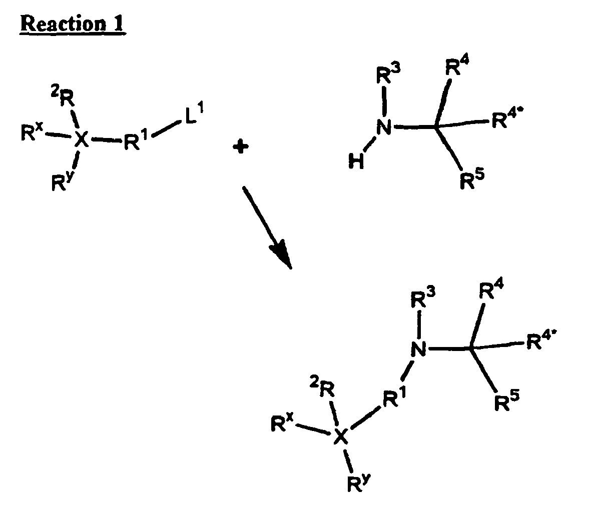 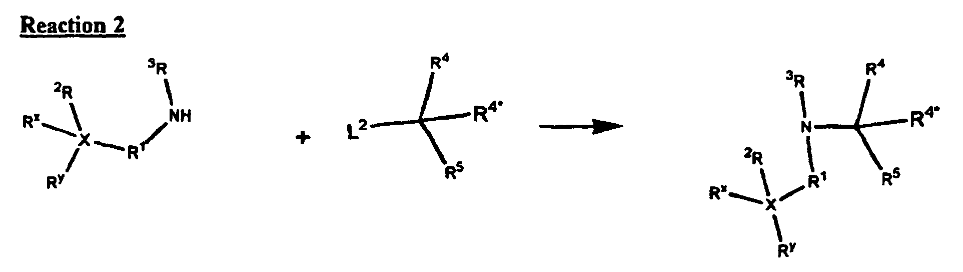 In Reaction 1 or Reaction 2, L¹ and L² are good nucleophilic substitution leaving groups such as a halide, especially a bromide, a tosylate, a brosylate (p-bromobenzenesulfonate), and the like. The reaction is preferably conducted in the presence of a base such as potassium carbonate or a tertiary amine such as diisopropylethylamine. Where the leaving group is a halide, the reaction is preferably conducted in the presence of an iodide salt such as potassium iodide. Suitable organic solvents include, for example, methanol, dioxane, acetonitrile or dimethyformamide. Reaction 1 is favorably conducted at a temperature range of about 50°C to about 100°C. Reaction 2 is favorably conducted at a temperature range of about 15°C to about 40°C. Avoiding more elevated temperatures helps decrease the formation of additional alkylation products. Those of ordinary skill will recognize that reaction 2 should be conducted with compounds that lack ring C.

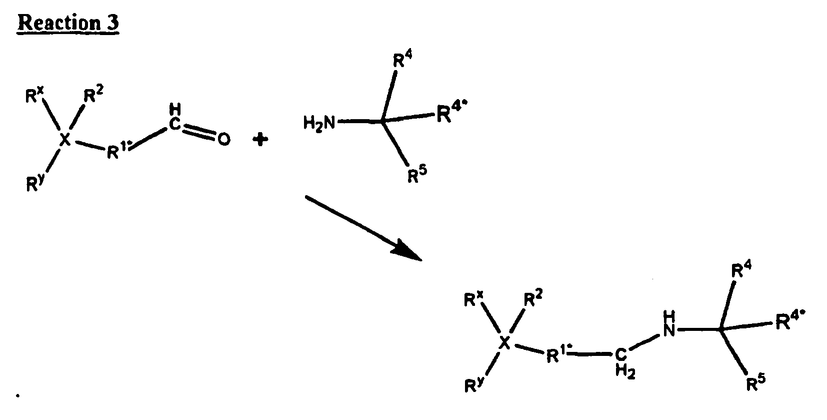

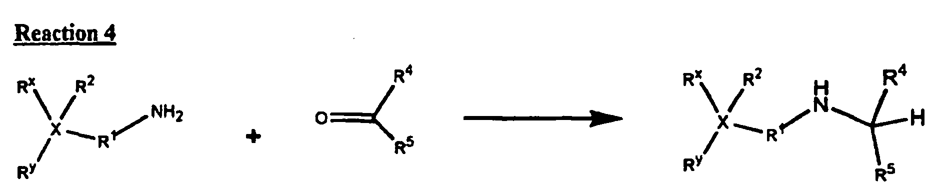

In Reaction 3, R^{1*} satisfies the definition of R¹ except for the absence of the carbon that is part of an aldehyde group in the starting material. The reductive alkylation of Reaction 3 or Reaction 4 can be effected by several known methods (see, for example, "Reductive Alkylation," W.S. Emerson in Organic Reactions, Vol. 4, John Wiley & Sons, 1948, p. 174 *et seq.)* including reaction with hydrogen in the presence of a catalyst such as palladium on carbon, reaction with sodium cyanoborohydride or reaction with sodium triacetoxyborohydride when groups labile to catalytic hydrogenation are present. It will be recognized that an intermediate Schiff's base is formed in the reaction, which Schiffs base is reduced to form the linkage. The intermediate Schiffs base can be isolated and then reduced in a separate reaction. Solvent selection will vary with such factors as the solubility of the starting materials, the degree to which the solvent favors the dehydration reaction forming the Schiffs base, and the suitability of the solvent in the reduction process. Suitable solvents using catalytic hydrogenation to reduce the Schiff's base include ethanol. Suitable solvents using a borohydride to reduce the Schiffs base include alcoholic solvents such as methanol or ethanol. In some cases, a drying process can be employed during the reaction to promote the dehydration reaction that forms the Schiffs base that is reduced. Such drying processes include refluxing under conditions selected to remove water as an azeotrope or the use of molecular sieves or other drying reagents. Suitable reaction temperatures include the range from about 20°C to the reflux temperature of the solvent employed.

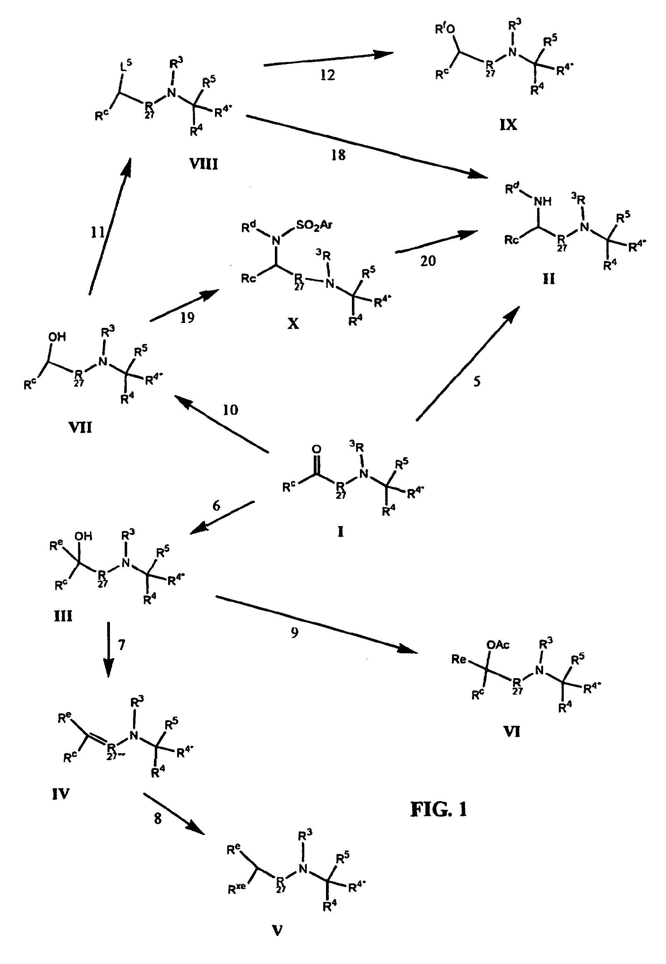

In Reaction 5, shown in Figure 1, R^{c} is independently the same as defined for R^{x}. The starting material **I** can be synthesized, for instance, using the chemistry of Reaction 13 (similar to Reaction 1), as follows: 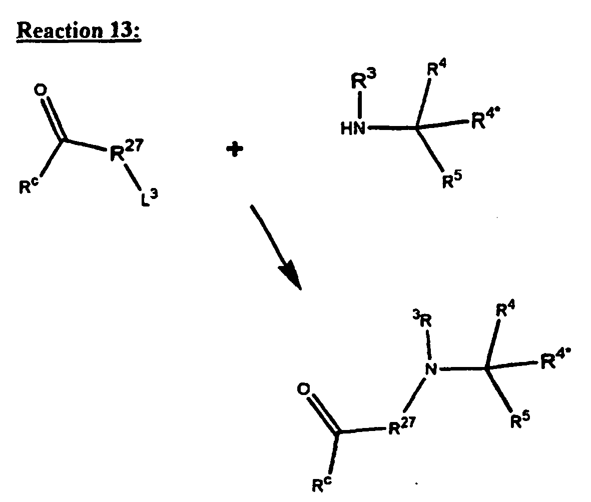 , wherein R²⁷ has the same definition as R ¹ except that it does not include a nitrogen, oxygen or sulfur and does not include any double bonds conjugated with the above-illustrated carbonyl, and wherein L³ is a good nucleophilic substitution leaving group such as a halide, especially a bromide, a tosylate, a brosylate (p-bromobenzenesulfonate), and the like. In Reaction 5 shown in Figure 1, R^{d}-NH₂ is reacted with **I** to give **II** under conditions that effect a reductive alkylation, as described for Reaction 3 and Reaction 4. R^{d} is independently the same as defined for R^{x}. Alternatively, **II** can be synthesized via Reaction 18 by reacting R^{d}-NH₂ with **VIII** under the conditions described for Reaction 1.

In Reaction 6, shown in Figure 1, R^{e} is independently the same as defined for R^{X}. In Reaction 6, **I** is reacted with a organometallic reagent such as an aryllithium or an aryl or arylalkyl Grignard reagent to form **III**, as described, for instance, in Section 5.1.2 of Cary and Sundberg, Advanced Organic Chemistry, Part 2, Plenum, New York, 1977, pp. 170 -180, and references cited therein. This reaction is described below in more detail for the synthesis of compound A32 (step 2 of Example 5A). Those of ordinary skill will be aware that in some cases where R⁵ includes an ester, the organometallic reagent may react with the ester group; in those such cases where the yield of the desired product is too low, the solvent, the organometallic reagent or the ester substitution can be varied.

In Reaction 7, shown in Figure 1, **III** is subjected to conditions suitable for dehydration to form the double bond of **IV.** Such conditions are, for instance, those described in H. Weiland, Ber. 45: 484 *et seq.* (1912), wherein **III** is refluxed with acetic anhydride. In the illustration, the double bond forms with the adjacent carbon atom of R²⁷. The double bond will typically form with this orientation where R^{c} and R^{e} are aryl or heteroaryl and the adjacent carbon of R²⁷ is saturated and not fully substituted, but other orientations are possible depending on the composition of R^{c}, R^{e} and R²⁷.

In Reaction 8, shown in Figure 1, **IV** is reduced to form **V,** for instance using any of a number of known methods for reducing carbon-carbon double bonds, such as catalytic hydrogenation in the presence of an appropriate hydrogenation catalyst. An example of this process is described below for compound A4 (Example 10).

In Reaction 9, shown in Figure 1, **III** is acylated, for instance, with acetic anhydride in the presence of an acylation catalyst such as 4-dimethylaminopyridine. In this context, R³ should not be hydrogen, though a hydrogen substituent can be restored to this position after Reaction 9 by using a suitable protecting group to mask the nitrogen.

In Reaction 10, shown in Figure 1, the ketone moiety of I is reduced, for instance by any of a number of known methods for selectively reducing ketones, such as reaction with lithium tri-*tert*-butoacyaluminohydride. An example of this process is described below for the preparation of compound A31 (step 1 of Example 8A).

For Reaction 11, shown in Figure 1, the hydroxyl of **VII** is replaced by a leaving group L⁵, wherein the leaving group is, for instance, chloro or bromo, by reacting **VII** with, for instance, thionyl chloride or thionyl bromide. An example of this process is described below for the preparation of compound A31 (step 2 of Example 8A).

For Reaction 12, shown in Figure 1, R^{f} is independently the same as defined for R^{x}. **VIII** is reacted with R^{f}OH in the presence of a base such as potassium carbonate or sodium hydride. Alternatively, the thio-containing analog of **IX** can be synthesized by reacting **VIII** with R^{f}SH. An example of this process is described below for the synthesis of compound A31 (step 3 of Example 8A). The transformations of Reactions 11 and 12 can be conducted in a single pot, for instance by a Mitzunobu reaction such as described in Examples 8C, Step 1 and 8D, Step 2. Alternatively, VII can be directly reacted with an aryl halide or chloride, preferably an aryl fluoride or chloride, to form IX, such as is described in U.S. Patent Nos. 5, 166,437 and 5,362,886. It will be recognized that typically the aryl halide used in this reaction will typically have an electron-withdrawing group that facilitates the reaction, such as a trifluoromethyl or nitro group in the para position. 1-fluoronaphthalene is also suitable for this reaction, since the ring fused to the fluoro-substituted ring is the electron withdrawing group.

In reaction 19, VII is reacted with R^{d}NHSO₂Ar to yield X, as described for example in Example 8C, Step 1. In reaction 20, X is coverted to II as described, for example, in Example 8C, Step 2.

A number of other well-known synthetic approaches can be applied. For instance, acids can be formed by the hydrolysis of the corresponding esters. Amine derivatives can be formed by the alkylation of primary, secondary or tertiary amines. A number of double bond containing compounds can be hydrogenated to form the corresponding single bond. The N-oxide compounds of the invention are typically formed from the corresponding tertiary nitrogen by known methods.

In some cases, the chemistries outlined above may have to be modified, for instance by use of protective groups, to prevent side reactions due to reactive groups, such as reactive groups incorporated into heterocyclic rings or attached as substituents.

Compounds of the invention may also be prepared by adapting the classical solution chemistries outlined above into solid-phase synthetic techniques. For example, R¹³, R¹⁵, R¹⁶, R¹⁷ and R²⁰ can be residues other than hydrogen representing functionalized resin or suitably selected linker attached to functionalized resin. The linker and the functional group represented by R⁵ should be stable under the conditions employed for the above-descnbed reactions. The compounds of the invention where R¹³, R¹⁵, R¹⁶, R¹⁷ is R²⁰ is hydrogen, are then cleaved from the resin or the linker leaving the remainder of the molecule intact. For example, solid-phase synthesis of peptoids [oligo(N-substituted glyeines)] using robotic synthesizer was described by Zuckermann et al., J. Am. Chem. Soc., 114, 10646-10647, (1992) and Spellmeyer et al., WO 95/04072. Under analogous conditions, acylation reaction of Rink amide polystyrene resin with bromoacetic acid in the presence of N,N'-diisopropylcarbodiimide followed by displacement of the bromine with N-substituted amine (Reaction 2) and cleavage can provide N-substituted glycinamides (R¹³ and R¹⁴ are hydrogen).

Using the reactions described herein, including hydrolysis of esters, alkylation of amines, or hydrogenation reactions, the following compounds of the invention have been synthesized:

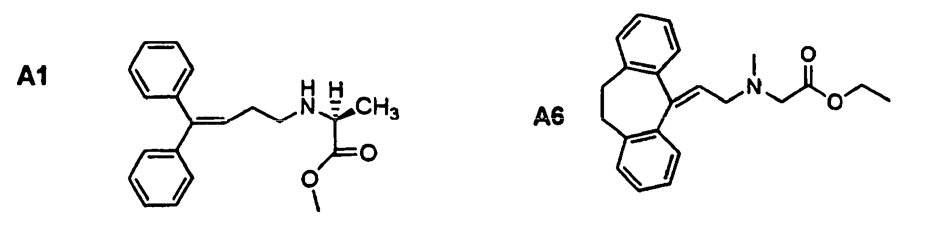

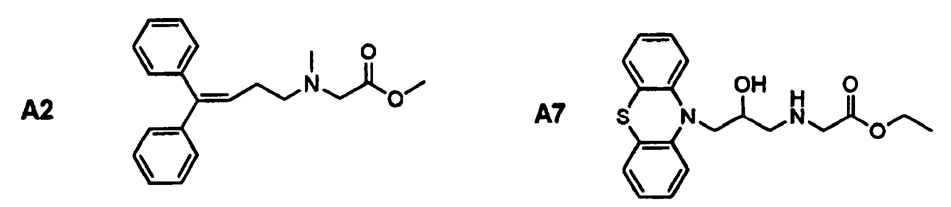

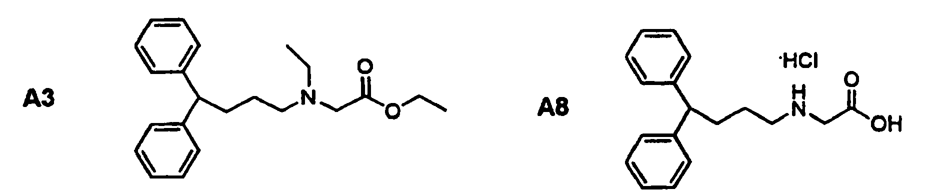

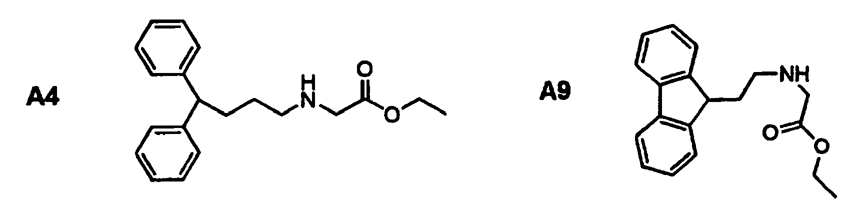

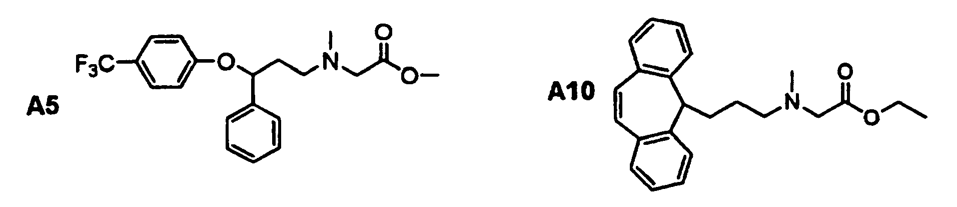

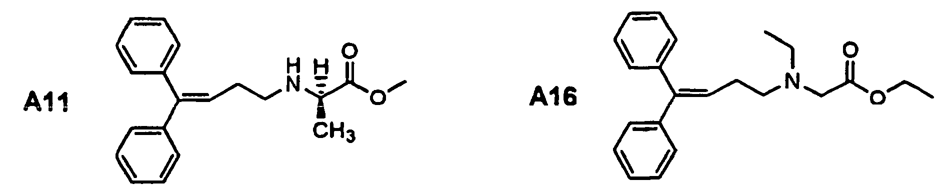

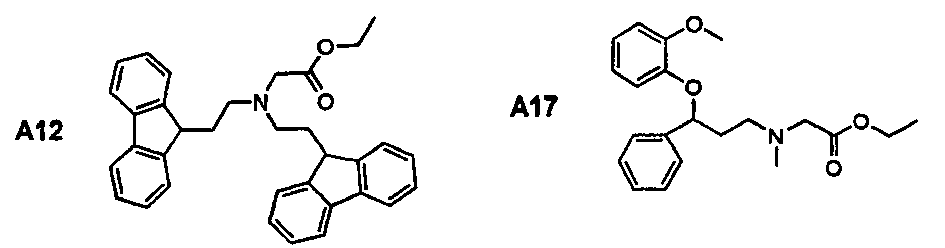

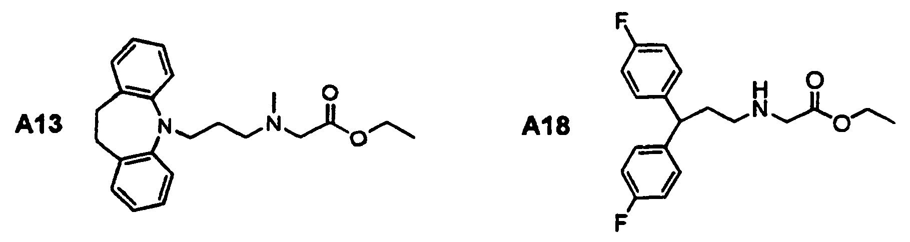

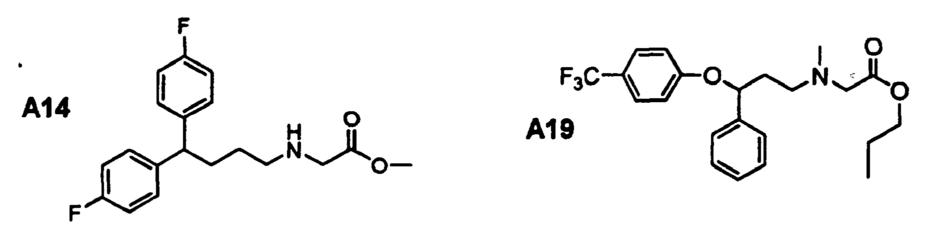

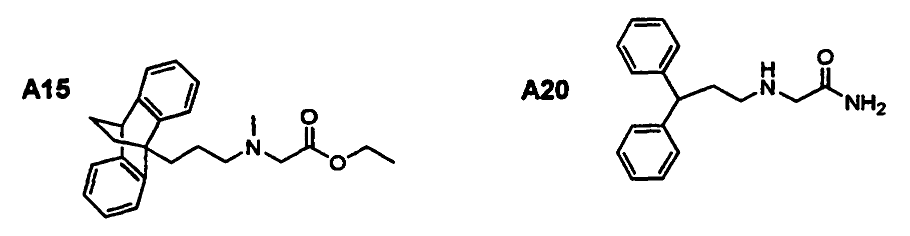

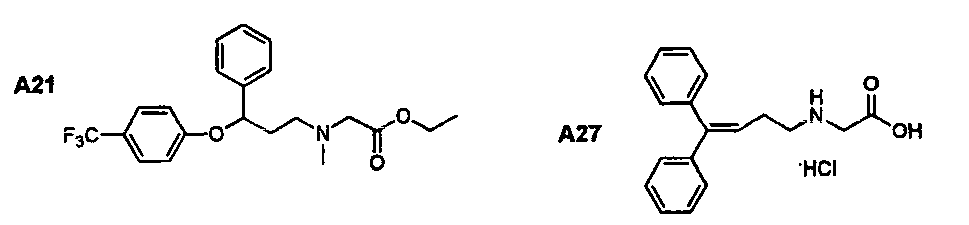

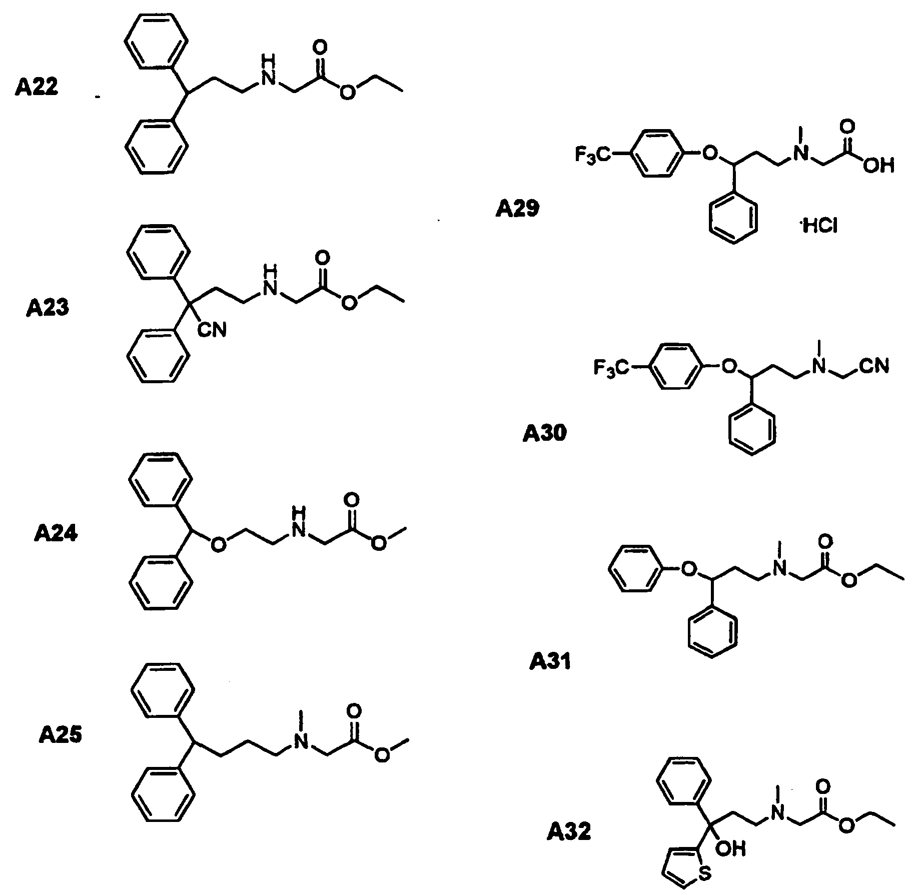

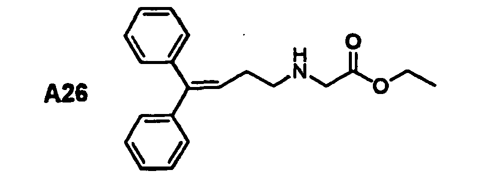

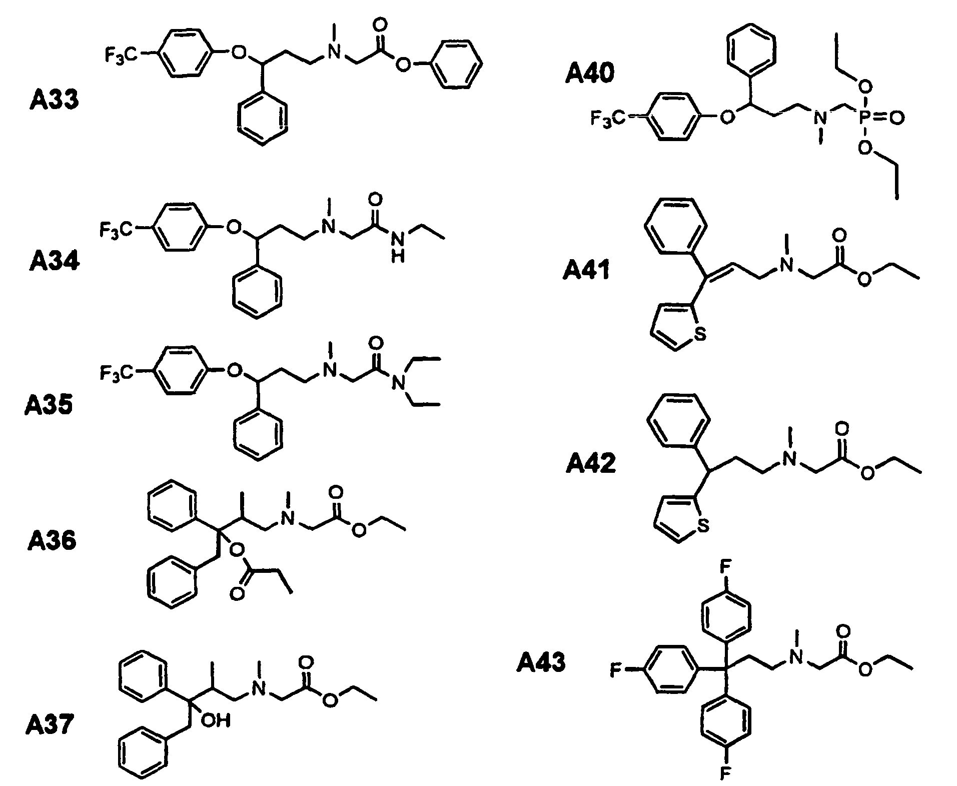

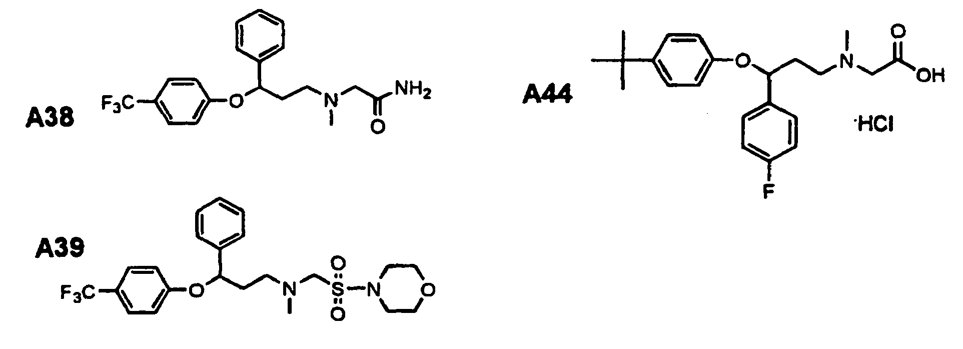

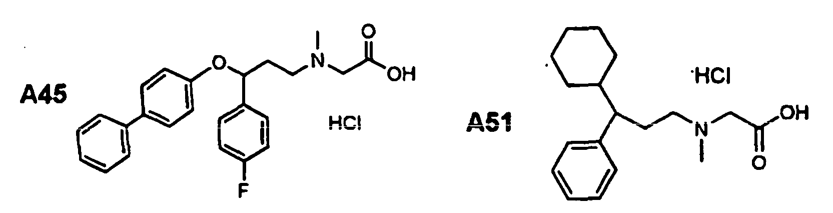

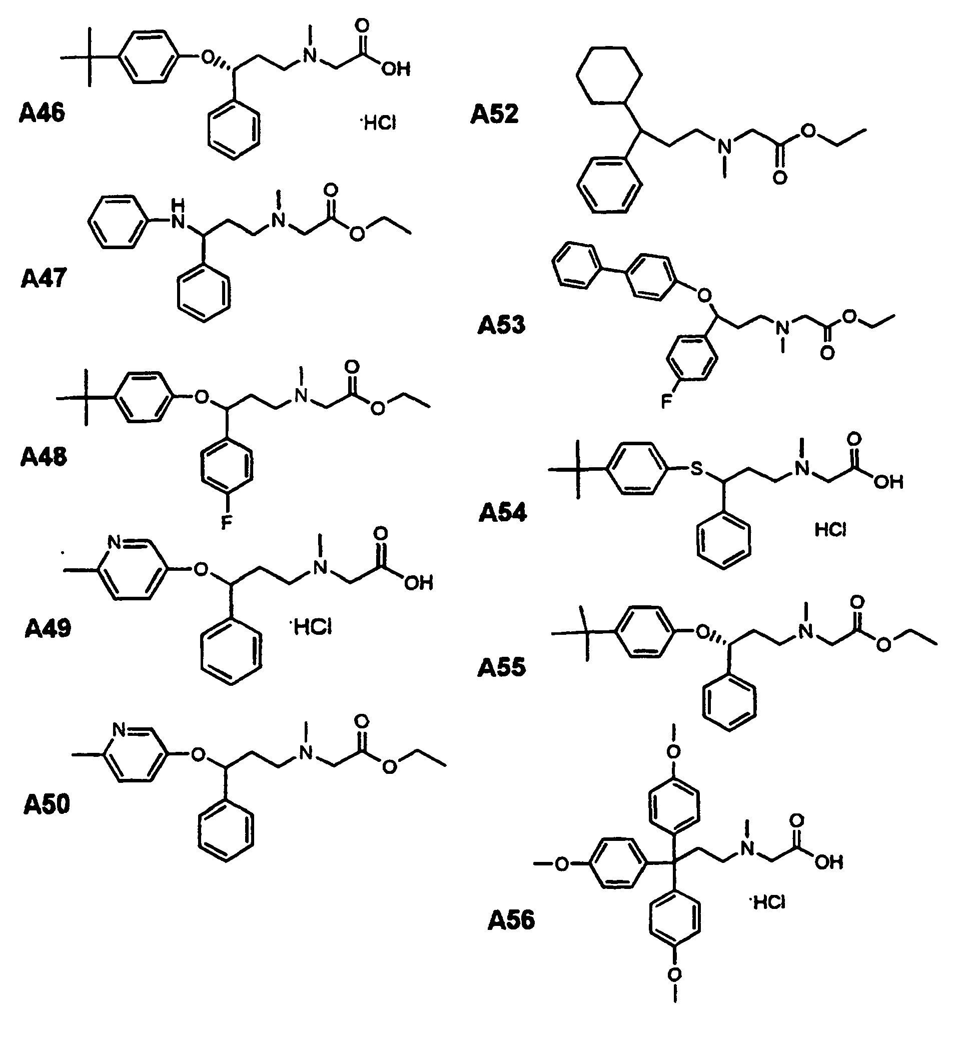

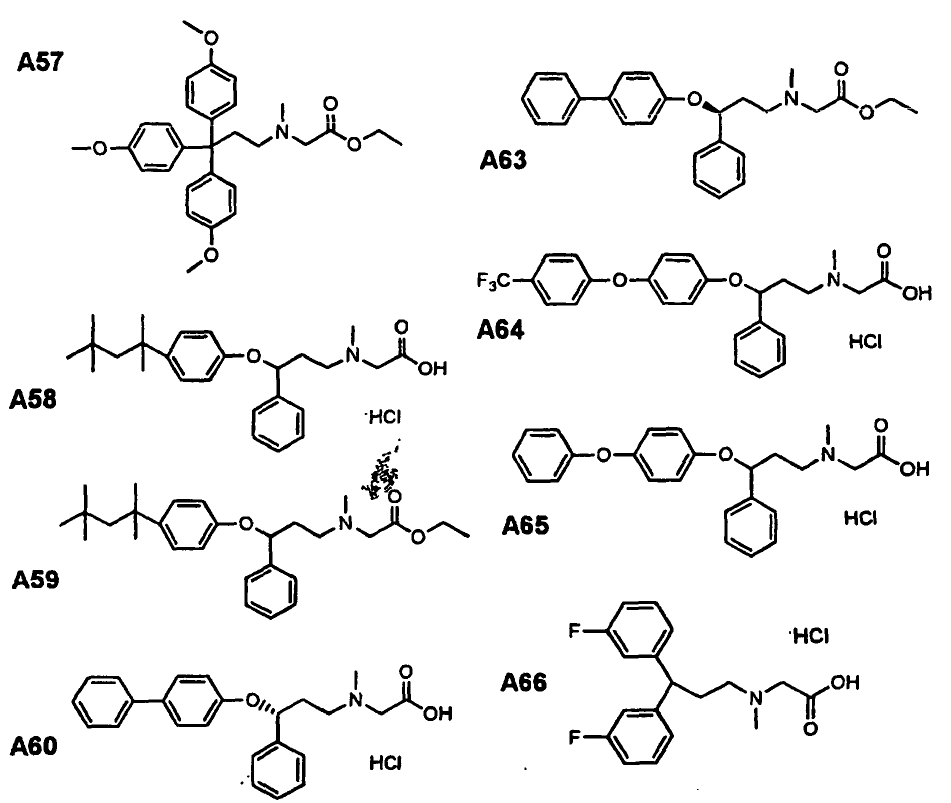

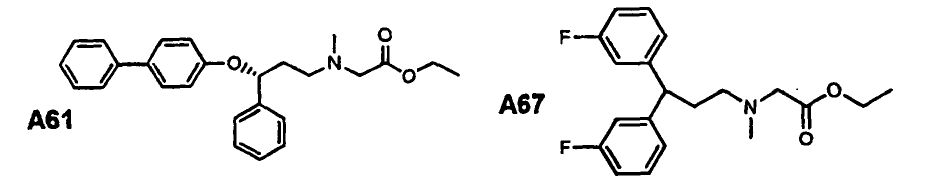

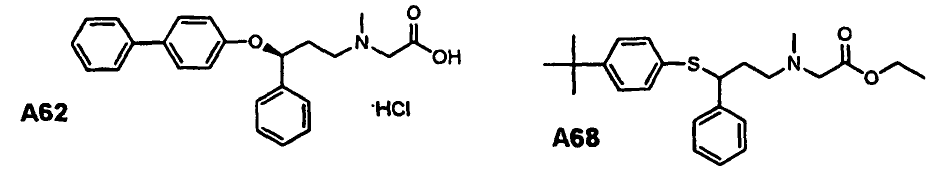

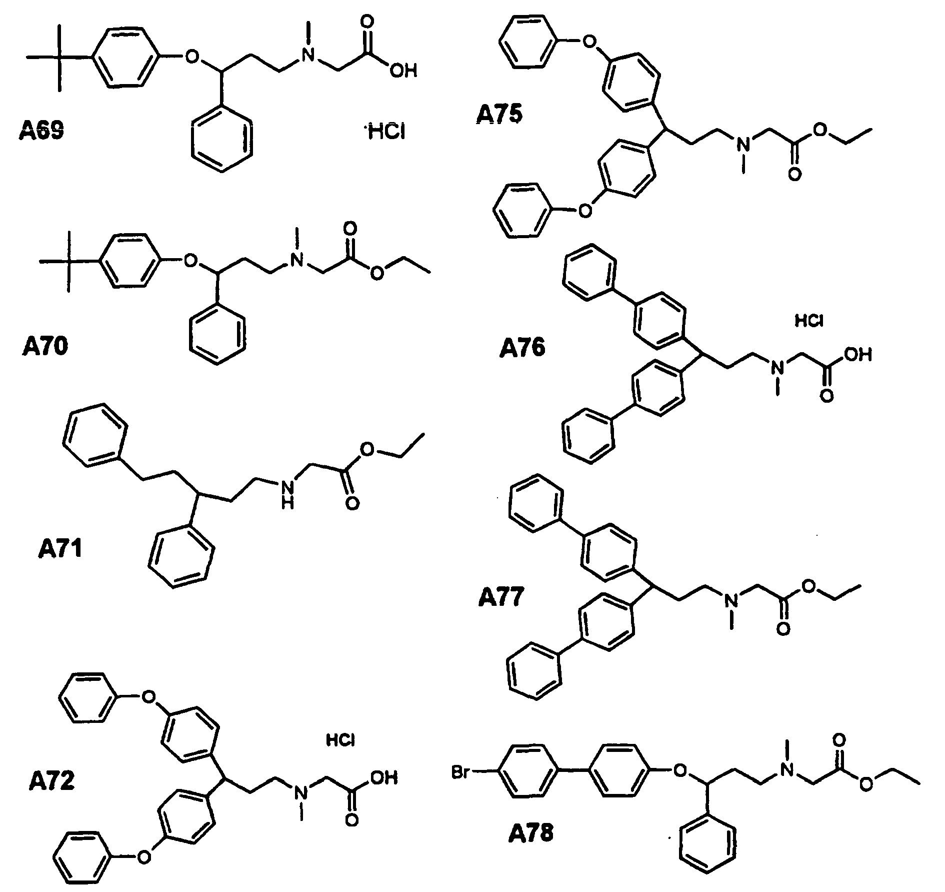

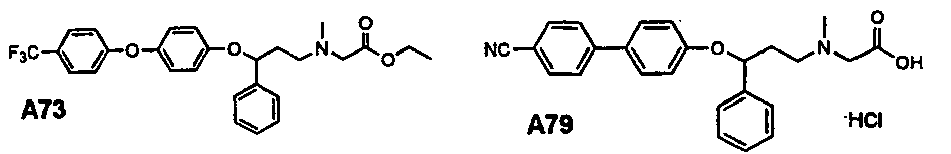

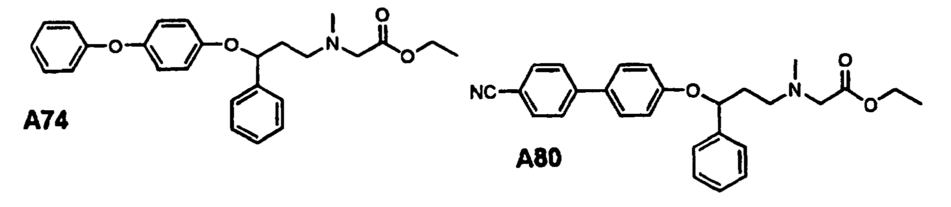

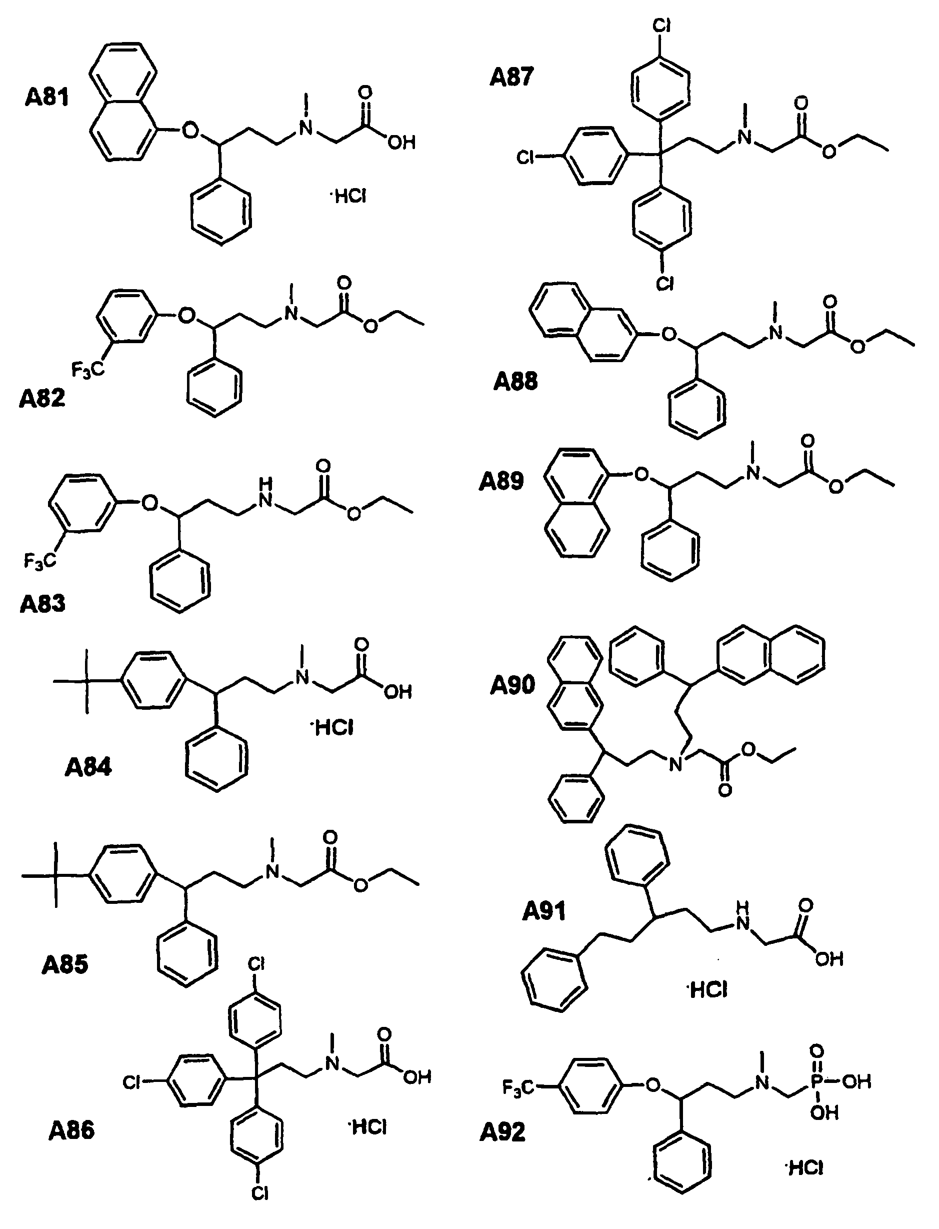

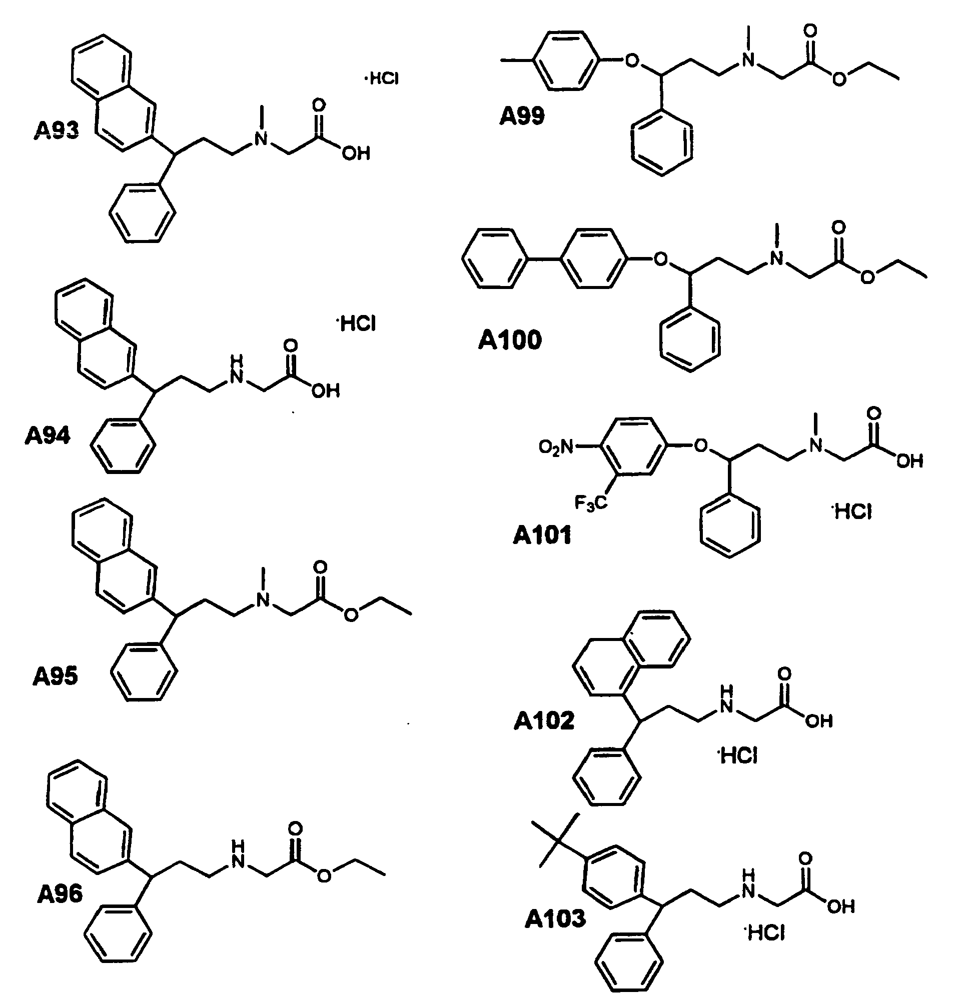

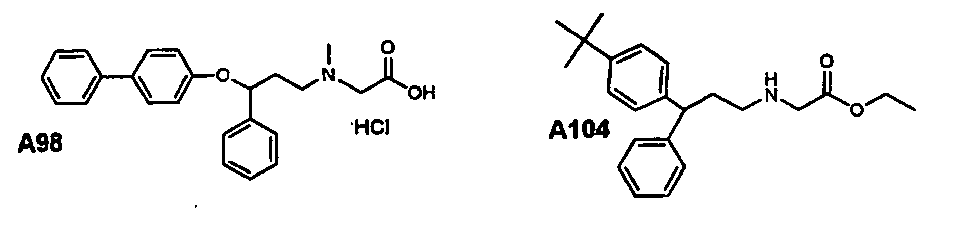

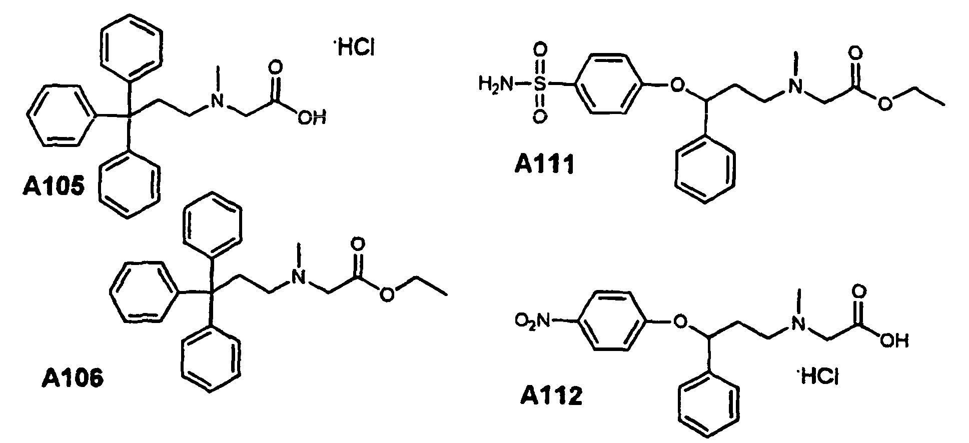

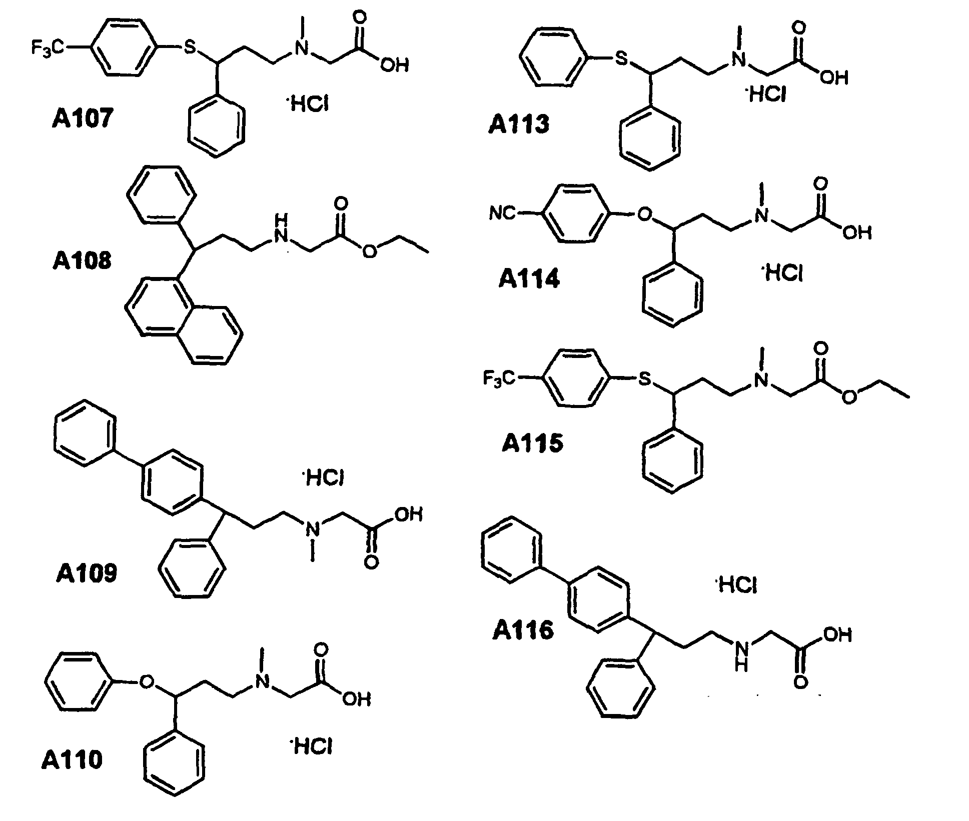

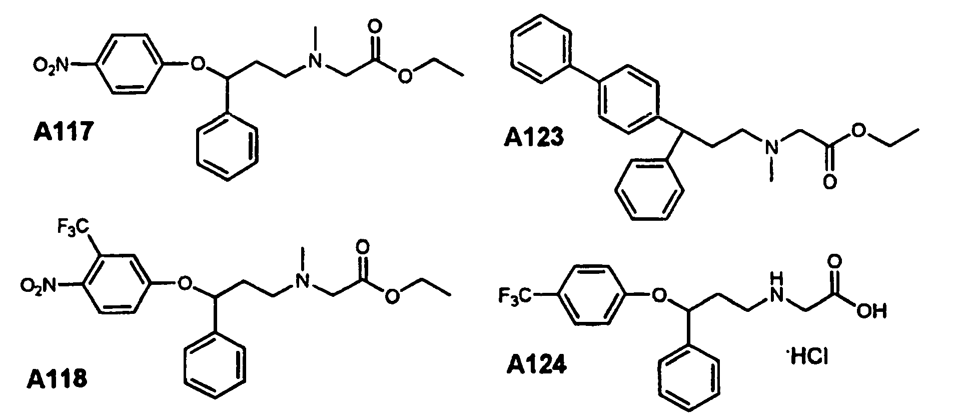

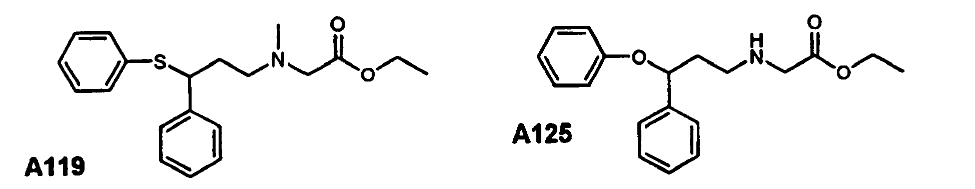

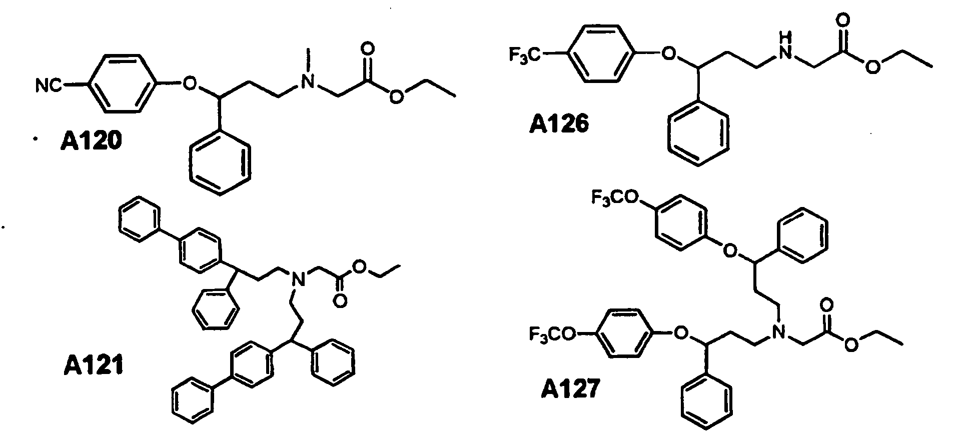

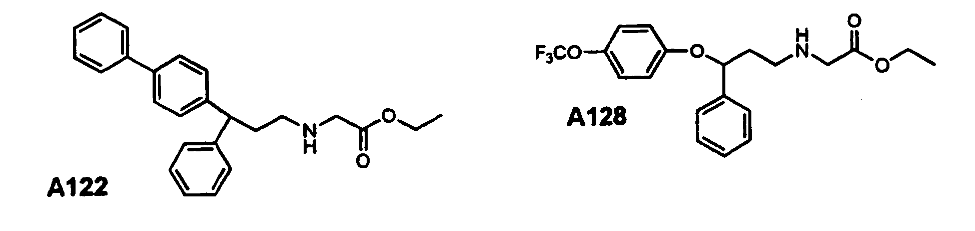

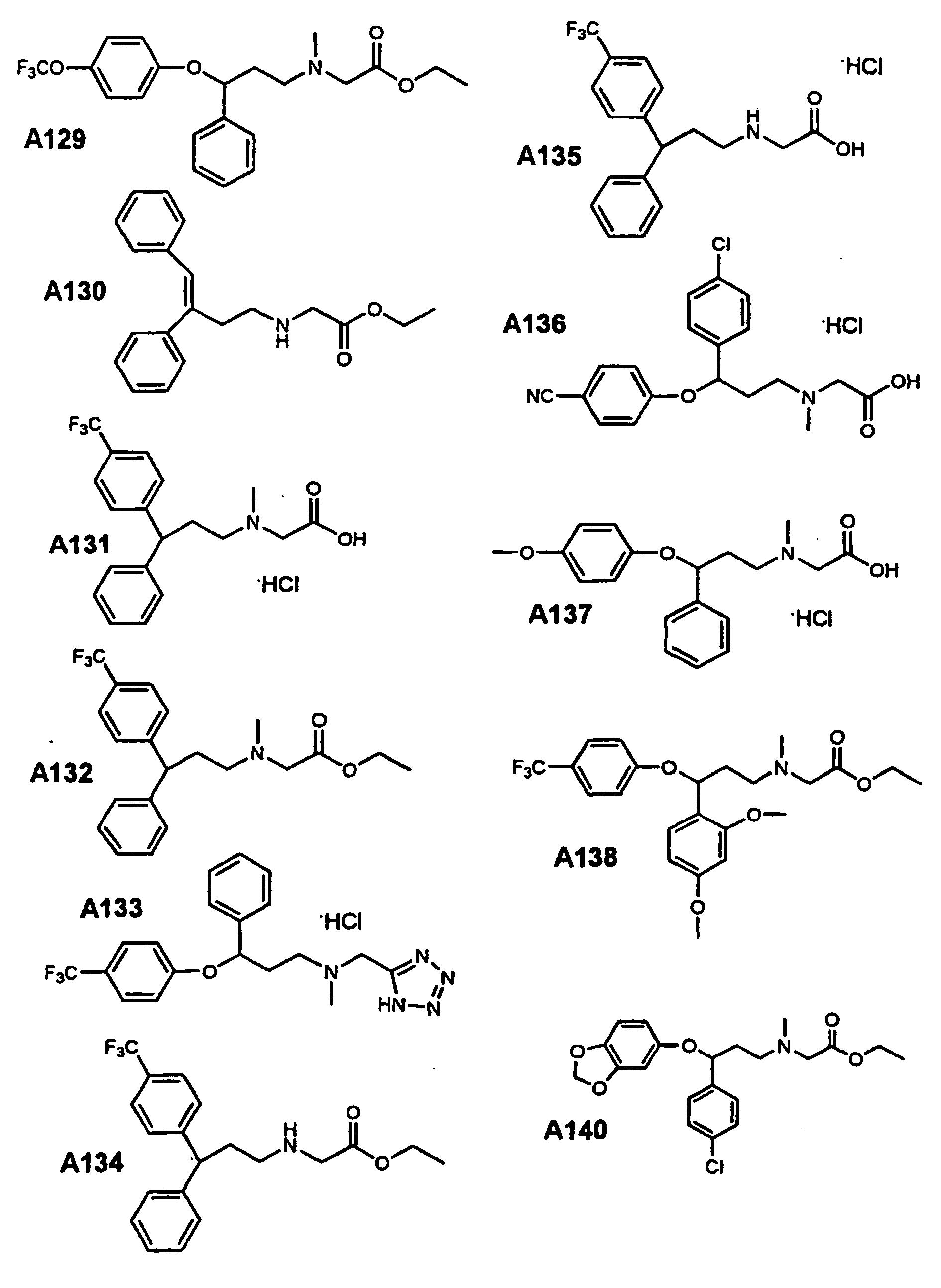

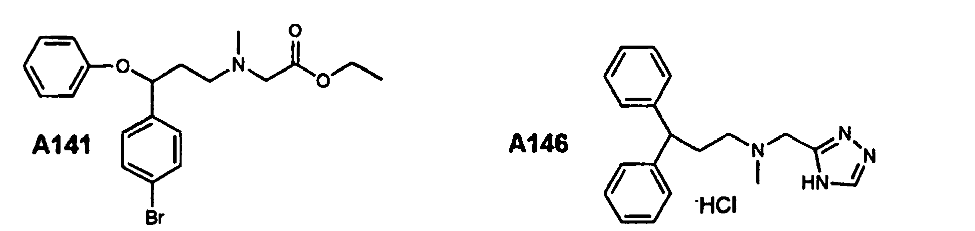

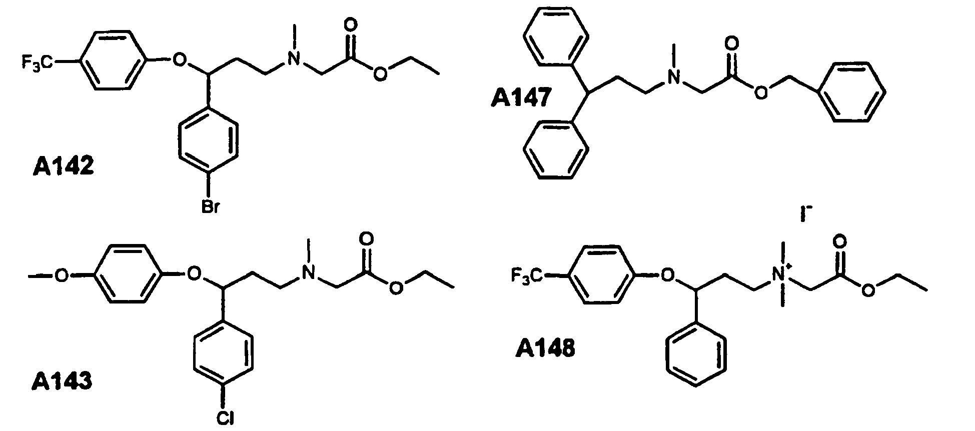

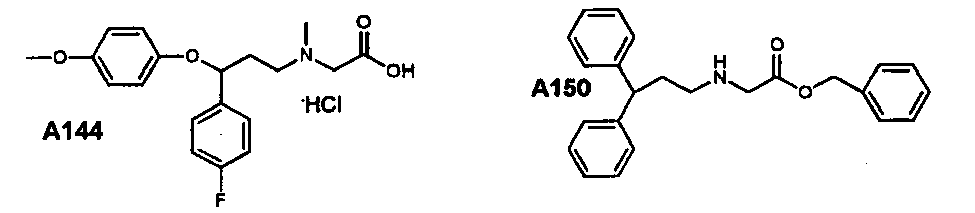

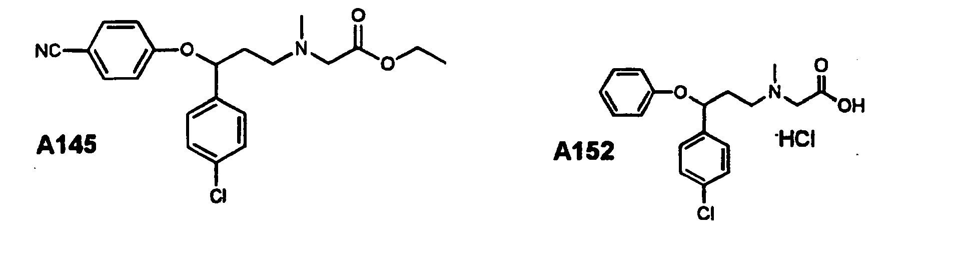

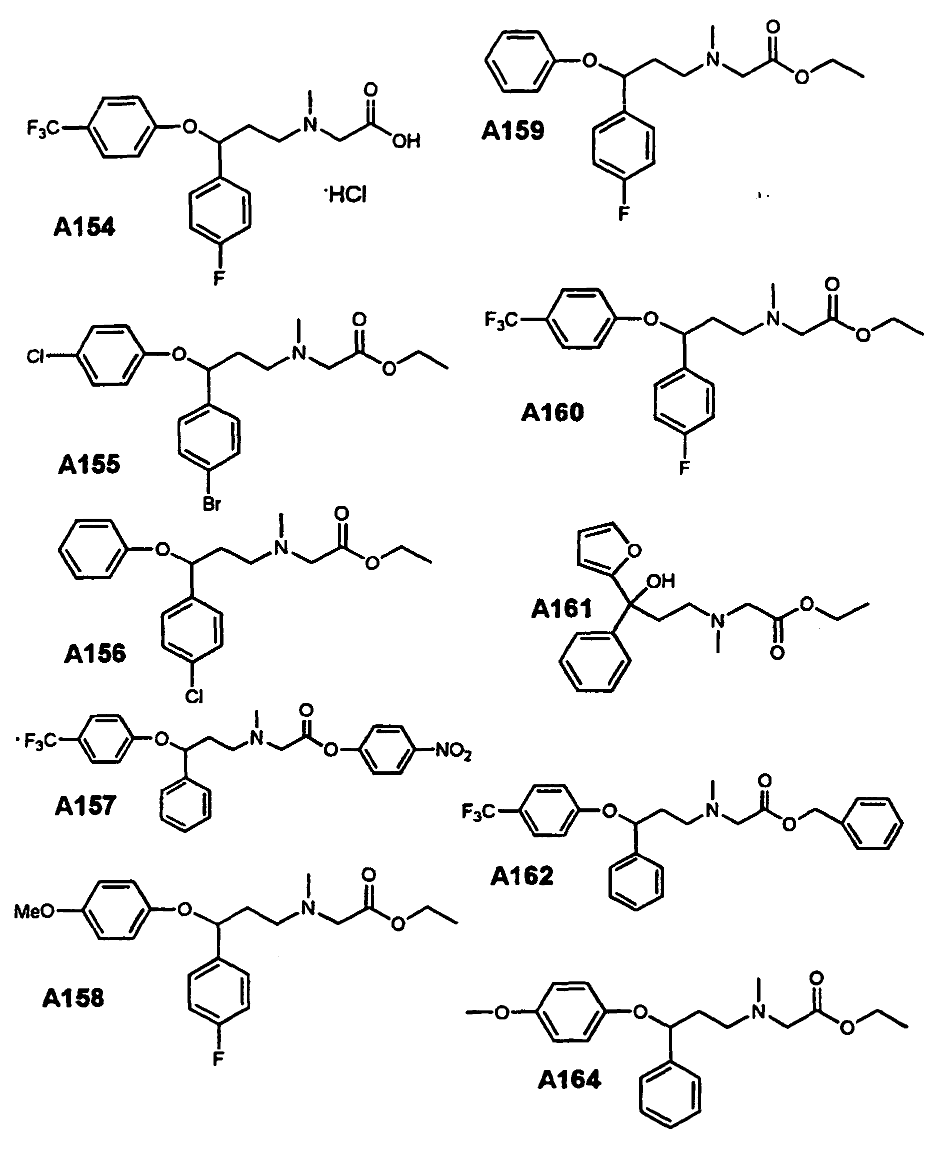

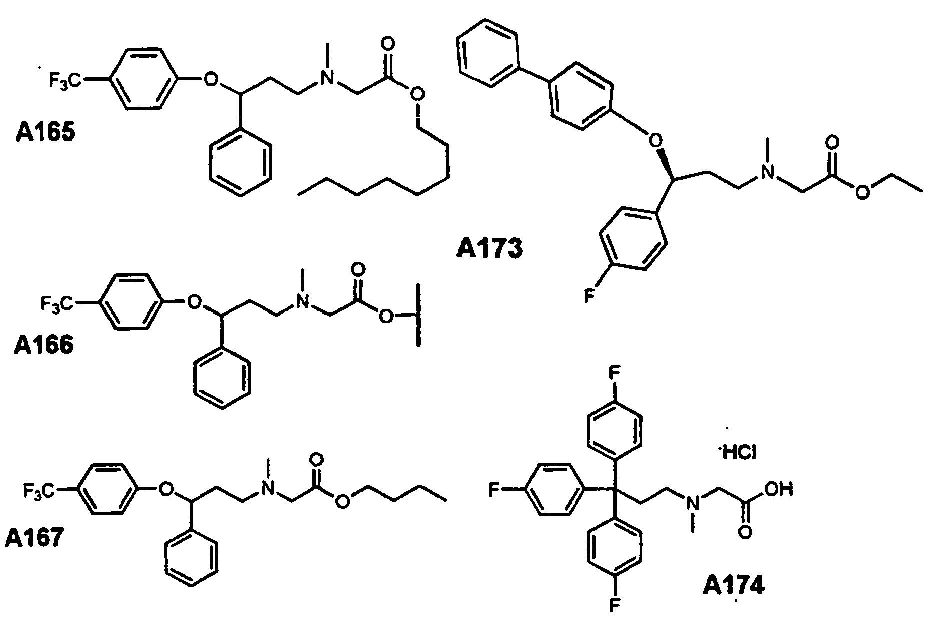

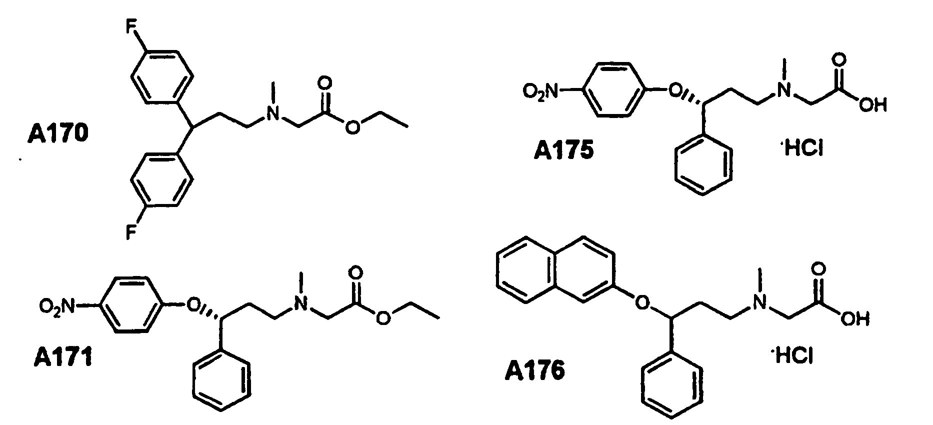

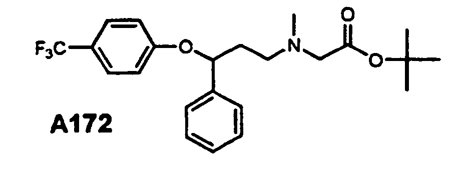

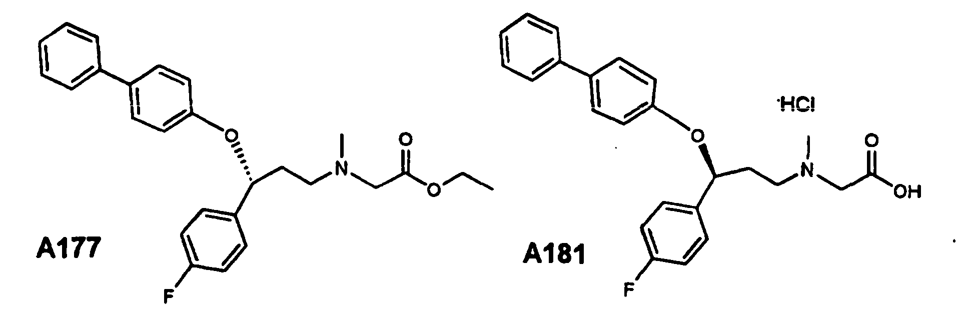

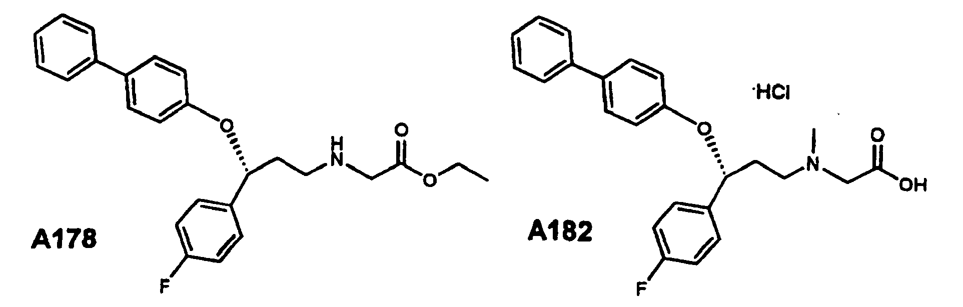

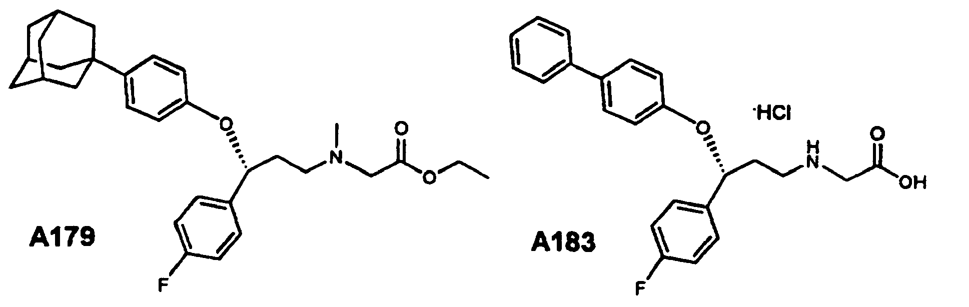

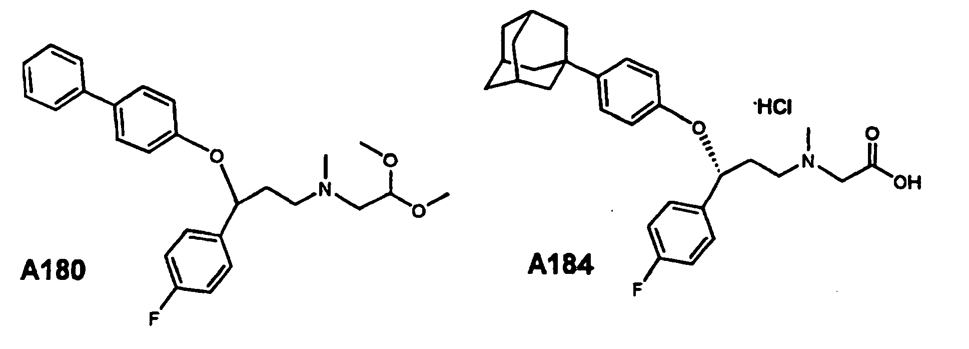

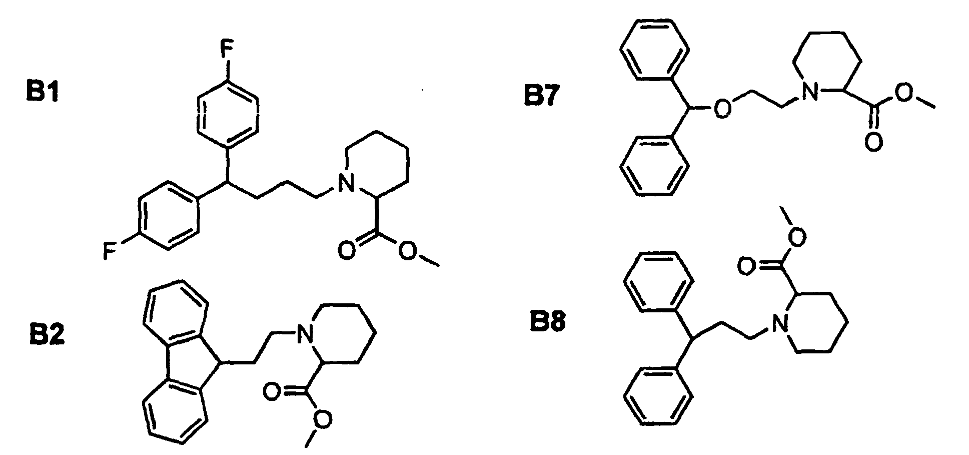

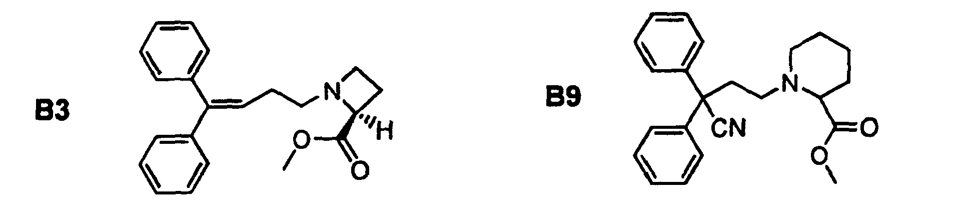

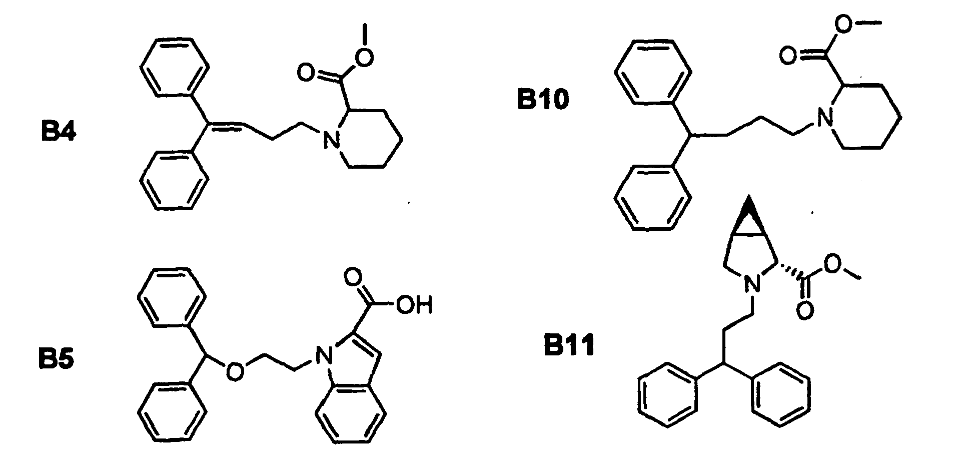

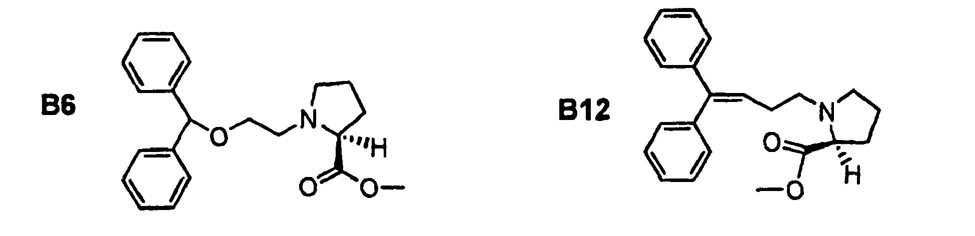

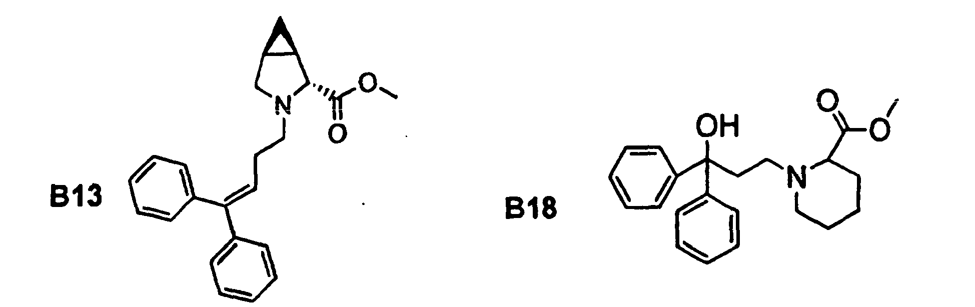

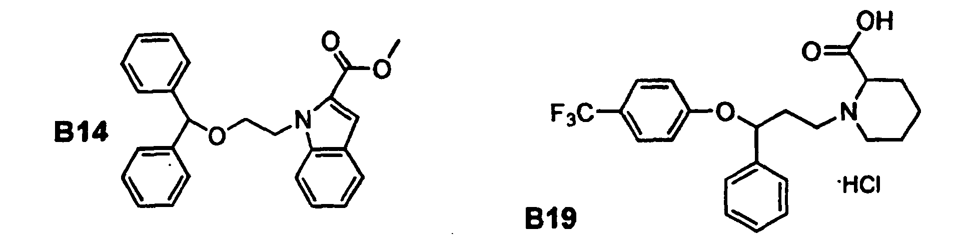

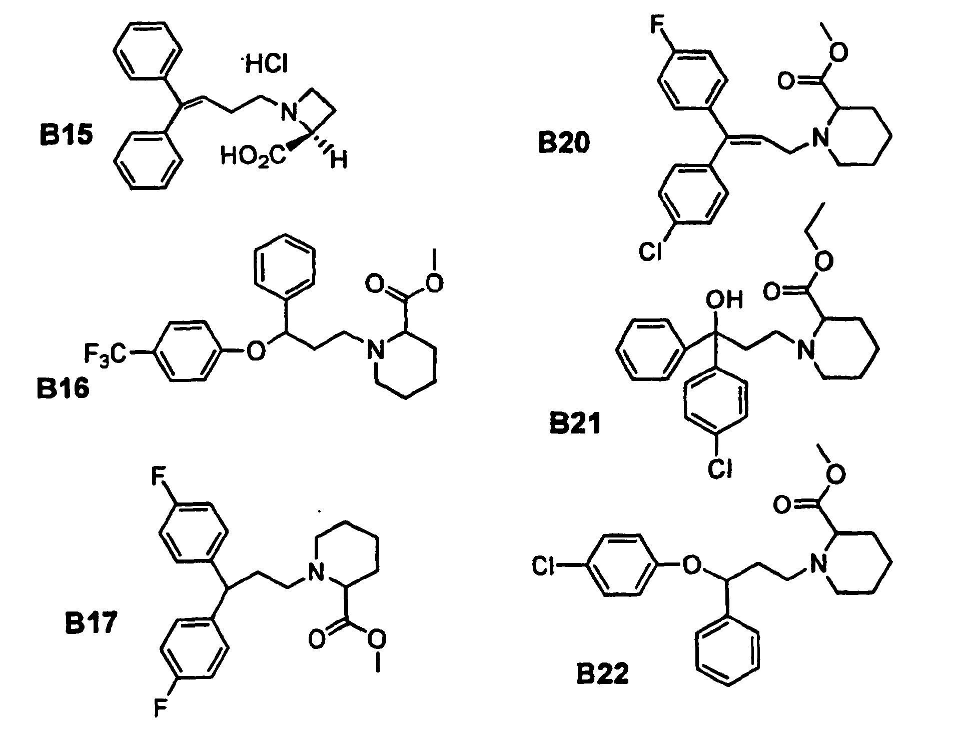

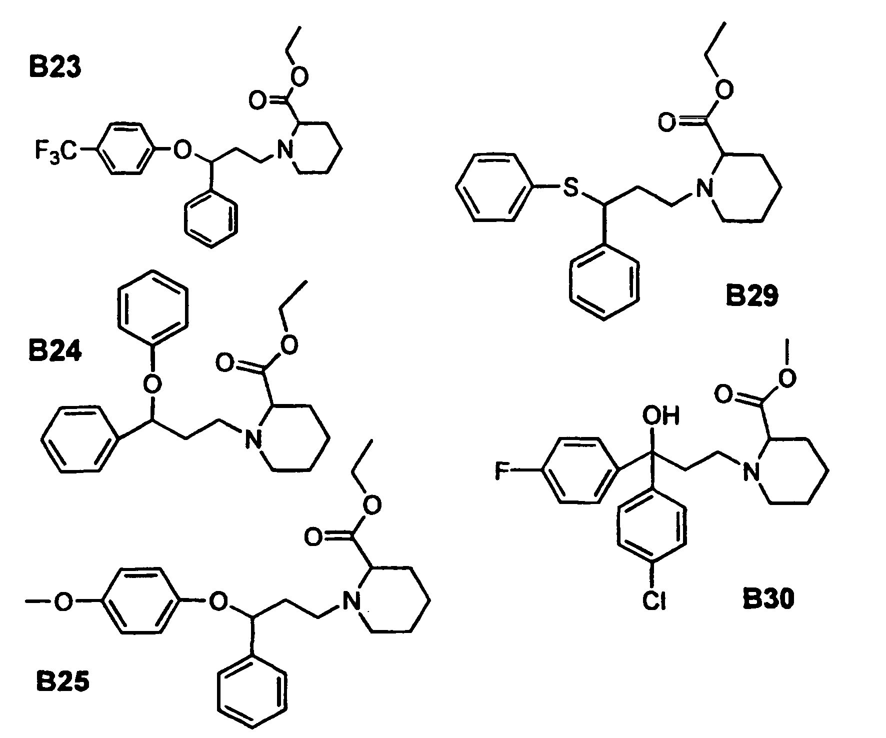

Compound A12 is a bis-alkylation byproduct of the synthesis of A9 using reaction I.

The compounds of the invention that incorporate =N-O- can be prepared, for example, by alkylating an amine (such as sarcosine or glycine) with O-(2-halogenethyl)alkanone oximes, which can be prepared by condensing alkanones with hydroxylamine, followed by O-alkylation (such as with 1,2-dihaloethane).

It will be recognized that numerous salt forms of the compounds herein described are available and suitable for use in the invention or during the synthesis of compounds of the invention. The invention contemplates that in certain instances where stereoisomers are available that one such isomer can be more active than another; in such a case, it will be desirable to isolate the particular isomeric form. The invention, of course, encompasses both the particular stereoisomers and racemic mixtures. As described herein, chemical approaches, starting with for example commercially available, optically pure starting materials (or made using enantioselective reactions), can also used to synthesize optically pure versions of the compounds of the invention. It will be recognized that such optically pure compounds are within the invention. Enantiomeric excess ("ee") can be enhanced by purification techniques such as crystallization or chromatography on chiral supports. Enantiomeric excess can be quantitated by a number of analytic techniques including NMR, optical rotation measurements and appropriate chromatography.

Additional, related compounds are described in two U.S. Patent Applications were filed concurrently with a parent hereof as U.S. Serial No. 08/655,912 (Docket No. 317743-106, Ognyanov et al.), U.S. Serial No. 08/655,847 (Docket No. 317743-107, Ognyanov et al.), U.S. Serial No. 08/807,682 (PHARMACEUTICAL FOR TREATMENT OF NEUROPSYCHIATRIC AND NEUROLOGICAL DISORDERS, Docket No. 317743-106A, Ognyanov et al.) and U.S. Serial No. 08/807,681 (PHARMACEUTICAL FOR TREATING OF NEUROLOGICAL AND NEUROPSYCHIATRIC DISORDERS, Docket No. 317743-107A, Ognyanov et al.

In a preferred embodiment, at least one of the following applies:
- if R¹⁵ is: hydrogen and R¹ is propylene, then at least one [preferably at least two, more preferably at least three] of the following applies (1) both R^{x} and R^{y} are not *p*-fluorophenyl, (2) one of R^{x} and R^{y} includes a heteroaryl, (3) RY is arylalkyl, heteroarylalkyl, aryloxy, heteroaryloxy, arylmethoxy, heteroarylmethoxy, arylthio, heteroarylthio, arylmethylthio, heteroarylmethylthio, Ar-N(R⁶)- or Ar-CH₂-N(R^{6*})-, (4) R² is R^{xa} R^{xb}-, (5) R^{2*} is not hydrogen, (6) R³ is not hydrogen, (7) n is one, or (8) R³ and R⁴ form ring Q;
- if R¹⁵ is: hydrogen and R¹ is ethylene or X-R¹ is prop-1-enylene, then at least one [preferably at least two, more preferably at least three] of the following applies (1) an aryl of at least one of R^{x} and R^{y} is substituted with a radical different from hydrogen, (2) one of R^{x} and R^{y} comprises a heteroaryl, (3) RY is arylalkyl, heteroarylalkyl, aryloxy, heteroaryloxy, arylmethoxy, heteroarylmethoxy, arylthio, heteroarylthio, arylmethylthio, heteroarylmethylthio, Ar-N(R⁶)- or Ar-CH₂-N(R^{6*})-, (4) R² is R^{xa} R^{xb}-, (5) R^{2*} is not hydrogen, (6) R³ is not hydrogen, (7) n is one, or (8) R³ and R⁴ form ring Q;
- if R⁵ is: C(O)NH₂, then at least one [preferably at least two, more preferably at least three] of the following applies (1) an aryl of at least one of R^{x} and R^{y} is substituted with a radical different from hydrogen, (2) one of R^{x} and RY comprises a heteroaryl, (3) RY is arylalkyl, heteroarylalkyl, aryloxy, heteroaryloxy, arylmethoxy, heteroarylmethoxy, arylthio, heteroarylthio, arylmethylthio, heteroarylmethylthio, Ar-N(R⁶)- or Ar-CH₂-N(R^{6*})-, (4) R² is R^{xa} R^{xb}-, (5) R^{2*} is not hydrogen, (6) R³ is not hydrogen, (7) n is one, (8) R¹ is not ethylene, or (9) R³ and R⁴ form ring Q;
- if R¹³: is hydrogen and R¹⁴ is (3,4-dihydro-2H-1-benzopyran-4-yl)methylene, then at least one [preferably at least two, more preferably at least three] of the following applies (1) an aryl of at least one of R^{x} and RY is substituted with a radical different from hydrogen, (2) one of R^{x} and RY comprises a heteroaryl, (3) RY is arylalkyl, heteroarylalkyl, aryloxy, heteroaryloxy, arylmethoxy, heteroarylmethoxy, arylthio, heteroarylthio, arylmethylthio, heteroarylmethylthio, Ar-N(R⁶)- or Ar-CH₂-N(R^{6*})-, (4) R² is R^{xa} R^{xb}-, (5) R^{2*} is not hydrogen, (6) R³ is not ethyl, (7) n is one, or (8) R³ and R⁴ form ring Q; and
- if R² is: phenyl, *p*-methylphenyl or *p*-methoxyphenyl, then at least one [preferably at least two, more preferably at least three] of the following applies (1) the aryls of R^{x} and RY are not substituted with *p*-methylphenyl or *p*-methoxyphenyl, (2) an aryl of at least one of R^{x} and R^{y} is substituted with a radical different from hydrogen, (3) one of R^{x} and RY comprises a heteroaryl, (4) RY is arylalkyl, heteroarylalkyl, aryloxy, heteroaryloxy, arylmethoxy, heteroarylmethoxy, arylthio, heteroarylthio, arylmethylthio, heteroarylmethylthio, Ar-N(R⁶)- or Ar-CH₂-N(R^{6*})-, (5) R¹ is not aminoethylene, OR⁸ or SR^{8*}, (6) n is one, or (7) R³ and R⁴ form ring Q.

In one preferred embodiment of the the methods, particularly treating or preventing epilepsy or spasticity or enhancing memory, the compound conforms with paragraph (f), above.

The glycine transporter genes and their respective gene products are responsible for the reuptake of glycine from the synaptic cleft into presynaptic nerve endings or glial cells, thus terminating the action of glycine. Neurological disorders or conditions associated with improperly controlled glycine receptor activity, or which could be treated with therapeutic agents that modulate glycine receptor activity, include spasticity (Becker, FASEB Journal, 4, 2767-2774 (1990)) and pain realization (Yaksh, Pain, 37, 111-123 (1989)). Additionally, glycine interacts at N-methyl-D-aspartate (NMDA) receptors, which have been implicated in learning and memory disorders and certain clinical conditions such as epilepsy, Alzheimer's and other cognition-related diseases, and schizophrenia. See Rison and Stanton, Neurosci. Biobehav. Rev., 19, 533-552 (1995); Danysz et al., Behavioral Pharmacol., 6, 455-474 (1995).

Compounds that inhibit GlyT-1 mediated glycine transport will increase glycine concentrations at NMDA receptors, which receptors are located in the forebrain, among other locations. This concentration increase elevates the activity of NMDA receptors, thereby alleviating schizophrenia and enhancing cognitive function. Alternatively, compounds that interact directly with the glycine receptor component of the NMDA receptor can have the same or similar effects as increasing or decreasing the availability of extracellular glycine caused by inhibiting or enhancing GlyT-1 activity, respectively. See, for example, Pitkänen et al., Eur. J. Pharmacol.. 253, 125-129 (1994); Thiels et al., Neuroscience. 46, 501-509 (1992); and Kretschmer and Schmidt, J. Neurosci., 16, 1561-1569 (1996). Compounds that inhibit GlyT-2 mediated glycine transport will increase glycine concentrations at receptors located primarily in the brain stem and spinal cord, where glycine acts as an inhibitor of synaptic transmission. These compounds are effective against epilepsy, pain and spasticity, myospasm and other such conditions. See, for example, Becker, FASEB J., 4 2767-2774 (1990) and Yaksh, Pain, 37 111-123 (1989).

The compounds of the invention are, for instance, administered orally, sublingually, rectally. nasally, vaginally, topically (including the use of a patch or other transdermal delivery device), by pubnonary route by use of an aerosol, or parenterally, including, for example, intramuscularly, subcutaneously, intraperitoneally, intraarterially, intravenously or intrathecally. Administration can be by means of a pump for periodic or continuous delivery. The compounds of the invention are administered alone, or are combined with a pharmaceutically-acceptable carrier or excipient according to standard pharmaceutical practice. For the oral mode of administration, the compounds of the invention are used in the form of tablets, capsules, lozenges, chewing gum, troches, powders, syrups, elixirs, aqueous solutions and suspensions, and the like. In the case of tablets, carriers that are used include lactose, sodium citrate and salts of phosphoric acid. Various disintegrants such as starch, and lubricating agents such as magnesium stearate and talc, are commonly used in tablets. For oral administration in capsule form, useful diluents are lactose and high molecular weight polyethylene glycols. If desired, certain sweetening and/or flavoring agents are added. For parenteral administration, sterile solutions of the compounds of the invention are usually prepared, and the pHs of the solutions are suitably adjusted and buffered. For intravenous use, the total concentration of solutes should be controlled to render the preparation isotonic. For ocular administration, ointments or droppable liquids may be delivered by ocular delivery systems known to the art such as applicators or eye droppers. Such compositions can include mucomimetics such as hyaluronic acid, chondroitin sulfate, hydroxypropyl methylcellulose or polyvinyl alcohol, preservatives such as sorbic acid, EDTA or benzylchromium chloride, and the usual quantities of diluents and/or carriers. For pulmonary administration, diluents and/or carriers will be selected to be appropriate to allow the formation of an aerosol.

Suppository forms of the compounds of the invention are useful for vaginal, urethral and rectal administrations. Such suppositories will generally be constructed of a mixture of substances that is solid at room temperature but melts at body temperature. The substances commonly used to create such vehicles include theobroma oil, glycerinated gelatin, hydrogenated vegetable oils, mixtures of polyethylene glycols of various molecular weight and fatty acid esters of polyethylene glycol. *See,* Remington's Pharmaceutical Sciences, 16th Ed., Mack Publishing, Easton, PA, 1980, pp. 1530-1533 for further discussion of suppository dosage forms. Analogous gels or cremes can be used for vaginal, urethral and rectal administrations.

Numerous administration vehicles will be apparent to those of ordinary skill in the art, including without limitation slow release formulations, liposomal formulations and polymeric matrices.

Examples of pharmaceutically acceptable acid addition salts for use in the present invention include those derived from mineral acids, such as hydrochloric, hydrobromic, phosphoric, metaphosphoric, nitric and sulfuric acids, and organic acids, such as tartaric, acetic, citric, malic, lactic, fumaric, benzoic, glycolic, gluconic, succinic, p-toluenesulphonic and arylsulphonic acids, for example. Examples of pharmaceutically acceptable base addition salts for use in the present invention include those derived from non-toxic metals such as sodium or potassium, ammonium salts and organoamino salts such as triethylamine salts. Numerous appropriate such salts will be known to those of ordinary skill.

The physician or other health care profesional can select the appropriate dose and treatment regimen based on the subject's weight, age, and physical condition. Dosages will generally be selected to maintain a serum level of compounds of the invention between about 0.01 µg/cc and about 1000 µg/cc, preferably between about 0.1 µg/cc and about 100 µg/cc. For parenteral administration, an alternative measure of preferred amount is from about 0.001 mg/kg to about 10 mg/kg (alternatively, from about 0.01 mg/kg to about 10 mg/kg), more preferably from about 0.01 mg/kg to about 1 mg/kg (from about 0.1 mg/kg to about 1 mg/kg), will be administered. For oral administrations, an alternative measure of preferred administration amount is from about 0.001 mg/kg to about 10 mg/kg (from about 0.1 mg/kg to about 10 mg/kg), more preferably from about 0.01 mg/kg to about I mg/kg (from about 0.1 mg/kg to about 1 mg/kg). For administrations in suppository form, an alternative measure of preferred administration amount is from about 0.1 mg/kg to about 10 mg/kg, more preferably from about 0.1 mg/kg to about 1 mg/kg.

For use in assaying for activity in inhibiting glycine transport eukaryokic cells, preferably QT-6 cells derived from quail fibroblasts, have been transfected to express one of the three known variants of human GlyT-1, namely GlyT-1a, GlyT-1b or GlyT-1c, or human GlyT-2. The sequences of these GlyT-1 transporters are described in Kim et al., *Molec. Pharm.* 45: 608-617, 1994, excepting that the sequence encoding the extreme N-terminal of GlyT-1a was merely inferred from the corresponding rat-derived sequence. This N-terminal protein-encoding sequence has now been confirmed to correspond to that inferred by Kim et al. The sequence of the human GlyT-2 is described by Albert et al., U.S. Application No. 08/700,013, filed August 20, 1996, which is incorporated herein by reference in its entirety. Suitable expression vectors include pRc/CMV (Invitrogen), Zap Express Vector (Stratagene Cloning Systems, LaJolla, CA; hereinafter "Stratagene"), pBk/CMV or pBk-RSV vectors (Stratagene), Bluescript II SK +/-Phagemid Vectors (Stratagene), LacSwitch (Stratagene), pMAM and pMAM neo (Clontech), among others. A suitable expression vector is capable of fostering expression of the included GlyT DNA in a suitable host cell, preferably a non-mammalian host cell, which can be eukaryotic, fungal, or prokaryotic. Such preferred host cells include amphibian, avian, fungal, insect, and reptilian cells.

As discussed above, the compounds of the invention have a number of pharmacological actions. The relative effectiveness of the compounds can be assessed in a number of ways, including the following:
◆ comparing the activity mediated through GlyT-1 and GlyT-2 transporters. This testing identifies compounds (a) that are more active against GlyT-1 transporters and thus more useful in treating or preventing schizophrenia, increasing cognition and enhancing memory or (b) that are more active against GlyT-2 transporters and thus more useful in treating or preventing epilepsy, pain, spasticity or myospasm.
◆ testing for NMDA receptor binding. This test establishes whether there is sufficient binding at this site, whether antagonist or agonist activity, to warrant further examination of the pharmacological effect of such binding.
◆ testing the activity of the compounds in enhancing or diminishing calcium fluxes in primary neuronal tissue culture. A test compound that increases calcium flux either (a) has little or no antagonist activity at the NMDA receptor and should not affect the potentiation of glycine activity through GlyT-1 transporter inhibition or (b), if marked increases are observed over GlyT-1 inhibitors used for comparison and that have little direct interaction with NMDA receptors, then the compound is a receptor agonist. In either of the above-described cases, the test confirms activity in treating or preventing schizophrenia, increasing cognition, or enhancing memory. In contrast, a test compound that decreases calcium flux has a net effect wherein receptor antagonist activity predominates over any activity the compound has in increasing glycine activity through inhibiting glycine transport. In this case, the test confirms activity in limiting or preventing the cell damage and cell death arising after stroke or other ischemia-inducing conditions, or in limiting or preventing the cell damage associated with neurodegenerative diseases.

All animal methods of treatment or prevention described herein are preferably applied to mammals, most preferably humans.

The following examples further illustrate the present invention, but of course, should not be construed as in any way limiting its scope.

### Example 1 - Synthesis of N-1[(4,4-Diphenyl)but-3-enyl]glycine ethyl ester (Compound A26)

A mixture of 5.95 g (20.7 mmol) 4-bromo-1,1-diphenyl-1-butene (prepared as described in F.A. Ali et al., J. Med. Chem., 28: 653-660, 1985), 4.71 g (33.7 mmol) glycine ethyl ester hydrochloride (Aldrich, Milwaukee, WI), 11.62 g (84 mmol) potassium carbonate and 1.06 g (6.38 mmol) potassium iodide in 50 ml acetonitrile was refluxed with stirring under argon for seven hours. The reaction mixture was filtered, the solvent evaporated and the residue chromatographed on silica gel column with 20% ethyl acetate in hexanes to give 3.70 g (yield 58%) of N-[(4,4-diphenyl)but-3-enyl]glycine ethyl ester (compound A26) as an oil. NMR spectra of the product showed: ¹H NMR (CDCl₃, 300 MHz) 7.60-7.00 (m, 10 H), 6.09 (t, 1 H), 4.16 (q, 2 H), 3.35 (s, 2 H), 2.71 (t, 2 H), 2.32 (dt, 2 H), 1.25 (t, 3 H), ¹³C NMR (CDCl₃, 75 MHz) 172.29, 143.25, 142.37, 139.82, 129.72, 128.13, 128.04, 127.97, 127.13, 126.92, 126.88, 126.68, 60.56, 50.73, 49.32, 30.33, 14.14.

### Example 2 - Additional Syntheses According to Reaction 1

Additional compounds were synthesized using Reaction 1, as follows:

| Compound | Reagent | Amino acid or precusor | Solvent | Yield |
|---|---|---|---|---|
| A1 | 1 | B | X | 27% |
| A2 | 1 | C | X | 35% |
| A7 | 7 | E | X | 9% |
| A9 | 4 | E | X | 47% |
| A11 | 1 | A | X | 70% |
| A12 | 4 | E | X | 7% |
| A14 | 2 | D | X | 15% |
| A18 | 6 | E | X | 50% |
| A23 | 5 | E | X | 26% |
| A24 | 3 | D | Y | 20% |
| A43 | 8 | F | X | 12% |
| A52 | 9 | F | X | 28% |
| A57 | 10 | F | X | 31% |
| A67 | 11 | F | X | 10% |
| A71 | 12 | E | X | 28% |
| A75 | 13 | F | X | 73% |
| A77 | 14 | F | X | 36% |
| A85 | 15 | F | X | 86% |
| A87 | 16 | F | X | 59% |
| A90 | 17 | E | X | 16% |
| A95 | 17 | F | X | 65% |
| A96 | 17 | E | X | 50% |
| A104 | 15 | E | X | 62% |
| A106 | 18 | F | X | 65% |
| A121 | 19 | E | X | 3% |
| A122 | 19 | E | X | 40% |
| A123 | 19 | F | X | 72% |
| A130 | 20 | E | X | 6% |
| A132 | 21 | F | X | 90% |
| A134 | 21 | E | X | 67% |
| A170 | 6 | F | X | 72% |
| A48 | 22 | F | x | 87% |
| A50 | 23 | F | X | 81% |
| A53 | 24 | F | X | 76% |
| A59 | 25 | F | X | 77% |
| A61 | 26 | F | X | 91% |
| A63 | 27 | F | X | 91% |
| A70 | 28 | F | X | 89% |
| A73 | 29 | F | X | 86% |
| A74 | 30 | F | X | 76% |
| A78 | 31 | F | X | 49% |
| A80 | 32 | F | X | 66% |
| A82 | 33 | F | X | 38% |
| A83 | 33 | E | X | 25% |
| A88 | 34 | F | X | 55% |
| A89 | 35 | F | X | 75% |
| A99 | 36 | F | X | 56% |
| A100 | 37 | F | X | 67% |
| A111 | 38 | F | X | 34% |
| A117 | 39 | F | X | 58% |
| A118 | 40 | F | X | 89% |
| A120 | 41 | F | X | 62% |
| A125 | 42 | F | X | 46% |
| A126 | 43 | E | X | 57% |
| A127 | 44 | E | X | 5% |
| A128 | 44 | E | X | 53% |
| A129 | 44 | F | X | 66% |
| A138 | 45 | F | X | 48% |
| A140 | 46 | F | X | 69% |
| A141 | 47 | F | X | 51% |
| A142 | 48 | F | X | 67% |
| A143 | 49 | F | X | 61% |
| A145 | 50 | F | X | 98% |
| A155 | 51 | F | X | 70% |
| A156 | 52 | F | X | 65% |
| A158 | 53 | F | X | 59% |
| A159 | 54 | F | X | 85% |
| A160 | 55 | F | X | 87% |
| A171 | 56 | F | X | 88% |
| A173 | 57 | F | X | 81% |
| A177 | 58 | F | X | 84% |
| A178 | 58 | F | X | 60% |
| A179 | 59 | F | X | 68% |
| A180 | 24 | G | X | 85% |

### Reagent:

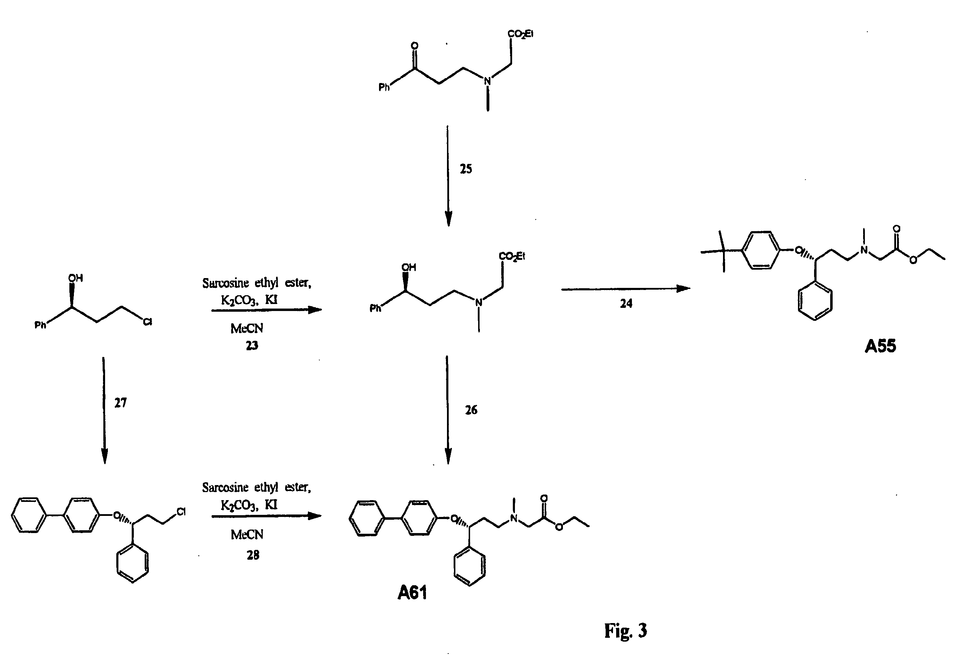

**1)** 4-bromo-1,1-diphenyl-1-butene, (prepared as described in F.A. Ali et al., J. Med. Chem., 28: 653-660, 1985); **2)** 1,1'-(4-chlorobutylidene)bis(4-fluorobenzene), (Acros Organics, Pittsburgh, PA); **3)** benzhydryl 2-bromoethyl ether, (prepared as described in M.R. Pavia et al., J. Med. Chem. 35: 4238-4248, 1992); **4)** 9-fluorenylethanol *p-*toluenesulfate, [prepared by LiAlH₄ reduction of 9-fluoreneacetic acid methyl ester (Aldrich) to 2-(9-fluorenyl)ethanol, followed by tosylation]; **5)** 4-bromo-2,2-diphenyl butyronitrile (Aldrich); **6)** 3-bis(4-fluorophenyl)propanol *p*-toluenesulfate [prepared by alkylation of diethyl malonate (Aldrich) with chlorobis(4-fluorophenyl)methane (Aldrich) followed by hydrolysis and decarboxylation, LiAlH₄ reduction of the monocarboxylic acid, and tosylation of the formed alcohol]; **7)** 10-(3-bromo-2-hydroxypropyl)phenothiazine [prepared essentially as described in British Patent 800,635]; **8)** 3-tris(4-fluorophenyl)propanol *p*-toluenesulfonate [prepared by alkylation of diethyl malonate (Aldrich) with 4,4',4"-trifluorotrityl bromide (TCI America, Portland, OR) followed by hydrolysis and decarboxylation, LiAlH₄ reduction of the monocarboxylic acid, and tosylation of the formed alcohol]; **9)** 3-cyclohexyl-3-phenylpropanol p-toluenesulfonate [prepared by Homer-Emmons reaction of the sodium ylide of triethyl phosphonoacetate (Aldrich) with cyclohexyl phenyl ketone (Aldrich) followed by catalytic hydrogenation of the intermediate α,β-unsaturated ester, LiAlH₄ reduction and tosylation of the formed alcohol]: **10)** 3-tris(4-methoxyphenyl)propanol *p*-toluenesulfonate [prepared by alkylation of diethyl malonate (Aldrich) with 4,4',4"-trimethoxytrityl chloride (Aldrich) followed by hydrolysis and decarboxylation, LiAlH₄, reduction of the monocarboxylic acid, and tosylation of the formed alcohol], **11)** 3-bis(3-fluorophenyl)propanol *p*-toluenesulfonate [prepared by Horner-Emmons reaction of the sodium ylide of triethyl phosphonoacetate (Aldrich) with 3,3'-difluorobenzophenone (Aldrich) followed by catalytic hydrogenation of the intermediate α,β-unsaturated ester, LiAIH₄ reduction and tosylation of the formed alcohol]; **12)** 3,5-diphenylpentanol *p*-toluenesulfonate [prepared by Horner-Emmons reaction of the sodium ylide of triethyl phosphonoacetate (Aldrich) with 3-phenylpropiophenone (Pfaltz & Bauer Chemicals Catalog, Waterbury, CT) followed by catalytic hydrogenation of the intermediate α,β-unsaturated ester, LiAlH₄ reduction and tosylation of the formed alcohol]; **13)** 3-bis(4-phenoxyphenyl)propanol-*p*-toluenesulfonate prepared by Horner-Emmons reaction of the sodium ylide of triethyl phosphonoacetate (Aldrich) with 4,4'-diphenoxybenzophenone (Lancaster, Windham, NH) followed by catalytic hydrogenation of the intermediate α,β-unsaturated ester, LiAlH₄ reduction and tosylation of the formed alcohol]; **14)** 3-bis(4-biphenyl)propanol-*p*-toluenesulfonate [prepared by Horner-Emmons reaction of the sodium ylide of triethyl phosphonoacetate (Aldrich) with 4-benzoylbiphenyl (Aldrich) followed by catalytic hydrogenation of the intermediate α,β-unsaturated ester, LiAlH₄ reduction and tosylation of the formed alcohol]; **15)** 3-(4-*tert*-butylphenyl-3-phenypropanol *p*-toluenesulfonant (prepared by Horner-Emmons reaction of the sodium ylide of triethyl phosphonoacetate with 4-*tert*-butylbenzophenone (Aldrich) followed by catalytic hydrogenation of the intermediate α,β-unsaturated ester, LiA1H₄ reduction and tosylation of the formed alcohol]; **16)** 3,3,3-tris(4-chlorophenyl)propanol *p*-toluenesulfonate [prepared by LiA1H₄ reduction of 3,3,3-tris(4-chloropropionic acid) (Aldrich) followed by tosylation of the formed alcohol]; **17)** 3-(2-naphthyl)-3-phenyl)propanol *p*-toluenesulfonate [prepared by Horner-Emmons reaction of the sodium ylide of triethyl phosphonoacetate with 2-benzoylnaphthalene (Aldrich) followed by catalytic hydrogenation of the intermediate α,β-unsaturated ester, LiAIH₄ reduction and tosylation of the formed alcohol]; **18)** 3,3,3-triphenylpropanol *p*-toluenesulfonate [prepared by LiAlH₄ reduction of 3,3,3-triphenylpropionic acid (Aldrich) followed by tosylation of the formed alcohol]; **19)** 3-(4-phenylphenyl)-3-phenylpropanol *p*-toluenesulfonate [prepared by Horner-Emmons reaction of the sodium ylide of triethyl phosphonoacetate with 4-benzoylbiphenyl (Aldrich) followed by catalytic hydrogenation of the intermediate α,β-unsaturated ester, LiAlH₄ reduction and tosylation of the formed alcohol]; **20)** 1,2-diphenylbutan-1,4-diol *p*-toluenesulfonate [prepared by C-alkylation of deoxybenzoin (Aldrich) with ethyl bromoacetate (Aldrich) followed by LiAlH₄ reduction of the intermediate β-ketoester and tosylation of the formed diol]; **21)** 3-phenyl-3-(4-trifluoromethylphenyl)propanol *p*-toluenesulfonate prepared by Homer-Emmons reaction of the sodium ylide of triethyl phosphonoacetate with 4-(trifluoromethyl)benzophenone (Aldrich) followed by catalytic hydrogenation of the intermediate α, β-unsaturated ester, LiA1H₄ reduction and tosylation of the formed alcohol]; **22)** 3-chloro-1-(4-*tert* -butylphenoxy)-1-(4-fluorophenyl)propane [prepared analogously to the method of U.S. Pat. 5,281,624 by reduction of 3-chloro-4'-fluoropropiophenone (Aldrich) with 1.0 M boranetetrahydrofuran complex ("BTC", Aldrich) followed by Mitzunobu reaction (diethyl azodicarboxylate ("DEAD"), Ph₃P, see Example 8C, Step 1) of the resuhing alcohol with 4-*tert*-butylphenol (Aldrich)]; **23)** 3-chloro-1-(2-me2hyl-5-pyridyloxy)-1-phenylpropane [prepared by reduction of 3-chloropropiophenone (Aldrich) with 1.0 M BTC followed by Mitzunobu reaction (DEAD, Ph₃P) of the resulting alcohol with 5-hydroxy-2-methylpyridine (Aldrich)]; **24)** 3-chloro-1-(4-phenylphenoxy)-1-(4-fluorophenyl)propane [prepared by reduction of 3-chloro-4'-fluoropropiophenone with 1.0 M BTC followed by Mitzunobu reaction (DEAD, Ph₃P) of the resulting alcohol with 4-phenylphenol (Aldrich)]; **25)** 3-chloro-1-(4-*tert*-octylphmo)-**1-phenylpropane** [prepared by reduction of 3-chloropropiophenone with 1.0 M BTC followed by Mitzunobu reaction (DEAD, Ph₃P) of the resulting alcohol with 4-*tert*-butylphol]; **26)** (*R*)-(+)-3-chloro-1-(4-phenylphenoxy)-1-phenylpropane [prepared by Mitzunobu reaction (DEAD, Ph₃P) of (R)-(+)-3-chloro-1-phenyl-1-propanol (Aldrich) with 4-phenylphenol (Aldrich)(see, e.g., U.S. Pat. 5,068,432) (Reaction illustrated in Fig. 3, Reaction 27)]; Compound A61 was prepared with [α]_{D}²⁵+54.9° (c 5.28, CHCl₃); **27)** (*S*)-(-)-3-chloro-1-(4-phenylphenoxy)-1-phenylpropane [prepared by Mitzunobu reaction (DEAD, Ph₃P) of (*S*)-(-)-3-chloro-1-phenyl-1-propanol (Aldrich) with 4-phenylphenol (see U.S. Patent No. 5,068,432); Compound A63 was prepared with [α]_{D}²⁵-54.6 (c 7.13, CHCl₃); **28)** 3-chloro-1-(4-tert-butylphenoxy)-1-phenylpropane [prepared by reduction of 3-chloropropiophenone with 1.0 M BTC followed by Mitzunobu reaction (DEAD, Ph₃P) of the resulting alcohol with 4-*tert*-butylphenol); **29)** 3-chtoro-1-{4-[4-{trifluoromethyl)phenoxy]phenoxy}-1-phenylpropane [prepared by reduction of 3-chloropropiophenone with 1.0 M BTC followed by Mitzunobu reaction (DEAD, Ph₃P) of the resulting alcohol with 4-[4-trifluoromethyl)phenoxy]phenol (Aldrich)]; **30)** 3-chloro-1-[4-(phenoxy)phenoxy]-1-phenylpropane [prepared by reduction of 3-chloropropiophenone with 1.0 M BTC followed by Mitzunobu reaction (DEAD, Ph₃P) of the resulting alcohol with 4-phenoxyphenol (Aldrich)]; **31)** 3-chloro-1-[4-(4-bromophenyl)phenoxy]-1-(4-fluorophenyl)propane [prepared by reduction of 3-chloropropiophenone with 1.0 M BTC followed by Mitzunobu reaction (DEAD, Ph₃P) of the resulting alcohol with 4-(4-bromophenyl)phenol (Aldrich)]; **32)** 3-chloro-1-[4-(4-cyanophenyl)phenoxy]-1-phenylpropane [prepared by reduction of 3-chloropropiophenone with 1.0 M BTC followed by Mitzunobu reaction (DEAD, Ph₃P) of the resulting alcohol with 4'-hydroxy-4-biphenylcarbonitrile (Aldrich)]; **33)** 3-chloro-1-(3-trifluoromethylphenoxy)-1-phenylpropane [prepared by reduction of 3-chloropropiophenone with 1.0 M BTC followed by Mitzunobu reaction (DEAD, Ph₃P) of the resulting alcohol with 3-trifluoromethylphenol (Aldrich)]; **34)** 3-chloro-1-(2-naphthyloxy)-1-phenylpropane [prepared by reduction of 3-chloropropiophenone with 1.0 M BTC followed by Mitzunobu reaction (DEAD, Ph₃P) of the resulting alcohol with 2-naphthol (Aldrich)]; **35)** 3-chloro-1-1-naphthyloxy)-1-phenylpropane [prepared by reduction of 3-chloropropiophenone with 1.0 M BTC followed by Mitzunobu reaction (DEAD, Ph₃P) of the resulting alcohol with 1-naphthol (Aldrich)]; **36)** 3-chloro-1-(4-methylphenoxy)-1-phenylpropane [prepared by reduction of 3-chloropropiophenone with 1.0 M BTC followed by Mitzunobu reaction (DEAD, Ph₃P) of the resulting alcohol with *p*-cresol (Aldrich)]; **37)** 3-chloro-1-(4-phenylphenoxy)-1-phenylpropane [prepared by reduction of 3-chloropropiophenone with 1.0 M BTC followed by Mitzunobu reaction (DEAD, Ph₃P) of the resulting alcohol with 4-phenylphenol]; **38)** 3-chloro-1-(4-amidosulfonylphenoxy)-1-phenylpropane [prepared by reduction of 3-chloropnopiophenane with 1.0 M BTC followed by Mitzunobu reaction (DEAD, Ph₃P) of the resulting alcohol with 4-hydroxybenzenesulfonamide, (TCI America, Portland, OR)]; **39)** 3-chloro-1-(4-nitrophenoxy)-phenylpropane [prepared by reduction of 3-chloropropiophenone with 1.0 M BTC followed by Mitzunobu reaction (DEAD, Ph₃P) of the resulting alcohol with 4-nitrophenol (Aldrich)]; **40)** 3-chloro-1-(4-nitro-3-trifluoromethylphenoxy)-1-phenylpropane [prepared by reduction of 3-chloropropiophenone with 1.0 M BTC followed by Mitzunobu reaction (DEAD, Ph₃P) of the resulting alcohol with 4-nitro-3-trifluoromethylphenol (Aldrich)]; **41)** 3-chloro-1-(4-cyanophenoxy)-1-phenylpropane [prepared by reduction of 3-chloropropiophenone with 1.0 M BTC followed by Mitzunobu reaction (DEAD, Ph₃P) of the resulting alcohol with 4-cyanophenol (Aldrich)]; **42)** 3-chloro-1-phenoxy-1-phenylpropane [prepared by reduction of 3-chloropropiophenone with 1.0 M BTC followed by Mitzunobu reaction (DEAD, Ph₃P) of the resulting alcohol with phenol (Aldrich)]; **43)** 3-chloro-1-(4-trifluoromethylphenoxy)-1-phenylpropane [prepared by reduction of 3-chloropropiophenone with 1.0 M BTC followed by Mitzunobu reaction (DEAD, Ph₃P) of the resulting alcohol with 4-trifluoromethylphenol]; **44)** 3-chloro-1-[(4 trifluoromethoxy)phenoxy]-1-phenylpropane [prepared by reducing 3-chloropropiophenone with 1.0 M BTC, and Mitzunobu reaction (DEAD, Ph₃P) of resulting alcohol with 4-(trifluoromethoxy)phenol (Aldrich)]; **45)** 3-chloro-1-(4-trifluoromethylphenoxy)-1-(2,4-dimethoxy)phenylpropane [prepared by reduction of 3-chloro-2',4'-dimethoxypropiophenone (Maybridge Chemical Co. Ltd., Cornwall, UK) with 1.0 M BTC followed by Mitzunobu reaction (DEAD, Ph₃P) of the resulting alcohol with 4-trifluoromethylphenol]; **46)** 3-chloro-1-(3,4-methylenedioxyphenoxy)-1-(4-chlorophenyl)propane [prepared by reduction of 3,4'-dichloropropiophenone (Aldrich) with 1.0 M BTC followed by Mitzunobu reaction (DEAD, Ph₃P) of the resulting alcohol with sesamol (Aldrich)]; **47)** 3-chloro-1-phenoxy-1-(4-bromophenyl)propane [prepared by reduction of 4-bromo-β-chloropropiophenone (Lancaster) with 1.0 M BTC followed by Mitzunobu reaction (DEAD, Ph₃P) of the resulting alcohol with phenol]; **48)** 3-chloro-1-(4-tnihoromethylphenoxy)-1-(4-bromophenyl)propane [prepared by reduction of 4-brceno-β-chloropropiophenone, with 1.0 M BTC followed by Mitzunobu reaction (DEAD, Ph₃P) of the resulting alcohol with 4-trifluoromethylphenol]; **49)** 3-chloro-1-(4-methoxyphenoxy)-1-(4-ch1orophenyl)propane [prepared by reduction of 3,4'-dichloropropiophenone with 1.0 M BTC followed by Mitzunobu reaction (DEAD, Ph₃P) of the resulting alcohol with 4-methoxyphenol (Aldrich)]; **50)** 3-chloro-1-(4-qwophenoxy)-1-(4-chlorophenyl)propane [prepared by reducing 3,4'-dichloropropiophenone with 1.0 M BTC followed by Mitzunobu reaction (DEAD, Ph₃P) of the resulting alcohol with 4-cyanophenol); **51)** 3-chloro-1-(4-chlorophenoxy)-1-(4-bromophenyl)propane [prepared by reduction of 4-bromo-β-chloropropiophenone with 1.0 M BTC followed by Mitzunobu reaction (DEAD, Ph₃P) of the resulting alcohol with 4-chlorophenol (Aldrich)]; **52**) 3-chloro-1-phenoxy-1-(4-chlorophenyl)propane [prepared by reduction of 3,4'-dichloropropiophenone with 1.0 M BTC followed by Mitzunobu reaction (DEAD, Ph₃P) of the resulting alcohol with phenol]; 53) 3-chloro-1-(4-methoxyphenoxy)-1-(4-fluorophenyl)propane [prepared by reducing 3-chloro-4'-fluoropropiophenone with 1.0 M BTC, and Mitzunobu reaction (DEAD, Ph₃P) of the resulting alcohol with 4-methoxyphenol]; 54) 3-chloro-1-phenoxy-1-(4-fluorophenyl)propane [prepared by reduction of 3-chloro-4'-fluoropropiophenone with 1.0 M BTC followed by Mitzunobu reaction (DEAD, Ph₃P) of the resulting alcohol with phenol]; 55) 3-chloro-1-(4-trifluoromethylphenoxy)-1-(4-fluorophenyl)propane [prepared by reduction of 3-chloro-4'-fluoropropiophenone with 1.0 M BTC followed by Mitzunobu reaction (DEAD, Ph₃P) of the resulting alcohol with 4-trifluoromethylphenol]; 56) (*R*)-(+)3-chloro-1-(4-nitrophenoxy)-1-phenylpropane [prepared (see, e.g., U.S. Patent No. 5,068,432) by Mitzunobu reaction (DEAD, Ph₃P) of (*R*)-(+)-3-chloro-1-phenyl-1-propanol (Aldrich) with 4-nitrophenol]; Compound A171 was prepared with [α]D²⁵+19.7⁰ (c 5.18, CHCl₃); 57) (*S*)-(-)-3-chloro-1-(4-phenylphenoxy)-1-(4-flurophenyl)propane [prepared with [α]_{D}²⁵-46.3° (c 2.49, CHCl₃) analogously to U.S. pat. 5,068,432 by reduction of 3-chloro-4'-fluoropropiophenone with (+) diisopinocampheylboron chloride (Aldrich) followed by Mitzunobu reaction (DEAD, Ph₃P) of the resulting (*R*)-(+)-3-chloro-1-(4-fluorophenyl)-1-propanol {[α]_{D}²⁵ + 22.1° (c 8.07, CHCl₃)} with 4-phenylphenol, (Aldrich)]; Compound A173 was prepared with [α]_{D}²⁵ -25.8° (c 3.03, CHCl₃); 58) (*R*)-(+)-3-chloro-1-(4-phenylphenoxy)-1-(4-fluorophenyl)propane [prepared with [α]_{D}²⁵ +46.6° (c 2.73, CHCl₃) analogously to U.S. pat. 5,068,432 by reduction of 3-chloro-4'-fluoropropiophenone with (-) diisopinocampheylboron chloride (Aldrich) followed by Mitzunobu reaction (DEAD, Ph₃P) of the resulting (*S*)-(-)-3-chloro-1-(4-fluorophenyl)-1-propanol {[α]_{D}²⁵ -22.2° (c 2.37, CHCl₃)} with 4-phenylphenol, (Aldrich)]; Compound A177 was prepared with [α]_{D}²⁵ +26.8° (c 3.10, CHCl₃); Compound A178 was prepared with [α]_{D}²⁵ +20.0° (c 3.13, CHCl₃); 59) (*R*)-(+)-3-chloro-1-[4-(1-adamantyl)phenoxy]-1-(4-flurophenyl)propane [prepared with [α]_{D}²⁵ +24.3° (c 2.19, CHCl₃) analogously to U.S. pat. 5,068,432 by reduction of 3-chloro-4-fluoropropiophenone with (-) diisopinocampheylboron chloride (Aldrich) followed by Mitzunobu reaction (DEAD, Ph₃P) of the resulting (*S*)-(-)-3-chloro-1-(4-fluorophenyl)-1-propanol {[α]_{D}²⁵ -22.2° (c 2.37, CHCl₃)} with 4-(1-adamantyl)phenol, (Aldrich)]; Compound A179 was prepared with [α]_{D}²⁵ +17.8° (c 2.98, CHCl₃).

### Amino acid or amino acid precursor:

**A)** L-alanine methyl ester hydrochloride, (Fluka, Ronkonkoma, NY); **B)** D-alanine methyl ester hydrochloride (Aldrich); **C)** sarcosine methyl ester hydrochloride, (Lancaster, Windham, NH); **D)** glycine methyl ester hydrochloride (Aldrich); **E)** glycine ethyl ester hydrochloride (Aldrich); **F)** sarcosine ethyl ester hydrochloride (Aldrich); and **G)** methylaminoacctaldehyde dimethyl acetal (Aldrich).

### Solvent: X) acetonitrile; Y) methanol.

For the synthesis of A61, the reaction is illustrated in Figure 3 (Reaction 28).

### Example 3 - Synthesis of N-[(3.3-Diphenyl]propyl]glycine ethyl ester (Compound A22)

2.132 g (10.1 mmol) 3,3-diphenylpropylamine (Aldrich, Milwaukee, WI) was added to a mixture of 0.853 g (5.11 mmol) ethyl bromoacetate (Aldrich) and 2.7 g (19.57 mmol) potassium carbonate in 14 ml acetonitrile at rom temperature. The mixture was stirred under argon for 18 hours. The reaction mixture was filtered, the solvent evaporated and the residue chromatographed on a silica gel column with 40% ethyl acetate in hexanes to give 1.05 g (yield 69%) N-[(3,3-diphenyl)propyl]glycine ethyl ester (Compound A22) as an oil. NMR spectra of the product showed: ¹H NMR (CDCl₃, 300 MHz) 7.40 - 7.10 (m, 10 H), 4.14 (q, 2H), 4.03 (t, 1H), 3.33 (s, 2 H), 2.56 (t, 2 H), 2.24 (dt, 2 H), 1.22 (t, 3 H); ¹³C NMR (CDCl₃, 75 MHz) 172.44, 144.66,128.43,127.75,126.15, 60.63,50.93,48.80,47.92,35.85,14.17. 0.019 g of A28 was also isolated from the silica gel column.

### Example 4 - Additional Syntheses Using Reaction 2

Additional compounds were synthesized using Reaction 2, as follows:

| Compound | Starting amine | Reagent | Solvent | Yield |
|---|---|---|---|---|
| A5 | 1 | A | X | 27% |
| A6 | 7 | B | Y | 89% |
| A10 | 9 | B | Y | 77% |
| A13 | 8 | B | Y | 95% |
| A15 | 6 | B | Y | 96% |
| A17 | 3 | B | X | 14% |
| A19 | 1 | C | X | 69% |
| A20 | 2 | E | X | 57% |
| A21 | 1 | B | X | 55% |
| A30 | 1 | H | X | 42% |
| A33 | 1 | D | X | 20% |
| A34 | 1 | G | X | 7% |
| A35 | 1 | F | X | 18% |
| A36 | 5 | B | X | 80% |
| A37 | 4 | B | X | 77% |
| A38 | 1 | E | X | 70% |
| A39 | 1 | I | X | 10% |
| A40 | 1 | J | X | 3% |
| A108 | 10 | B | X | 9% |
| A150 | 2 | K | X | 56% |
| A157 | 1 | L | X | 30% |
| A162 | 1 | K | X | 36% |
| A165 | 1 | M | X | 59% |
| A166 | 1 | N | X | 51% |
| A167 | 1 | O | X | 50% |
| A172 | 1 | P | X | 46% |

Starting amine: 1) Fluoxetine [N-methyl-3-(p-trifluoromethylphenoxy)-3-phenylpropylamine hydrochloride], (Sigma, St. Louis); 2) 3,3-diphenylpropylamine (Aldrich); 3) Nisoxetine hydrochloride [(±)-γ-(2-methoxyphenoxy)-N-methyl-benzenepropanamine hydrochloride], (RBI, Natick, MA); 4) 1,2-diphenyl-3-methyl-4-(methylamino)-2-butanol hydrochloride, (Sigma-Aldrich Library of Rare Chemicals); 5) d-Norpropoxyphene (1,2-diphenyl-3-methyl-4-methylamino-2-butyl propionate maleate salt), (Sigma); 6) Maprotyline hydrochloride [N-Methyl-9, 10-ethanoanthracene-9(10H)-propanamine hydrochloride], (Sigma); 7) Nortriptyline hydrochloride {3-(10,11-dihydro-5H-dibenzo[a,d]cyclohepten-5-ylidene)-N-methyl-1-propanamine hydrochloride}, (Sigma); 8) Desipiramine hydrochloride {10,11-dihydro-N-methyl-5H-dibenz[b,f]azepine-5-propanamine hydrochloride}, (Sigma); 9) Protriptyline hydrochloride {N-Methyl-5H-dibenzo[a,d]cycloheptene-5-propanamine hydrochloride}, (Sigma); 10) 3-(1-naphthyl)-3-phenylpropylamine [prepared by Horner-Emmons reaction of the sodium ylide of diethyl cyanomethylphosphonate (Aldrich) with α-benzoylnaphthalene, (Pfaltz & Bauer, , Waterbury, CT) followed by catalytic hydrogenation of the intermediate α,β-unsaturated nitrile]. Reagent: A) methyl bromoacetate (Aldrich); B) ethyl bromoacetate (Aldrich); C) propyl bromoacetate (Aldrich); D) phenyl bromoacetate (Aldrich); E) 2-bromoacetamide (Aldrich); F) 2-chloro-N,N-diethylacetamide (Aldrich); G) N-ethylchloroacetamide (Lancaster); H) bromoacetonitrile (Aldrich); I) 4-(bromornethylsulfonyl)rnorpholine, (Sigma - Aldrich Library of Rare Chemicals); J) diethyl chloromethylphosphonate (Aldrich); K) benzyl 2-bromoacetate, (Aldrich); L) *p*-nitrophenyl bromoacetate, (Lancaster); M) octyl chloroacetate, (Sigma-Aldrich Library of Rare Chemicals); N) isopropyl bromoacetate, (Aldrich); O) *n*-butyl bromoacetate, (Pfatz & Bauer), Waterbury, CT); P) *tert*-butyl bromoacetate, (Aldrich).
Solvent: X) acetonitrile; Y) ethanol.

### Example 5A - Synthesis of N-{[3-Hydroxy-3-phenyl-3-(thien-2-yl)]propyl} sarcosine ethyl ester (Compound A32)

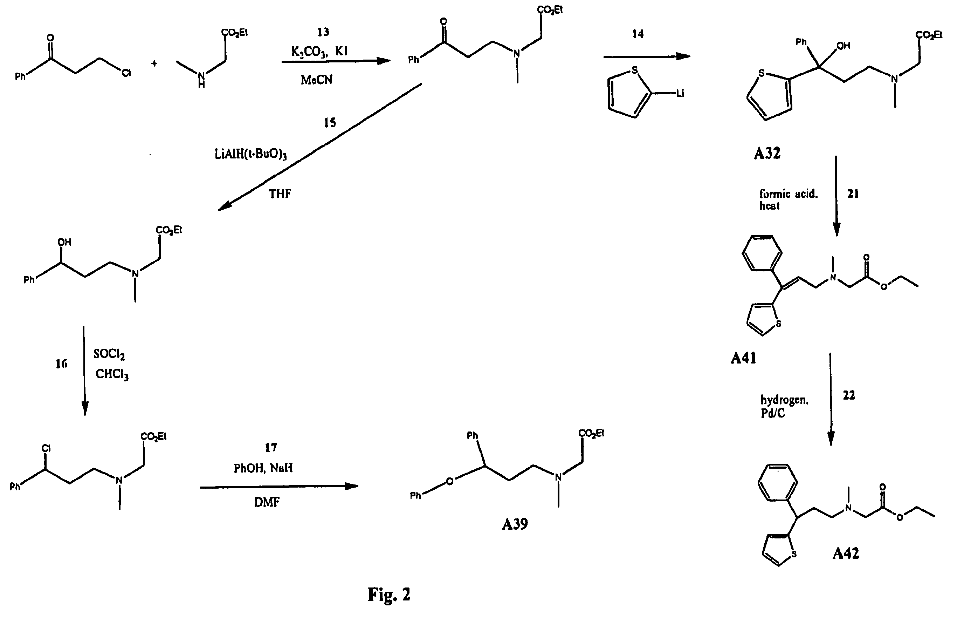

Step 1: N-[(3-Oxo-3-phenyl)propyl]sarcosine ethyl ester: A mixture of 3.37 g (20 mmol) 3-chloropropiophenone (Aldrich), (3.07 g, (20 mmol) sarcosine ethyl ester hydrochloride, 3.32 g (20 mmol) potassium iodide and 2.5 g potassium carbonate in 140 ml acetonitrile was heated under reflux with stirring for 2 hours (see Reaction 13, Figure 2). The reaction mixture was filtered and the solvent evaporated. The residue was dissolved in dichloromethane, washed with water and dried over sodium sulphate. Evaporation of the solvent gave N-[(3-oxo-3-phenyl)propyl]sarcosine ethyl ester as a yellow oil which was used in step 2 without purification.

Step 2: 2-Thienyllithium [generated by adding 1 ml of butyllithium (2.5 M in tetrahydrofuran) to 0.21 g (2.5 mmol) thiophene in 10 ml tetrahydrofuran at -78°C] was added dropwise into a solution of 0.623 g (2.5 mmol) of N-[(3-oxo-3-phenyl)propyl]sarcosine ethyl ester (from step 1) in 30 ml of tetrahydrofuran at -78°C (see Reaction 14, Figure 2). After stirring at -78°C for 1 h and at 20°C for 1 h, the reaction was quenched by adding 20 ml 10% ammonium hydroxide solution at 0°C. The mixture was extracted with methylene chloride, the solvent evaporated and the residue chromatographed on silica gel column with 16% ethyl acetate in hexanes to give 0.43 g (yield 52%) N-{[3-hydroxy-3-phenyl-3-(thien-2-yl)]propyl}sarcosine ethyl ester (compound A32) as a beige solid.

### Example 5B - Synthesis of N-{[3-Hydroxy-3-phenyl-3-(furan-2-yl)propyl} sarcosine

### ethyl ester (Compound A161)

N-{[3-Hydroxy-3-phenyl-3-(furan-2-yl)]propyl} sarcosine ethyl ester was synthesized essentially as described in Example 5A (replacing 2-thienyllithium with 2-furanyllithium) (yield 14%).

### Example 6 - Synthesis of N-13-Phenvl-3-(thien-2-y))-2-prooenyl]sarcosine ethyl ester (Compound A41)

N-{[3-Hydroxy-3-phenyl-3-(thien-2-yl)]propyl}sarcosine ethyl ester (Compound 32 from Example 5), 0.118g (0.354 mmol) was dissolved in 2 ml of formic acid. The solution was heated at 110°C for 0.5 hour (see Reaction 19, Figure 2). The deep red reaction mixture was concentrated and the residue was partitioned between water and CH₂Cl₂. The aqueous phase was extracted with CH₂Cl₂ and the CH₂Cl₂ solution was dried over Na₂SO₄. After evaporating the solvent, the residue was purified by preparative TLC with 1:3 ethyl acetate:hexanes to give 0.091 g (82 %) N-[3-phenyl-3-(thien-2-yl)-2-propenyl]sarcosine ethyl ester (Compound A41) as a deep red oil.

### Example 7 - Synthesis of N-13-Phenyl-3-(thien-2-yl)propyl]sarcosine ethyl ester (Compound A42)

0.055 g (0.174 mmol) N-[3-Phenyl-3-(thien-2-yl)-2-propenyl] sarcosine ethyl ester (Compound 41 from Example 6) was hydrogenated over 0.055 g 10% Pd/C in 2 ml of EtOH. The hydrogenation was conducted at 40 psi for 16 hours at room temperature (see Reaction 20, Figure 2). After filtering off the catalyst the solution was concentrated and the residue was purified by preparative TLC with 1:2 ethyl acetate:hexanes to give 0.012 g (22 %) N-[3-phenyl-3-(thien-2-yl)propyl]sarcosine ethyl ester (Compound A42) as a yellow oil.

### Example 8A - Synthesis of N-1(3-Phenyl-3-phenoxy)propyl]sarcosine ethyl ester (compound A31)

Step 1: N-[(3-Hydroxy-3-phenyl)propyl]sarcosine ethyl ester: 2.40 ml of LiAl(t-BuO)₃ [lithium tri-*tert*-butoxyaluminohydride (Aldrich) (1 M in THF)] was added into a solution of 0.593 g (2.38 mmol) N-[(3-oxo-3-phenyl)propyl]sarcosine ethyl ester (step 1 of Example 5A) in 10 ml of tetrahydrofuran at -78°C (see Reaction 15 in Figure 2). After stirring at -78°C for 1 h and 1 h at room temperature, the reaction was quenched by adding 10 ml 10% ammonium chloride solution at 0°C and filtered through celite. The mixture was extracted with methylene chloride and dried over sodium sulphate. Evaporation of the solvent gave N-[(3-hydroxy-3-phenyl)propyl]sarcosine ethyl ester as a yellow oil which was used in the next step without further purification.

Step 2: N-[(3-Chloro-3-phenyl)propyl]sarcosine ethyl ester: The yellow oil of step 1 was dissolved in 20 ml of chloroform, 1 ml of SOCl₂ was added and the mixture heated under reflux for 2 h (see Reaction 16 in Figure 2). After addition of crushed ice, the reaction mixture was neutralized with a saturated solution of potassium carbonate and extracted with methylene chloride. The combined extracts were evaporated and the residue purified by preparative silica gel TLC with 20% ethyl acetate in hexanes to give 0.165 g N-[(3-chloro-3-phenyl)propyl]sarcosine ethyl ester (yield 26% in two steps).

Step 3: N-[(3-Phenyl-3-phenoxy)propyl]sarcosine ethyl ester (compound A31): A solution of 0.075 g (0.278 mmol) N-[(3-chloro-3-phenyl)propyl]sarcosine ethyl ester (from step 2) in 3 ml of anhydrous dimethylformamide was added into a solution of sodium phenoxide (generated by adding 0.022 g of 60% NaH in mineral oil to 0.054 g phenol in 2 ml dimethylformamide) at room temperature (see Reaction 17 in Figure 2). The reaction mixture was stirred at room temperature for 30 hours, the solvent was evaporated under vacuum and the residue purified by preparative silica gel TLC with 35% ethyl acetate in hexanes to give 0.014 g (yield 15%) N-[(3-phenyl-3-phenoxy)propyl]sarcosine ethyl ester (compound A31) as a yellow oil.

### Example 8B - Additional Syntheses Using the Procedure of Example 8A

Compound A164 was prepared by alkylation of 4-methoxyphenol (Aldrich) with N-(3-chloro-3-phenylpropyl)sarcosine ethyl ester as described above in Example 8A (Step 3) - yield 5%.

Compound A119 was prepared by alkylation of thiophenol (Aldrich) with N-(3-chloro-3-phenylpropyl)sarcosine ethyl ester as described above in Example 8A (Step 3) yield 62%.

Compound A115 was prepared by alkylation of 4-(trifluoromethyl)thiophenol (Lancaster) with N-(3-chloro-3-phenylpropyl)sarcosine ethyl ester as described above in Example 8A (Step 3) - yield 93 %.

Compound A68 was prepared by alkylation of 4-*tert*-butylthiophenol (Lancaster) with N-(3-chloro-3-phenylpropyl)sarcosme ethyl ester as described above in Example 8A (Step 3) - yield 5%.

### Example 8C Synthesis of N-[3-Phenyl-3-(phenylaminopropyl]sarcosine ethyl ester (Compound A47)

Step 1:N-[3-Phenyl-3-(*p*-toluenesulfonanitido)propyl]sarcosine ethyl ester: 0 465 g (2.67 mmol) diethyl azodicarboxylate ("DEAD", Aldrich) was added dropwise to a solution of 0.511 g (2.03 mmol) N-(3-hydroxy-3-phenylpropyl)sarcosine ethyl ester (from Example 8A, Step 1), 0.571 g (2.31 mmol) *p*-toluenesulfonanilide, (TCI America, Portland, 0R) and 0.712 g (2.71 mmol) triphenylphosphine in 2 ml anhydrous tetrahydrofuran with stirring under nitrogen and cooling with an ice bath. The mixture was stirred at room temperature for 4 hours, the solvent evaporated and the residue chromatographed on silica gel with 25% ethyl acetate in hexanes to give 0.730 g (yield 74%) N-[3-phenyl-3-(*p*-toluenesulfonanilido)propyl]sarcosine ethyl ester. ¹H NMR (CDCl₃, 300 MHz) 7.58 (d, 2 H), 7.40-6.90 (m, 10 H), 6.62 (d, 2 H), 5.55 (t, 1H), 4.14 (q, 2 H), 3.20 (s, 2 H), 2.60-2.20 (m, 2 H), 2.39 (s, 3 H), 2.33 (s, 3 H), 2.20-1.80 (m, 2 H), 1.12 (t, 3 H); ¹³C NMR (CDCl₃, 75 MHz) 170.74, 142.90, 138.33, 138.08, 134.88, 132.78, 129.14, 128.60, 128.36, 128.28, 127.93, 127.79, 127.46, 60.51, 60.26, 58.57, 53.93, 42.16, 30.60, 21.36,14.12.

Step 2: N-[3-Phenyl-3-(phenylamino)propyl]sarcosine ethyl ester (Compound A47): A solution of 0.284 g (0.6 mmol) N-[3-phenyl-3-(*p*-toluenesulfonanilido)propyl]sarcosine ethyl ester (from Step 1) in 3 ml anhydrous ethylene glycol dimethyl ether was added dropwise within 1 hour into solution of sodium naphthalenide [prepared from 0.545 g (5.04 mmol) naphthalene and 0.110 g (5.16 mmol) sodium) in 8 ml anhydrous ethylene glycol dimethyl ether with stirring under nitrogen and cooling with an ice bath. The mixture was stirred at room temperature for 1 hour, quenched with ice and extracted with ethyl acetate. The combined organic extracts were washed with brine, the solvent evaporated and the residue chromatographed on silica gel with 25% ethyl acetate in hexanes to give 0.092 g (yield 47%) N-[3-phenyl-3-(phenylamino)propyl]sarcosine ethyl ester (Compound A47). ¹H NMR (CDCl₃, 300 MHz) 7.50-7.00 (m, 7 H), 6.70-6.40 (m, 3 H), 5.75 (br. s, 1H), 4.47 (t, 1H), 4.18 (q, 2 H), 3.24 (s, 2 H), 2.57 (t, 2 H), 2.37 (s, 3H), 2.10-1.70 (m, 2 H), 1.18 (t, 3 H); ¹³C NMR (CDCl₃, 75 MHz) 170.73, 147.82, 143.89, 128.87, 128.43, 126.69, 126.26, 116.57, 113.17, 60.47, 58.53, 57.92, 54.47, 42.32, 35.19, 14.18.

### Example 8D Synthesis of [R]-(+)-N-[3-Phenyl-3-(4-tert-butylphenoxy)propylsarcosine ethyl ester (Compound A55) {[α]_{D}²⁵+18.6° (c 7.84. CHCl₃)}

Step 1: [*S*]-(-)-N-(3-Hydroxy-3-phenylpropyl)sarcosine ethyl ester {[α]_{D}²⁵-35° (c 4.88, CHCl₃)}; prepared by alkylation of sarcosine ethyl ester with (R)-(+)-3-chloro-l phenyl-1-propanol (Aldrich) under the conditions described in Example 1 - yield 72 %. See Reaction 23, Figure 3.

Step 2: [*R*]-(+)-N-[3-Phenyl-3-(4-*tert*-butylphenoxy)propyl]sarcosine ethyl ester: prepared by Mitzunobu reaction (analogously to Example 8C, Step 1) of [*S*]-(-)-N-(3-hydroxy-3-phenylpropyl)sarcosine ethyl ester (from step 1) with 4-*tert*-butylphenol (Aldrich) - yield 41 %; [α]_{D}²⁵ +18.6° (c 7.84, CHCl₃). See Reaction 24, Figure 3

### Example 8E - Synthesis of [R]-(+)-N-[3-Phenyl-3-(4-phenylphenoxy)propyl]sarcosine ethyl ester (Compound A61) {[α]_{D}²⁵ + 22.3° (c 8.1, CHCl₃)}

Another synthesis of compound A61 with [α]D²⁵ +54.9° (c 5.28, CHCl₃) was already described in Example 2.

Step 1: [S]-(-)-N-(3-Hydroxy-3-phenylpropyl)sarcosine ethyl ester: prepared analogously to the method of U.S. Pat. 5,068,432 by reduction of N-[(3-oxo-3-phenyl)propyl]sarcosine ethyl ester (from step I of Example 5A) with (-) diisopinocampheylboron chloride (Aldrich) - yield 12%; [α]D²⁵ -24.6° (c 3.63, CHCl₃) (see Reaction 25, Figure 3). Another synthesis of [*S*]-(-)-N-(3-hydroxy-3-phenylpropyl)sarcosine ethyl ester with [a]_{D}²⁵-35° (c 4.88, CHCl₃) was already described in Example 8D (Step 1). See Reaction 23, Figure 3.

Step 2: [*R*]-(+)-N-[3-Phenyl-3-(4-phenylphenoxy)propyl]sarcosine ethyl ester (Compound A61): prepared by Mitzunobu reaction (analogously to Example 8C, Step 1) of [*S*]-(-)-N-(3-hydroxy-3-phenylpropyl)sarcosine ethyl ester (from step 1) with 4-phenylphenol (Aldrich) - yield 22%; [α]_{D}²⁵+22.3° (c 8.1, CHCl₃). See Reaction 26, Figure 3.

### Example 9A - Synthesis of N-[(4,4-Diphenyl)but-3-enyl]-N-ethylglycine ethyl ester (Compound A16)

A mixture of 0.158 g (0.5 mmol) of N-[(4,4-diphenyl)but-3-enyl]glycine ethyl ester (Compound A26), 0.234 g (2.1 mmol) bromoethane, 0.281 g (2 mmol) potassium carbonate and 0.068 g (0.4 mmol) potassium iodide was stirred under argon for 20 hours at room temperature. The reaction mixture was filtered, the solvent evaporated, and the residue chromatographed on a silica gel column with 20% ethyl acetate in hexanes to yield 0.112 g (66%) N-[(4,4-diphenyl)but-3-enyl]-N-ethylglycine ethyl ester (Compound A16) as an oil. NMR spectra showed: ¹H NMR (CDCl₃, 300 MHz) 7.60-7.00 (m, 10 H), 6.09 (t, 1 H), 4.13 (q. 2 H), 3.27 (s, 2 H), 2.72 (t, 2 H), 2.61 (q, 2 H), 2.28 (dt, 2 H), 1.23 (t, 3 H), 1.01 (t, 3 H); ¹³C NMR (CDCl₃, 75 MHz) 171.77, 142.96, 142.86, 140.33, 130.09, 128.49, 128.35, 127.48, 127.27, 127.19, 60.58, 54.90, 53.98, 48.20, 28.19, 14.57, 12.70.

### Example 9B Additional Syntheses Using the Procedure of Example 9A

Compound A147 was prepared by treatment of compound A150 with iodomethane under the conditions described in Example 9A - yield 30%.

### Example 10 - Synthesis of N-[(4,4-Diphenyl)butyl]glycine ethyl ester (Compound A4)

0.072 g (0.23 mmol) of N-[(4,4-diphenyl)but-3-enyl]glycine ethyl ester (compound A26) was hydrogenated over 0.072 g 10% Pd/C in 5 ml ethanol under 40 psi for 3 hours at room temperature. The mixture was filtered from the catalyst through celite and the solvent evaporated to give 0.065 g (yield 90%) N-[(4,4-diphenyl)butyl]glycine ethyl ester (compound A4) as an oil. NMR spectra of the product showed: ¹H NMR (CDCl₃, 300 MHz) 7.40-7.10 (m, 10H), 4.17 (q, 2 H), 3.89 (t, 1 H), 3.34 (s, 2 H), 2.61 (t, 2H), 2.08 (dt, 2 H), 1.50-1.40 (m, 2 H), 1.25 (t, 3 H), ¹³C NMR (CDCl₃, 75 MHz) 172.47, 144.89, 148.36, 127.77, 126.05, 60.63, 51.17, 50.90, 49.44, 33.19, 28.50, 14.17.

### Example 11 - Additional Syntheses Using the Procedure of Example 10

Compound A25 was prepared by catalytic hydrogenation, using 10% palladium on carbon, of compound A2 - yield 90%.

Compound A3 was prepared by catalytic hydrogenation, using 10% palladium on carbon, of compound A16 - yield 90%.

### Example 12 - Synthesis of N-[(4,4-Diphenyl)but-3-enyl]glycine hydrochloride (compound A27)

To a solution of 0.093 g (0.3 mmol) of N-[(4,4-diphenyl)but-3-enyl]glycine ethyl ester (compound A26) in 2 ml methanol was added 3.4 ml 1N sodium hydroxide and the mixture was heated under reflux for four hours. The reaction mixture was concentrated to half volume, acidified with 4 N hydrochloric acid, and extracted 4 times with methylene chloride. The combined extracts were dried and evaporated to give 0.100 g (yield 86%) of N-[(4,4-diphenyl)but-3-enyl]glycine hydrochloride (compound A27). NMR spectra of the product showed: ¹H NMR (CD₃OD, 300 MHz) 7.40-7.00 (m, 10 H), 5.96 (t, 1H), 3.81 (s, I H), 3.69 (s, 2 H), 3.04 (br.s, 2 H), 2.42 (br.s, 2 H); ¹³C NMR (CD₃OD, 75 MHz) 166.78, 145.86, 145.82, 141.73, 139.34, 129.42, 128.42, 127.96, 127.41, 127.35, 127.02, 121.97, 121.87, 52.28, 26.43.

### Example 13A - Additional Syntheses Using the Procedure of Example 12

The following N-modified amino acids were prepared by hydrolysis of the corresponding esters with 1N sodium hydroxide in methanol, or with 1N lithium hydroxide in ethanol at room temperature, followed by acidification with hydrochloric acid as described above in Example 12, where the parenthetical lists the starting ester, yield, and - where applicable, [α]_{D}²⁵:

| | | |
|---|---|---|
| A8 (A4, 86%) | A29 (A5, 70%) | A44 (A48, 98%) |
| A45 (A53, 98%) | A46 (A55, 98%, +2.38° (c 2.4, CHCl₃)) | A49 (A50, 95%) |
| A51 (A52, 82%) | A54 (A68, 52%) | A56 (A57, 71%) |
| A58 (A59, 98%) | A60 (A61, 80%, +25.3° (c 2.13, MeOH) | A62 (A63, 69%, -25.6 (c 2.4, MeOH)) |
| A64 (A73, 90%) | A65 (A74, 90%) | A66 (A67,60%) |
| 69 (A70,99%) | A72 (A75,98%) | A76(A77,75%) |
| A79(A80,62%) | A81(A89, 64%) | A84 (A85, 93%) |
| A86 (A87, 98%) | A91 (A71, 54%) | A92 (A40, 90%) |
| A93 (A95, 95%) | A94 (A96, 95%) | |
| A98 (A100, 95%) | A101 (A118, 53%) | A102 (A108, 61%) |
| A 103 (A104, 83%) | A105 (A106, 86%) | A107 (A115, 76%) |
| A109 (A123, 98%) | A110 (A169, 68%) | A112 (A117, 62%) |
| A113 (A119, 56%) | A114 (A120, 98%) | A116 (A122, 35%) |
| A124 (A126, 62%) | A131 (A132, 82%); | A135 (A134, 92%) |
| A136 (A145, 98%) | A137 (A164, 85%) | A144 (A158, 43%) |
| A152 (A156, 58%) | A154 (A160, 98%) | A174 (A43, 91%) |
| A175 (A171,38%, +10 (c 2.9, MeOH)) | A176 (A88, 61%) | A181 (A173, 82%, -16.6° (c 3.11, MeOH)) |
| A182 (A177, 78%, +19.0° (c 2.93, MeOH)) | A183 (A178, 72%, +13.7° (c 2.68, MeOH)) | A184 (A179, 98%, +13.5° (*c* 2.5, MeOH)) |

### Example 13B Synthesis of N-Methyl-N-[(1H-tetrazol-5-yl)methyl]-3,3-diphenylpropylamine hydrochloride (Compound A146)

Step 1: A mixture of 2.11 g (10 mmol) 3,3-diphenylpropylamine (Aldrich), (0.54 g, 4.54 mmol) bromoacetonitrile (Aldrich), and 2.5 g potassium carbonate in 5 ml acetonitrile was stirred at room temperature for 16 hours. The reaction mixture was diluted with dichloromethane, washed with water, the solvent evaporated, and the residue chromatographed on silica gel column with 30% ethyl acetate in hexanes to give 1.24 g (yield 50%) N-cyanomethyl-3,3-diphenylpropylamine as an oil which solidified on standing. ¹H NMR (CDCl₃, 300 MHz) 7.45-7.10 (m, 10 H), 4.05 (t, 1H), 3.50 (s, 2 H), 2.67 (t, 2H), 2.23 (dt, 2H); ¹³C NMR (CDCl₃, 75 MHz) 144.25, 128.53, 127.68, 126.33, 117.72, 48.58, 47.13, 37.19, 35.14.

Step 2: A mixture of 0.72 g (2.9 mmol) N-cyanomethyl-3,3-diphenylpropylamine (from step 1), 0.49g (3.4 mmol) iodomethane and 1.6 g potassium carbonate in 5 ml acetonitrile was stirred at room temperature for 16 hours. The reaction mixture was diluted with dichloromethane, washed with water, the solvent evaporated, and the residue chromatographed on silica gel column with 20% ethyl acetate in hexanes to give 0.33 g (yield 43%) N-methyl-N-cyanomethyl-3,3-diphenylpropylamine as an oil which solidified on standing. ¹H NMR ((CDCl₃, 300 MHz) 7.30-7.10 (m, 10 H), 4.02 (t, I H), 3.47 (s, 3 H), 2.38 (t, 2 H), 2.32 (s, 3H), 2.19 (dt, 2H);

Step 3: A mixture of 0.132 g (0.5 mmol) N-methyl-N-cyanomethyl-3,3-diphenylpropylamine (from step 2) and 0.183 g (0.55 mmol) azidotributyltin (Aldrich) was stirred at 80°C under argon for 16 hours. The reaction mixture was suspended with 1 M solution of hydrogen chloride in diethyl ether (Aldrich) and the precipitated yellow wax was purified by preparative TLC with 10% methanol in ethyl acetate to give 0.06 g (yield 35%) N-methyl-N-[(1*H*-tetrazol-5-yl)methyl]-3,3-diphenylpropylamine hydrochloride (Compound A146) as a white powder. ¹H NMR (DMSO-*d*₆, 300 MHz) 7.30-7.16 (m, 10 H), 4.11 (s, 2 H), 3.97 (t, 1H), 2.60 (br. s, 2 H), 2.45 (s, 3H), 2.36 (br. s, 2H).

### Example 13C Additional Syntheses Usine the Procedure of Example 13B:

Compound A133 was prepared by treatment of compound A30 with azidotributyltin as described above in Example 13B (Step 3) - yield 11%.

### Example 13D Synthesis of Dimethyl(ethoxycarbonylmethyl)[3-phenyl-3-(4-trifluoromethylphenoxy)propylammonium iodide (Compound A148)

A solution of 0.152 g (0.38 mmol) N-[3-phenyl-3-(4-trifluoromethylphenoxy)propyl] sarcosine ethyl ester (Compound A21) and 0.273 g (1.93 mmol) iodomethane in 2 ml benzene was heated under reflux for 2 hours and the solvent evaporated. The residue was washed three times with anhydrous diethyl ether and dried under vacuum to give 0.175g (yield 85%) dimethyl(ethoxycarbonylmethyl)[3-phenyl-3-(4-trifluoromethylphenoxy)propyl]ammonium iodide (Compound A148) as a pale yellow hygroscopic powder.

### Example 14 - Preparation of Cells Expressing GlyT-1 and GlyT-2

This example sets forth methods and materials used for growing and transfecting QT-6 cells.

QT-6 cells were obtained from American Type Culture Collection (Accession No. ATCC CRL-1708). Complete QT-6 medium for growing QT-6 is Medium 199 (Sigma Chemical Company, St. Louis, MO; hereinafter "Sigma") supplemented to be 10% tryptose phosphate; 5% fetal bovine serum (Sigma); 1% penicillin-streptomycin (Sigma); and 1% sterile dimethylsulfoxide (DMSO; Sigma). Other solutions required for growing or transfecting QT-6 cells included:
DNA/DEAE Mix: 450 µl TBS, 450 µl DEAE Dextran (Sigma), and 100 µl of DNA (4 µg) in TE, where the DNA includes GlyT-1a, GlyT-1b, GlyT-1c, or GlyT-2, in a suitable expression vector. The DNA used was as defined below.
PBS: Standard phosphate buffered saline, pH 7.4 including 1 mM CaCl₂ and I mM MgCl₂ sterilized through 0.2 µ filter.
TBS: One ml of Solution B, 10 ml of Solution A; brought to 100 ml with distilled H₂O; filter-sterilized and stored at 4°C.
TE: 0.01 M Tris, 0.001 M EDTA, pH 8.0.
DEAE dextran: Sigma, #D-9885. A stock solution was prepared consisting of 0.1 % (1 mg/ml) of the DEAE dextran in TBS. The stock solution was filter sterilized and frozen in 1 ml aliquots.
Chloroquine: Sigma, #C-6628. A stock solution was prepared consisting of 100 mM chloroquine in H₂O. The stock solution was filter-sterilized and stored in 0.5 ml aliquots, frozen.

### Solution A (10X):

| | |
|---|---|
| NaCl | 8.00 g |
| KCl | 0.38 g |
| Na₂HPO₄ | 0.20 g |
| Tris base | 3.00 g |

The solution was adjusted to pH 7.5 with HCl, brought to 100.0 ml with distilled H₂O and filter-sterilized and stored at room temperature.

### Solution B (100X):

| | |
|---|---|
| CaCl₂·2H₂O | 1.5 g |
| MgCl₂·6H₂O | 1.0 g |

The solution was brought to 100 ml with distilled H₂O, and filter-sterilized; the solution was then stored at room temperature.

HBSS: 150 mM NaCl, 20 mM HEPES, I mM CaCl₂, 10 mM glucose, 5 mM KCl, 1 mM MgCl₂ H₂O; adjusted with NaOH to pH 7.4.

Standard growth and passaging procedures used were as follows: Cells were grown in 225 ml flasks. For passaging, cells were washed twice with warm HBSS (5 ml each wash). Two ml of a 0.05% trypsin/EDTA solution was added, the culture was swirled, then the trypsin/EDTA solution was aspirated quickly. The culture was then incubated about 2 minutes (until cells lift off), then 10 ml of QT-6 media was added and the cells were further dislodged by swirling the flask and tapping its bottom. The cells were removed and transferred to a 15 ml conical tube, centrifuged at 1000 x g for 10 minutes, and resuspended in 10 ml of QT-6 medium. A sample was removed for counting, the cells were then diluted further to a concentration of I x 10⁵ cells/ml using QT-6 medium, and 65 ml of the culture was added per 225 ml flask of passaged cells.

Transfection was accomplished using cDNA's prepared as follows:

The rat GlyT-2 (rGlyT-2) clone used contains the entire sequence of rGlyT-2 cloned into pBluescript SK+(Stratagene) as an Eco RI - Hind III fragment, as described in Liu et al., J. Biol. Chem. 268, 22802-22808 (1993). GlyT-2 was then subcloned into the pRc/RSV vector as follows: A PCR fragment corresponding to nucleotides 208 to 702 of the rGlyT-2 sequence was amplified by PCR using the oligonucleotide: 5'GGGGGAAGCTTATGGATTGCAGTGCTCC 3' as the 5' primer and the oligonucleotide: 5' GGGGGGGTACCCAACACCACTGTGCTCTG 3' as the 3' primer. This created a Hind III site immediately upstream of the translation start site. This fragment, which contained a Kpn I site at the 3' end, along with a Kpn I - Pvu II fragment containing the remainder of the coding sequence of rGlyT-2, were cloned into pBluescript SK+ previously digested with Hind III and Sma I, in a three part ligation. A Hind III - Xba 1 fragment from this clone was then subcloned into the pRc/RSV vector. The resulting construct contains nucleotides 208 to 2720 of the rGlyT-2 nucleic acid in the pRc/RSV expression vector.

The human GlyT-1a (hGlyT-1a) clone used contains the sequence of hGlyT-1a from nucleotide position 183 to 2108 cloned into the pRc/CMV vector (Invitrogen, San Diego, CA) as a Hind III-Xba 1 fragment as described in Kim et al., Mol. Pharmacol., 45, 608-617, 1994. This cDNA encoding GlyT-1a actually contained the first 17 nucleotides (corresponding to the first 6 amino acids) of the GlyT-1a sequence from rat. To determine whether the sequence of human GlyT-1a was different in this region, the 5' region of hGlyT-1a from nucleotide 1 to 212 was obtained by rapid amplification of cDNA end using the 5' RACE system supplied by Gibco BRL (Gaithersburg, MD). The gene specific primer: 5' CCACATTGTAGTAGATGCCG 3' corresponding to nucleotides 558 to 539 of the hGlyT-1a sequence, was used to prime cDNA synthesis from human brain mRNA, and the gene specific primer. 5' GCAAACTGGCCGAAGGAGAGCTCC 3', corresponding to nucleotides 454 to 431 of the hGlyT-1a sequence, was used for PCR amplification. Sequencing of this 5' region of GlyT-1a confirmed that the first 17 nucleotides of coding sequence are identical in human and rat GlyT-1a.

The human GlyT-1b (hGlyT-1b) clone used contains the sequence ofhGlyT-1b from nucleotide position 213 to 2274 cloned into the pRc/CMV vector as a Hind III - Xba I fragment as described in Kim et al., Mol. Pharmacol., 45, 608-617, 1994.

The human GlyT-1c (hGlyT-1c) clone used contains the sequence of hGlyT-1c from nucleotide position 213 to 2336 cloned into the pRc/CMV vector (Invitrogen) as a Hind III - Xba I fragment as described in Kim et al., Mol. Pharmacol., 45, 608-617, 1994. The Hind III - Xba fragment of hGlyT-1c from this clone was then subcloned into the pRc/RSV vector. Transfection experiments were performed with GlyT-1c in both the pRc/RSV and pRc/CMV expression vectors.

The following four day procedure for the tranfections was used:
On day 1, QT-6 cells were plated at a density of 1x10⁶ cells in 10 ml of complete QT-6 medium in 100 mm dishes.
On day 2, the media was aspirated and the cells were washed with 10 ml of PBS followed by 10 ml of TBS. The TBS was aspirated, and then 1 ml of the DEAE/DNA mix was added to the plate. The plate was swirled in the hood every 5 minutes. After 30 minutes, 8 ml of 80 µM chloroquine, in QT-6 medium was added and the culture was incubated for 2.5 hours at 37°C and 5% CO₂. The medium was then aspirated and the cells were washed two times with complete QT-6 media, then 100 ml complete QT-6 media was added and the cells were returned to the incubator.
On day 3, the cells were removed with trypsin/EDTA as described above, and plated into the wells of 96-well assay plates at approximately 2x10⁵ cells/well.
On day 4, glycine transport was assayed (see Example 15).

### Example 15 - Assay of Transport Via GlyT-1 or GlyT-2 transporters

This example illustrates a method for the measurement of glycine uptake by transfected cultured cells.

Transient GlyT-transfected cells grown in accordance with Example 14 were washed three times with HEPES buffered saline (HBS). The cells were then incubated 10 minutes at 37°C, after which a solution was added containing 50 nM [³H]glycine (17.5 Ci/mmol) and either (a) no potential competitor, (b) 10 mM nonradioactive glycine or (c) a concentration of a candidate drug. A range of concentrations of the candidate drug was used to generate data for calculating the concentration resulting in 50% of the effect (e.g., the IC₅₀s, which are the concentrations of drug inhibiting glycine uptake by 50%). The cells were then incubated another 10 minutes at 37°C, after which the cells were aspirated and washed three times with ice-cold HBS. The cells were harvested, scintillant was added to the cells, the cells were shaken for 30 minutes, and the radioactivity in the cells was counted using a scintillation counter. Data were compared between the same cells contacted or not contacted by a candidate agent, and between cells having GlyT-1 activity versus cells having GlyT-2 activity, depending on the assay being conducted.

### Example 16 - Assay of Binding to NMDA Receptors

This example illustrates binding assays to measure interaction of compounds with the glycine site on the NMDA receptor.

Direct binding of [³H]glycine to the NMDA-glycine site was performed according to the method of Grimwood et al., Molecular Pharmacology, 41, 923-930 (1992); Yoneda et al., J. Neurochem. 62, 102-112 (1994).

Preparation of membranes for the binding test required application of a series of standard methods. Unless otherwise specified, tissues and homogenates were kept on ice and centrifugations were conducted at 4°C. Homogenizations were conducted with an effort to minimize resulting rise in tissue/homogenate temperature. The membrane preparation included the following steps:
A. Sacrifice and decapitate four rats; remove cortices and hippocampi.
B. Homogenize tissue in twenty volumes of 0.32 M sucrose/5 mM Tris-Acetate (pH 7.4) with 20 strokes of a glass/teflon homogenizer.
C. Centrifuge tissue at 1000 x g, 10 minutes. Save supernatant. Resuspend pellet in small volume of buffer and homogenize again. Centrifuge the homogenized pellet and combine the supernatant with the previous supernatant.
D. Centrifuge the combined supernatants at 40,000 x g, for 30 minutes. Discard the supernatant.
E. Resuspend the pellet in 20 volumes of 5 mM Tris-Acetate (pH 7.4). Stir the suspension on ice for one hour. Centrifuge the suspension at 40,000 x g for 30 minutes. Discard the supernatant and freeze the pellet for at least 24 hours.
F. Resuspend the pellet from step 5 in Tris Acetate buffer (5 mM, pH 7.4) containing 0.1% saponin (w/v; Sigma Chemical Co., St. Louis) to a protein concentration of 1 mg/ml. Leave on ice for 20 minutes. Centrifuge the suspension at 40,000 x g for 30 minutes. Resuspend the pellet in saponin-free buffer and centrifuge again. Resuspend the pellet in Tris-Acetate buffer at a concentration of 10 mg/ml and freeze in aliquots.
G. On day three, remove an aliquot of membranes and thaw on ice. Dilute the suspension into 10 ml Tris-Acetate buffer and centrifuge at 40,000 x g for 30 minutes. Repeat the wash step twice more for a total of 3 washes. Resuspend the final pellet at a concentration of I mg/ml in glycine-free Tris-Acetate buffer.

The binding test was performed in eppendorf tubes containing 150 µg of membrane protein and 50 nM [³H]glycine in a volume of 0.5 ml. Non-specific binding was determined with 1 mM glycine. Drugs were dissolved in assay buffer (50 mM Tris-acetate, pH 7.4) or DMSO (final concentration of 0.1%). Membranes were incubated on ice for 30 minutes and bound radioligand was separated from free radioligand by filtration on Whatman GF/B glass fiber filters or by centrifugation (18,000 x g, 20 min). Filters or pellet was washed three times quickly with ice-cold 5 mM Tris-acetate buffer. Filters were dried and placed in scintillation tubes and counted. Pellets were dissolved in deoxycholate/NaOH (0.1 N) solution overnight, neutralized and radioactivity was determined by scintillation counting.

A second binding test for the NMDA-glycine site used [³H]dichlorokynurenic acid (DCKA) and membranes prepared as above. See, Yoneda et al., J. Neurochem., 60 ,634 -645 (1993). The binding assay was performed as described for [³H]glycine above except that [³H]DCKA was used to label the glycine site. The final concentration of **[**^{**3**}**H]DCKA** was 10 nM, and the assay was performed for 10 minutes on ice.

A third binding test used for the NMDA-glycine site used indirect assessment of affinity of ligands for the site by measuring the binding of [³H]MK-801 (dizocilpine). See, Palmer and Burns, J. Neurochem., 62, 187-196 (1994). Preparation of membranes for the test was the same as above. The binding assay allowed separate detection of antagonists and agonists.

The third binding test was operated to identify antagonists as follows: 100 µg of membranes were added to wells of a 96-well plate, along with glutamate (10 µM) and glycine (200 nM) and various concentrations of the ligand to be tested. The assay was started by the addition of 5 nM [³H]MK-801 (23.9 Ci/mmol), which binds to the ion channel associated with NMDA receptors. The final volume of the assay was 200 µl. The assay was performed for 1 hour at room temperature. Bound radioactivity was separated from free by filtration, using a TOMTEC harvester. Antagonist activity was indicated by decreasing radioactivity associated with the NMDA receptor with increasing concentration of the tested ligand.

The third binding test was operated to identify agonists by performing the test as above, except that the concentration of glycine was 200 nM. Agonist activity was indicated by increasing radioactivity associated with the NMDA receptor with increasing concentration of the tested ligand.

### Example 17 - Assay of Calcium Flux

This example illustrates a protocol for measuring calcium flux in primary neuronal calls.

The calcium flux measurement is performed in primary neuronal cell cultures, which are prepared from rat fetal cortices dissected from pregnant rats using standard procedures and techniques that require sterile dissecting equipment, a microscope and defined medium. The protocol used was adapted from Lu et al., Proc. Nat'l. Acad. Sci. USA, 88, 6289-6292 (1991).

Defined medium is prepared in advance in accordance with the following recipe:

| Components | Source (catalogue #) | Final Concentration |
|---|---|---|
| D-glucose | Sigma (G-7021) | 0.6% |
| transferrin | Sigma (T-2252) | 100 µg/ml |
| insulin | Sigma (1-5500) | 25 µg/ml |
| progesterone | Sigma (P-6149) | 20 nM |
| putrescine | Sigma (P-7505) | 60 µM |
| selenium | Sigma (S-5261) | 30 nM |
| pen-strcp▲ | GIBCO (15070-014) | 0.5 U-0.5 µg/ml |
| L-glutamine* | GIBCO (25030-016) | 146 mg/l |
| MEM° | GIBCO (11095 or 11090) | 500 ml/l |
| F-12 | GIBCO (11765) | 500 ml/l |

| | | |
|---|---|---|
| ▲ pen-strep: 5,000 U/ml penicillin and 5,000 µg/ml steptomycin | | |
| *add only when MEM without L-glutamine is used | | |
| °with L-glutamine or without L-glutamine, respectively | | |

Before starting the dissection, tissue culture plates were treated with polylysine (100 µg/ml for at least 30 minutes at 37°C) and washed with distilled water. Also, a metal tray containing two sets of sterile crude dissecting equipment (scissors and tweezers) and several sets of finer dissecting tools was autoclaved. A pair of scissors and tweezers were placed into a sterile beaker with 70% alcohol and brought to the dissecting table. A petri dish with cold phosphate buffered saline (PBS) was placed on ice next to the place of dissection.

A pregnant rat (E15 or 16 on arrival from Hilltop Lab Animals (Scottdale, PA), E17 or 18 at dissection) was placed in a CO₂/dry ice chamber until it was unconscious. The rat was removed, pinned to a backing, the area of dissection was swabbed with 70% alcohol, and skin was cut and removed from the area of interest. A second pair of scissors was used to cut through and remove the prenatal pups in their sacs. The string of sacs was placed into the cold PBS and transported to a sterile hood.

The prenatal pups were removed from the sacs and decapitated. The skulls were then removed and the brains were carefully dislodged and placed into a clean petri dish with cold PBS. At this point, it was necessary to proceed with a dissecting microscope. The brain was turned so that the cortices were contacting the plate and the tissue between the dissector and the cortex (striatum and other brain parts) was scooped out. The hippocampus and olfactory bulb were cut away from the cortex. Then the tissue was turned over and the meninges were removed with tweezers. The remaining tissue (cortex) was placed in a small petri dish with defined media.

The tissue was chopped with a scalpel and then triturated with a glass pipet that had been fire polished. The chopped, triturated tissue was then transferred to a sterile plastic tube and continued to be triturated with a glass pipet with a finer opening. Cells were counted in a suitable counting chamber. Cells were plated at roughly 40,000 cells/well in 100 µl of defined medium for 96-well plates, 200,000 cells/well in 500 µl in 24-well plates, 400,000 cells/well in 1 ml in 12-well plates, 1.5 × 10⁸ cells/35 mm dish in 1.5 ml and 10 × 10⁸ cells/100 mm dish in 10 ml. To inhibit glia growth, cultures were treated with 100 µM 5-flouro-2-deoxyuridine (FDUR, Sigma (F-0503)) or 50/µM uridine (Sigma (U-3003)) and 50 µM FDUR.

The cortical cultures for the standard calcium flux assay were grow in 24-well plates in the defined medium described above for 7 days and fed once with serum containing media (10% heat inactivated fetal calf serum, 0.6% glucose in MEM) by exchanging half of the medium. Cultures were used after 12 days of incubation in *vitro.* The cultures were rinsed three times with HCSS (i.e. HEPES-buffered control salt solution, containing 120 mM NaCl, 5.4 mM KCl, 1.8 mM CaCl₂ 25 mM HEPES, and 15 mM glucose, in HPLC water and adjusted to pH 7.4 by NaOH, which was also made in HPLC water). In the third wash, the culture was incubated at 37°C for 20 to 30 minutes.

Solutions containing ⁴⁵Ca⁺⁺ (5000 dpm/ml) and drugs for testing or controls were prepared in HCSS. Immediately before the above ⁴⁵Ca⁺⁺ solutions were added, cultures were washed twice with HCSS, and 250 µl of ⁴⁵Ca⁺⁺ solution per well was added, one plate at a time. The cultures were incubated for 10 minutes at room temperature, rinsed three times with HCSS, and 1 ml scintillation liquid per well was added, followed by shaking for at least 15 minutes. Retained radioactivity was counted in a scintillation counter.

### Example 18 - Synthesis of N-(3-Cyano-3,3-diphenyl)propyl-2-piperidinecarboxylic acid methyl ester (Compound B9)

A mixture of 0.3 g (1 mmol) of 4-bromo-2,2-diphenyl butyronitrile (Aldrich, Milwaukee, WI), 0.359 g (2 mmol) methyl pipecolinate hydrochloride (Aldrich), 0.553 g (4 mmol) potassium carbonate and 0.166 g (1 mmol) potassium iodide in 5 ml acetonitrile was refluxed under argon for 20 hours. The reaction mixture was filtered, the solvent evaporated and the residue chromatographed on silica gel column with 30% ethyl acetate in hexanes to give 0.173 g (yield 48%) of N-(3-cyano-3,3-diphenyl)propyl-2-piperidinecarboxylic acid methyl ester (compound B9) as an oil. NMR spectra of the product showed: ¹H NMR (CDCl₃, 300 MHz) 7.50-7.20 (m, 10 H), 3.58 (s. 3 H), 3.10 - 3.00 (m, 2 H), 2.70 - 2.50 (m, 3 H), 2.50 - 2.35 (m, 1H), 2.25 - 2.10 (m, 1H), 1.90 - 1.50 (m, 4 H), 1.40 - 1.20 (m, 2 H); ¹³C NMR (CDCl₃, 75 MHz) 173.59, 140.00, 139.00, 128.71, 127.72, 126.58, 126.46, 121.73, 103.85, 65.09, 52.88, 51.47, 50.92, 49.70, 36.35, 29.27, 24.82, 22.27.

### Example 19 - Additional Syntheses Usine Reaction 1

Additional compounds were synthesized using Reaction 1 as follows:

| Compound | Reagent | Aminoacid | Solvent | Yield |
|---|---|---|---|---|
| B1 | A | 1 | X | 70% |
| B2 | E | 1 | X | 28% |
| B3 | B | 2 | Y | 13% |
| B4 | B | 1 | X | 57% |
| B6 | C | 3 | Z | 24% |
| B7 | C | 1 | Z | 48% |
| B8 | D | 1 | X | 77% |
| B11 | D | 4 | X | 61% |
| B 12 | B | 3 | X | 43% |
| B13 | B | 4 | X | 39% |
| B14 | C | 5 | Z | 63% |
| B17 | F | 1 | X | 65% |

Reagent: A) 1,1'-(4-chlorobutylidene)bis(4-fluorobenzene) (Acros Organics, Pittsburgh, PA); B) 4-bromo-1,1-diphenyl-1-butene [prepared as described in F.A. Ali et al., J. Med. Chem., 28: 653-660, 1985]; C) benzhydryl 2-bromoethyl ether, [prepared as described in M.R. Pavia et al., J. Med. Chem., 35: 4238-4248, 1992]; D) 3,3-diphenylpropyl tosylate [prepared by LiAlH₄ reduction of 3,3-diphenylpropionic acid (Aldrich) to 3,3-diphenylpropanol, followed by tosylation]; E) 9-fluorenylethyl tosylate [prepared by LiAlH₄ reduction of 9-fluoreneacetic acid methyl ester (Aldrich) to 2-(9-fluorenyl)ethanol, followed by tosylation]; and F) 3,3-bis(4-fluorophenyl)propyl tosylate [prepared by alkylation of diethyl malonate (Aldrich) with chlorobis(4-fluorophenyl)methane (Aldrich), followed by hydrolysis and decarboxylation, LiAlH₄ reduction of the monocarboxylic acid, and tosylation of the formed alcohol].

Amino acid: 1) methyl pipecolinate hydrochloride (Aldrich); 2) methyl (S-(-)-2-azetidinecarboxylate hydrochloride [prepared by methylation of S-(-)-2-azetidinecarboxylic acid (Aldrich) with chlorotrimethylsilane (Aldrich) in methanol according to the general procedure described in M.A. Brook et al., Synthesis, p. 201, 1983]; 3) L-proline methyl ester hydrochloride (Aldrich); 4) methyl (±)-*trans*-3-azabicyclo[3.1.0]hexane-2-carboxylate hydrochloride [prepared by methylation of (±)-*trans-*3-azabicyclo[3.1.0]hexane-2-carboxylic acid (Aldrich) with chlorotrimethylsilane (Aldrich) in methanol according to the general procedure described in M.A. Brook et al., Synthesis, 201, 1983]; 5) indole-2-carboxylic acid methyl ester hydrochloride [prepared by methylation of indole-2-carboxylic acid (Aldrich) with chlorotrimethylsilane (Aldrich) in methanol according to the general procedure described in M.A. Brook et al., Synthesis. 201, 1983].

Solvent: X) acetonitrile; Y) dioxane; Z) methanol

### Example 20A - Synthesis of N-r(3,3-Diphenyl-3-hydroxy)propyl]pipecolic acidmethyl ester (compound B18)

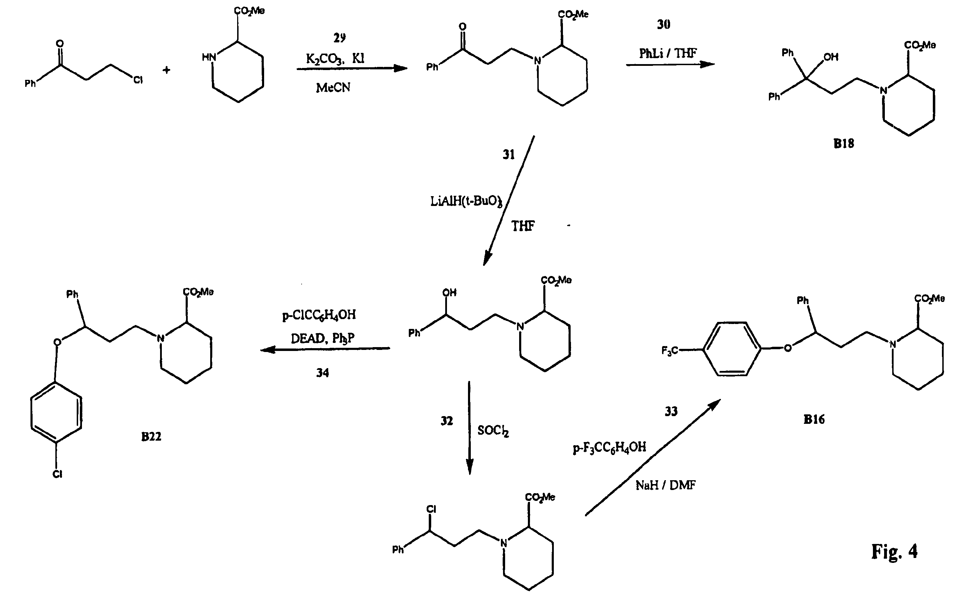

Step 1: N-[(3-Oxo-3-phenyl)propyl]pipecolic acid methyl ester: A mixture of 3.37 g (20 mmol) 3-chloropropiophenone (Aldrich), 3.59 g (20 mmol) methyl pipecolinate hydrochloride (Aldrich), 3.32 g (20 mmol) potassium iodide and 2.5 g potassium carbonate in 140 ml of acetonitrile was heated under reflux with stirring for 2h (Reaction 29, Fig. 4). The reaction mixture was filtered, the solvent evaporated and the residue dissolved in dichloromethane, washed with water and dried over sodium sulphate. Evaporation of the solvent gave N-[(3-oxo-3-phenyl)propyl]pipecolic acid methyl ester as a yellow oil which was used in the next step without further purification.

Step 2: 0.21 ml of phenyllithium (1.8 M in cyclohexane-ether, Aldrich) was added dropwise into a solution of 0.101 g (0.367 mmol) of N-[(3-oxo-3-phenyl)propyl]pipecolic acid methyl ester (from step 1) in 5 ml of tetrahydrofuran at -78°C (Reaction 30, Fig. 4). After stirring at -78°C for 0.5 h and at 20°C for 0.5 h, the reaction was quenched by adding 5 ml 10% ammonium chloride solution at 0°C. The mixture was extracted with methylene chloride, the solvent evaporated and the residue purified by preparative TLC with 40% ethyl acetate in hexanes to give 0.072 g (yield 56%) N-[(3,3-diphenyl-3-hydroxy)propyl]pipecolic acid methyl ester (compound B18) as a pale yellow oil.

### Example 20B - N-[3-(4-Chlorophenyl)-3-(4-fluorophenyl)-3-hydroxypropyl]pipecolic acid methyl ester (Compound B30)

Step 1: N-[3-(4-Fluorophenyl)-3-oxopropyl]pipecolic acid methyl ester was prepared in 92% yield by alkylation of methyl pipecolinate with 3-chloro-4'-fluoropropiophenone (Aldrich) as described in Example 20A (Step 1).

Step 2: N-[3-(4-Chlorophenyl)-3-(4-fluorophenyl)-3-hydroxypropyl]pipecolic acid methyl ester (Compound B30): 7ml (2 mmol) of 0.28 M solution of 4-chlorophenylmagnesium iodide in diethyl ether [prepared from 1-chloro-4-iodobenzene (Aldrich) and magnesium] was added dropwise to an ice-cooled solution of 0.605 g (2 mmol) N-[3-(4-fluorophenyl)-3-oxopropyl]pipecolic acid methyl ester (from Step 1) in 12 ml anhydrous diethyl ether with stirring under nitrogen. The mixture was stirred at room temperature for 16 hours, poured onto crushed ice and extracted with dichloromethane. The combined organic extracts were washed with brine, concentrated and the residue purified by preparative silica gel TLC with 25% ethyl acetate in hexanes to give 0.037 g (yield 4.5%) N-[3-(4-chlorophenyl)-3-(4-fIuorophenyl)-3-hydroxypropyl]pipecolic acid methyl ester (Compound B30).

Compound B21 was prepared in 4% yield analogously to Step 2 by reaction of N-(3-oxo-3-phenylpropyl)pipecolic acid methyl ester [synthesized analogously to Step I of Example 20A from ethyl pipecolinate (Aldrich)] with 4-chlofophenylmagnesium iodide.

### Example 20C - N-[3-(4-Chloronhenyl)-3-(4-fluorouhenyl)prop-2-enyl]pipecolic acid methyl ester (Compound B20)

A solution of 0.035 g (0.086 mmol) N-[3-(4-chlorophenyl)-3-(4 fluorophenyl)-3-hydroxypropyl]pipecolic acid methyl ester (Compound B30) in I ml 99% formic acid was heated under reflux for 0.5 hours. The mixture was concentrated under vacuum, the residue dissolved in ethyl acetate, washed with saturated sodium bicarbonate solution and brine, and the solvent evaporated. The residue was purified by preparative silica gel TLC with 5% diethyl ether in dichlorometane to give 0.018 g (yield 54%) N-[3-(4-chlorophenyl)-3-(4-fluorophenyl)prop-2-enyl]pipecolic acid methyl ester (Compound B20)

### Example 21A - Synthesis of N-[3-Phenyl-3-(p-trifluoromethylphenoxy)propyl]pipecolic acid methyl ester (Compound B16)

Step 1: 0.70 ml of lithium tri-tert-butoxyaluminohydride (Aldrich) (1 M in THF) was added into a solution of 0.190 g, (0.69 mmol) N-[(3-oxo-3-phenyl)propyl]pipecolic acid methyl ester (prepared in step 1 of Example 20A) in 10 ml of THF at -78°C (Reaction 31, Fig. 4). After stirring at -78°C for 0.5 h and at room temperature for 20 h, the reaction was quenched by adding 10 ml 10% ammonium chloride solution at 0°C, filtered, and extracted with methylene chloride. After evaporation of the solvent, the residue was chromatographed on silica gel column with 30% ethyl acetate in hexanes to give 0.171 g (yield 89%) N-[(3-hydroxy-3-phenyl)propyl]pipecolic acid methyl ester as a pale yellow oil.

Step 2: To an ice cooled solution of 2.27 g (8.2 mmol) of N-[(3-hydroxy-3-phenyl)propyl]pipecolic acid methyl ester (from step 1) in 10 ml anhydrous methylene chloride was added dropwise 4 ml (51 mmol) thionyl chloride and the mixture heated under reflux for one hour (Reaction 32, Fig. 4). After addition of crushed ice, the reaction mixture was neutralized with saturated solution of potassium carbonate and extracted with methylene chloride. The combined extracts were evaporated and the residue chomatographed on silica gel column with 20% diethyl ether in hexanes to give 1.45 g (yield 60%) N-[(3-chloro-3-phenyl)propyl]pipecolic acid methyl ester as an oil.

Step 3: A solution of 0.082 g (0.28 mmol) of N-[(3-chloro-3-phenyl)propyl]pipecolic acid methyl ester (from step 2) in 1 ml of anhydrous dimethylformamide was added into a solution of sodium 4-trifluoromethylphenoxide in 2ml anhydrous dimethylformamide at room temperature (Reaction 33, Fig. 4). The sodium 4-trifluoromethylphenoxide was generated by adding 0.040 g of 60% sodium hydride in mineral oil to a solution of 0.165 g (1 mmol) of α,α,α-trifluoro-p-cresol (Aldrich) in 2 ml of dimethylformamide. The reaction mixture was stirred at room temperature for 30 h, the solvent evaporated under vacuo and the residue purified by preparative TLC with 30% ethyl acetate in hexanes to give 0.079 g (yield 68%) N-(3-phenyl-3-(p-trifluoromethylphenoxy)propyl]pipecolic acid methyl ester (Compound B 16) as a pale yellow oil.

### Example 21B - additional Syntheses Usine the Procedure of Example 21A

Compound B23 was prepared by alkylation of 4-trifluoromethylphenol (Aldrich) with N-(3-chloro-3-phenylpropyl)pipecolic acid ethyl ester as described above in Example 21A (Step 3) - yield 6.5%.

Compound B24 was prepared by alkylation of phenol (Aldrich) with N-(3-chloro-3-phenylpropyl)pipecolic acid ethyl ester as described above in Example 21A (Step 3) - yield 4%.

Compound B25 was prepared by alkylation of 4-methoxyphenol (Aldrich) with N-(3-chloro-3-phenylpropyl)pipecolic acid ethyl ester as described above in Example 21A (Step 3) - yield 8%.

Compound B29 was prepared by alkylation ofthiophenol (Aldrich) with N-(3-chloro-3-phenylpropyl)pipecolic acid ethyl ester as described above in Example 21A (Step 3) - yield 12%.

### Example 21C - Synthesis of N-13-(4-chloror)henoxy)-3-phenylpropyl]pipecolic acid ethyl ester (Compound B22)

0.133g (0.76 mmol) diethyl azodicarboxylate (Aldrich) was added dropwise to a solution of 0.142 g (0.51 mmol) N-(3-hydroxy-3-phenylpropyl)pipecolic acid methyl ester (from Example 21A, Step 1), 0.083 g (0.64 mmol)*p*-chlorophenol (Aldrich) and 0.197 g (0.75 mmol) triphenylphosphine in 5 ml anhydrous tetrahydrofuran with stirring under nitrogen and cooling with an ice bath. The mixture was stirred at room temperature for 4 hours, the solvent evaporated and the residue purified by preparative silica gel TLC with 30% ethyl acetate in hexanes to give 0.09 g (yield 46%) N-[3-(4-chlorophenoxy)-3-phenylpropyl]pipecolic acid ethyl ester (Compound B22). (See Reaction 34, Figure 4.)

### Example 22 - Synthesis of N-(4,4-Diphenyl)butyl-2-piperidine carboxylic acid methyl ester (compound B10)

0.040 g (0.11 mmol) of N-[4,4-diphenyl)but-3-enyl]-2-piperidine carboxylic acid methyl ester (compound B4) was hydrogenated over 0.030 g 10%Pd/C in 5 ml ethanol under 40 psi for 4 hours at room temperature. The mixture was separated from the catalyst by filtration through celite and the solvent evaporated to give 0.028 g (yield 70%) N-(4,4-diphenyl)butyl-2-piperidine carboxylic acid methyl ester (compound B10) as an oil. NMR spectra of the product showed: ¹H NMR (CDCl₃, 300 MHz) 7.40 - 7.10 (m, 10H), 3.88 (t, 1 H), 3.65 (s, 3H), 3.10 - 2.90 (m, 2 H), 2.60 - 2.45 (m, 1 H), 2.35 - 2.20 (m, 1H), 2.10 - 1.90 (m, 3 H), 1.85 - 1.10 (m, 8 H); ¹³C NMR (CDCl₃, 75 MHz) 174.57, 145.36, 145.23, 128.66, 128.12, 128.10, 126.34, 126.33, 65.66, 56.81, 51.78, 51.44, 50.78, 33.81, 29.88, 25.53, 25.39, 22.92.

### Example 23 - Synthesis of N-[(4,4-Diphenyl)but-3-enyl]-L-2-azetidine carboxylic acid hydrochloride (compound B15)

To a solution of 0.050 g (0.3 mmol) of N-[(4,4-diphenyl)but-3-enyl]-L-2-azetidine carboxylic acid methyl ester (compound B3) in 2.4 ml ethanol was added 1.2 ml 1N lithium hydroxide and the mixture was stirred at room temperature for 20 hours. The reaction mixture was concentrated to half volume, acidified with 4 N hydrochloric acid, and extracted 4 times with methylene chloride. The combined extracts were dried and evaporated to give 0.041 g (yield 80%) of N-[(4,4-diphenyl)but-3-enyl]-L-2-azetidine carboxylic acid hydrochloride (compound B15). ¹H NMR (CD₃OD, 300 MHz) 7.50 - 7.00 (m, 10 H), 6.08 (t, 1 H), 4.62 (t, I H), 4.00 - 3.75 (m, 3 H), 3.30 - 3.20 (m, I H), 2.75 - 2.55 (m, 1H), 2.50 - 2.30 (m, 3 H).

Compound B5 was prepared by hydrolysis of the corresponding ester, compound B 14.

Compound B 19 was prepared by hydrolysis of the corresponding ester, compound B23.

## Claims

1. A compound of the following formula: 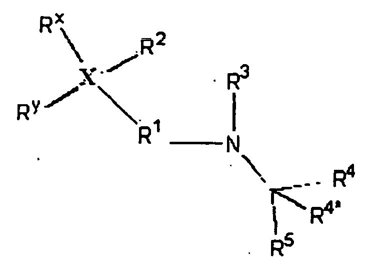 or a pharmaceutically acceptable salt thereof,
wherein:
(1) X is nitrogen or carbon, and R² is not present when X is nitrogen;
(2) R² (a) is hydrogen; (C1-C6) alkyl, (C1-C6) alkoxy, cyano, (C2-C7) alkanoyl, aminocarbonyl, (C1-C6) alkylaminocarbonyl or dialkylaminocarbonyl wherein each alkyl is independently C1 to C6, (b) comprises (where R¹ is not aminoethylene, -O-R⁸ or -S-R⁸*) hydroxy, fluoro, chloro, bromo or (C2-C7) alkanoyloxy, (c) forms a double bond with an adjacent carbon or nitrogen from one of either R¹, R^{xb} or R^{yb}, or (d) is R^{2a} linked by R^{2b} to X, or (e) ethylene forming a third bridging structure as set forth in (2ⁱⁱⁱ)(b)(i);
(2ⁱ) R^{x} is R^{xa} linked by R^{xb} to X;
(2ⁱⁱ) RY is R^{ya} linked by R^{yb} to X;
(2ⁱⁱⁱ) R^{xa}, R^{ya} and R^{2a}, are independently Ar which is aryl or heteroaryl, adamantyl or a 5 to 7-membered non-aromatic ring having from 0 to 2 heteroatoms selected from the group consisting of oxygen, sulfur and nitrogen, wherein at least one of R^{xa}, R^{ya} and R^{2a} is phenyl, and wherein:
(a) aryl is phenyl or naphthyl,
(b) heteroaryl comprises a five-membered ring, a six-membered ring, a six-membered ring fused to a five-membered ring, a five-membered ring fused to a six-membered ring, or a six-membered ring fused to a six-membered ring, wherein the heteroaryl is aromatic and contains heteroatoms selected from the group consisting of oxygen, sulfur and nitrogen, with the remaining ring atoms being carbon,
(c) each of R^{xa}, R^{ya} and R^{2a} can be substituted or independently substituted with one of R^{q}, R^{r}O- or R^{s}S-, wherein each of R^{q}, R^{r} and R^{s} are independently Ar, adamantyl or a 5 to 7-membered non-aromatic ring as these ring structures are defined for R^{xa}, and
(d) R^{xa}, R^{ya}, R^{2a}, R^{q}, R^{r} and R^{s} can be additionally substituted with one or more substituents selected from the group consisting of fluoro, chloro, bromo, nitro, hydroxy, cyano, trifluoromethyl, amidosulfonyl which can have up to two independent (C1-C6) N-alkyl substitutions, adamantyl, (C1-C12) alkyl, (C2-C12) alkenyl, amino, (C1-C6) alkylamino, dialkylamino wherein each alkyl is independently C1 to C6, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be substituted for hydrogen with up to two independent (C1-C6) alkyl, (C1-C6) alkylsulfonyl, amidino that can be independently substituted with up to three (C1-C6) alkyl group, or methylenedioxy or ethylenedioxy with the two oxygens bonded to adjacent positions on the aryl or heteroaryl ring structure, which methylenedioxy or ethylenedioxy can be substituted with up to two independent (C1-C6) alkyl, wherein:
(i.) the substitutions of R^{xa}, R^{ya} and R^{2a} can be combined to form a second bridge between two of R^{xa}, R^{ya} and R^{2a} comprising (1) (C1-C2) alkyl or (C2) alkenyl, which can be independently substituted with one or more (C1-C6) alkyl or by R², wherein R² is ethylene to form a third bridging structure, (2) sulfur, (3) oxygen, (4) amino, which can be substituted for hydrogen with one (C1-C6) alkyl, (5) carbonyl, (6) -CH₂C(=O)-, which can be substituted for hydrogen with up to two independent (C1-C6) alkyl, (7) -C(=O)-O-, (8) -CH₂-O-, which can be substituted for hydrogen with up to two independent (C1-C6) alkyl, (9) -C(=O) N(R²⁴) , wherein R²⁴ is hydrogen or (C1-C6) alkyl, (10) -CH₂-NH-, which can be substituted for hydrogen with up to three (C1-C6) alkyl, or (11) -CH=N-, which can be substituted for hydrogen with (C1-C6) alkyl, or wherein two of R^{xa}, R^{ya} and R^{2a} can be directly linked by a single bond;
(2^{iv}) R^{xb} and R^{2b} are independently a single bond or (C1-C2) alkylene;
(2^{v}) R^{yb} is a single bond, oxo, (C1-C2) alkylene, ethenylene or -CH= (where the double bond is with X), thio, methyleneoxy or methylenethio, or either -N(R⁶) or -CH₂-N(R^{6*})-, wherein R⁶ and
R^{6*} are hydrogen or (C1-C6) alkyl, wherein when X is nitrogen X is not bonded to another heteroatom;
(3) R¹ comprises: a straight-chained (C2-C3) aliphatic group; where X is carbon, =N-O-(ethylene), wherein the unmatched double bond is linked to X, in which X is carbon and Ryb does not include a heteroatom attached to X, -O-R⁸ or -S-R^{8*} wherein R⁸ or R^{8*}is a ethylene or ethenylene and O or S is bonded to X, in which X is carbon and Ryb does not include a heteroatom attached to X; aminoethylene where the amino is bonded to X:
wherein R¹ can be substituted with up to one hydroxy, up to one (C1-C6) alkoxy or up to one (C2-C7) alkanoyloxy, with up to two independent (C1-C6) alkyl, with up to one oxo, up to one (C1-C6) alkylidene, with the proviso that the hydroxy, alkoxy, alkanoyloxy or oxo substituents are not bonded to a carbon that is bonded to a nitrogen or oxygen;
wherein the alkyl or alkylidene substituents of R¹ can be linked to form a 3 to 7-membered non-aromatic ring; and
wherein if X is nitrogen, X is linked to R¹ by a single bond and the terminal carbon of R¹ that links R¹ to N is saturated;
(4) R³ is hydrogen, (C1-C6) alkyl, or phenyl or phenylalkyl wherein the alkyl is C1 to C6 and either such phenyl can be substituted with the same substituents defined above for the aryl or heteroaryl of R^{xa} ; and (5) R4 and R4* are hydrogen; and
(6) R⁵ is (CO)NR¹³R¹⁴, (CO)OR¹⁵, (CO)SR¹⁶, (SO₂)NR¹⁷R¹⁸, (PO)(OR¹⁹)(OR²⁰), (CR²²)(OR²³)(OR²⁴), CN or tetrazol-5-yl, wherein (a) R¹³, R¹⁴, R¹⁵, R¹⁶, R¹⁷, R¹⁸ R¹⁹ and R²⁰ are independently hydrogen, (C1-C8) alkyl which can include a (C3-C8) cycloalkyl, wherein the carbon linked to the oxygen of R¹⁵ or the sulfur of R¹⁶ has no more than secondary branching and , ^{l}(C2-C6) hydroxyalkyl, aminoalkyl where the alkyl is C2 to C6 and the amino can be substituted with up to two independent (C1-C6) alkyls, Ar-alkyl wherein the alkyl is C1-C6 or Ar, (b) R²² is hydrogen or OR²⁵ and (c) R²³ , R²⁴ and R²⁵ are (C1-C6) alkyl, phenyl, benzyl, acetyl or, where R²² is hydrogen, the alkyls of R²³ and R²⁴ can be combined to include 1,3-dioxolane or 1,3-dioxane:
wherein the Ar groups of R¹³, R¹⁴, R¹⁵, R¹⁶, R¹⁷, R¹⁸, R¹⁹, R²⁰, R²², R²³ or R²⁴ can be substituted with substituents selected from the group consisting of fluoro, chloro, bromo, nitro, cyano, hydroxy, trifluoromethyl, amidosulfonyl which can have up to two independent (C1-C6) N-alkyl substitutions, (C1-C6) alkyl, (C2-C6) alkenyl, (C1-C6) alkylamine, dialkylamine wherein each alkyl is independently C1 to C6, amino, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be N-substituted with up to two independent (C1-C6) alkyl, (C1-C6) alkylsulfonyl, amidino that can substituted with up to three (C1-C6) alkyl, or methylenedioxy or ethylenedioxy with the two oxygens bonded to adjacent positions on the aryl or heteroaryl ring structure, which methylenedioxy or ethylenedioxy can be substituted with up to two independent (C1-C6) alkyl;
wherein R¹³ and R¹⁴ together with the nitrogen can form a 5 to 7-membered ring that can contain one additional heteroatom selected from oxygen and sulfur, and wherein the following provisos apply:
if R¹⁵ is hydrogen and R¹ is propylene, then at least one of the following applies (1) both R^{x} and R^{y} are notp-fluorophenyl, (2) one of R^{x} and R^{y} includes a heteroaryl, (3) RY is Ar-(C1-C2)alkyl, Ar-oxy, Ar-methoxy, Ar-thio, Ar-methylthio, Ar-N(R⁶)- or Ar-CH₂-N(R^{6*})-, (4) R² is R^{2a}R^{2b}-, (5) R² is not hydrogen, or (6) R³ is not hydrogen
if R¹⁵ is hydrogen and R¹ is ethylene or X-R¹ is prop-1-enylene, then at least one of the following applies (1) an aryl of at least one of R^{x} and R^{y} is substituted with a radical different from hydrogen, (2) one of R^{x} and RY comprises a heteroaryl, (3) RY is Ar-(C1-C2)alkyl, Ar-oxy, Ar-methoxy, Ar-thio, Ar-methylthio, Ar-N(R⁶)- or Ar-CH₂₋N(R^{6*})-, (4) R² is R^{2a} R^{2b}-, (5) R^{2*} is not hydrogen, or (6) R³ is not hydrogen;
if R⁵ is C(O)NR¹³R¹⁴, wherein R¹³ and R¹⁴ are hydrogen, (C1-C8)alkyl, phenyl or substituted phenyl, then at least one of the following applies (1) an aryl of at least one of R^{x} and R^{y} is substituted with a radical different from hydrogen, (2) one of R^{x} and RY comprises a heteroaryl, (3) RY is Ar-(C1-C2)alkyl, Ar-oxy, Ar-methoxy, Ar-thio, Ar-methylthio, Ar-N(R⁶)- or Ar-CH₂-N(R⁶*)-, (4) R² is R^{2a} R^{2b}-, (5) R²* is not hydrogen, (6) R³ is not hydrogen, or (7) R¹ is not ethylene;
wherein if R² is phenyl or *p*-methylphenyl, then at least one of the following applies (1) the aryls of R^{x} and R^{y} are not substituted with *p*-methylphenyl or *p*-methoxyphenyl, (2) an aryl of at least one of R^{x} and RY is substituted with a radical different from hydrogen, (3) one of R^{x} and RY comprises a heteroaryl, (4) RY is Ar-(C1-C2)alkyl, Ar-oxy, Ar-methoxy, Ar-thio, Ar-methylthio, Ar-N(R⁶)- or Ar-CH₂ N(R^{6*})-, or (5) R¹ is not aminoethylene, OR⁸ or SR⁸*;
wherein if R^{2a} is *p*-methoxyphenyl, then at least one of the following applies (1) an Ar of at least one of R^{x} and RY is substituted with a radical different from hydrogen, (2) RY is Ar-(C1-C2)alkyl, Ar-oxy, Ar-methoxy, Ar-thio, Ar-methylthio, Ar-N(R⁶)- or Ar-CH₂-N(R^{6*})-, or (3) R¹ is not OR⁸ or SR^{8*}.

2. A compound according to claim 1 of the following formula: 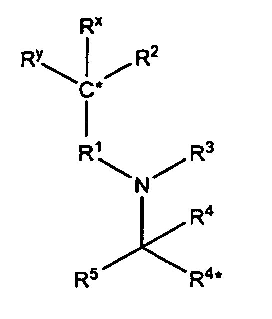 or a pharmaceutically acceptable salt thereof,
wherein:
(1) C* is a substituted carbon;
(2) R² (a) is hydrogen, (C1-C6) alkyl, (C1-C6) alkoxy, cyano, (C2-C7) alkanoyl, aminocarbonyl, (C1-C6) alkylaminocarbonyl, or dialkylaminocarbonyl wherein each alkyl is independently C1 to C6, (b) comprises (where R¹ is not aminoethylene, -O-R⁸ or -S-R⁸*) hydroxy, fluoro, chloro, bromo or (C2-C7) alkanoyloxy, (c) forms a double bond with an adjacent carbon or nitrogen from one of either R¹, R^{xb}, R^{yb}, (d) is R^{2a} linked by R^{2b} to C*, or (e) ethylene forming a third bridging structure as set forth in (2ⁱⁱⁱ)(b)(i);
(2ⁱ) R^{x} is R^{xa} linked by R^{xb} to C*;
(2ⁱⁱ) R^{y} is R^{ya} linked by R^{yb} to C*;
(2ⁱⁱⁱ) R^{xa}, R^{ya} and R^{2a}, are independently Ar, which is phenyl or naphthyl, or a 5 to 7-membered non-aromatic ring having 0 heteroatoms wherein:
(a) each of R^{xa} and R^{ya} can be independently substituted with one of R^{q}, R^{r}O- or R^{s}S-, wherein each of R^{q}, R^{r} and R^{s} are independently Ar or adamantly, and
(b) R^{xa}, R^{ya}, R^{2a}, R^{q}, R^{r} and R^{s} can be substituted or additionally substituted with one or more substituents selected from the group consisting of fluoro, chloro, bromo, nitro, hydroxy, cyano, trifluoromethyl, amidosulfonyl which can have up to two independent (C1-C6) N-alkyl substitutions, (C1-C12) alkyl, (C2-C12) alkenyl, amino, (C1-C6) alkylamino, dialkylamino wherein each alkyl of dialkylamino is independently C1 to C6, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be substituted for hydrogen with up to two independent (C1-C6) alkyl, (C1-C6) alkylsulfonyl, or amidino, that can independently substituted with up to three (C1-C6) alkyl, wherein:
(i.) the substitutions of R^{xa} and R^{ya} can be combined to form a second bridge between R^{xa} and R^{ya} comprising (1) methylene or ethylene, which methylene or ethylene can be substituted by an R² when R² is ethylene to form the third bridging structure, or, (2) -CH=CH- or wherein R^{xa} and R^{ya} can be directly linked by a single bond;
(2^{iv}) R^{xb} and R^{2b} are independently a single bond or (C1-C2) alkylene; .
(2^{v}) R^{yb} is a single bond, oxa, (C1-C2) alkylene, ethenylene or -CH= (where the double bond is with C*), thia, methyleneoxy or methylenethio, or either -N(R⁶) or -CH₂-N(R⁶*)-, wherein R⁶ and R⁶* are hydrogen or (C1-C6) alkyl;
(3) R' comprises: a straight-chained (C2-C3) aliphatic group; =N-O-(ethylene), wherein the unmatched double bond is linked to C*; -O-R⁸ or -S-R⁸* wherein R⁸ or R⁸* is a ethylene or ethenylene and O or S is bonded to C*; aminoethylene where the amino is bonded to C*:
wherein R¹ can be substituted with up to one hydroxy, up to one (C1-C6) alkoxy or up to one (C2-C7) alkanoyloxy, with up to two independent (C1-C6) alkyl, with up to one oxo, up to one (C1-C6) alkylidene, with the proviso that the hydroxyl, alkoxy, alkanoyloxy or oxo substituents are not bonded to a carbon that is bonded to a nitrogen or oxygen; and
wherein the alkyl or alyklidene substituents of R¹ can be linked to form a 3 to 7-membered non aromatic ring;
(4) R³ (a) is hydrogen, (C1-C6) alkyl, or phenyl or phenylalkyl wherein the alkyl is C1 to C6 and the phenyl or phenyl of phenylalkyl can be substituted with the same substituents defined above for the phenyl of R^{xa}, (b) is -R¹²C(R^{xx})(R^{yy})(R¹¹) ,wherein R¹² is bonded to N, R^{xx} is independently the same as R^{x}, R^{yy} is independently the same as R^{y}, R¹¹ is independently the same as R² and R¹² is independently the same as R¹;
(5) R⁴ and R⁴* are independently hydrogen and
(6) R⁵ is (CO)NR¹³R¹⁴, (CO)OR¹⁵, (CO)SR¹⁶, (SO₂)NR¹⁷R¹⁸, (PO)(OR¹⁹)(OR²⁰), (CR²²)(OR²³)(OR²⁴), CN or tetrazol-5-yl, wherein (a) R¹³, R¹⁴, R¹⁵,R¹⁶, R¹⁷, R¹⁸ R¹⁹ and R²⁰ are independently hydrogen, (C1-C8) alkyl which can include a (C3-C8) cycloalkyl, wherein the carbon linked to the oxygen of R¹⁵ or the sulfur of R¹⁶ has no more than secondary branching and, (C2-C6) hydroxyalkyl, aminoalkyl where the alkyl is C2 to C6 and the amino can be substituted with up to two independent (C1-C6) alkyls, Ar-alkyl wherein the alkyl is C1-C6 or Ar, and (b) R²² is hydrogen or OR²⁵ and R²³, R²⁴ and R²⁵ are (C1-C6) independently alkyl, phenyl, benzyl or acetyl or, the alkyls of R²³ and R²⁴ can be combined to include 1,3-dioxolane or 1,3-dioxane:
wherein the phenyl or naphthyl groups of R¹³, R¹⁴, R¹⁵, R¹⁶, R¹⁷, R¹⁸, R¹⁹, R²⁰, R²², R²³ or R²⁴ can be substituted with substituents selected from the group consisting of fluoro, chloro, bromo, nitro, cyano, hydroxy, trifluoromethyl, amidosulfonyl which can have up to two independent (C1-C6) N-alkyl substitutions, (C1-C6) alkyl, (C2-C6) alkenyl, (C1-C6) alkylamine, dialkylamine wherein each alkyl is independently C1 to C6, amino, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be N-substituted with up to two independent (C1-C6) alkyl, (C1-C6) alkylsulfonyl, or amidino that can substituted with up to three (C1-C6) alkyl;
wherein R¹³ and R¹⁴ together with the attached nitrogen can form a 5 to 7-membered ring; and wherein further the following provisos apply:
if R¹⁵ is hydrogen and R¹ is propylene, then at least one of the following applies (1) both R^{xa} and R^{ya} are not *p*-fluorophenyl, (2) R^{y} is Ar-alkyl, Ar-oxy, Ar-methoxy, Ar-thio, Ar-methylthio, Ar-N(R⁶)- or Ar-CH₂-N(R^{6*})-, (3) R² is R^{2a} R^{2b}-, (4) R² is not hydrogen, or (5) R³ is not hydrogen;
if R¹⁵ is hydrogen and R¹ is ethylene or C*R¹ is prop-1-enylene, then at least one of the following applies (1) an aryl of at least one of R^{xa} and R^{ya} is, substituted with a radical different from hydrogen, (2) R^{y} is Ar-alkyl, Ar-oxy, Ar-methoxy, Ar-thio, Ar-methylthio, Ar-N(R⁶)- or Ar-CH₂-N(R^{6*})-, (3) R² is R^{2a} R^{2b}-, (4) R² is not hydrogen, or (5) R³ is not hydrogen;
if R⁵ is C(O)NR¹³R¹⁴, wherein R¹³ and R¹⁴ are hydrogen, (C1-C8)alkyl, phenyl or substituted phenyl, then at least one of the following applies (1) an aryl of at least one of R^{x} and R^{y} is substituted with a radical different from hydrogen, fluoro, chloro, or bromo (2) R^{y} is Ar-alkyl, Ar-oxy, Ar-methoxy, Ar-thio, Ar-methylthio, Ar-N(R⁶)- or Ar-CH₂-N(R^{6*})-, (3) R² is R^{xa} R^{2a} R^{2b}-, (4) R²is not hydrogen, (5) R³ is not hydrogen, or (6) R¹ is not ethylene;
if R^{2a} is phenyl or *p*-methylphenyl, then at least one of the following applies (1) the aryls of R^{x} and R^{y} are not substituted with *p*-methylphenyl or *p*-methoxyphenyl, (2) an aryl of at least one of R^{x} and R^{y} is substituted with a radical different from hydrogen, (3) R^{y} is Ar-alkyl, Ar-oxy, Ar-methoxy, Ar-thio, Ar-methylthio, Ar-N(R⁶)- or Ar-CH₂-N(R⁶*)-, or (5) R¹ is not aminoethylene, OR⁸ or SR⁸*;
if R^{2a} is *p*-methoxyphenyl, then at least one of the following applies (1) an Ar of at least one of R^{x} and R^{y} is substituted with a radical different from hydrogen, (2) R^{y} is Ar-(C1-C2)alkyl, Ar-oxy, Ar-methoxy, Ar-thio, Ar methylthio, Ar-N(R⁶)-, or Ar-CH2N(R⁶*)-, or (3) R¹ is not - OR⁸ or -SR⁸*.

3. The compound of claim 1 or 2, wherein (A) at least one of R^{xa}, R^{ya} and R^{2a} is substituted with fluoro, chloro, bromo, hydroxy, trifluoromethyl, trifluoromethoxy, nitro, cyano, (C3-C8) alkyl, R^{q}, R^{r}O-, R^{s}S-, (B) R³ is hydrogen, (C1-C6) alkyl, or phenyl or phenylalkyl wherein the alkyl is C1 to C6 and either such phenyl can be substituted with the same substituents defined above for the aryl or heteroaryl of R^{xa} or (C) the ring structures of R^{xa}, R^{ya} and R^{2a}, including substituents thereto, otherwise include at least two aromatic ring structures that together include from 15 to 20 ring atoms.

4. The compound of claim 4, wherein at least one of R^{xa}, R^{ya} and R^{2a} is substituted with fluoro, trifluoromethyl, trifluoromethoxy, nitro, cyano, or (C3-C8) alkyl.

5. The compound of claim 1 or 2, wherein at least one of R^{xa}, R^{ya} and R^{2a} is substituted with R^{q}, R^{r}O-, or R^{s}S-.

6. The compound of claim 1 or 2, wherein R^{yb} is oxy, methyleneoxy, thio or methylenethio.

7. The compound of claim 6, wherein R^{yb} is oxy or thio.

8. The compound of claim 1 or 2, wherein R⁵ is (CO)NR¹³R¹⁴, (CO)OR¹⁵ or (CO)SR¹⁶.

9. The compound of claim 8, wherein R¹⁵ is (C2-C6) alkyl, (C2-C4) hydroxyalkyl, phenyl, phenylalkyl wherein the alkyl is C1-C3, or aminoalkyl where the alkyl is C2-C6 and the amino can be substituted with up to two independent (C1-C3) alkyls, wherein the phenyl or the phenyl of phenylalkyl can be substituted.

10. The compound of claim 8, wherein R¹⁵ is hydrogen.

11. The compound of claim 1 or 2, wherein R⁴ is hydrogen, methyl or hydroxymethyl and R^{4*} is hydrogen.

12. The compound of claim 1, wherein at least one of R^{xa}, R^{ya} and R^{2a} is a heteroaryl comprising diazolyl, triazolyl, tetrazolyl, thiazolyl, isothiazolyl, oxazolyl, isoxazolyl, thiolyl, diazinyl, triazinyl, benzoazolyl, benzodiazolyl, benzothiazolyl, benzoxazolyl, benzoxolyl, benzothiolyl, quinolyl, isoquinolyl, benzodiazinyl, benzotriazinyl, pyridyl, thienyl, furanyl, pyrrolyl, indolyl, isoindoyl or pyrimidyl.

13. The compound of claim 1 or 2, wherein R¹ is -O-R⁸ or -S-R^{8*}.

14. The compound of claim 1 or 2, wherein said second bridge between two of R^{xa}, R^{ya} and R^{2a} is L, and satisfies the following formula: 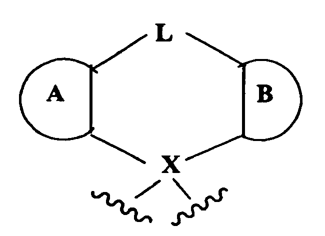 , wherein A and B are aryl or heteroaryl groups of R^{xa} and R^{ya}, respectively.

15. The compound of claim 12, wherein R^{xa}-R^{xb}-, R^{ya}-R^{yb}- and X form: 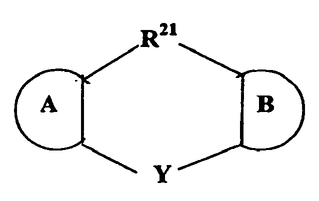 wherein Y is a carbon bonded to R¹ by a single or double bond or a nitrogen that is bonded to R¹ and wherein R²¹ either (i.) completes a single bond linking two aryl or heteroaryl rings of R^{x} and R^{y}, (ii.) is (C1-C2) alkylene or alkenylene, (iii.) is sulfur or (iv.) is oxygen, and wherein R^{x} and R^{y} can be substituted as set forth above.

16. The compound of claim 15, wherein R²¹ is CH₂CH₂ or CH=CH.

17. The compound of claim 1, wherein the alkylenedioxy substitution of R^{xa}, R^{ya} or R^{2a} is as follows: 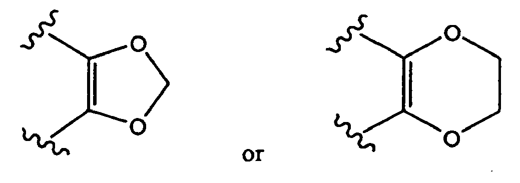 wherein the alkylenedioxy can be substituted with up to two independent (C1-C3) alkyl.

18. The compound of claim 1 or 2, wherein R^{xa} and R^{ya} together can be substituted with up to six substituents, R^{2a}, R^{q}, R^{r} and R^{s} can each be substituted with up to 3 substituents, and wherein the presence of each of R^{q}, R^{r} or R^{s} is considered a substitution to the respective ring structure of R^{xa}, R^{ya} and R^{2a}.

19. The compound of claim 1 or 2, wherein a phenyl of R³ is substituted with up to three substituents.

20. The compound of claim 1 or 2, wherein the aryl, heteroaryl, aryl of arylalkyl or the heteroaryl of heteroarylalkyl of R¹³, R¹⁴, R¹⁵, R¹⁶ R¹⁷, R¹⁸ R¹⁹ or R²⁰ is substituted with up to three substituents.

21. The compound of claim 1 or 2, wherein the compound is an optically pure enantiomer.

22. The compound of claim 1 or 2, wherein:
(1) R² is hydrogen,
(2) R^{xa} and R^{ya} are both phenyl and at least one of R^{xa} and R^{ya} is substituted with one of phenyl, phenoxy, or phenylthio,
(3) R^{xb} is a single bond and R^{yb} is a single bond or oxa, and
(4) R⁵ is (CO)NR¹³R¹⁴ or (CO)OR¹⁵, wherein R¹³, R¹⁴, and R¹⁵ are independently hydrogen; (C1-C8) alkyl which can include a (C3-C8) cycloalkyl, wherein the carbon linked to the oxygen of OR¹⁵ has no more than secondary branching; (C2-C6) hydroxyalkyl or aminoalkyl where the alkyl is C2 to C6 and the amino can be substituted with up to two independent (C1-C6) alkyls or phenylalkyls, wherein the alkyl is C1-C6 and the phenyl can be substituted with substituents selected from the group consisting of fluoro, chloro, bromo, nitro, cyano, hydroxy, trifluoromethyl, amidosulfonyl which can have up to two independent (C1-C6) N-alkyl substitutions, (C1-C6) alkyl, (C2-C6) alkenyl, (C1-C6) alkylamine, dialkylamine
wherein each alkyl is independently C1 to C6, amino, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be N-substituted with up to two independent (C1-C6) alkyl, (C1-C6) alkylsulfonyl, amidino that can substituted with up to three (C1-C6) alkyl.

23. A pharmaceutical composition comprising the compound of claim 1 or 2 and a pharmaceutically acceptable excipient.

24. The pharmaceutical composition of claim 23, wherein the compound of claim 1 or 2 is present in an effective amount for:
(1) treating or preventing schizophrenia,
(2) enhancing treating or preventing dementia,
(3) treating or preventing epilepsy,
(4) treating or preventing spasticity,
(5) treating or preventing muscle spasm,
(6) treating or preventing pain,
(7) preventing neural cell death after stroke,
(8) preventing neural cell death in an animal suffering from a neurodegenerative disease,
(9) treating or preventing mood disorders,
(10) enhancing memory or learning, or
(11) treating or preventing learning disorders.

25. Use of a compound of formula: 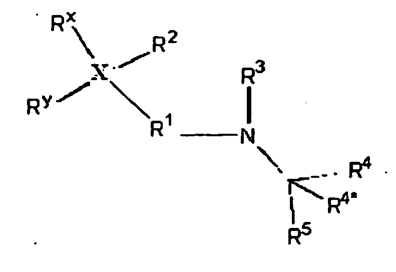 or a pharmaceutically acceptable salt thereof,
wherein:
(1) X is nitrogen or carbon, and R² is not present when X is nitrogen;
(2) R² (a) is hydrogen, (C1-C6) alkyl, (C1-C6) alkoxy, cyano, (C2-C7) alkanoyl, aminocarbonyl, (C1-C6) alkylaminocarbonyl or dialkylaminocarbonyl wherein each alkyl is independently C1 to C6, (b) comprises (where R¹ is not aminoethylene, -O-R⁸ or -S-R⁸*) hydroxy, fluoro, chloro, bromo or (C2-C7) alkanoyloxy, (c) forms a double bond with an adjacent carbon or nitrogen from one of either R¹, R^{xb} or R^{yb}, or (d) is R^{2a} linked by R^{2b} to X, or (e) ethylene forming a third bridging structure as set forth in (2ⁱⁱⁱ)(b)(i);
(2ⁱ) R^{x} is R^{xa} linked by R^{xb} to X;
(2ⁱⁱ) R^{y} is R^{ya} linked by R^{yb} to X;
(2ⁱⁱⁱ) R^{xa}, R^{ya} and R^{2a}, are independently Ar which is aryl or heteroaryl, adamantyl or a 5 to 7-membered non-aromatic ring having from 0 to 2 heteroatoms selected from the group consisting of oxygen, sulfur and nitrogen, wherein at least one of R^{xa}, R^{ya} and R^{2a} is phenyl, and wherein:
(a) aryl is phenyl or naphthyl,
(b) heteroaryl comprises a five-membered ring, a six-membered ring, a six-membered ring fused to a five-membered ring, a five-membered ring fused to a six-membered ring, or a six-membered ring fused to a six-membered ring, wherein the heteroaryl is aromatic and contains heteroatoms selected from the group consisting of oxygen, sulfur and nitrogen, with the remaining ring atoms being carbon,
(c) each of R^{xa}, R^{ya} and R^{2a} can be substituted or independently substituted with one of R^{q}, R^{r}O- or R^{s}S-, wherein each of R^{q}, R^{r} anf R^{s} are independently Ar, adamantly or a 5 to 7-membered non-aromatic ring as these ring structures are defined for R^{xa}, and
(d) R^{xa}, R^{ya}, R^{2a}, R^{q}, k^{r} and R^{s} can be additionally substituted with one or more substituents selected from the group consisting of fluoro, chloro, bromo, nitro, hydroxy, cyano, trifluoromethyl, amidosulfonyl which can have up to two independent (C1-C6) N-alkyl substitutions, adamantyl, (C1-C12) alkyl, (C2-C 12) alkenyl, amino, (C1-C6) alkylamino, dialkylamino wherein each alkyl is independently C1 to C6, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be substituted for hydrogen with up to two independent (C1-C6) alkyl, (C1-C6) alkylsulfonyl, amidino that can be independently substituted with up to three (C1-C6) alkyl group, or methylenedioxy or ethylenedioxy with the two oxygens bonded to adjacent positions on the aryl or heteroaryl ring structure, which methylenedioxy or ethylenedioxy can be substituted with up to two independent (C1-C6) alkyl, wherein:
(i.) the substitutions of R^{xa}, R^{ya} and R^{2a} can be combined to form a second bridge between two of R^{xa}, R^{ya} and R^{2a} comprising (1) (C1-C2) alkyl or (C2) alkenyl, which can be independently substituted with one or more (C1-C6) alkyl or by R², wherein R² is ethylene to form a third bridging structure, (2) sulfur, (3) oxygen, (4) amino, which can be substituted for hydrogen with one (C1-C6) alkyl, (5) carbonyl, (6) -CH₂C(=0)-, which can be substituted for hydrogen with up to two independent (C1-C6) alkyl, (7) -C(=0)-0-, (8) -CH₂-0-, which can be substituted for hydrogen with up to two independent (C1-C6) alkyl, (9) -C(=0) N(R²⁴), wherein R²⁴ is hydrogen or (C1-C6) alkyl, (10) -CH₂-NH-, which can be substituted for hydrogen with up to three (C1-C6) alkyl, or (11) -CH=N-, which can be substituted for hydrogen with (C1-C6) alkyl, or wherein two of R^{xa}, R^{ya} and R^{2a} can be directly linked by a single bond;
(2^{iv}) R^{xb} and R^{2b} are independently a single bond or (C1-C2) alkylene;
(2^{v}) R^{yb} is a single bond, oxo, (C1-C2) alkylene, ethylene or -CH= (where the double bond is with X), thio, methyleneoxy or methylenethio, or either -N(R⁶) or -CH₂-N(R⁶*)-, wherein R⁶ and R⁶* are hydrogen or (C1-C6) alkyl, wherein when X is nitrogen X is not bonded to another heteroatom;
(3) R¹ comprises: a straight-chained (C2-C3) aliphatic group; where X is carbon, =NO-(ethylene), wherein the unmatched double bond is linked to X; (where X is carbon and R^{yb} does not include a heteroatom attached to X), -O-R⁸ or -S-R⁸* wherein R⁸ or R⁸* is a ethylene or ethenylene and O or S is bonded to X; (where X is carbon and R^{yb} does not include a heteroatom attached to X), aminoethylene where the amino is bonded to X:
wherein R¹ can be substituted with up to one hydroxy, up to one (C1-C6) alkoxy or up to one (C2-C7) alkanoyloxy, with up to two independent (C1-C6) alkyl, with up to one oxo, up to one (C1-C6) alkylidene, with the proviso that the hydroxy, alkoxy, alkanoyloxy or oxo substituents are not bonded to a carbon that is bonded to a nitrogen or oxygen;
wherein the alkyl or alkylidene substituents of R¹ can be linked to form a 3 to 7-membered non-aromatic ring; and
wherein if X is nitrogen, X is linked to R¹ by a single bond and the terminal carbon of R¹ that links R¹ to N is saturated;
(4) R³ is hydrogen, (C1-C6) alkyl, or phenyl or phenylalkyl wherein the alkyl is C1 to C6 and either such phenyl can be substituted with the same substituents defined above for the aryl or heteroaryl of R^{xa},
(5) R⁴ and R^{4*} are independently hydrogen;
(6) R⁵ is (CO)NR¹³R¹⁴, (CO)OR¹⁵, (CO)SR¹⁶, (SO₂)NR¹⁷R¹⁸, (PO)(OR¹⁹)(OR²⁰), (CR²²)(OR²³)(OR²⁴), CN or tetrazol-5-yl, wherein (a) R¹³, R¹⁴, R¹⁵, R¹⁶, R¹⁷ , R¹⁸ R¹⁹ and R²⁰ are independently hydrogen, (C1-C8) alkyl which can include a (C3-C8) cycloalkyl, wherein the carbon linked to the oxygen of R¹⁵ or the sulfur of R¹⁶ has no more than secondary branching and, (C2-C6) hydroxyalkyl, aminoalkyl where the alkyl is C2 to C6 and the amino can be substituted with up to two independent (C1-C6) alkyls, Ar-alkyl wherein the alkyl is C1-C6 or Ar, (b) R²² is hydrogen or OR²⁵ and (c) R²³, R²⁴ and R²⁵ are (C1-C6) alkyl, phenyl, benzyl, acetyl or, where R²² is hydrogen, the alkyls of R²³ and R²⁴ can be combined to include 1,3-dioxolane or 1,3-dioxane:
wherein the Ar groups of R³, R¹⁴, R¹⁵, R¹⁶ R¹⁷, R¹⁸, R¹⁹, R²⁰ R²², R²³ or R²⁴ can be substituted with substituents selected from the group consisting of fluoro, chloro, bromo, nitro, cyano, hydroxy, trifluoromethyl, amidosulfonyl which can have up to two independent (C1-C6) N-alkyl substitutions, (C1-C6) alkyl, (C2-C6) alkenyl, (C1-C6) alkylamine, dialkylamine wherein each alkyl is independently C1 to C6, amino, (C1-C6) alkoxy, (C2-C7) alkanoyl, (C2-C7) alkanoyloxy, trifluoromethoxy, hydroxycarbonyl, (C2-C7) alkyloxycarbonyl, aminocarbonyl that can be N-substituted with up to two independent (C1-C6) alkyl, (C1-C6) alkylsulfonyl, amidino that can substituted with up to three (C1-C6) alkyl, or methylenedioxy or ethylenedioxy with the two oxygens bonded to adjacent positions on the aryl or heteroaryl ring structure, which methylenedioxy or ethylenedioxy can be substituted with up to two independent (C1-C6) alkyl;
wherein R¹³ and R¹⁴ together with the nitrogen can form a 5 to 7-membered ring that can contain one additional heteroatom selected from oxygen and sulfur;
for the manufacture of a medicament for treating or preventing schizophrenia, (2) for treating or preventing dementia, (3) for treating or preventing epilepsy, (4) for treating or preventing spasticity, (5) for treating or preventing muscle spasm, (6) for treating or preventing pain, (7) for preventing neural cell death after stroke, (8) for preventing neural cell death in an animal suffering from a neurodegenerative disease, (9) for treating or preventing mood disorders, (10) for enhancing memory or learning, or (11) for treating or preventing learning disorders.

26. Use according to claim 25, wherein spasticity is treated or prevented and the spasticity is associated with epilepsy.

27. Use according to claim 25, wherein spasticity is treated or prevented and the spasticity is associated with stroke, head trauma, multiple sclerosis, spinal cord injury or dystonia.

28. Use according to claim 25, (1) for treating or preventing schizophrenia, (2) for treating or preventing dementia or (3) for treating or preventing pain.

29. Use according to claim 25, wherein the neurodegenerative disease is Parkinson's disease.

30. Use according to claim 25, wherein the neurodegenerative disease is Alzheimer's disease, multi-infarct dementia, AIDS dementia, Huntington's disease, amyotrophic lateral sclerosis or stroke or head trauma.

31. A method of synthetizing a compound of claim 1 or 2 comprising:
A) reacting a compound of one of the following formulas 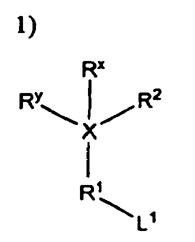 , wherein L¹ is a nucleophilic substitution leaving group, with a compound of the formula 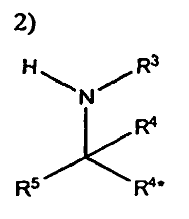
or B) reacting a compound of the formula 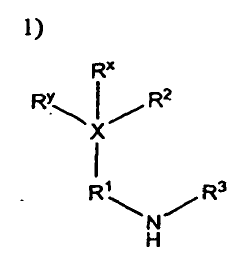 , with a compound of the formula 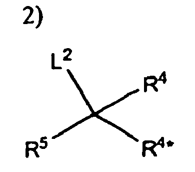 , wherein L² is a nucleophilic substitution leaving group.

32. A method of synthesizing a compound of claim 1 or 2 comprising:
A) reductively alkylating a compound of the formula 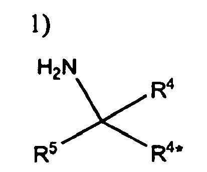 with a compound of the formula 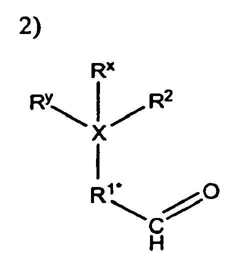 , where R¹* differs from R¹ in that it lacks the carbon that is part of the illustrated aldehyde carbonyl,
OR B) reductively alkylating a compound of the formula 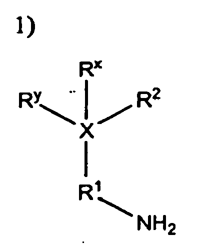 with a compound of the formula

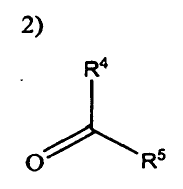

33. A method of synthesizing a compound of claim 1 or 2 comprising reductively alkylating R^{d}NH₂ with a compound of the formula 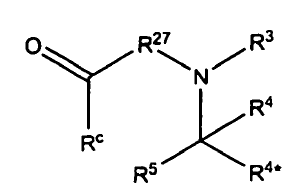 , wherein R^{d} and R^{c} are independently the same as defined for R^{x}, and wherein R²⁷ has the same definition as R¹ except that it does not include a nitrogen, oxygen or sulfur and does not include any double bonds conjugated with the above-illustrated carbonyl.

34. A method of synthesizing a compound of claim 1 or 2 comprising reacting R^{f}OH or R^{f*}SH with a compound of the formula 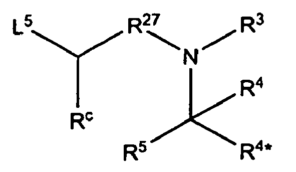 to form an ether or a thioether, respectively, wherein R^{c}, R^{f} and R^{f}* are independently the same as defined for R^{x}, wherein R²⁷ has the same definition as R¹ except that it does not include a nitrogen, oxygen or sulfur and does not include any double bonds at the atom bonded to the above-illustrated L⁵-substituted carbon and wherein L⁵ is a nucleophilic substitution leaving group.

35. The method of claim 34, further comprising synthesizing the compound of formula 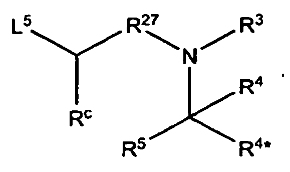 by replacing the hydroxyl of formula 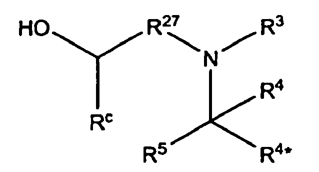 with another nucleophilic substitution leaving group.

36. The method of claim 35, comprising reacting a compound of formula 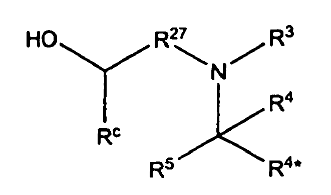 with a azodicarboxylate in the presence of a phosphine compound.

37. A method of synthesizing a compound of claim 1 or 2 comprising reacting R^{e}M with a compound of the formula 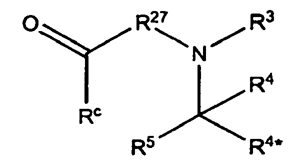 to form a compound of the formula 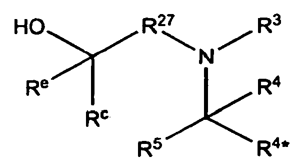 , wherein R^{e} and R^{c} are independently the same as defined for R^{x}, wherein M is a metal-containing substituent such that R^{e}M is a organometallic reagent, and wherein R²⁷ has the same definition as R¹ except that it does not include a nitrogen, oxygen or sulfur and does not include any double bonds conjugated with the above-illustrated carbonyl.

38. A method of synthesizing a compound of claim 1 or 2 comprising dehydrating a compound of the formula 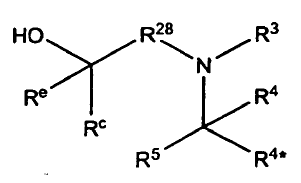 to form a compound of the formula 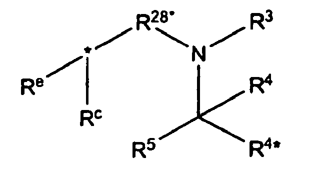 wherein C* has a double bond with an adjacent carbon, and wherein R^{e} and R^{c} are independently the same as defined for R^{x}, and wherein R²⁸ and R²⁸* have the same definition as R¹ except R²⁸ and R^{28*} do not include a nitrogen, oxygen or sulfur.

39. A method of synthesizing a compound of claim 1 or 2 comprising reducing a compound of the formula 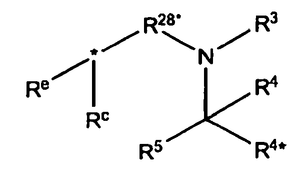 wherein C* has a double bond with an adjacent carbon and R^{c} is independently the same as defined for R^{x}, to form a compound of the formula 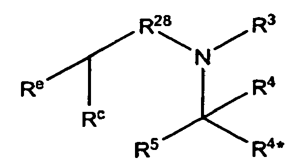 wherein R^{e} is independently the same as defined for R^{x}, and wherein R²⁸ and R^{28*} have the same definition as R¹ except that R²⁸ and R²⁸* do not include a nitrogen, oxygen or sulfur.

## Patentansprüche

1. Verbindung der folgenden Formel: 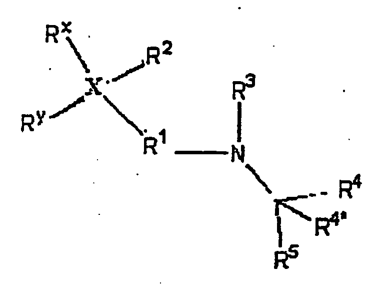 oder ein pharmazeutisch annehmbares Salz derselben, in der:
(1) X Stickstoff oder Kohlenstoff ist und R² nicht anwesend ist, wenn X Stickstoff ist;
(2) R² (a) Wasserstoff, (C1-C6)-Alkyl, (C1-C6)-Alkoxy, Cyano, (C2-C7)-Alkanoyl, Aminocarbonyl, (C1-C6)-Alkylaminocarbonyl oder Dialkylaminocarbonyl ist, worin jedes Alkyl unabhängig C1 bis C6 ist, (b) (wenn R¹ nicht Aminoethylen, -O-R⁸ oder -S-R⁸* ist) Hydroxy, Fluor,-Chlor, Brom oder (C2-C7)-Alkanoyloxy umfasst, (c) eine Doppelbindung mit einem benachbarten Kohlenstoff oder Stickstoff von einem aus entweder R¹, R^{xb} oder R^{yb} bildet oder (d) R^{2a} ist, das durch R^{2b} mit X verknüpft ist, oder (e) Ethylen ist, das eine dritte Verbrückungsstruktur bildet, wie in (2ⁱⁱⁱ)(b)(i) angegeben;
(2ⁱ) R^{x} für R^{xa} steht, das durch R^{xb} mit X verknüpft ist;
(2ⁱⁱ) R^{y} für R^{ya} steht, das durch R^{yb} mit X verknüpft ist;
(2ⁱⁱⁱ) R^{xa}, R^{ya} und R^{2a} unabhängig Ar, das Aryl oder Heteroaryl ist, Adamantyl oder ein 5- bis 7-gliedriger nicht-aromatischer Ring mit 0 bis 2 Heteroatomen sind, die ausgewählt sind aus der Gruppe bestehend aus Sauerstoff, Schwefel und Stickstoff, wobei mindestens eines von R^{xa}, R^{ya} und R^{2a} Phenyl ist, und worin:
(a) Aryl Phenyl oder Naphthyl ist,
(b) Heteroaryl einen fünfgliedrigen Ring, einen sechsgliedrigen Ring, einen sechsgliedrigen Ring, der mit einem fünfgliedrigen Ring kondensiert ist, einen fünfgliedrigen Ring, der mit einem sechsgliedrigen Ring kondensiert ist, oder einen sechsgliedrigen Ring, der mit einem sechsgliedrigen Ring kondensiert ist, umfasst, wobei das Heteroaryl aromatisch ist und Heteroatome enthält, die ausgewählt sind aus der Gruppe bestehend aus Sauerstoff, Schwefel und Stickstoff, wobei die verbleibenden Ringatome Kohlenstoff sind,
(c) jedes von R^{xa}, R^{ya} und R^{2a} mit einem von R^{q}, R^{r}O- oder R^{s}S-substituiert oder unabhängig substituiert sein kann, worin jedes von R^{q}, R^{r} und R^{s} unabhängig Ar, Adamantyl oder ein 5-bis 7-gliedriger nicht-aromatischer Ring ist, wie diese Ringstrukturen für R^{xa} definiert sind, und
(d) R^{xa}, R^{ya}, R^{2a}, R^{q}, R^{r} und R^{s} zusätzlich mit einem oder mehreren Substituenten substituiert sein können, die ausgewählt sind aus der Gruppe bestehend aus Fluor, Chlor, Brom, Nitro, Hydroxy, Cyano, Trifluormethyl, Amidosulfonyl, das bis zu zwei unabhängige N-(C1-C6)-Alkyl-Substitutionen aufweisen kann, Adamantyl, (C1-C12)-Alkyl, (C2-C12)-Alkenyl, Amino, (C1-C6)-Alkylamino, Dialkylamino, worin jedes Alkyl unabhängig C1 bis C6 ist, (C1-C6)-Alkoxy, (C2-C7)-Alkanoyl, (C2-C7)-Alkanoyloxy, Trifluormethoxy, Hydroxycarbonyl, (C2-C7)-Alkyloxycarbonyl, Aminocarbonyl, das anstelle von Wasserstoff mit bis zu zwei unabhängigen (C1-C6)-Alkyl substituiert sein kann, (C1-C6)-Alkylsulfonyl, Amidino, das unabhängig mit bis zu drei (C1-C6)-Alkylgruppen substituiert sein kann, oder Methylendioxy oder Ethylendioxy, wobei die zwei Sauerstoffe an benachbarte Positionen der Aryl- oder Heteroaryl-Ringstruktur gebunden sind, wobei das Methylendioxy oder Ethylendioxy mit bis zu zwei unabhängigen (C1-C6)-Alkyl substituiert sein kann, wobei:
(i.) die Substitutionen von R^{xa}, R^{ya} und R^{2a} vereinigt sein können, um einen zweite Brücke zwischen zwei von R^{xa}, R^{ya} und R^{2a} zu bilden, welche umfasst: (1) (C1-C2)-Alkyl oder (C2)-Alkenyl, die unabhängig mit einem oder mehreren (C1-C6)-Alkyl oder durch R² substituiert sein können, wobei R² Ethylen ist, um eine dritte Verbrückungsstruktur zu bilden, (2) Schwefel, (3) Sauerstoff, (4) Amino, das anstelle von Wasserstoff mit einem (C1-C6)-Alkyl substituiert sein kann, (5) Carbonyl, (6) -CH₂C(=O)-, das anstelle von Wasserstoff mit bis zu zwei unabhängigen (C1-C6)-Alkyl substituiert sein kann, (7) -C(=O)-O-, (8) -CH₂-O-, das anstelle von Wasserstoff mit bis zu zwei unabhängigen (C1-C6)-Alkyl substituiert sein kann, (9) -C(=O)N(R²⁴), worin R²⁴ Wasserstoff oder (C1-C6)-Alkyl ist, (10) -CH₂-NH-, das anstelle von Wasserstoff mit bis zu drei (C1-C6)-Alkyl substituiert sein kann, oder (11) -CH=N-, das anstelle von Wasserstoff mit (C1-C6)-Alkyl substituiert sein kann, oder worin zwei von R^{xa}, R^{ya} und R^{2a} direkt durch eine Einfachbindung verknüpft sein können;
(2^{iv}) R^{xb} und R^{2b} unabhängig eine Einfachbindung oder (C1-C2)-Alkylen sind;
(2^{v}) R^{yb} eine Einfachbindung, Oxo, (C1-C2)-Alkylen, Ethenylen oder -CH= (worin die Doppelbindung mit X ausgebildet ist), Thio, Methylenoxy oder Methylenthio oder entweder -N(R⁶) oder -CH₂₋N(R^{6*})- ist, worin R⁶ und R⁶* Wasserstoff oder (C1-C6)-Alkyl sind, wobei, wenn X Stickstoff ist, X nicht an ein weiteres Heteroatom gebunden ist;
(3) R¹ umfasst: eine geradkettige aliphatische (C2-C3)-Gruppe; wenn X Kohlenstoff ist, =N-O-(Ethylen), worin die freie Doppelbindung an X geknüpft ist, wobei X Kohlenstoff ist und R^{yb} kein Heteroatom einschließt, das an X geknüpft ist, -O-R⁸ oder -S-R^{8*}, worin R⁸ oder R^{8*} ein Ethylen oder Ethenylen ist und O oder S an X gebunden ist, wobei X Kohlenstoff ist und R^{yb} kein Heteroatom einschließt, das an X geknüpft ist; Aminoethylen, wobei das Amino an X gebunden ist:
worin R¹ mit bis zu einem Hydroxy, bis zu einem (C1-C6)-Alkoxy oder bis zu einem (C2-C7)-Alkanoyloxy, mit bis zu zwei unabhängigen (C1-C6)-Alkyl, mit bis zu einem Oxo, bis zu einem (C1-C6)-Alkyliden substituiert sein kann, mit der Maßgabe, dass der Hydroxy-, Alkoxy-, Alkanoyloxy- oder Oxo-Substituent nicht an einen Kohlenstoff gebunden ist, der an einen Stickstoff oder Sauerstoff gebunden ist;
worin die Alkyl- oder Alkyliden-Substituenten von R¹ unter Bildung eines 3- bis 7-gliedrigen nicht-aromatischen Rings verbunden sein können; und
worin, wenn X Stickstoff ist, X durch eine Einfachbindung an R¹ gebunden ist und der endständige Kohlenstoff von R¹, der R¹ mit N verknüpft, gesättigt ist;
(4) R³ Wasserstoff, (C1-C6)-Alkyl oder Phenyl oder Phenylalkyl ist, worin das Alkyl C1 bis C6 ist und jedes derartige Phenyl mit den gleichen Substituenten substituiert sein kann, die oben für das Aryl oder Heteroaryl von R^{xa} definiert wurden; und (5) R⁴ und R^{4*} Wasserstoff sind; und
(6) R⁵ für (CO)NR¹³R¹⁴, (CO)OR¹⁵, (CO)SR¹⁶, (SO₂)NR¹⁷R¹⁸, (PO)(OR¹⁹)(OR²⁰), (CR²²)(OR²³)(OR²⁴), CN oder Tetrazol-5-yl steht,
worin (a) R¹³, R¹⁴, R¹⁵, R¹⁶, R¹⁷, R¹⁸, R¹⁹ und R²⁰ unabhängig Wasserstoff, (C1-C8)-Alkyl, das (C3-C8)-Cycloalkyl einschließen kann, wobei der Kohlenstoff, der an den Sauerstoff von R¹⁵ oder den Schwefel von R¹⁶ gebunden ist, nicht mehr als eine sekundäre Verzweigung aufweist, und (C2-C6)-Hydroxyalkyl, Aminoalkyl, worin Alkyl C2 bis C6 ist und das Amino mit bis zu zwei unabhängigen (C1-C6)-Alkyl substituiert sein kann, Ar-alkyl, worin das Alkyl C1-C6 ist, oder Ar sein können, (b) R²² Wasserstoff oder OR²⁵ ist und (c) R²³, R²⁴ und R²⁵ für (C1-C6)-Alkyl, Phenyl, Benzyl, Acetyl steht oder, wenn R²² Wasserstoff ist, die Alkyle von R²³ und R²⁴ vereinigt sein können, um 1,3-Dioxolan oder 1,3-Dioxan einzuschließen:
wobei die Ar-Gruppen von R¹³, R¹⁴, R¹⁵, R¹⁶, R¹⁷ , R¹⁸, R¹⁹, R²⁰, R²², R²³ oder R²⁴ mit Substituenten substituiert sein können, die ausgewählt sind aus der Gruppe bestehend aus Fluor, Chlor, Brom, Nitro, Cyano, Hydroxy, Trifluormethyl, Amidosulfonyl, das bis zu zwei unabhängige (C1-C6)-N-Alkyl-Substitutionen aufweisen kann, (C1-C6)-Alkyl, (C2-C6)-Alkenyl, (C1-C6)-Alkylamin, Dialkylamin, worin jedes Alkyl unabhängig C1 bis C6 ist, Amino, (C1-C6)-Alkoxy, (C2-C7)-Alkanoyl, (C2-C7)-Alkanoyloxy, Trifluormethoxy, Hydroxycarbonyl, (C2-C7)-Alkyloxycarbonyl, Aminocarbonyl, das mit bis zu zwei unabhängigen (C1-C6)-Alkyl N-substituiert sein kann, (C1-C6)-Alkylsulfonyl, Amidino, das mit bis zu drei (C1-C6)-Alkyl substituiert sein kann, oder Methylendioxy oder Ethylendioxy, wobei die zwei Sauerstoffe an benachbarte Positionen an der Aryl- oder Heteroaryl-Ringstruktur gebunden sind, wobei das Methylendioxy oder Ethylendioxy mit bis zu zwei unabhängigen (C1-C6)-Alkyl substituiert sein kann; worin R¹³ und R¹⁴ zusammen mit dem Stickstoff einen 5- bis 7-gliedrigen Ring bilden können, der ein zusätzliches Heteroatom enthalten kann, das aus Sauerstoff und Schwefel ausgewählt ist;
und wobei die folgenden Maßgaben gelten:
wenn R¹⁵ Wasserstoff ist und R¹ Propylen ist, dann gilt mindestens eines der Folgenden: (1) sowohl R^{x} als auch R^{y} sind nicht p-Fluorphenyl, (2) eines von R^{x} und R^{y} schließt ein Heteroaryl ein, (3) R^{y} ist Ar-(C1-C2)-alkyl, Ar-oxy, Ar-methoxy, Ar-thio, Ar-methylthio, Ar-N(R⁶)- oder Ar-CH₂-N(R⁶_{*})-, (4) R² ist R^{2a}R^{2b}-, (5) R²_{*} ist nicht Wasserstoff oder (6) R³ ist nicht Wasserstoff;
wenn R¹⁵ Wasserstoff ist und R¹ Ethylen ist oder X-R¹ Prop-1-enylen ist, dann gilt mindestens eines der Folgenden: (1) ein Aryl von mindestens einem von R^{x} und R^{y} ist mit einem von Wasserstoff verschiedenen Rest substituiert, (2) eines von R^{x} und R^{y} umfasst ein Heteroaryl, (3) R^{y} ist Ar-(C1-C2)-Alkyl, Ar-oxy, Ar-methoxy, Ar-thio, Ar-methylthio, Ar-N(R⁶)- oder Ar-CH₂-N(R⁶*)-, (4) R² ist R^{2a}R^{2b}-, (5) R²* ist nicht Wasserstoff oder (6) R³ ist nicht Wasserstoff;
wenn R⁵ für C(O)NR¹³R¹⁴ steht, worin R¹³ und R¹⁴ Wasserstoff, (C1-C8)-Alkyl, Phenyl oder substituiertes Phenyl sind, dann gilt mindestens eines der Folgenden: (1) ein Aryl von mindestens einem von R^{x} und R^{y} ist mit einem von Wasserstoff verschiedenen Rest substituiert, (2) eines von R^{x} und R^{y} umfasst ein Heteroaryl, (3) R^{y} ist Ar-(C1-C2)-alkyl, Ar-oxy, Ar-methoxy, Ar-thio, Ar-methylthio, Ar-N(R⁶)- oder Ar-CH₂-N(R^{6*})-, (4) R² ist R^{2a}R^{2b}-, (5) R²* ist nicht Wasserstoff, (6) R³ ist nicht Wasserstoff oder (7) R¹ ist nicht Ethylen;
wobei, wenn R² Phenyl oder p-Methylphenyl ist, mindestens eines der Folgenden zutrifft: (1) die Aryle von R^{x} und R^{y} sind nicht mit p-Methylphenyl oder p-Methoxyphenyl substituiert, (2) ein Aryl von mindestens einem von R^{x} und R^{y} ist mit einem von Wasserstoff verschiedenen Rest substituiert, (3) eines von R^{x} und R^{y} umfasst ein Heteroaryl, (4) R^{y} ist Ar-(C1-C2)-alkyl, Ar-oxy, Ar-methoxy, Ar-thio, Ar-methylthio, Ar-N(R⁶)- oder Ar-CH₂-N(R^{6*})- oder (5) R¹ ist nicht Aminoethylen, OR⁸ oder SR^{8*};
wobei, wenn R^{2a} für p-Methoxyphenyl steht, mindestens eines der Folgenden zutrifft: (1) ein Ar von mindestens einem von R^{x} und R^{y} ist mit einem von Wasserstoff verschiedenen Rest substituiert, (2) R^{y} ist Ar-(C1-C2)-alkyl, Ar-oxy, Ar-methoxy, Ar-thio, Ar-methylthio, , Ar-N(R⁶)- oder Ar-CH₂-N(R^{6*})-, oder (3) R¹ ist nicht OR⁸ oder SR⁸*.

2. Verbindung nach Anspruch 1 der folgenden Formel: 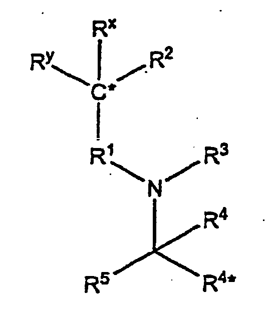 oder ein pharmazeutisch annehmbares Salz derselben, in der:
(1) C* ein substituierter Kohlenstoff ist;
(2) R² (a) Wasserstoff, (C1-C6)-Alkyl, (C1-C6)-Alkoxy, Cyano, (C2-C7)-Alkanoyl, Aminocarbonyl, (C1-C6)-Alkylaminocarbonyl oder Dialkylaminocarbonyl ist, worin jedes Alkyl unabhängig C1 bis C6 ist, (b) (wenn R¹ nicht Aminoethylen, -O-R⁸ oder -S-R⁸* ist) Hydroxy, Fluor, Chlor, Brom oder (C2-C7)-Alkanoyloxy umfasst, (c) mit einem benachbarten Kohlenstoff oder Stickstoff von entweder R^{1,} R^{xb}, R^{yb} eine Doppelbindung bildet, (d) R^{2a} ist, das durch R^{2b} mit C* verknüpft ist, oder (e) Ethylen ist, das eine dritte Verbrückungsstruktur bildet, wie in (2ⁱⁱⁱ)(b)(i) angegeben;
(2ⁱ) R^{x} für R^{xa} steht, das durch R^{xb} an C* geknüpft ist;
(2") R^{y} für R^{ya} steht, das durch R^{yb} an C* geknüpft ist;
(2ⁱⁱⁱ) R^{xa}, R^{ya} und R^{2a} unabhängig Ar, das Phenyl oder Naphthyl ist, oder ein 5- bis 7-gliedriger nicht-aromatischer Ring mit 0 Heteroatomen sind, worin:
(a) jedes von R^{xa} und R^{ya} unabhängig mit einem von R^{q}, R^{r}O- oder R^{s}S-substituiert sein kann, worin jedes von R^{q}, R^{r} und R^{s} unabhängig Ar oder Adamantyl ist, und
(b) R^{xa}, R^{ya}, R^{2a}, R^{q}, R^{r} und R^{s} mit einem oder mehreren Substituenten substituiert oder zusätzlich substituiert sein können, die ausgewählt sind aus der Gruppe bestehend aus Fluor, Chlor, Brom, Nitro, Hydroxy, Cyano, Trifluormethyl, Amidosulfonyl, das bis zu zwei unabhängige N-(C1-C6)-Alkyl-Substitutionen aufweisen kann, (C1-C12)-Alkyl, (C2-C12)-Alkenyl, Amino, (C1-C6)-Alkylamino, Dialkylamino, worin jedes Alkyl von Dialkylamino unabhängig C1 bis C6 ist, (C1-C6)-Alkoxy, (C2-C7)-Alkanoyl, (C2-C7)-Alkanoyloxy, Trifluormethoxy, Hydroxycarbonyl, (C2-C7)-Alkyloxycarbonyl, Aminocarbonyl, das anstelle von Wasserstoff mit bis zu zwei unabhängigen (C1-C6)-Alkyl substituiert sein kann, (C1-C6)-Alkylsulfonyl oder Amidino, das unabhängig mit bis zu drei (C1-C6)-Alkyl substituiert sein kann, worin:
(i.) die Substituenten von R^{xa} und R^{ya} vereinigt sein können, um eine zweite Brücke zwischen R^{xa} und R^{ya} zu bilden, die umfasst: (1) Methylen oder Ethylen, wobei das Methylen oder Ethylen mit einem R² substituiert sein kann, wenn R² Ethylen ist, um die dritte Verbrückungsstruktur zu bilden, oder (2) -CH=CH-, oder worin R^{xa} und R^{ya} durch eine Einfachbindung verbunden sein können;
(2^{iv}) R^{xb} und R^{2b} unabhängig eine Einfachbindung oder (C1-C2)-Alkylen sind;
(2^{v}) R^{yb} eine Einfachbindung, Oxa, (C1-C2)-Alkylen, Ethenylen oder -CH= (worin die Doppelbindung mit C* ausgebildet ist), Thia, Methylenoxy oder Methylenthio oder entweder -N(R⁶) oder -CH₂N(R⁶*)- ist, worin R⁶ und R⁶* Wasserstoff oder (C1-C6)-Alkyl sind;
(3) R¹ umfasst: eine geradkettige aliphatische (C2-C3)-Gruppe; =N-O-(Ethylen), worin die ungepaarte Doppelbindung an C^{*} geknüpft ist; -O-R⁸ oder -S-R^{8*} , worin R⁸ oder R^{8*} Ethylen oder Ethenylen ist und O oder S an C^{*} gebunden ist; Aminoethylen, worin das Amino an C^{*} gebunden ist;
wobei R¹ mit bis zu einem Hydroxy, bis zu einem (C1-C6)-Alkoxy oder bis zu einem (C2-C7)-Alkanoyloxy, mit zu zwei unabhängigen (C1-C6)-Alkyl, mit bis zu einem Oxo, bis zu einem (C1-C6)-Alkyliden substituiert sein kann, mit der Maßgabe, dass der Hydroxyl-, Alkoxy-, Alkanoyloxy- oder Oxo-Substituent nicht an einen Kohlenstoff gebunden ist, der an einen Stickstoff oder Sauerstoff gebunden ist; und
wobei die Alkyl- oder Alkyliden-Substituenten von R¹ unter Bildung eines 3- bis 7-gliedrigen nicht-aromatischen Rings verknüpft sein können;
(4) R³ (a) Wasserstoff, (C1-C6)-Alkyl oder Phenyl oder Phenylalkyl ist, worin das Alkyl C1 bis C6 ist und das Phenyl oder Phenyl von Phenylalkyl mit den gleichen Substituenten substituiert sein kann, die oben für das Phenyl von R^{xa} definiert wurden, (b) -R¹²C(R^{xx})(R^{yy})(R¹¹) ist, worin R¹² an N gebunden ist, R^{xx} unabhängig das gleiche wie R^{x} ist, R^{yy} unabhängig das gleiche wie R^{y} ist, R¹¹ unabhängig das gleiche wie R² ist und R¹² unabhängig das gleiche wie R¹ ist;
(5) R⁴ und R^{4*} unabhängig Wasserstoff sind; und
(6) R⁵ für (CO)NR¹³R¹⁴, (CO)OR¹⁵, (CO)SR¹⁶, (SO₂)NR¹⁷R¹⁸, (PO)(OR¹⁹)(OR²⁰), (CR²²)(OR²³)(OR²⁴), CN oder Tetrazol-5-yl steht, worin (a) R¹³, R¹⁴, R¹⁵, R¹⁶, R¹⁷, R¹⁸, R¹⁹ und R²⁰ unabhängig sind: Wasserstoff, (C1-C8)-Alkyl, das (C3-C8)-Cycloalkyl einschließen kann, wobei der Kohlenstoff, der an den Sauerstoff von R¹⁵ oder den Schwefel von R¹⁶ gebunden ist, nicht mehr als eine sekundäre Verzweigung aufweist, und (C2-C6)-Hydroxyalkyl, Aminoalkyl, worin das Alkyl C2 bis C6 ist und das Amino mit bis zu zwei unabhängigen (C1-C6)-Alkylen substituiert sein kann, Ar-alkyl, worin das Alkyl C1-C6 ist, oder Ar, (b) R²² Wasserstoff oder OR²⁵ ist und R²³, R²⁴ und R²⁵ für (C1-C6)-Alkyl, Phenyl, Benzy oder Acetyl stehen oder die Alkyle von R²³ und R²⁴ vereinigt sein können, um 1,3-Dioxolan oder 1,3-Dioxan einzuschließen:
worin die Phenyl- oder Naphthylgruppen von R¹³, R¹⁴, R¹⁵, R¹⁶, R¹⁷, R¹⁸, R¹⁹, R²⁰, R²², R²³ oder R²⁴ mit Substituenten substituiert sein können, die ausgewählt sind aus der Gruppe bestehend aus Fluor, Chlor, Brom, Nitro, Cyano, Hydroxy, Trifluormethyl, Amidosulfonyl, das bis zu zwei unabhängige N-(C1-C6)-Alkyl-Substitutionen aufweisen kann, (C1-C6)-Alkyl, (C2-C6)-Alkenyl, (C1-C6)-Alkylamin, Dialkylamin, worin jedes Alkyl unabhängig C1 bis C6 ist, Amino, (C1-C6)-Alkoxy, (C2-C7)-Alkanoyl, (C2-C7)-Alkanoyloxy, Trifluormethoxy, Hydroxycarbonyl, (C2-C7)-Alkyloxycarbonyl, Aminocarbonyl, das mit bis zu zwei unabhängigen (C1-C6)-Alkyl N-substituiert sein kann, (C1-C6)-Alkylsulfonyl oder Amidino, das mit bis zu drei (C1-C6)-Alkyl substituiert sein kann,
worin R¹³ und R¹⁴ zusammen mit dem angeknüpften Stickstoff einen 5- bis 7-gliedrigen Ring bilden können;
und wobei weiter die folgenden Maßgaben zutreffen:
wenn R¹⁵ Wasserstoff ist und R¹ Propylen ist, dann gilt mindestens eines der Folgenden: (1) sowohl R^{xa} als auch R^{ya} sind nicht p-Fluorphenyl, (2) R^{y} ist Ar-alkyl, Ar-oxy, Ar-methoxy, Ar-thio, Ar-methylthio, Ar-N(R⁶)- oder Ar-CH₂-N(R^{6*})-, (3) R² ist R^{2a}R^{2b-}, (4) R² ist nicht Wasserstoff oder (5) R³ ist nicht Wasserstoff;
wenn R¹⁵ Wasserstoff ist und R¹ Ethylen ist oder C*R¹ Prop-1-enylen ist, dann gilt mindestens eines der Folgenden: (1) ein Aryl von mindestens einem von R^{xa} und R^{ya} ist mit einem von Wasserstoff verschiedenen Rest substituiert, (2) R^{y} ist Ar-alkyl, Ar-oxy, Ar-methoxy, Ar-thio, Ar-methylthio, Ar-N(R⁶)- oder Ar-CH₂-N(R^{6*})-, (3) R² ist R^{2a}R^{2b}-, (4) R² ist nicht Wasserstoff oder (5) R³ ist nicht Wasserstoff;
wenn R⁵ für C(O)NR¹³R¹⁴ steht, worin R¹³ und R¹⁴ Wasserstoff, (C1-C8)-Alkyl, Phenyl oder substituiertes Phenyl sind, dann gilt mindestens eines der Folgenden: (1) ein Aryl von mindestens einem von R^{x} und R^{y} ist mit einem von Wasserstoff, Fluor, Chlor oder Brom verschiedenen Rest substituiert, (2) R^{y} ist Ar-alkyl, Ar-oxy, Ar-methoxy, Ar-thio, Ar-methylthio, Ar-N(R⁶)- oder Ar-CH₂-N(R⁶*)-, (3) R² ist R^{xa}R^{2a}R^{2b}-, (4) R² ist nicht Wasserstoff, (5) R³ ist nicht Wasserstoff oder (6) R¹ ist nicht Ethylen;
wenn R^{2a} Phenyl oder p-Methylphenyl ist, dann gilt mindestens eines der Folgenden: (1) die Aryle von R^{x} und R^{y} sind nicht mit p-Methylphenyl oder p-Methoxyphenyl substituiert, (2) ein Aryl von mindestens einem von R^{x} und R^{y} ist mit einem von Wasserstoff verschiedenen Rest substituiert, (3) R^{y} ist Ar-alkyl, Ar-oxy, Ar-methoxy, Ar-thio, Ar-methylthio, Ar-N(R⁶)- oder Ar-CH₂-N(R^{6*})-, oder (4) R¹ ist nicht Aminoethylen, OR⁸ oder SR^{8*};
wenn R^{2a} für p-Methoxyphenyl steht, dann gilt mindestens eines der Folgenden: (1) ein Ar von mindestens einem von R^{x} und R^{y} ist mit einem von Wasserstoff verschiedenen Rest substituiert, (2) R^{y} ist Ar-alkyl, Ar-oxy, Ar-methoxy, Ar-thio, Ar-methylthio, Ar-N(R⁶)- oder Ar-CH₂-N(R^{6*})- oder (3) R¹ ist nicht OR⁸ oder SR^{8*}.

3. Verbindung nach Anspruch 1 oder 2, in der (A) mindestens eines von R^{xa}, R^{ya} und R^{2a} mit Fluor, Chlor, Brom, Hydroxy, Trifluormethyl, Trifluormethoxy, Nitro, Cyano, (C3-C8)-Alkyl, R^{q}, R^{r}O-, R^{s}S- substituiert ist, (B) R³ Wasserstoff, (C1-C6)-Alkyl oder Phenyl oder Phenylalkyl ist, worin das Alkyl C1 bis C6 ist und jedes derartige Phenyl mit den gleichen Substituenten substituiert sein kann, die oben für das Aryl oder Heteroaryl von R^{xa} definiert wurden, oder (C) die Ringstrukturen von R^{xa}, R^{ya} und R^{2a}, einschließlich deren Substituenten, ansonsten mindestens zwei aromatische Ringstrukturen einschließen, die zusammen 15 bis 20 Ringatome einschließen.

4. Verbindung nach Anspruch 4, in der mindestens eines von R^{xa}, R^{ya} und R^{2a} mit Fluor, Trifluormethyl, Trifluormethoxy, Nitro, Cyano oder (C3-C8)-Alkyl substituiert ist.

5. Verbindung nach Anspruch 1 oder 2, in der mindestens eines von R^{xa}, R^{ya} und R^{2a} mit R^{q}, R^{r}O- oder R^{s}S- substituiert ist.

6. Verbindung nach Anspruch 1 oder 2, in der R^{yb} für Oxy, Methylenoxy, Thio oder Methylenthio steht.

7. Verbindung nach Anspruch 6, in der R^{yb} Oxy oder Thio ist.

8. Verbindung nach Anspruch 1 oder 2, in der R⁵ für (CO)NR¹³R¹⁴, (CO)OR¹⁵ oder (CO)SR¹⁶ steht.

9. Verbindung nach Anspruch 8, in der R¹⁵ für (C2-C6)-Alkyl, (C2-C4)-Hydroxyalkyl, Phenyl, Phenylalkyl, worin das Alkyl C1-C3 ist, oder Aminoalkyl steht, worin das Alkyl C2-C6 ist und das Amino mit bis zu zwei unabhängigen (C1-C3)-Alkylen substituiert sein kann, wobei das Phenyl oder Phenyl von Phenylalkyl substituiert sein kann.

10. Verbindung nach Anspruch 8, in der R¹⁵ Wasserstoff ist.

11. Verbindung nach Anspruch 1 oder 2, in der R⁴ Wasserstoff, Methyl oder Hydroxymethyl ist und R^{4*} Wasserstoff ist.

12. Verbindung nach Anspruch 1, in der mindestens eines von R^{xa}, R^{ya} und R^{2a} ein Heteroaryl ist, das Diazolyl, Triazolyl, Tetrazolyl, Thiazolyl, Isothiazolyl, Oxazolyl, Isoxazolyl, Thiolyl, Diazinyl, Triazinyl, Benzoazolyl, Benzodiazolyl, Benzothiazolyl, Benzoxazolyl, Benzoxolyl, Benzothiolyl, Chinolyl, lsochinolyl, Benzodiazinyl, Benzotriazinyl, Pyridyl, Thienyl, Furanyl, Pyrrolyl, Indolyl, Isoindolyl oder Pyrimidyl umfasst.

13. Verbindung nach Anspruch 1 oder 2, in der R¹ für -O-R⁸ oder -S-R^{8*} steht.

14. Verbindung nach Anspruch 1 oder 2, in der die zweite Brücke zwischen zwei von R^{xa}, R^{ya} und R^{2a} L ist und der folgenden Formel genügt: 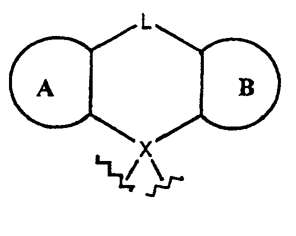 in der A und B Aryl- oder Heteroarylgruppen von R^{xa} bzw. R^{ya} sind.

15. Verbindung nach Anspruch 12, in der R^{xa}-R^{xb}-, R^{ya}-R^{yb}- und X 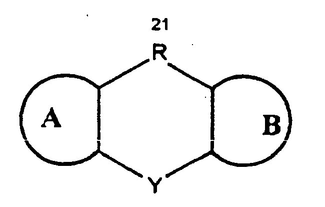 bilden, worin Y ein Kohlenstoff, der durch eine Einfach- und Doppelbindung an R¹ gebunden ist, oder ein Stickstoff ist, der an R¹ gebunden ist, und worin R²¹ entweder (i.) eine Einfachbindung vervollständigt, die zwei Aryl- oder Heteroarylringe von R^{x} und R^{y} verknüpft, (ii.) (C1-C2)-Alkylen oder -Alkenylen ist, (iii.) Schwefel ist oder (iv.) Sauerstoff ist und worin R^{x} und R^{y} substituiert sein können, wie oben angegeben.

16. Verbindung nach Anspruch 15, worin R²¹ für CH₂CH₂ oder CH=CH steht.

17. Verbindung nach Anspruch 1, in der die Alkylendioxy-Substitution von R^{xa}, R^{ya} oder R^{2a} wie folgt ist: 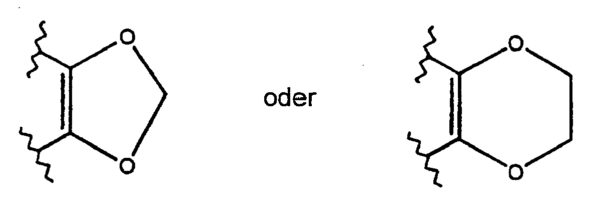 worin das Alkylendioxy mit bis zu zwei unabhängigen (C1-C3)-Alkyl substituiert sein kann.

18. Verbindung nach Anspruch 1 oder 2, in der R^{xa} und R^{ya} zusammen mit bis zu sechs Substituenten substituiert sein können, R^{2a}, R^{q}, R^{r} und R^{s} jeweils mit bis zu drei Substituenten substituiert sein können und worin die Anwesenheit von jedem von R^{q}, R^{r} oder R^{s} als Substitution der jeweiligen Ringstruktur von R^{xa}, R^{ya} und R^{2a} angesehen wird.

19. Verbindung nach Anspruch 1 oder 2, in der ein Phenyl von R³ mit bis zu drei Substituenten substituiert ist.

20. Verbindung nach Anspruch 1 oder 2, in der das Aryl, Heteroaryl, Aryl von Arylalkyl oder das Heteroaryl von Heteroarylalkyl von R¹³, R¹⁴, R¹⁵, R¹⁶, R¹⁷, R¹⁸, R¹⁹ oder R²⁰ mit bis zu drei Substituenten substituiert ist.

21. Verbindung nach Anspruch 1 oder 2, in der die Verbindung ein optisch reines Enantiomer ist.

22. Verbindung nach Anspruch 1 oder 2, in der:
(1) R² Wasserstoff ist,
(2) R^{xa} und R^{ya} beide Phenyl sind und mindestens eines von R^{xa} und R^{ya} mit einem von Phenyl, Phenoxy oder Phenylthio substituiert ist,
(3) R^{xb} eine Einfachbindung ist und R^{yb} eine Einfachbindung oder Oxa ist und
(4) R⁵ für (CO)NR¹³R¹⁴ oder (CO)OR¹⁵ steht, worin R¹³, R¹⁴ und R¹⁵ unabhängig Wasserstoff; (C1-C8)-Alkyl, das (C3-C8)-Cycloalkyl einschließen kann, wobei der Kohlenstoff, der an den Sauerstoff von OR¹⁵ gebunden ist, nicht mehr als eine sekundäre Verzweigung aufweist; (C2-C6)-Hydroxyalkyl oder Aminoalkyl sind, worin das Alkyl C2 bis C6 ist und das Amino mit bis zu zwei unabhängigen (C1-C6)-Alkylen oder Phenylalkylen substituiert sein kann, worin das Alkyl C1-C6 ist und das Phenyl mit Substituenten substituiert sein kann, die ausgewählt sind aus der Gruppe bestehend aus Fluor, Chlor, Brom, Nitro, Cyano, Hydroxy, Trifluormethyl, Amidosulfonyl, das bis zu zwei unabhängige N-(C1-C6)-Alkyl-Substitutionen aufweisen kann, (C1-C6)-Alkyl, (C2-C6)-Alkenyl, (C1-C6)-Alkylamin, Dialkylamin, worin jedes Alkyl unabhängig C1 bis C6 ist, Amino, (C1-C6)-Alkoxy, (C2-C7)-Alkanoyl, (C2-C7)-Alkanoyloxy, Trifluormethoxy, Hydroxycarbonyl, (C2-C7)-Alkyloxycarbonyl, Aminocarbonyl, das mit bis zu zwei unabhängigen (C1-C6)-Alkyl substituiert sein kann, (C1-C6)-Alkylsulfonyl, Amidino, das mit bis zu drei (C1-C6)-Alkyl substituiert sein kann.

23. Pharmazeutische Zusammensetzung, welche die Verbindung von Anspruch 1 oder 2 und einen pharmazeutisch annehmbaren Hilfsstoff umfasst.

24. Pharmazeutische Zusammensetzung von Anspruch 23, in der die Verbindung von Anspruch 1 oder 2 in einer wirksamen Menge vorliegt für:
(1) Behandlung oder Verhütung von Schizophrenie,
(2) Verstärkung der Behandlung oder Verhütung von Demenz,
(3) Behandlung oder Verhütung von Epilepsie,
(4) Behandlung oder Verhütung von Spastizität,
(5) Behandlung oder Verhütung von Muskelspasmus,
(6) Behandlung oder Verhütung von Schmerzen,
(7) Verhütung von Nervenzellentod nach Schlaganfall,
(8) Verhütung von Nervenzellentod in einem Lebewesen, das an einer neurodegenerativen Krankheit leidet,
(9) Behandlung oder Verhütung von Stimmungsstörungen,
(10) Verstärkung von Gedächtnis oder Lernen oder
(11) Behandlung oder Verhütung von Lernstörungen.

25. Verwendung einer Verbindung der Formel: 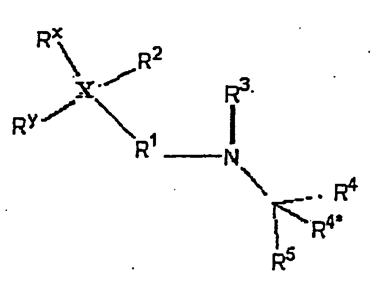 oder eines pharmazeutisch annehmbaren Salzes derselben, in der:
(1) X Stickstoff oder Kohlenstoff ist und R² nicht anwesend ist, wenn X Stickstoff ist;
(2) R² (a) Wasserstoff, (C1-C6)-Alkyl, (C1-C6)-Alkoxy, Cyano, (C2-C7)-Alkanoyl, Aminocarbonyl, (C1-C6)-Alkylaminocarbonyl oder Dialkylaminocarbonyl ist, worin jedes Alkyl unabhängig C1 bis C6 ist, (b) (wenn R¹ nicht Aminoethylen, -O-R⁸ oder -S-R⁸* ist) Hydroxy, Fluor, Chlor, Brom oder (C2-C7)-Alkanoyloxy umfasst, (c) eine Doppelbindung mit einem benachbarten Kohlenstoff oder Stickstoff von einem aus entweder R¹, R^{xb} oder R^{yb} bildet oder (d) R^{2a} ist, das durch R^{2b} an X geknüpft ist, oder (e) Ethylen ist, das eine dritte Verbrückungsstruktur bildet, wie in (2ⁱⁱⁱ)(b)(i) angegeben;
(2ⁱ) R^{x} für R^{xa} steht, das durch R^{xb} an X geknüpft ist;
(2") R^{y} für R^{ya} steht, das durch R^{yb} an X geknüpft ist;
(2ⁱⁱⁱ) R^{xa}, R^{ya} und R^{2a} unabhängig Ar, das Aryl oder Heteroaryl ist, Adamantyl oder ein 5- bis 7-gliedriger nicht-aromatischer Ring mit 0 bis 2 Heteroatomen sind, die ausgewählt sind aus der Gruppe bestehend aus Sauerstoff, Schwefel und Stickstoff, wobei mindestens eines von R^{xa}, R^{ya} und R^{2a} Phenyl ist, und worin:
(a) Aryl Phenyl oder Naphthyl ist,
(b) Heteroaryl einen fünfgliedrigen Ring, einen sechsgliedrigen Ring, einen sechsgliedrigen Ring, der mit einem fünfgliedrigen Ring kondensiert ist, einen fünfgliedrigen Ring, der mit einem sechsgliedrigen Ring kondensiert ist, oder einen sechsgliedrigen Ring, der mit einem sechsgliedrigen Ring kondensiert ist, umfasst, wobei das Heteroaryl aromatisch ist und Heteroatome enthält, die ausgewählt sind aus der Gruppe bestehend aus Sauerstoff, Schwefel und Stickstoff, wobei die verbleibenden Ringatome Kohlenstoff sind,
(c) jedes von R^{xa}, R^{ya} und R^{2a} mit einem von R^{q}, R^{r}O- oder R^{s}S-substituiert oder unabhängig substituiert sein kann, worin jedes von R^{q}, R^{r} und R^{s} unabhängig Ar, Adamantyl oder ein 5-oder 7-gliedriger nicht-aromatischer Ring ist, wie diese Ringstrukturen für R^{xa} definiert sind, und
(d) R^{xa}, R^{ya}, R^{2a}, R^{q}, R^{r} und R^{s} zusätzlich mit einem oder mehreren Substituenten substituiert sein können, die ausgewählt sind aus der Gruppe bestehend aus Fluor, Chlor, Brom, Nitro, Hydroxy, Cyano, Trifluormethyl, Amidosulfonyl, das bis zu zwei unabhängige N-(C1-C6)-Alkyl-Substitutionen aufweisen kann, Adamantyl, (C1-C12)-Alkyl, (C2-C12)-Alkenyl, Amino, (C1-C6)-Alkylamino, Dialkylamino, worin jedes Alkyl unabhängig C1 bis C6 ist, (C1-C6)-Alkoxy, (C2-C7)-Alkanoyl), (C2-C7)-Alkanoyloxy, Trifluormethoxy, Hydroxycarbonyl, (C2-C7)-Alkyloxycarbonyl, Aminocarbonyl, das anstelle von Wasserstoff mit bis zu zwei unabhängigen (C1-C6)-Alkyl substituiert sein kann, (C1-C6)-Alkylsulfonyl, Amidino, das unabhängig mit bis zu drei (C1-C6)-Alkylgruppen substituiert sein kann, oder Methylendioxy oder Ethylendioxy,
wobei die zwei Sauerstoffe an benachbarte Positionen der Aryl- oder Heteroaryl-Ringstruktur gebunden sind, wobei das Methylendioxy oder Ethylendioxy mit bis zu zwei unabhängigen (C1-C6)-Alkyl substituiert sein kann, wobei:
(i.) die Substitutionen von R^{xa}, R^{ya} und R^{2a} vereinigt sein können, um einen zweite Brücke zwischen zwei von R^{xa}, R^{ya} und R^{2a} zu bilden, welche umfasst: (1) (C1-C2)-Alkyl oder (C2)-Alkenyl, die unabhängig mit einem oder mehreren (C1-C6)-Alkyl oder durch R² substituiert sein können, wobei R² Ethylen ist, um eine dritte Verbrückungsstruktur zu bilden, (2) Schwefel, (3) Sauerstoff, (4) Amino, das anstelle von Wasserstoff mit einem (C1-C6)-Alkyl substituiert sein kann, (5) Carbonyl, (6) -CH₂C(=O)-, das anstelle von Wasserstoff mit bis zu zwei unabhängigen (C1-C6)-Alkyl substituiert sein kann, (7) -C(=O)-O-, (8) -CH₂-O-, das anstelle von Wasserstoff mit bis zu zwei unabhängigen (C1-C6)-Alkyl substituiert sein kann, (9) -C(=O)N(R²⁴), worin R²⁴ Wasserstoff oder (C1-C6)-Alkyl ist, (10) -CH₂-NH-, das anstelle von Wasserstoff mit bis zu drei (C1-C6)-Alkyl substituiert sein kann, oder (11) -CH=N-, das anstelle von Wasserstoff mit (C1-C6)-Alkyl substituiert sein kann, oder worin zwei von R^{xa}, R^{ya} und R^{2a} direkt durch eine Einfachbindung verknüpft sein können;
(2^{iv}) R^{xb} und R^{2b} unabhängig eine Einfachbindung oder (C1-C2)-Alkylen sind;
(2^{v}) R^{yb} eine Einfachbindung, Oxo, (C1-C2)-Alkylen, Ethenylen oder -CH= (worin die Doppelbindung mit X ausgebildet ist), Thio, Methylenoxy oder Methylenthio oder entweder -N(R⁶) oder -CH₂₋N(R^{6*})- ist, worin R⁶ und R^{6*} Wasserstoff oder (C1-C6)-Alkyl sind, wobei, wenn X Stickstoff ist, X nicht an ein weiteres Heteroatom gebunden ist;
(3) R¹ umfasst: eine geradkettige aliphatische (C2-C3)-Gruppe; wenn X Kohlenstoff ist, =N-O-(Ethylen), worin die freie Doppelbindung an X geknüpft ist (wobei X Kohlenstoff ist und R^{yb} kein Heteroatom einschließt, das an X geknüpft ist), -O-R⁸ oder -S-R^{8*}, worin R⁸ oder R^{8*} ein Ethylen oder Ethenylen ist und O oder S an X gebunden ist; (wobei X Kohlenstoff ist und R^{yb} kein Heteroatom einschließt, das an X geknüpft ist), Aminoethylen, wobei das Amino an X gebunden ist:
worin R¹ mit bis zu einem Hydroxy, bis zu einem (C1-C6)-Alkoxy oder bis zu einem (C2-C7)-Alkanoyloxy, mit bis zu zwei unabhängigen (C1-C6)-Alkyl, mit bis zu einem Oxo, bis zu einem (C1-C6)-Alkyliden substituiert sein kann, mit der Maßgabe, dass der Hydroxy-, Alkoxy-, Alkanoyloxy- oder Oxo-Substituent nicht an einen Kohlenstoff gebunden ist, der an einen Stickstoff oder Sauerstoff gebunden ist;
worin die Alkyl- oder Alkyliden-Substituenten von R¹ unter Bildung eines 3- bis 7-gliedrigen nicht-aromatischen Rings verbunden sein können; und
worin, wenn X Stickstoff ist, X durch eine Einfachbindung an R¹ gebunden ist und der endständige Kohlenstoff von R¹, der R¹ mit N verknüpft, gesättigt ist;
(4) R³ Wasserstoff, (C1-C6)-Alkyl oder Phenyl oder Phenylalkyl ist, worin das Alkyl C1 bis C6 ist und jedes derartige Phenyl mit den gleichen Substituenten substituiert sein kann, die oben für das Aryl oder Heteroaryl von R^{xa} definiert wurden,
(5) R⁴ und R⁴* unabhängig Wasserstoff sind; und
(6) R⁵ für (CO)NR¹³R¹⁴, (CO)OR¹⁵, (CO)SR¹⁶, (SO₂)NR¹⁷R¹⁸, (PO)(OR¹⁹)(OR²⁰), (CR²²)(OR²³)(OR²⁴), CN oder Tetrazol-5-yl steht, worin (a) R¹³, R¹⁴, R¹⁵, R¹⁶, R¹⁷, R¹⁸, R¹⁹ und R²⁰ unabhängig Wasserstoff, (C1-C8)-Alkyl, das (C3-C8)-Cycloalkyl einschließen kann, wobei der Kohlenstoff, der an den Sauerstoff von R¹⁵ oder den Schwefel von R¹⁶ gebunden ist, nicht mehr als eine sekundäre Verzweigung aufweist, und (C2-C6)-Hydroxyalkyl, Aminoalkyl, worin das Alkyl C2 bis C6 ist und das Amino mit bis zu zwei unabhängigen (C1-C6)-Alkylen substituiert sein kann, Ar-alkyl, worin das Alkyl C1-C6 ist, oder Ar sind, (b) R²² Wasserstoff oder OR²⁵ ist und (c) R²³, R²⁴ und R²⁵ für (C1-C6)-Alkyl, Phenyl, Benzyl, Acetyl stehen oder, wenn R²² Wasserstoff ist, die Alkyle von R²³ und R²⁴ vereinigt sein können, um 1,3-Dioxolan oder 1,3-Dioxan einzuschließen:
wobei die Ar-Gruppen von R¹³, R¹⁴, R¹⁵ R¹⁶, R¹⁷, R¹⁸, R¹⁹, R²⁰, R²², R²³ oder R²⁴ mit Substituenten substituiert sein können, die ausgewählt sind aus der Gruppe bestehend aus Fluor, Chlor, Brom, Nitro, Cyano, Hydroxy, Trifluormethyl, Amidosulfonyl, das bis zu zwei unabhängige N-(C1-C6)-Alkyl-Substitutionen aufweisen kann, (C1-C6)-Alkyl, (C2-C6)-Alkenyl, (C1-C6)-Alkylamin, Dialkylamin, worin jedes Alkyl unabhängig C1 bis C6 ist, Amino, (C1-C6)-Alkoxy, (C2-C7)-Alkanoyl, (C2-C7)-Alkanoyloxy, Trifluormethoxy, Hydroxycarbonyl, (C2-C7)-Alkyloxycarbonyl, Aminocarbonyl, das mit bis zu zwei unabhängigen (C1-C6)-Alkyl N-substituiert sein kann, (C1-C6)-Alkylsulfonyl, Amidino, das mit bis zu drei (C1-C6)-Alkyl substituiert sein kann, oder Methylendioxy oder Ethylendioxy, wobei die zwei Sauerstoffe an benachbarte Positionen an der Aryl- oder Heteroaryl-Ringstruktur gebunden sind, wobei das Methylendioxy oder Ethylendioxy mit bis zu zwei unabhängigen (C1-C6)-Alkyl substituiert sein kann;
worin R¹³ und R¹⁴ zusammen mit dem Stickstoff einen 5- bis 7-gliedrigen Ring bilden können, der ein zusätzliches Heteroatom enthalten kann, das aus Sauerstoff und Schwefel ausgewählt ist;
für die Herstellung eines Medikaments zur Behandlung oder Verhütung von Schizophrenie, (2) zur Behandlung oder Verhütung von Demenz, (3) zur Behandlung oder Verhütung von Epilepsie, (4) zur Behandlung oder Verhütung von Spastizität, (5) zur Behandlung oder Verhütung von Muskelspasmus, (6) zur Behandlung oder Verhütung von Schmerzen, (7) zur Verhütung von Nervenzellentod nach Schlaganfall, (8) zur Verhütung von Nervenzellentod in einem Lebewesen, das an einer neurodegenerativen Krankheit leidet, (9) zur Behandlung oder Verhütung von Stimmungsstörungen, (10) zur Verstärkung von Gedächtnis und Lernen oder (11) zur Behandlung oder Verhütung von Lernstörungen.

26. Verwendung nach Anspruch 25, bei der Spastizität behandelt oder verhütet wird und die Spastizität mit Epilepsie verbunden ist.

27. Verwendung nach Anspruch 25, bei der Spastizität behandelt oder verhütet wird und die Spastizität mit Schlaganfall, Kopftrauma, Multipler Sklerose, Wirbelsäulenverletzung oder Dystonie verbunden ist.

28. Verwendung nach Anspruch 25 (1) zur Behandlung oder Verhütung von Schizophrenie, (2) zur Behandlung oder Verhütung von Demenz oder (3) zur Behandlung oder Verhütung von Schmerzen.

29. Verwendung nach Anspruch 25, bei der die neurodegenerative Krankheit Parkinson-Krankheit ist.

30. Verwendung nach Anspruch 25, bei der die neurodegenerative Krankheit Alzheimer-Krankheit, Multiinfarkt-Demenz, AIDS-Demenz, Chorea Huntington, amyotrophe Lateralsklerose oder Schlaganfall oder Kopftrauma ist.

31. Verfahren zur Synthese einer Verbindung nach Anspruch 1 oder 2, umfassend:
(A) Umsetzen einer Verbindung mit einer der folgenden Formeln 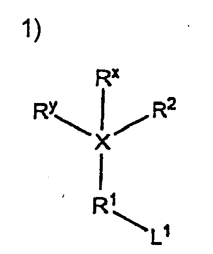 worin L¹ eine Abgangsgruppe bei nukleophiler Substitution ist, mit einer Verbindung der Formel 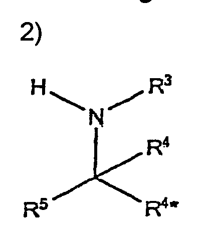 oder
(B) Umsetzen einer Verbindung der Formel 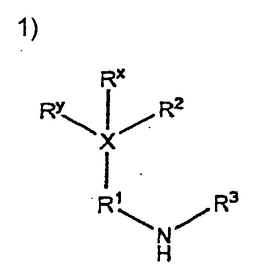 mit einer Verbindung der Formel 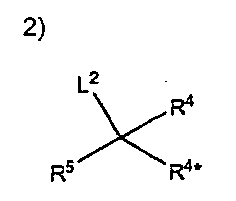 in der L² eine Abgangsgruppe bei nukleophiler Substitution ist.

32. Verfahren zur Synthese einer Verbindung nach Anspruch 1 oder 2, umfassend:
A) die reduktive Alkylierung einer Verbindung der Formel 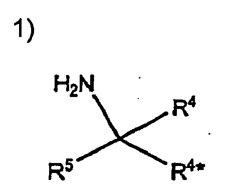 mit einer Verbindung der Formel 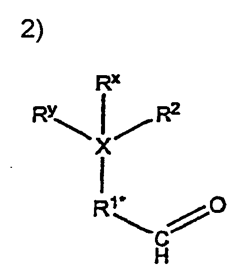 worin sich R¹* von R¹ unterscheidet, indem ihm der Kohlenstoff fehlt, der Teil des veranschaulichten Aldehydcarbonyls ist, oder
B) die reduktive Alkylierung einer Verbindung der Formel 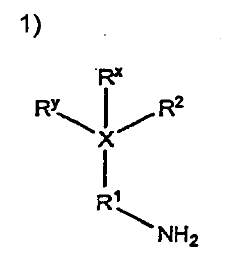 mit einer Verbindung der Formel

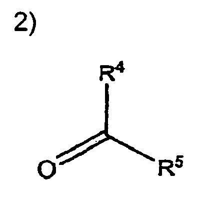

33. Verfahren zur Synthese einer Verbindung nach Anspruch 1 oder 2, umfassend die reduktive Alkylierung von R^{d}NH₂ mit einer Verbindung der Formel 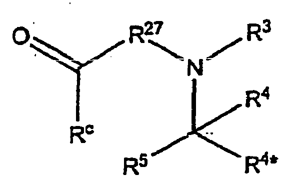 in der R^{d} und R^{c} unabhängig die gleichen sind wie für R^{x} definiert und in der R²⁷ die gleiche Definition wie R¹ aufweist, außer dass es keinen Stickstoff, Sauerstoff oder Schwefel einschließt und keine Doppelbindungen einschließt, die mit dem oben veranschaulichten Carbonyl konjugiert sind.

34. Verfahren zur Synthese einer Verbindung nach Anspruch 1 oder 2, umfassend die Umsetzung von R^{f}OH oder R^{f}*SH mit einer Verbindung der Formel 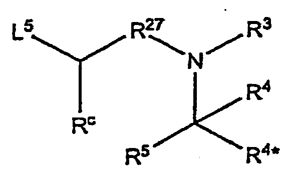 um einen Ether bzw. einen Thioether zu bilden, worin R^{c}, R^{f} und R^{f}* unabhängig die gleichen sind wie für R^{X} definiert, worin R²⁷ die gleiche Definition wie R¹ aufweist, außer dass es keinen Stickstoff, Sauerstoff oder Schwefel einschließt und keine Doppelbindungen an dem Atom einschließt, das an den oben veranschaulichten L⁵-substituierten Kohlenstoff gebunden ist, und worin L⁵ eine Abgangsgruppe bei einer nukleophilen Substitution ist.

35. Verfahren nach Anspruch 34, weiter umfassend das Synthetisieren der Verbindung der Formel 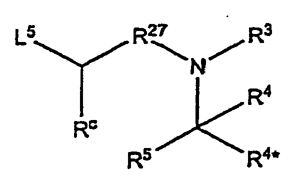 durch Ersetzen des Hydroxyls der Formel 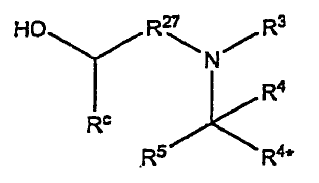 durch eine andere Abgangsgruppe bei nukleophiler Substitution.

36. Verfahren nach Anspruch 35, umfassend das Umsetzen einer Verbindung der Formel 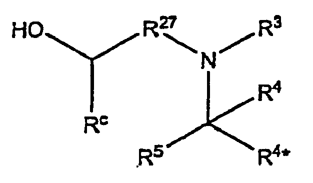 mit einem Azodicarboxylat in Anwesenheit einer Phosphin-Verbindung.

37. Verfahren zur Synthese einer Verbindung nach Anspruch 1 oder 2, umfassend das Umsetzen von R^{e}M mit einer Verbindung der Formel 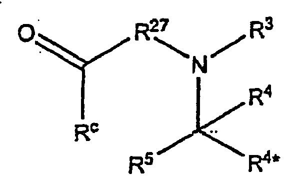 um eine Verbindung der Formel 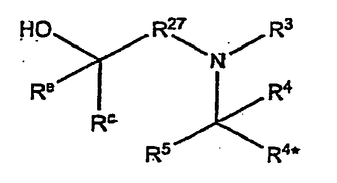 zu bilden, worin R^{e} und R^{c} unabhängig die gleichen sind wie für R^{x} definiert, worin M ein metallhaltiger Substituent ist, so dass R^{e}M ein organometallisches Reagens ist, und worin R²⁷ die gleiche Definition wie R¹ aufweist, außer dass es keinen Stickstoff, Sauerstoff oder Schwefel einschließt und keine Doppelbindungen einschließt, die mit dem oben veranschaulichten Carbonyl konjugiert sind.

38. Verfahren zur Synthese einer Verbindung nach Anspruch 1 oder 2, umfassend das Dehydratisieren einer Verbindung der Formel 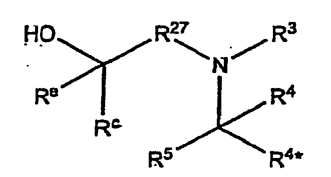 um eine Verbindung der Formel 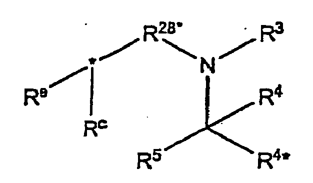 zu bilden, worin C* eine Doppelbindung mit einem benachbarten Kohlenstoff aufweist und worin R^{e} und R^{c} unabhängig die gleichen sind wie für R^{x} definiert und worin R²⁸ und R^{28*} die gleiche Definition wie R¹ aufweisen, außer dass R²⁸ und R^{28*} keinen Stickstoff, Sauerstoff oder Schwefel einschließen.

39. Verfahren zur Synthese einer Verbindung nach Anspruch 1 oder 2, umfassend die Reduktion einer Verbindung der Formel 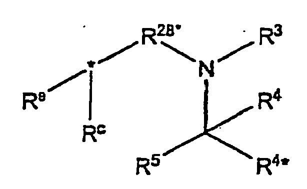 in der C^{*} eine Doppelbindung mit einem benachbarten Kohlenstoff aufweist und R^{c} unabhängig das gleiche ist wie für R^{x} definiert, um eine Verbindung der Formel 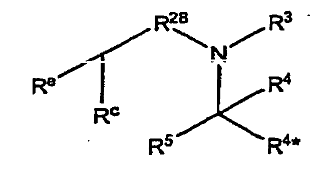 zu bilden, in der R^{e} unabhängig das gleiche ist wie für R^{x} definiert und worin R²⁸ und R^{28*} die gleiche Definition wie R¹ aufweisen, außer dass R²⁸ und R^{28*} keinen Stickstoff, Sauerstoff oder Schwefel einschließen.

## Revendications

1. Composé de formule suivante : 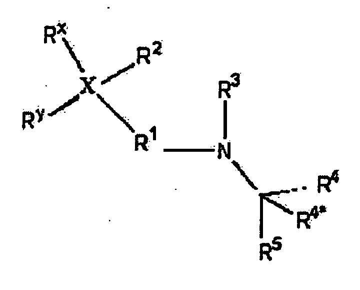 ou un sel pharmaceutiquement acceptable de celui-ci,
dans laquelle :
(1) X est un azote ou carbone, et R² n'est pas présent lorsque X est un azote ;
(2) R² (a) est un hydrogène, alkyle en C₁ à C₆, alcoxy en C₁ à C₆, cyano, alcanoyle en C₂ à C₇, aminocarbonyle, (alkyle en C₁ à C₆) aminocarbonyle ou dialkyl-aminocarbonyle dans lequel chaque alkyle est indépendamment en C₁ à C₆, (b) comprend (lorsque R¹ n'est pas un aminoéthylène, -O-R⁸ ou -S-R⁸*) un hydroxy, fluoro, chloro, bromo ou alcanoyloxy en C₂ à C₇, (c) forme une double liaison avec un carbone ou azote adjacent de l'un dé R¹, R^{xb} ou R^{yb}, ou (d) est R^{2a} lié par R^{2b} à X, ou (e) un éthylène formant une troisième structure de pontage comme présenté en (2ⁱⁱⁱ) (b) (i) ;
(2ⁱ) R^{x} est R^{xa} lié par R^{xb} à X ;
(2ⁱⁱ) R^{y} est R^{ya} lié par R^{yb} à X ;
(2ⁱⁱⁱ) R^{xa} , R^{ya} et R^{2a}, sont indépendamment un Ar qui est aryle ou hétéroaryle, adamantyle, ou un cycle non aromatique à 5 à 7 chaînons ayant de 0 à 2 hétéro-atomes choisis dans le groupe constitué d'un oxygène, soufre et azote, dans lequel au moins l'un de R^{xa}, R^{ya} et R^{2a} est un phényle, et dans lequel :
(a) aryle est un phényle ou naphtyle,
(b) hétéroaryle comprend un cycle à cinq chaînons, un cycle à six chaînons, un cycle à six chaînons accolé à un cycle à cinq chaînons, un cycle à cinq chaînons accolé à un cycle à six chaînons, ou un cycle à six chaînons accolé à un cycle à six chaînons, dans lequel l'hétéroaryle est aromatique et contient des hétéro-atomes choisis dans le groupe constitué d'un oxygène, soufre et azote, les atomes de cycle restant étant le carbone,
(c) chacun de R^{xa}, R^{ya} et R^{2a} peut être substitué ou indépendamment substitué avec l'un de R^{q}, R^{r}O- ou R^{s}S-, dans lequel chacun de R^{q}, R^{r} et R^{s} est indépendamment Ar, adamantyle ou un cycle non aromatique à 5 à 7 chaînons tel que ces structures cycliques sont définies pour R^{xa}, et
(d) R^{xa}, R^{ya}, R^{2a}, R^{q}, R^{r} et R^{s} peuvent être additionnellement substitués par un ou plusieurs substituants choisis dans le groupe constitué d'un fluoro, chloro, bromo, nitro, hydroxy, cyano, trifluorométhyle, amidosulfonyle qui peut avoir jusqu'à deux substitutions N-alkyle en C₁ à C₆ indépendantes, adamantyle, alkyle en C₁ à C₁₂, alcényle en C₂ à C₁₂, amino, alkylamino en C₁ à C₆, dialkylamino dans lequel chaque alkyle est indépendamment en C₁ à C₆, alcoxy en C₁ à C₆, alcanoyle en C₂ à C₇, alcanoyloxy en C₂ à C₇, trifluorométhoxy, hydroxycarbonyle, alkyloxycarbonyle en C₂ à C₇, aminocarbonyle qui peut avoir des hydrogènes substitués avec jusqu'à deux alkyles en C₁ à C₆ indépendants, alkylsulfonyle en C₁ à C₆, amidino qui peut être indépendamment substitué avec jusqu'à trois groupes alkyle en C₁ à C₆, ou méthylènedioxy ou éthylènedioxy avec les deux oxygènes liés à des positions adjacentes sur la structure cyclique aryle ou hétéroaryle, lequel méthylènedioxy ou éthylènedioxy peut être substitué avec jusqu'à deux alkyles en C₁ à C₆ indépendants, dans lesquels :
(i.) les substitutions de R^{xa}, R^{ya} et R^{2a} peuvent être combinées pour former un second pont entre deux de R^{xa}, R^{ya} et R^{2a} comprenant (1) un alkyle en C₁ à C₂ ou un alcényle en C₂ qui peut être indépendamment substitué par un ou plusieurs alkyles en C₁ à C₆ ou par R²,
dans lequel R² est un éthylène pour former une troisième structure de pontage, (2) le soufre, (3) l'oxygène, (4) un amino, qui peut avoir des hydrogènes substitués avec un alkyle en C₁ à C₆, (5) carbonyle, (6) -CH₂C(=O)-, qui peut avoir des hydrogènes substitués par jusqu'à deux alkyles en C₁ à C₆ indépendants, (7) -C(=O)-O-, (8) -CH₂-O-, qui peut avoir des hydrogènes substitués par jusqu'à deux alkyles en C₁ à C₆ indépendants, (9) -C(=O)N(R²⁴) , dans lequel R²⁴ est un hydrogène ou alkyle en C₁ à C₆, (10) -CH₂-NH-, qui peut avoir des hydrogènes substitués avec jusqu'à trois alkyles en C₁ à C₆, (11) -CH (=N) -, qui peut avoir des hydrogènes substitués par un alkyle en C₁ à C₆, ou dans lequel deux de R^{xa}, R^{ya} et R^{2a} peuvent être directement liés par une liaison simple ;
(2^{iv}) R^{xb} et R^{2b} sont indépendamment une liaison simple ou alkylène en C₁ à C₂ ;
(2^{v}) R^{yb} est une liaison simple, oxo, alkylène en C₁ à C₂, éthénylène ou -CH= (où la double liaison est avec X), thio, méthylèneoxy ou méthylènethio, ou -N(R⁶) ou -CH₂-N(R^{6*}) -, dans lesquels R⁶ et R^{6*} sont un hydrogène ou alkyle en C₁ à C₆, dans lesquels lorsque X est un azote, X n'est pas lié à un autre hétéroatome ;
(3) R¹ comprend : un groupe aliphatique en C₂ à C₃ à chaîne linéaire ; dans lequel X est un carbone, =N-O-(éthylène), dans lequel la double liaison non jointe est liée à X, dans lequel X est un carbone et R^{yb} ne comprend pas d'hétéroatome lié à X, -O-R⁸ ou -S-R^{8*} dans lequel R⁸ ou R^{8*} est un éthylène ou éthénylène et O ou S est lié à X, dans lequel X est un carbone et R^{yb} ne comprend pas un hétéroatome lié à X ; aminoéthylène où l'amino est lié à X :
dans lequel R¹ peut être substitué avec jusqu'à un hydroxy, jusqu'à un alcoxy en C₁ à C₆ ou jusqu'à un alcanoyloxy en C₂ à C₇, avec jusqu'à deux alkyles en C₁ à C₆ indépendants, avec jusqu'à un oxo, jusqu'à un alkylidène en C₁ à C₆, à la condition que les substituants hydroxy, alcoxy, alcanoyloxy ou oxo ne soient pas liés à un carbone qui est lié à un azote ou oxygène ;
dans lequel les substituants alkyle ou alkylidène de R¹ peuvent être liés pour former un cycle non aromatique à 3 à 7 chaînons ; et
dans lequel si X est un azote, X est lié à R¹ par une liaison simple et le carbone terminal de R¹ qui lie R¹ à N est saturé ;
(4) R³ est un hydrogène, alkyle en C₁ à C₆, ou phényle ou phénylalkyle dans lequel l'alkyle est en C₁ à C₆ et un tel phényle peut être substitué par les mêmes substituants définis ci-dessus pour un aryle ou hétéroaryle de R^{xa}; et (5) R⁴ et R^{4*} sont un hydrogène ; et
(6) R⁵ est (CO)NR¹³R¹⁴, (CO)OR¹⁵, (CO)SR¹⁶, (SO₂)NR¹⁷R¹⁸, (PO) (OR¹⁹) (OR²⁰), (CR²²) (OR²³) (OR²⁴) , CN ou un tétrazol-5-yle, dans lequel (a) R¹³, R¹⁴, R¹⁵, R¹⁶, R¹⁷, R¹⁸, R¹⁹ et R²⁰ sont indépendamment un hydrogène, alkyle en C₁ à C₈ qui peut comprendre un cycloalkyle en C₃ à C₈, dans lequel le carboné lié à l'oxygène de R¹⁵ ou au soufre de R¹⁶ n'a pas plus d'une ramification secondaire, et hydroxyalkyle en C₂ à C₆, aminoalkyle où l'alkyle est en C₂ à C₆ et l'amino peut être substitué avec jusqu'à deux alkyles en C₁ à C₆ indépendants, Ar-alkyle dans lequel l'alkyle est en C₁ à C₆ ou Ar, (b) R²² est un hydrogène ou OR²⁵ et (c) R²³, R²⁴ et R²⁵ sont un alkyle en C₁ à C₆, phényle, benzyle, acétyle ou, lorsque R²² est un hydrogène, les alkyles de R²³ et R²⁴ peuvent être combinés pour inclure le 1,3-dioxolane ou 1,3-dioxane :
dans lequel les groupes Ar de R¹³, R¹⁴, R¹⁵, R¹⁶, R¹⁷, R¹⁸, R¹⁹, R²⁰, R²², R²³ ou R²⁴ peuvent être substitués avec des substituants choisis dans le groupe constitué d'un fluoro, chloro, bromo, nitro, cyano, hydroxy, trifluorométhylé, amidosulfonyle qui peut avoir jusqu'à deux substitutions N-alkyle en C₁ à C₆ indépendantes, alkyle en C₁ à C₆, alcényle en C₂ à C₆, alkylamine en C₁ à C₆, dialkylamine dans laquelle chaque alkyle est indépendamment en C₁ à C₆, amino, alcoxy en C₁ à C₆, alcanoyle en C₂ à C₇, alcanoyloxy en C₂ à C₇, trifluorométhoxy, hydroxycarbonyle, alkyloxycarbonyle en C₂ à C₇, aminocarbonyle qui peut être N-substitué avec jusqu'à deux alkyles en C₁ à C₆ indépendants, alkyl sul f onyl e en C₁ à C₆, amidino qui peut être substitué avec jusqu'à trois alkyles en C₁ à C₆, ou méthylènedioxy ou éthylènedioxy avec les deux oxygènes liés à des positions adjacentes sur la structure cyclique aryle ou hétéroaryle, lequel méthylènedioxy ou éthylènedioxy peut être substitué avec jusqu'à deux alkyles en C₁ à C₆ indépendants ;
dans lequel R¹³ et R¹⁴ ensemble avec l'azote peuvent former un cycle à 5 à 7 chaînons qui peut contenir un hétéroatome additionnel choisi parmi l'oxygène et le soufre ;
et dans lequel les conditions suivantes s'appliquent :
si R¹⁵ est un hydrogène et R¹ un propylène, alors au moins l'une des propositions suivantes s'applique (1) à la fois R^{x} et R^{y} ne sont pas un p-fluorophényle, (2) l'un de R^{x} et R^{y} comprend un hétéroaryle, (3) R^{y} est Ar- (alkyle en C₁ à C₂), Ar-oxy, Ar-méthoxy, Ar-thio, Ar-méthylthio, Ar-N(R⁶)- ou Ar-CH₂-N(R^{6*}) -, (4) R² est R^{2a}R^{2b}-, (5) R² n'est pas un hydrogène, ou (6) R³ n'est pas un hydrogène;
si R¹⁵ est un hydrogène et R¹ un éthylène ou X-R¹ est un prop-1-énylène, alors au moins l'une des propositions suivantes s'applique (1) un aryle d'au moins un de R^{x} et R^{y} est substitué par un radical différent d'un hydrogène, (2) l'un de R^{x} et R^{y} comprend un hétéroaryle, (3) R^{y} est Ar-(alkyle en C₁ à C₂), Ar-oxy, Ar-méthoxy, Ar-thio, Ar-méthylthio, Ar-N(R⁶)- ou Ar-CH₂-N(R^{6*}) -, (4) R² est R^{2a}R^{2b}- (5) R^{2*} n'est pas un hydrogène, ou (6) R³ n'est pas un hydrogène ;
si R⁵ est un C (O) NR¹³R¹⁴, dans lequel R¹³ et R¹⁴ sont un hydrogène, alkyle en C₁ à C₈, phényle ou phényle substitué, alors au moins l'une des propositions suivantes s'applique (1) un aryle d'au moins un de R^{x} et R^{y} est substitué par un radical différent d'un hydrogène, (2) l'un de R^{x} et R^{y} comprend un hétéroaryle, (3) R^{y} est Ar-(alkyle en C₁ à C₂), Ar-oxy, Ar-méthoxy, Ar-thio, Ar-méthylthio, Ar-N(R⁶)- ou Ar-CH₂-N(R^{6*}) -, (4) R² est R^{2a}R^{2b}-, (5) R^{2*} n'est pas un hydrogène, (6) R³ n'est pas un hydrogène, ou (7) R¹ n'est pas un éthylène ;
dans lequel si R² est un phényle ou p-méthylphényle, alors au moins l'une des propositions suivantes s'applique (1) les aryles de R^{x} et R^{y} se sont pas substitués par un *p*-méthylphényle ou *p*-méthoxyphényle, (2) un aryle d'au moins un de R^{x} et R^{y} est substitué avec un radical différent d'un hydrogène, (3) l'un de R^{x} et R^{y} comprend un hétéroaryle, (4) R^{y} est Ar- (alkyle en C₁ à C₂), Ar-oxy, Ar-méthoxy, Ar-thio, Ar-méthylthio, Ar-N(R⁶)- ou Ar-CH₂-N (R⁶*) -, ou (5) R¹ n'est pas l'aminoéthylène, OR⁸ ou SR⁸*;
dans lequel si R^{2a} est le *p*-méthoxyphényle, alors au moins l'une des propositions suivantes s'applique (1) un Ar d'au moins un de R^{x} et R^{y} est substitué par un radical différent d'un hydrogène, (2) R^{y} est Ar-(alkyle en C₁ à C₂), Ar-oxy, Ar-méthoxy, Ar-thio, Ar-méthylthio, Ar-N(R⁶) - ou Ar-CH₂-N(R^{6*}) -, ou (3) R¹ n'est pas OR⁸ ou SR^{8*}.

2. Composé selon la revendication 1 de formule suivante : 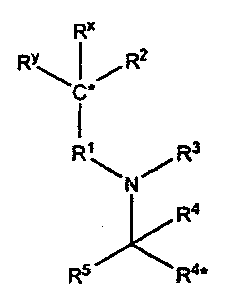 ou un sel pharmaceutiquement acceptable de celui-ci,
dans laquelle :
(1) C^{*} est un carbone substitué ;
(2) R² (a) est un hydrogène, alkyle en C₁ à C₆, alcoxy en C₁ à C₆, cyano, alcanoyle en C₂ à C₇, aminocarbonyle, (alkyle en C₁ à C₆)aminocarbonyle ou dialkylaminocarbonyle dans lequel chaque alkyle est indépendamment en C₁ à C₆, (b) comprend (lorsque R¹ n'est pas un aminoéthylène, -O-R⁸ ou -S-R^{8*}) un hydroxy, fluoro, chloro, bromo ou alcanoyloxy en C₂ à C₇, (c) forme une double liaison avec un carbone ou azote adjacent de l'un ou des deux de R¹, R^{xb}, R^{yb}, (d) est R^{2a} lié par R^{2b} à C*, ou (e) un éthylène formant une troisième structure de pontage comme présenté en
(2ⁱⁱⁱ) (b) (i) ;
(2ⁱ) R^{x} est R^{xa} lié par R^{xb} à C* ;
(2ⁱⁱ) R^{y} est R^{ya} lié par R^{yb} à C* ;
(2ⁱⁱⁱ) R^{xa} , R^{ya} et R^{2a}, sont indépendamment un Ar, qui est un phényle ou naphtyle, ou un cycle non aromatique de 5 à 7 chaînons ayant 0 hétéroatome dans lequel .
(a) chacun de R^{xa} et R^{ya} peut être indépendamment substitué par un de R^{q}, R^{r}O- ou R^{s}S-, dans lequel chacun de R^{q}, R^{r} et R^{s} est indépendamment Ar ou adamantyle, et
(b) R^{xa}, R^{ya}, R^{2a}, R^{q}, R^{r} et R^{s} peuvent être substitués ou additionnellement substitués par un ou plusieurs substituants choisis dans le groupe constitué d'un fluoro, chloro, bromo, nitro, hydroxy, cyano, trifluorométhyle, amidosulfonyle qui peut avoir jusqu'à deux substitutions N-alkyle en C₁ à C₆ indépendantes, alkyle en C₁ à C₁₂, alcényle en C₂ à C₁₂, amino, alkylamino en C₁ à C₆, dialkylamino dans lequel chaque alkyle de dialkylamino est indépendamment en C₁ à C₆, alcoxy en C₁ à C₆, alcanoyle en C₂ à C₇, alcanoyloxy en C₂ à C₇, trifluorométhoxy, hydroxycarbonyle, alkyloxycarbonyle en C₂ à C₇, aminocarbonyle dont les hydrogènes peuvent être substitués avec jusqu'à deux alkyles en C₁ à C₆ indépendants, alkylsulfonyle en C₁ à C₆ ou amidino qui peut être indépendamment substitué avec jusqu'à trois alkyles en C₁ à C₆, dans lesquels :
(i.) les substitutions de R^{xa} et R^{ya} peuvent être combinées pour former un second pont entre R^{xa} et R^{ya} comprenant (1) le méthylène ou l'éthylène, lequel méthylène ou éthylène peut être substitué par un R² lorsque R² est l'éthylène pour former la troisième structure de pontage, (2) -CH=CH- ou dans laquelle R^{xa} et R^{ya} peuvent être directement liés par une liaison simple ;
(2^{iv}) R^{xb} et R^{2b} sont indépendamment une liaison simple ou un alkylène en C₁ à C₂ ;
(2^{v}) R^{yb} est une liaison simple, oxa, alkylène en C₁ à C₂, éthénylène ou -CH= (où la double liaison est avec C*), thia, méthylèneoxy ou méthylènethio, ou l'un de -N (R⁶) ou -CH₂N (R^{6*}) -, dans lesquels R⁶ et R^{6*} sont un hydrogène ou alkyle en C₁ à C₆ ;
(3) R¹ comprend : un groupe aliphatique en C₂ à C₃ à chaîne linéaire ; =N-O-(éthylène), dans lequel la double liaison non jointe est liée à C* ; -O-R⁸ ou -S-R-8* dans lequel R⁸ ou R^{8*} est un éthylène ou éthénylène et O ou S est lié à C* ; aminoéthylène où l'amino est lié à C* :
dans lequel R¹ peut être substitué avec jusqu'à un hydroxy, jusqu'à un alcoxy en C₁ à C₆ ou jusqu'à un alcanoyloxy en C₂ à C₇, avec jusqu'à deux alkyles en C₁ à C₆ indépendants, avec jusqu'à un oxo, jusqu'à un alkylidène en C₁ à C₆, sous réserve que les substituants hydroxyle, alcoxy, alcanoyloxy ou oxo ne soient pas liés à un carbone qui est lié à un azote ou oxygène ; et
dans lequel les substituants alkyle ou alkylidène de R¹ peuvent être liés pour former un cycle non aromatique à 3 à 7 chaînons ;
(4) R³ (a) est un hydrogène, alkyle en C₁ à C₆, ou phényle ou phénylalkyle dans lequel l'alkyle est en C₁ à C₆ et le phényle ou phényle de phénylalkyle peut être substitué par les mêmes substituants définis ci-dessus pour le phényle de R^{xa}, (b) est -R¹²C (R^{xx}) (R^{yy}) (R¹¹), dans lequel R¹² est lié à N, R^{xx} est indépendamment identique à R^{x}, R^{yy} est indépendamment identique à R^{y}, R¹¹ est indépendamment identique à R² et R¹² est indépendamment identique à R¹ ;
(5) R⁴ et R^{4*} sont indépendamment un hydrogène ; et
(6) R⁵ est (CO)NR¹³R¹⁴, (CO)OR¹⁵, (CO)SR¹⁶, (SO₂)NR¹⁷R¹⁸, (PO) (OR¹⁹) (OR²⁰), (CR²²) (OR²³) (OR²⁴) , CN ou un tétrazol-5-yle, dans lequel (a) R¹³, R¹⁴, R¹⁵, R¹⁶, R¹⁷, R¹⁸, R¹⁹ et R²⁰ sont indépendamment un hydrogène, alkyle en C₁ à C₈ qui peut comprendre un cycloalkyle en C₃ à C₈, dans lequel le carboné lié à l'oxygène de R¹⁵ ou au soufre de R¹⁶ n'a pas plus d'une ramification secondaire et, hydroxyalkyle en C₂ à C₆, aminoalkyle où l'alkyle est en C₂ à C₆ et l'amino peut être substitué avec jusqu'à deux alkyles en C₁ à C₆ indépendants, Ar-alkyle dans lequel l'alkyle est en C₁ à C₆ ou Ar, et (b) R²² est un hydrogène ou OR²⁵ et R²³, R²⁴ et R²⁵ sont indépendamment un alkyle en C₁ à C₆, phényle, benzyle ou acétyle ou, les alkyles de R²³ et R²⁴ peuvent être combinés pour comprendre le 1,3-dioxolane ou 1,3-dioxane :
dans lequel les groupes phényle ou naphtyle de R¹³, R¹⁴, R¹⁵, R¹⁶, R¹⁷, R¹⁸, R¹⁹, R²⁰, R²², R²³ ou R²⁴ peuvent être substitués avec des substituants choisis dans le groupe constitué d'un fluoro, chloro, bromo, nitro, cyano, hydroxy, trifluorométhyle, amidosulfonyle qui peut avoir jusqu'à deux substitutions N-alkyle en C₁ à C₆ indépendantes, alkyle en C₁ à C₆, alcényle en C₂ à C₆, alkylamine en C₁ à C₆, dialkylamine dans laquelle chaque alkyle est indépendamment en C₁ à C₆, amino, alcoxy en C₁ à C₆, alcanoyle en C₂ à C₇, alcanoyloxy en C₂ à C₇, trifluorométhoxy, hydroxycarbonyle, alkyloxycarbonyle en C₂ à C₇, aminocarbonyle qui peut être N-substitué avec jusgu'à deux alkyles en C₁ à C₆ indépendants, alkylsulfonyle en C₁ à C₆, ou amidino qui peut être substitué avec jusqu'à trois alkyles en C₁ à C₆ ;
dans lesquels R¹³ et R¹⁴ ensemble avec l'azote lié peuvent former un cycle à 5 à 7 chaînons ; et dans lesquels en outre les conditions suivantes s'appliquent :
si R¹⁵ est un hydrogène et R¹ un propylène, alors au moins l'une des propositions suivantes s'applique (1) à la fois R^{xa} et R^{ya} ne sont pas un *p*-fluorophényle, (2) R^{y} est Ar-alkyle, Ar-oxy, Ar-méthoxy, Ar-thio, Ar-méthylthio, Ar-N(R⁶)- ou Ar-CH₂-N(R⁶*)-, (3) R² est R^{2a}R^{2b}-, (4) R² n'est pas un hydrogène, ou (5) R³ n'est pas un hydrogène ;
si R¹⁵ est un hydrogène et R¹ un éthylène ou C*R¹ est le prop-1-énylène, alors au moins l'une des propositions suivantes s'applique (1) un aryle d'au moins l'un de R^{x} et R^{y} est substitué par un radical différent de l'hydrogène, (2) R^{y} est Ar-alkyle, Ar-oxy, Ar-méthoxy, Ar-thio, Ar-méthylthio, Ar-N (R⁶) - ou Ar-CH₂-N (R^{6*}) -, (3) R² est R^{2a}R^{2b}-, (4) R² n'est pas un hydrogène, ou (5) R³ n'est pas un hydrogène ;
si R⁵ est un C(O)NR¹³R¹⁴, dans lequel R¹³ et R¹⁴ sont un hydrogène, alkyle en C₁ à C₈, phényle ou phényle substitué, alors au moins l'une des propositions suivantes s'applique (1) un aryle d'au moins un de R^{x} et R^{y} est substitué par un radical différent d'un hydrogène, fluoro, chloro ou bromo, (2) R^{y} est Ar-alkyle, Ar-oxy, Ar-méthoxy, Ar-thio, Ar-méthylthio, Ar-N(R⁶) - ou Ar-CH₂-N(R^{6*}) -, (3) R² est R^{2a}R^{2b}- (4) R² n'est pas un hydrogène, (5) R³ n'est pas un hydrogène, ou (6) R¹ n'est pas un éthylène ;
si R^{2a} est un phényle ou *p*-méthylphényle, alors au moins l'une des propositions suivantes s'applique (1) les aryles de R^{x} et R^{y} se sont pas substitués par un p-méthylphényle ou *p*-méthoxyphényle, (2) un aryle d'au moins un de R^{x} et R^{y} est substitué par un radical différent d'un hydrogène, (3) R^{y} est Ar-alkyle, Ar-oxy, Ar-méthoxy, Ar-thio, Ar-méthylthio, Ar-N(R⁶)- ou Ar-CH₂-N(R^{6*})-, ou (5) R¹ n'est pas un aminoéthylène, OR⁸ ou SR^{8*};
si R^{2a} est un p-méthoxyphényle, alors au moins l'une des propositions suivantes s'applique (1) un Ar d'au moins un de R^{x} et R^{y} est substitué par un radical différent d'un hydrogène, (2) R^{y} est Ar-(alkyle en C₁ à C₂), Ar-oxy, Ar-méthoxy, Ar-thio, Ar-méthylthio, Ar-N(R⁶)- ou Ar-CH₂-N(R^{6*})-, ou (3) R¹ n'est pas OR⁸ ou SR⁸*.

3. Composé selon la revendication 1 ou 2, dans lequel (A) au moins un de R^{xa}, R^{ya} et R^{2a} est substitué par un fluoro, chloro, bromo, hydroxy, trifluorométhyle, trifluorométhoxy, nitro, cyano, alkyle en C₃ à C₈, R^{q}, R^{r}O-, R^{s}S-, (B) R³ est un hydrogène, alkyle en C₁ à C₆, ou phényle ou phénylalkyle dans lequel l'alkyle est en C₁ à C₆ et un tel phényle peut être substitué par les mêmes substituants que définis ci-dessus pour l'aryle ou hétéroaryle de R^{xa} ou (C) les structures cycliques de R^{xa}, R^{ya} et R^{2a}, y compris leurs substituants, comprennent autrement au moins deux structures cycliques aromatiques qui comprennent ensemble de 15 à 20 atomes de cycle.

4. Composé selon la revendication 4, dans lequel au moins un de R^{xa}, R^{ya} et R^{2a} est substitué par un fluoro, trifluorométhyle, trifluorométhoxy, nitro, cyano ou alkyle en C₃ à C₈.

5. Composé selon la revendication 1 ou 2, dans lequel au moins un de R^{xa}, R^{ya} et R^{2a} est substitué par R^{q}, R^{r}O- ou R^{s}S- .

6. Composé selon la revendication 1 ou 2, dans lequel R^{yb} est un oxy, méthylèneoxy, thio ou méthylènethio.

7. Composé selon la revendication 6, dans lequel R^{yb} est un oxy ou thio.

8. Composé selon la revendication 1 ou 2, dans lequel R⁵ est (CO)NR¹³R¹⁴, (CO)OR¹⁵ ou (CO)SR¹⁶.

9. Composé selon la revendication 8, dans lequel R¹⁵ est un alkyle en C₂ à C₆, hydroxyalkyle en C₂ à C₄, phényle, phénylalkyle dans lequel l'alkyle est en C₁ à C₃, ou aminoalkyle où l'alkyle est en C₂ à C₆ et l'amino peut être substitué avec jusqu'à deux alkyles en C₁ à C₃ indépendants, dans lequel le phényle ou le phényle du phénylalkyle peut être substitué.

10. Composé selon la revendication 8, dans lequel R¹⁵ est un hydrogène.

11. Composé selon la revendication 1 ou 2, dans lequel R⁴ est un hydrogène, méthyle ou hydroxyméthyle et R^{4*} est un hydrogène.

12. Composé selon la revendication 1, dans lequel au moins un de R^{xa}, R^{ya} et R^{2a} est un hétéroaryle comprenant un diazolyle, triazolyle, tétrazolyle, thiazolyle, isothiazolyle, oxazolyle, isoxazolyle, thiolyle, diazinyle, triazinyle, benzoazolyle, benzodiazolyle, benzothiazolyle, benzoxazolyle, benzoxolyle, benzothiolyle, quinoléyle, isoquinoléyle, benzodiazinyle, benzotriazinyle, pyridyle, thiényle, furanyle, pyrrolyle, indolyle, isoindolyle ou pyrimidyle.

13. Composé selon la revendication 1 ou 2, dans lequel R¹ est -O-R⁸ ou -S-R^{8*}.

14. Composé selon la revendication 1 ou 2, dans lequel ledit second pont entre deux de R^{xa}, R^{ya} et R^{2a} est L, et satisfait à la formule suivante : 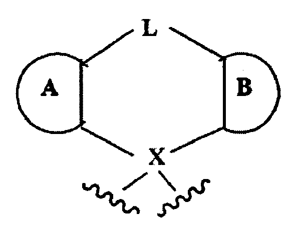 dans laquelle A et B sont des groupes aryle ou hétéroaryle de R^{xa} et R^{ya}, respectivement.

15. Composé selon la revendication 12, dans lequel R^{xa}-R^{xb}, R^{ya}-R^{yb} et X forment : 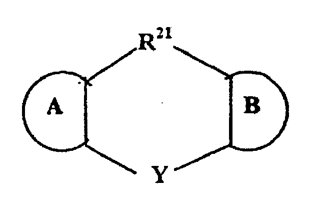 dans lequel Y est un carbone lié à R¹ par une liaison simple ou double ou un azote qui est lié à R¹ et dans lequel R²¹ (i.) représente une liaison simple liant deux cycles aryle ou hétéroaryle de R^{x} et R^{y}, (ii.) est un alkylène en C₁ à C₂ ou alcénylène, (iii.) est un soufre ou (iv.) est un oxygène, et dans lequel R^{x} et R^{y} peuvent être substitués comme présenté ci-dessus.

16. Composé selon la revendication 15, dans lequel R²¹ est CH₂CH₂ ou CH=CH.

17. Composé selon la revendication 1, dans lequel la substitution alkylènedioxy de R^{xa}, R^{ya} ou R^{2a} est comme suit : 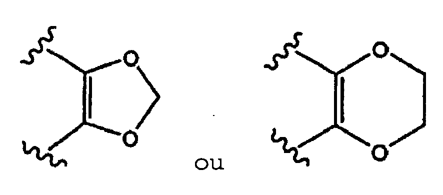 dans lequel l'alkylènedioxy peut être substitué avec jusqu'à deux alkyles en C₁ à C₃ indépendants.

18. Composé selon la revendication 1 ou 2, dans lequel R^{xa} et R^{ya} peuvent être substitués ensemble avec jusqu'à six substituants, R^{2a}, R^{a}, R^{r} et R^{s} peuvent chacun être substitués avec jusqu'à 3 substituants, et dans lequel la présence de chacun de R^{q}, R^{r} ou R^{s} est considérée comme une substitution à la structure cyclique respective de ^{Rxa}_{,} ^{Rya} et R^{2a}.

19. Composé selon la revendication 1 ou 2, dans lequel un phényle de R³ est substitué avec jusqu'à trois substituants.

20. Composé selon la revendication 1 ou 2, dans lequel l'aryle, hétéroaryle, aryle ou arylalkyle ou l'hétéroaryle ou hétéroarylalkyle de R¹³, R¹⁴, R¹⁵, R¹⁶, R¹⁷,R¹⁸, R¹⁹ ou R²⁰ est substitué avec jusqu'à trois substituants.

21. Composé selon la revendication 1 ou 2, dans lequel le composé est un énantiomère optiquement pur.

22. Composé selon la revendication 1 ou 2, dans lequel :
(1) R² est un hydrogène,
(2) R^{xa} et R^{ya} sont tous deux un phényle et au moins un de R^{xa} et R^{ya} est substitué par un du phényle, phénoxy ou phénylthio,
(3) R^{xb} est une liaison simple et R^{yb} est une liaison simple ou oxa, et
(4) R⁵ est (CO)NR¹³R¹⁴ ou (CO)OR¹⁵, dans lequel R¹³, R¹⁴ et R¹⁵ sont indépendamment un hydrogène, alkyle en C₁ à C₈ qui peut comprendre un cycloalkyle en C₃ à C₈, dans lequel le carboné lié à l'oxygène de OR¹⁵ n'a pas plus d'une ramification secondaire ; hydroxyalkyle en C₂ à C₆ ou aminoalkyle où l'alkyle est en C₂ à C₆ et l'amino peut être substitué avec jusqu'à deux alkyles en C₁ à C₆ ou phénylalkyles, dans lequel l'alkyle est en C₁ à C₆ et le phényle peut être substitué par des substituants choisis dans le groupe constitué d'un fluoro, chloro, bromo, nitro, cyano, hydroxy, trifluorométhyle, amidosulfonyle qui peut avoir jusqu'à deux substitutions N-alkyle en C₁ à C₆ indépendantes, alkyle en C₁ à C₆, alcényle en C₂ à C₆, alkylamine en C₁ à C₆, dialkylamine dans laquelle chaque alkyle est indépendamment en C₁ à C₆, amino, alcoxy en C₁ à C₆, alcanoyle en C₂ à C₇, alcanoyloxy en C₂ à C₇, trifluorométhoxy, hydroxycarbonyle, alkyloxycarbonyle en C₂ à C₇, aminocarbonyle qui peut être N-substitué avec jusqu'à deux alkyles en C₁ à C₆ indépendants, alkylsulfonyle en C₁ à C₆, amidino qui peut être substitué avec jusqu'à trois alkyles en C₁ à C₆.

23. Composition pharmaceutique comprenant le composé selon la revendication 1 ou 2 et un excipient pharmaceutiquement acceptable.

24. Composition pharmaceutique selon la revendication 23, dans laquelle le composé selon la revendication 1 ou 2 est présent en une quantité efficace pour :
(1) traiter ou prévenir la schizophrénie,
(2) améliorer le traitement ou la prévention de la démence,
(3) traiter ou prévenir l'épilepsie,
(4) traiter ou prévenir la spasticité,
(5) traiter ou prévenir un spasme musculaire,
(6) traiter ou prévenir la douleur,
(7) prévenir la mort cellulaire neurale après un accident vasculaire cérébral,
(8) prévenir la mort cellulaire neurale chez un animal souffrant d'une maladie neurodégénérative,
(9) traiter ou prévenir les troubles de l'humeur,
(10) améliorer la mémoire ou l'apprentissage, ou
(11) traiter ou prévenir les troubles de l'apprentissage.

25. Utilisation d'un composé de formule : 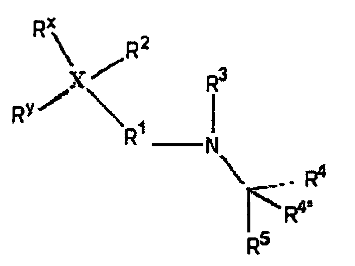 ou d'un sel pharmaceutiquement acceptable de celui-ci,
dans laquelle
(1) X est un azote ou carbone, et R² n'est pas présent lorsque X est un azote ;
(2) R² (a) est un hydrogène, alkyle en C₁ à C₆, alcoxy en C₁ à C₆, cyano, alcanoyle en C₂ à C₇, aminocarbonyle, (alkyle en C₁ à C₆)aminocarbonyle ou dialkylaminocarbonyle, dans lequel chaque alkyle est indépendamment en C₁ à C₆, (b) comprend (lorsque X n'est pas un aminoéthylène, -O-R⁸ ou -S-R^{8*}) un hydroxy, fluoro, chloro, bromo ou alcanoyloxy en C₂ à C₇, (c) forme une double liaison avec un carbone ou azote adjacent de l'un de R¹, R^{xb} ou R^{yb}, ou (d) est R^{2a} lié par R^{2b} à X, ou (e) un éthylène formant une troisième structure de pontage comme présenté dans
(2ⁱⁱⁱ) (b) (i) ;
(2ⁱ) R^{x} est R^{xa} lié par R^{xb} à X ;
(2ⁱⁱ) R^{y} est R^{ya} lié par R^{yb} à X;
(2ⁱⁱⁱ) R^{xa}, R^{ya} et R^{2a}, sont indépendamment un Ar qui est aryle ou hétéroaryle, adamantyle ou un cycle non aromatique à 5 à 7 chaînons ayant de 0 à 2 hétéro-atomes choisis dans le groupe constitué d'un oxygène, soufre et azote, dans lequel au moins un de R^{xa}, R^{ya} et R^{2a} est un phényle, et dans lequel :
(a) aryle est un phényle ou naphtyle,
(b) hétéroaryle comprend un cycle à cinq chaînons, un cycle à six chaînons, un cycle à six chaînons accolé à un cycle à cinq chaînons, un cycle à cinq chaînons accolé à un cycle à six chaînons ou un cycle à six chaînons accolé à un cycle à six chaînons, dans lequel l'hétéroaryle est aromatique et contient des hétéro-atomes choisis dans le groupe constitué d'un oxygène, soufre et azote, les atomes de cycle restant étant du carbone,
(c) chacun de R^{xa}, R^{ya} et R^{2a} peut être substitué ou indépendamment substitué avec l'un de R^{q}, R^{r}O- ou R^{s}S-, dans lequel chacun de R^{q}, R^{r} et R^{s} est indépendamment Ar, adamantyle ou un cycle non aromatique à 5 à 7 chaînons tel que ces structures cycliques sont définies pour R^{xa}, et
(d) R^{xa}, R^{ya}, R^{2a}, R^{q}, R^{r} et R^{s} peuvent être additionnellement substitués par un ou plusieurs substituants choisis dans le groupe constitué d'un fluoro, chloro, bromo, nitro, hydroxy, cyano, trifluorométhyle, amidosulfonyle qui peut avoir jusqu'à deux substitutions N-alkyle en C₁ à C₆ indépendantes, adamantyle, alkyle en C₁ à C₁₂, alcényle en C₂ à C₁₂, amino, alkylamino en C₁ à C₆, dialkylamino dans lequel chaque alkyle est indépendamment en C₁ à C₆, alcoxy en C₁ à C₆, alcanoyle en C₂ à C₇, alcanoyloxy en C₂ à C₇, trifluorométhoxy, hydroxycarbonyle, alkyloxycarbonyle en C₂ à C₇, aminocarbonyle qui peut avoir des hydrogènes substitués avec jusqu'à deux alkyles en C₁ à C₆ indépendants, alkylsulfonyle en C₁ à C₆, amidino qui peut être indépendamment substitué par jusqu'à trois groupes alkyle en C₁ à C₆, ou méthylènedioxy ou éthylènedioxy avec les deux oxygènes liés à des positions adjacentes sur la structure cyclique aryle ou hétéroaryle, lequel méthylènedioxy ou éthylènedioxy peut être substitué avec jusqu'à deux alkyles en C₁ à C₆ indépendants, dans lesquels :
(i.) les substitutions de R^{xa}, R^{ya} et R^{2a} peuvent être combinées pour former un second pont entre deux de R^{xa}, R^{ya} et R^{2a} comprenant (1) un alkyle en C₁ à C₂ ou un alcényle en C₂ qui peut être indépendamment substitué par un ou plusieurs alkyles en C₁ à C₆ ou par R², dans lequel R² est l'éthylène pour former une troisième structure de pontage, (2) soufre, (3) oxygène, (4) amino, dont les hydrogènes peuvent être substitués par un alkyle en C₁ à C₆, (5) carbonyle, (6) -CH₂C(=O)-, dont les hydrogènes peuvent être substitués par jusqu'à deux alkyles en C₁ à C₆ indépendants, (7) -C(=O)-O-, (8) -CH₂-O-, dont les hydrogènes peuvent être substitués par jusqu'à deux alkyles en C₁ à C₆ indépendants, (9) -C(=O)N(R²⁴) , dans lequel R²⁴ est un hydrogène ou alkyle en C₁ à C₆, (10) -CH₂-NH-, dont les hydrogènes peuvent être substitués par jusqu'à trois alkyles en C₁ à C₆, ou (11) -CH(=N)-, dont l'hydrogène peut être substitué par un alkyle en C₁ à C₆, ou dans lequel deux de R^{xa}, R^{ya} et R^{2a} peuvent être directement liés par une liaison simple ;
(2^{iv}) R^{xb} et R^{2b} sont indépendamment une liaison simple ou un alkylène en C₁ à C₂ ;
(2^{v}) R^{yb} est une liaison simple, oxo, alkylène en C₁ à C₂, éthénylène ou -CH= (où la double liaison est avec X), thio, méthylèneoxy ou méthylènethio, ou -N(R⁶) ou -CH₂-N(R^{6*})-, dans lesquels R⁶ et R^{6*} sont un hydrogène ou alkyle en C₁ à C₆, dans lesquels lorsque X est un azote, X n'est pas lié à un autre hétéroatome ;
(3) R¹ comprend un groupe aliphatique à chaîne linéaire en C₂ à C₃ ; où X est un carbone, =N-O-(éthylène), dans lequel la double liaison non jointe est liée à X ; (où X est un carbone et R^{yb} ne comprend pas d'hétéroatome lié à X), -O-R⁸ ou -S-R^{8*} dans lequel R⁸ ou R^{8*} est un éthylène ou éthénylène et O ou S est lié à X ; (où X est un carbone et R^{yb} ne comprend pas d'hétéroatome lié à X), aminoéthylène où l'amino est lié à X :
dans lequel R¹ peut être substitué avec jusqu'à un hydroxy, jusqu'à un alcoxy en C₁ à C₆ ou jusqu'à un alcanoyloxy en C₂ à C₇, avec jusqu'à deux alkyles en C₁ à C₆ indépendants, avec jusqu'à un oxo, jusqu'à un alkylidène en C₁ à C₆, sous réserve que les substituants hydroxy, alcoxy, alcanoyloxy ou oxo ne soient pas liés à un carbone qui est lié à un azote ou oxygène ;
dans lequel les substituants alkyle ou alkylidène de R¹ peuvent être liés pour former un cycle non aromatique à 3 à 7 chaînons ; et
dans lequel si X est un azote, X est lié à R¹ par une liaison simple et le carbone terminal de R¹ qui lie R¹ à N est saturé ;
(4) R³ est un hydrogène, alkyle en C₁ à C₆, ou phényle ou phénylalkyle dans lequel l'alkyle est en C₁ à C₆ et un tel phényle peut être substitué par les mêmes substituants définis ci-dessus pour l'aryle ou hétéroaryle de R^{xa};
(5) R⁴ et R^{4*} sont indépendamment un hydrogène ; (6) R⁵ est (CO)NR¹³R¹⁴, (CO)OR¹⁵, (CO)SR¹⁶, (SO₂)NR¹⁷R¹⁸, (PO) (OR¹⁹) (OR²⁰), (CR²²) (OR²³) (OR²⁴) , CN ou un tétrazol-5-yle, dans lequel (a) R¹³, R¹⁴, R¹⁵, R¹⁶, R¹⁷, R¹⁸, R¹⁹ et R²⁰ sont indépendamment un hydrogène, alkyle en C₁ à C₈ qui peut comprendre un cycloalkyle en C₃ à C₈, dans lequel le carboné lié à l'oxygène de R¹⁵ ou au soufre de R¹⁶ n'a pas plus d'une liaison ramifiée secondaire et, hydroxyalkyle en C₂ à C₆, aminoalkyle où l'alkyle est en C₂ à C₆ et l'amino peut être substitué avec jusqu'à deux alkyles en C₁ à C₆ indépendants, Ar-alkyle dans lequel l'alkyle est en C₁ à C₆ ou Ar, (b) R²² est un hydrogène ou OR²⁵ et (c) R²³, R²⁴ et R²⁵ sont un alkyle en C₁ à C₆, phényle, benzyle, acétyle ou, lorsque R²² est un hydrogène, les alkyles de R²³ et R²⁴ peuvent être combinés pour comprendre le 1,3-dioxolane ou 1,3-dioxane :
dans lequel les groupes Ar de R¹³, R¹⁴, R¹⁵, R¹⁶, R¹⁷, R¹⁸, R¹⁹, R²⁰, R²², R²³ ou R²⁴ peuvent être substitués par des substituants choisis dans le groupe constitué d'un fluoro, chloro, bromo, nitro, cyano, hydroxy, trifluorométhyle, amidosulfonyle qui peut avoir jusqu'à deux substitutions N-alkyle en C₁ à C₆ indépendantes, alkyle en C₁ à C₆, alcényle en C₂ à C₆, alkylamine en C₁ à C₆, dialkylamine dans laquelle chaque alkyle est indépendamment en C₁ à C₆, amino, alcoxy en C₁ à C₆, alcanoyle en C₂ à C₇, alcanoyloxy en C₂ à C₇, trifluorométhoxy, hydroxycarbonyle, alkyloxycarbonyle en C₂ à C₇, aminocarbonyle qui peut être N-substitué avec jusqu'à deux alkyles en C₁ à C₆ indépendants, alkylsulfonyle en C₁ à C₆, amidino qui peut être substitué avec jusqu'à trois alkyles en C₁ à C₆, ou méthylènedioxy ou éthylènedioxy avec les deux oxygènes liés à des positions adjacentes sur la structure cyclique aryle ou hétéroaryle, lequel méthylènedioxy ou éthylènedioxy peut être substitué avec jusqu'à deux alkyles en C₁ à C₆ indépendants ;
dans lequel R¹³ et R¹⁴ ensemble avec l'azote peuvent former un cycle à 5 à 7 chaînons qui peut contenir un hétéroatome additionnel choisi parmi l'oxygène et le soufre ;
pour la fabrication d'un médicament destiné au traitement ou à la prévention de la schizophrénie, (2) pour le traitement ou la prévention de la démence, (3) pour le traitement ou la prévention de l'épilepsie, (4) pour le traitement ou la prévention de la spasticité, (5) pour le traitement ou la prévention des spasmes musculaires, (6) pour le traitement ou la prévention de la douleur, (7) pour la prévention de la mort cellulaire neurale après un accident vasculaire cérébral, (8) pour prévenir la mort cellulaire neurale chez un animal souffrant d'une maladie neurodégénérative, (9) pour le traitement ou la prévention des troubles de l'humeur, (10) pour améliorer la mémoire ou l'apprentissage, ou (11) pour traiter ou prévenir les troubles de l'apprentissage.

26. Utilisation selon la revendication 25, dans laquelle on traite ou on prévient la spasticité et la spasticité est associée avec l'épilepsie.

27. Utilisation selon la revendication 25, dans laquelle on traite ou on prévient la spasticité et la spasticité est associée avec un accident vasculaire cérébral, un traumatisme crânien, la sclérose en plaques, une blessure ou dystonie médullaire.

28. Utilisation selon la revendication 25, (1) pour traiter ou prévenir la schizophrénie, (2) pour traiter ou prévenir la démence ou (3) pour traiter ou prévenir la douleur.

29. Utilisation selon la revendication 25, dans laquelle la maladie neurodégénérative est la maladie de Parkinson.

30. Utilisation selon la revendication 25, dans laquelle la maladie neurodégénérative est la maladie d'Alzheimer, la démence multi-infarctus, la démence du SIDA, la maladie de Huntington, la sclérose latérale amyotrophique ou l'accident vasculaire cérébral ou le traumatisme crânien.

31. Procédé de synthèse d'un composé selon la revendication 1 ou 2 comprenant :
A) la réaction d'un composé de l'une des formules suivantes 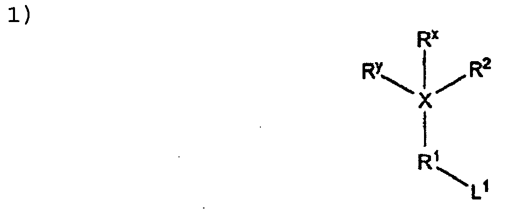 dans laquelle L¹ est un groupe partant de substitution nucléophile, avec un composé de formule 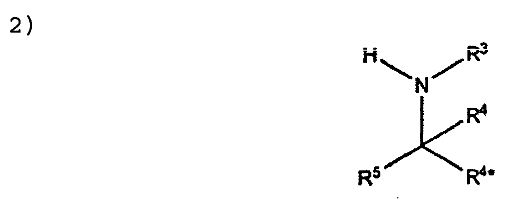
ou B) la réaction d'un composé de formule 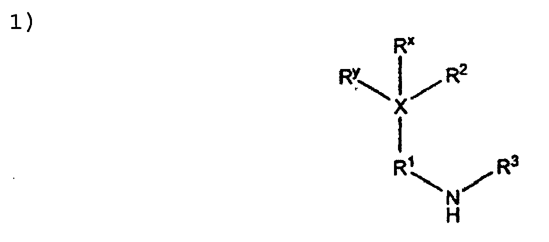 avec un composé de formule 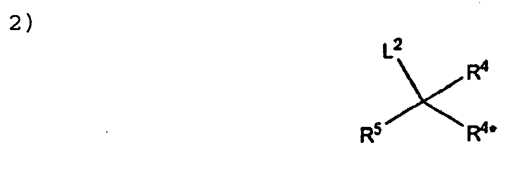 dans laquelle L² est un groupe partant de substitution nucléophile.

32. Procédé de synthèse d'un composé selon la revendication 1 ou 2 comprenant :
A) l'alkylation réductrice d'un composé de formule 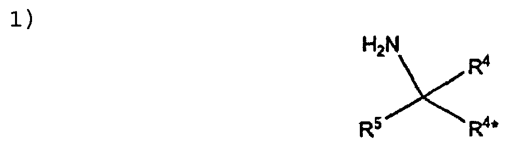 avec un composé de formule 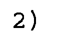 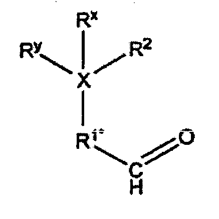 dans laquelle R^{1*} diffère de R¹ en ce qu'il manque le carbone qui est une partie du carbonylaldéhyde illustré,
ou B) l'alkylation réductrice d'un composé de formule 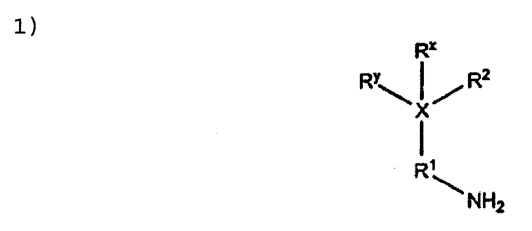 avec un composé de formule

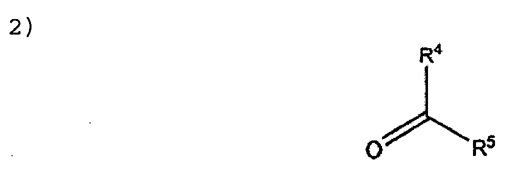

33. Procédé de synthèse d'un composé selon la revendication 1 ou 2 comprenant l'alkylation réductrice de R^{d}NH₂ avec un composé de formule 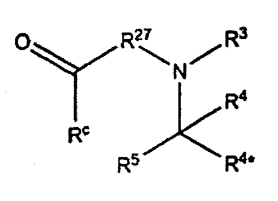 dans laquelle R^{d} et R^{c} sont indépendamment identiques à ce qui est défini pour R^{x}, et dans laquelle R²⁷ a la même définition que R¹ sauf qu'il ne comprend pas un azote, oxygène ou soufre et n'inclut pas de doubles liaisons conjuguées avec le carbonyle illustré ci-dessus.

34. Procédé de synthèse d'un composé selon la revendication 1 ou 2 comprenant la réaction de R^{f} OH ou R^{f}*SH avec un composé de formule 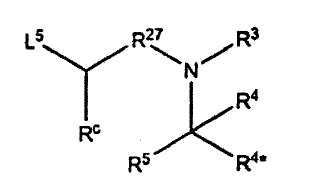 pour former un éther ou un thioéther, respectivement, dans laquelle R^{c}, R^{f} et R^{f*} sont indépendamment identiques à ce qui est défini pour R^{x}, dans laquelle R²⁷ a la même définition que R¹ sauf qu'il ne comprend pas un azote, oxygène ou soufre et n'inclut pas de doubles liaisons sur l'atome lié au carbone substitué par L⁵ illustré ci-dessus et dans laquelle L⁵ est un groupe partant de substitution nucléophile.

35. Procédé selon la revendication 34, comprenant en outre la synthèse du composé de formule 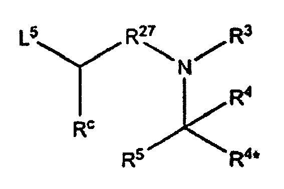 en remplaçant l'hydroxyle de formule 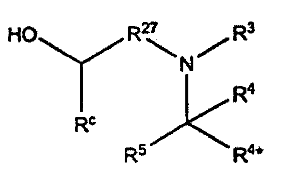 avec un autre groupe partant de substitution nucléophile.

36. Procédé selon la revendication 35, comprenant la réaction d'un composé de formule 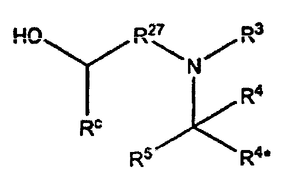 avec un azodicarboxylate en présence d'un composé phosphine.

37. Procédé de synthèse d'un composé selon la revendication 1 ou 2 comprenant la réaction de R^{e}M avec un composé de formule 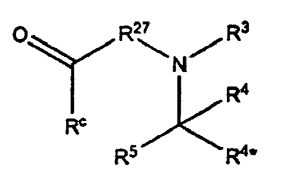 pour former un composé de formule 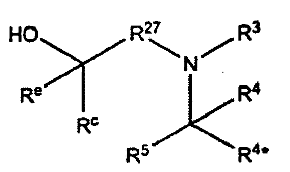 dans lesquelles R^{c} et R^{e} sont indépendamment identiques à ce qui est défini pour R^{x}, dans lesquelles M est un substituant contenant un métal tel que R^{e}M est un réactif organométallique, et dans lesquelles R²⁷ a la même définition que R¹ sauf qu'il ne comprend pas un azote, oxygène ou soufre et n'inclut pas de doubles liaisons conjuguées avec le carbonyle illustré ci-dessus.

38. Procédé de synthèse d'un composé selon la revendication 1 ou 2 comprenant la déshydratation d'un composé de formule 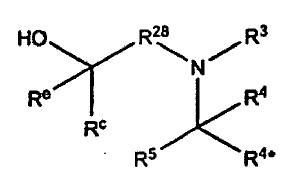 pour former un composé de formule 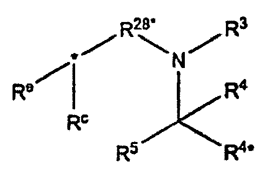 dans laquelle C* a une double liaison avec un carbone adjacent, et dans laquelle R^{e} et R^{c} sont indépendamment identiques à ce qui est défini pour R^{x}, et dans laquelle R²⁸ et R^{28*} ont la même définition que R¹ sauf que R²⁸ et R^{28*} ne comprennent pas un azote, oxygène ou soufre.

39. Procédé de synthèse d'un composé selon la revendication 1 ou 2 comprenant la réduction d'un composé de formule 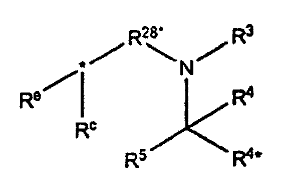 dans laquelle C* a une double liaison avec un carbone adjacent et R^{c} est indépendamment identique à ce qui est défini pour R^{x}, pour former un composé de formule 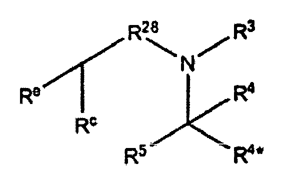 dans laquelle R^{e} est indépendamment identique à ce qui est défini pour R^{x}, et dans laquelle R²⁸ et R²⁸* ont la même définition que R¹ sauf que R²⁸ et R²⁸* ne comprennent pas un azote, oxygène ou soufre.
